# EUROPEAN PATENT APPLICATION

(11) **EP 4 105 328 A1**
(43) Date of publication of application: **21.12.2022**
(21) Application number: 21179667.7
(22) Date of filing: 15.06.2021
(51) Int. Cl.: C12N 15/113, A61K 31/712, A61P 13/12

(54) **ANTISENSE-OLIGONUCLEOTIDES FOR PREVENTION OF KIDNEY DYSFUNCTION PROMOTED BY ENDOTHELIAL DYSFUNCTION BY EPHRIN-B2 SUPPRESSION**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to antisense-oligonucleotides capable of hybridizing with a region of the gene encoding *Efnb2*, or with a region of the mRNA encoding *Efnb2*, and salts and optical isomers of said antisense-oligonucleotides for use in prevention of kidney dysfunction promoted by endothelial dysfunction.

## Description

The present invention relates to antisense-oligonucleotides capable of hybridizing with a region of the gene encoding *Efnb2,* or with a region of the mRNA encoding *Efnb2,* and salts and optical isomers of said antisense-oligonucleotides for prevention of kidney dysfunction promoted by endothelial dysfunction.

### Background of the invention

End stage renal disease (ESRD) is a worldwide public health problem with an enormous financial burden for healthcare systems. Endothelial dysfunction is known to induce microangiopathy in the kidney often coupled with podocyte foot process effacement, which is the key process of nephropathy. In the developed and developing world, diabetes is the major cause of microangiopathy, resulting in ESRD. Hyperglycemia in diabetes leads to many complications such as hypertension and nephropathy. Under the condition, hypertension is a major pathogenic factor driving the development of microvascular dysfunction in diabetes. In the kidney, increased blood pressure attenuates glomerular filtration barriers between the blood and urinary space, resulting in proteinuria, the major risk of developing ESRD. Compromised kidney filtration function in diabetic nephropathy is the major cause of kidney failure.

Glomeruli are specialized filtration barriers between the blood and urinary space, comprised of podocytes, glomerular basement membrane (GBM) and fenestrated glomerular endothelial cells (GECs). The morphology of healthy podocyte foot processes is essential for kidney filtration function. Effacement of foot processes is observed in most proteinuric diseases including diabetic nephropathy.

Recent studies have shown that nephrin, a transmembrane protein localized at slit diaphragm of foot process, and nephrin phosphorylation are important for maintenance of the podocyte foot processes. Decreased expression of nephrin and nephrin phosphorylation are known to result in renal dysfunction characterized by pathologic foot process remodeling. Loss of nephrin results in podocyte effacement and causes proteinuria, which is often seen in the onset of ESRD. In diabetes, hyperglycemia causes endothelial dysfunction, leading to foot process effacement, suggesting cellular communication from endothelial cells (ECs) to podocytes is important for development of the disease. However, the molecular link connecting endothelial cells to podocytes across the glomerular basement membrane (GBM) is not known.

Current treatment options for diabetic nephropathy are limited to control of blood pressure and blood glucose levels. It is believed that hypertension in diabetes causes microvascular dysfunction, including the glomerulus, leading to proteinuria. However, the effect of those treatments is limited.

It is the objective of the present invention to provide pharmaceutically active agents, especially for use in the treatment of nephropathy coupled with podocyte foot process effacement, such as diabetic nephropathy as well as compositions comprising at least one of those compounds as pharmaceutically active ingredients. The objective of the present application is also to provide pharmaceutically active compounds for use in controlling nephrin function in podocytes.

The objective of the present invention is solved by the teaching of the independent claims. Further advantageous features, aspects and details of the invention are evident from the dependent claims, the description, the figures, and the examples of the present application.

### Brief description of the invention

The present invention relates to antisense-oligonucleotide consisting of 10 to 28 nucleotides, wherein the antisense-oligonucleotide is capable of hybridizing with a region of the gene encoding *Efnb2,* or with a region of the mRNA encoding *Efnb2,* wherein the region of the gene encoding *Efnb2,* or the region of the mRNA encoding *Efnb2,* comprises the sequence AATTCAGCCCTAACCT (Seq. ID No. 1), AAATGCCTTGCTTGTA (Seq. ID No. 2), or CTGAATTTTGCAATGT (Seq. ID No. 3), and the antisense-oligonucleotide comprises a sequence capable of hybridizing with said sequence AATTCAGCCCTAACCT (Seq. ID No. 1), AAATGCCTTGCTTGTA (Seq. ID No. 2), or CTGAATTTTGCAATGT (Seq. ID No. 3), and salts and optical isomers of said antisense-oligonucleotide.

Antisense-oligonucleotides containing LNAs (LNA^{®}: Locked Nucleic Acids) were found the most promising candidates for the uses disclosed herein. It is therefore particularly preferred that at least two of the 10 to 28 nucleotides are LNAs. It is further preferred that at least four of the 10 to 28 nucleotides are LNAs. In preferred embodiments the antisense-oligonucleotide has therefore a Gapmer structure with 1 to 5 LNA units at the 3' terminal end and 1 to 5 LNA units at the 5' terminal end.

In preferred embodiments the antisense-oligonucleotide hybridizes selectively only with the sequence AATTCAGCCCTAACCT (Seq. ID No. 1) of the region of the gene encoding *Efnb2,* or of the region of the mRNA encoding the *Efnb2.*

In preferred embodiments the antisense-oligonucleotide hybridizes selectively only with the sequence AAATGCCTTGCTTGTA (Seq. ID No. 2) of the region of the gene encoding *Efnb2,* or of the region of the mRNA encoding the *Efnb2.*

In preferred embodiments the antisense-oligonucleotide hybridizes selectively only with the sequence CTGAATTTTGCAATGT (Seq. ID No. 3) of the region of the gene encoding *Efnb2* or of the region of the mRNA encoding the *Efnb2.*

In preferred embodiments, the antisense oligonucleotide binds with 100% complementarity to the region of the gene encoding *Efnb2* or to the mRNA encoding *Efnb2* and do not bind to any other region in the human transcriptome.

In preferred embodiments the antisense-oligonucleotide consists of a sequence of 10 to 16 nucleotides of the sequence AGGTTAGGGCTGAATT (Seq. ID No. 4) which is complementary to the sequence AATTCAGCCCTAACCT (Seq. ID No. 1) of the region of the gene encoding *Efnb2,* or of the region of the mRNA encoding the *Efnb2.*

Preferably the antisense-oligonucleotide consists of a sequence of 10 to 16 nucleotides of the sequence AGGTTAGGGCTGAATT selected from:

| **Seq ID No.** | **L** | **Sequence, 5'-3'** | **Seq ID No.** | **L** | **Sequence, 5'-3'** |
|---|---|---|---|---|---|
| 8 | 10 | AGGTT AGGGC | 33 | 11 | AGGTT AGGGCT |
| 9 | 10 | GGTTAGGGCT | 21 | 11 | GGTT AGGGCTG |
| 10 | 10 | GTTAGGGCTG | 22 | 11 | GTTAGGGCTGA |
| 11 | 10 | TTAGGGCTGA | 23 | 11 | TTAGGGCTGAA |
| 12 | 10 | TAGGGCTGAA | 24 | 11 | TAGGGCTGAAT |
| 13 | 10 | AGGGCTGAA T | 25 | 11 | AGGGCTGAA TT |
| 14 | 10 | GGGCTGAA TT | 26 | 13 | AGGTT AGGGCTGA |
| 15 | 12 | AGGTT AGGGCTG | 27 | 13 | GGTT AGGGCTGAA |
| 16 | 12 | GGTT AGGGCTGA | 28 | 13 | GTTAGGGCTGAA T |
| 17 | 12 | GTTAGGGCTGAA | 29 | 13 | TTAGGGCTGAATT |
| 18 | 12 | TTAGGGCTGAAT | 30 | 14 | AGGTT AGGGCTGAA |
| 36 | 12 | TAGGGCTGAATT | 31 | 14 | GGTT AGGGCTGAA T |
| 19 | 15 | AGGTTAGGGCTGAA T | 32 | 14 | GTT AGGGCTGAA TT |
| 20 | 15 | GGTT AGGGCTGAA TT | 4 | 16 | AGGTTAGGGCTGAATT |

In preferred embodiments the antisense-oligonucleotide consists of a sequence of 10 to 16 nucleotides of the sequence TACAAGCAAGGCATTT (Seq. ID No. 5) which is complementary to the sequence AAATGCCTTGCTTGTA (Seq. ID No. 2) of the region of the gene encoding *Efnb2,* or of the region of the mRNA encoding the *Efnb2.*

Preferably the antisense-oligonucleotide consists of a sequence of 10 to 16 nucleotides of the sequence TACAAGCAAGGCATTT selected from:

| **Seq ID No.** | **L** | **Sequence, 5'-3'** | **Seq ID No.** | **L** | **Sequence, 5'-3'** |
|---|---|---|---|---|---|
| 37 | 10 | TACAAGCAAG | 51 | 11 | TACAAGCAAGG |
| 38 | 10 | ACAAGCAAGG | 52 | 11 | ACAAGCAAGGC |
| 39 | 10 | CAAGCAAGGC | 53 | 11 | CAAGCAAGGCA |
| 40 | 10 | AAGCAAGGCA | 54 | 11 | AAGCAAGGCAT |
| 41 | 10 | AGCAAGGCAT | 55 | 11 | AGCAAGGCA TT |
| 42 | 10 | GCAAGGCA TT | 56 | 11 | GCAAGGCA TTT |
| 43 | 10 | CAAGGCA TTT | 57 | 13 | TACAAGCAAGGCA |
| 44 | 12 | TACAAGCAAGGC | 58 | 13 | ACAAGCAAGGCAT |
| 45 | 12 | ACAAGCAAGGCA | 59 | 13 | CAAGCAAGGCA TT |
| 46 | 12 | CAAGCAAGGCAT | 60 | 13 | AAGCAAGGCA TTT |
| 47 | 12 | AAGCAAGGCA TT | 61 | 14 | TACAAGCAAGGCAT |
| 48 | 12 | AGCAAGGCA TTT | 62 | 14 | ACAAGCAAGGCA TT |
| 49 | 15 | TACAAGCAAGGCATT | 63 | 14 | CAAGCAAGGCA TTT |
| 50 | 15 | ACAAGCAAGGCA TTT | 5 | 16 | TACAAGCAAGGCATTT |

In preferred embodiments the antisense-oligonucleotide consists of a sequence of 10 to 16 nucleotides of the sequence ACATTGCAAAATTCAG (Seq. ID No. 6) which is complementary to the sequence CTGAATTTTGCAATGT (Seq. ID No. 3) of the region of the gene encoding *Efnb2,* or of the region of the mRNA encoding the *Efnb2.*

Preferably the antisense-oligonucleotide consists of a sequence of 10 to 16 nucleotides of the sequence ACATTGCAAAATTCAG selected from:

| **Seq ID No.** | **L** | **Sequence, 5'-3'** | **Seq ID No.** | **L** | **Sequence, 5'-3'** |
|---|---|---|---|---|---|
| 67 | 10 | ACATTGCAAA | 81 | 11 | ACATTGCAAAA |
| 68 | 10 | CATTGCAAAA | 82 | 11 | CATTGCAAAAT |
| 69 | 10 | ATTGCAAAAT | 83 | 11 | ATTGCAAAATT |
| 70 | 10 | TTGCAAAATT | 84 | 11 | TTGCAAAATTC |
| 71 | 10 | TGCAAAATTC | 85 | 11 | TGCAAAATTCA |
| 72 | 10 | GCAAAATTCA | 86 | 11 | GCAAAATTCAG |
| 73 | 10 | CAAAATTCAG | 87 | 13 | ACATTGCAAAATT |
| 74 | 12 | TGCAAAATTCAG | 88 | 13 | CATTGCAAAATTC |
| 75 | 12 | ACATTGCAAAAT | 89 | 13 | ATTGCAAAATTCA |
| 76 | 12 | CATTGCAAAATT | 90 | 13 | TTGCAAAATTCAG |
| 77 | 12 | ATTGCAAAATTC | 91 | 14 | ACATTGCAAAATTC |
| 78 | 12 | TTGCAAAATTCA | 92 | 14 | CATTGCAAAATTCA |
| 79 | 15 | ACATTGCAAAATTCA | 93 | 14 | ATTGCAAAATTCAG |
| 80 | 15 | CATTGCAAAATTCAG | 6 | 16 | ACATTGCAAAATTCAG |

The antisense-oligonucleotides of the present invention may be used as a pharmaceutical active agent for suppression of ephrin-B2 function or secretion providing renal protective effects. Thus, antisense-oligonucleotides of the present invention are suitable for use in controlling nephrin function, preferably for use in the prophylaxis and treatment of proteinuria in diabetes and/or of diabetic nephropathy.

The present invention further relates to a pharmaceutical composition containing at least one antisense-oligonucleotide together with at least one pharmaceutically acceptable carrier, excipient, adjuvant, solvent or diluent.

### Description of the invention

Surprisingly, it has been found that suppression of ephrin-B2 in endothelial cells strongly restore podocyte foot process effacement and kidney function induced by decreased nephrin expression.

EphB receptor tyrosine kinases and their transmembrane ligand, ephrin-B2 mediate cell-to-cell contact dependent signaling and thus regulate cell migration and cytoskeletal organization in many different cell types and tissues. The binding of ephrin-B2 induces clustering and auto-phosphorylation of EphB receptors, resulting in downstream signal activation in the cells expressing EphBs (termed as "forward signaling"). Simultaneously, the cytoplasmic tail of ephrin-B2, via its C-terminal PDZ binding motif or phosphorylation of tyrosine residues, engages in "reverse signaling". In the vasculature, ephrin-B2 controls VEGF receptor trafficking and downstream signaling, thereby regulating endothelial sprouting behavior during angiogenesis. In mature tissues, ephrin-B2 is a marker of arterial and arterial derived ECs, including glomerular endothelial cells (GECs). However, the role of ephrin-B2 in mature endothelial cells (ECs) is largely unknown.

The inventors have surprisingly found that cell-to-cell contact independent ephrin-B2/EphB4 forward signaling mediated by extracellular vesicles such as exosomes plays a pivotal role in cellular communication from endothelial cells (ECs) to podocytes.

It has been found that this signaling pathway is over-activated in the diabetic condition. This pathway is over-activated in diabetes to cause podocyte foot process effacement, and suppression of ephrin-B2/EphB4 by cell type specific gene deletion has been found to prevent glomerular dysfunction in mice. Proteinuria and podocyte effacement in diabetic mice are restored in both ephrin-B2 endothelial specific and EphB4 podocyte specific inducible knockout mice.

Surprisingly, it has been found that antisense-oligonucleotides capable of hybridizing with a region of the gene encoding *Efnb2,* or with a region of the mRNA encoding *Efnb2,* and salts and optical isomers of said antisense-oligonucleotides solve the above objective.

Thus, the present invention relates to antisense-oligonucleotides consisting of 10 to 28 nucleotides, wherein the antisense-oligonucleotides are capable of hybridizing with a region of the gene encoding *Efnb2,* or with a region of the mRNA encoding *Efnb2,* wherein the region of the gene encoding *Efnb2,* or the region of the mRNA encoding the *Efnb2,* comprises the sequence AATTCAGCCCTAACCT (Seq. ID No. 1), AAATGCCTTGCTTGTA (Seq. ID No. 2), or CTGAATTTTGCAATGT (Seq. ID No. 3), and the antisense-oligonucleotides comprise a sequence capable of hybridizing with said sequence AATTCAGCCCTAACCT (Seq. ID No. 1), AAATGCCTTGCTTGTA (Seq. ID No. 2), or CTGAATTTTGCAATGT (Seq. ID No. 3), and salts and optical isomers of said antisense-oligonucleotides.

Slightly reworded the present invention relates to antisense-oligonucleotides consisting of 10 to 28 nucleotides, wherein the antisense-oligonucleotides are capable of hybridizing with a region of the open reading frame of the gene encoding *Efnb2,* or with a region of the mRNA encoding *Efnb2,* wherein the region of the open reading frame of the gene encoding *Efnb2,* or the region of the mRNA encoding the *Efnb2,* comprises the sequence AATTCAGCCCTAACCT (Seq. ID No. 1), AAATGCCTTGCTTGTA (Seq. ID No. 2), or CTGAATTTTGCAATGT (Seq. ID No. 3), and the antisense-oligonucleotides comprise a sequence capable of hybridizing with said sequence AATTCAGCCCTAACCT (Seq. ID No. 1), AAATGCCTTGCTTGTA (Seq. ID No. 2), or CTGAATTTTGCAATGT (Seq. ID No. 3), and salts and optical isomers of said antisense-oligonucleotides.

The present invention therefore relates to an antisense-oligonucleotide consisting of 10 to 28 nucleotides, wherein the antisense-oligonucleotides is capable of hybridizing with a region of the gene encoding *Efnb2,* or with a region of the mRNA encoding *Efnb2,* wherein the region of the gene encoding *Efnb2,* or the region of the mRNA encoding the *Efnb2,* comprises the sequence AATTCAGCCCTAACCT (Seq. ID No. 1), and the antisense-oligonucleotides comprise a sequence capable of hybridizing with said sequence AATTCAGCCCTAACCT (Seq. ID No. 1), and salts and optical isomers of said antisense-oligonucleotide.

Preferably, the antisense-oligonucleotide hybridizes selectively only with the sequence AATTCAGCCCTAACCT (Seq. ID No. 1) of the region of the gene encoding *Efnb2,* or of the region of the mRNA encoding the *Efnb2.*

The complementary sequence to the sequence AATTCAGCCCTAACCT (Seq. ID No. 1) is AGGTTAGGGCTGAATT (**5'-3'**) (Seq. ID No. 4). The sequence AATTCAGCCCTAACCT (Seq. ID No. 1) is also written in **5'-3'** direction.

Preferred antisense-oligonucleotides of the present invention comprise a sequence that overlaps with or corresponds to the sequence AGGTTAGGGCTGAATT (**5'-3'**) (Seq. ID No. 4). According to the present invention the antisense-oligonucleotides consist of 10 to 28 nucleotides and preferably comprise a sequence of at least 10 consecutive nucleotides that corresponds to a sequence of 10 consecutive nucleotides of the sequence AGGTTAGGGCTGAATT (**5'-3'**) (Seq. ID No. 4).

The present invention therefore relates to an antisense-oligonucleotide consisting of 10 to 28 nucleotides, wherein the antisense-oligonucleotide is capable of hybridizing with a region of the gene encoding *Efnb2,* or with a region of the mRNA encoding *Efnb2,* wherein the region of the gene encoding *Efnb2,* or the region of the mRNA encoding the *Efnb2,* comprises the sequence AAATGCCTTGCTTGTA (Seq. ID No. 2), and the antisense-oligonucleotides comprise a sequence capable of hybridizing with said sequence AAATGCCTTGCTTGTA (Seq. ID No. 2), and salts and optical isomers of said antisense-oligonucleotide.

Preferably, the antisense-oligonucleotide hybridizes selectively only with the sequence AAATGCCTTGCTTGTA (Seq. ID No. 2) of the region of the gene encoding *Efnb2,* or of the region of the mRNA encoding the *Efnb2.*

The complementary sequence to the sequence AAATGCCTTGCTTGTA (Seq. ID No. 2) is TACAAGCAAGGCATTT (**5'-3'**) (Seq. ID No. 5). The sequence AAATGCCTTGCTTGTA (Seq. ID No. 2) is also written in **5'-3'** direction.

Preferred antisense-oligonucleotides of the present invention comprise a sequence that overlaps with or corresponds to the sequence TACAAGCAAGGCATTT (**5'-3'**) (Seq. ID No. 4). According to the present invention the antisense-oligonucleotides consist of 10 to 28 nucleotides and preferably comprise a sequence of at least 10 consecutive nucleotides that corresponds to a sequence of 10 consecutive nucleotides of the sequence TACAAGCAAGGCATTT (**5'-3'**) (Seq. ID No. 5).

The present invention therefore relates to an antisense-oligonucleotide consisting of 10 to 28 nucleotides, wherein the antisense-oligonucleotides is capable of hybridizing with a region of the gene encoding *Efnb2,* or with a region of the mRNA encoding *Efnb2,* wherein the region of the gene encoding *Efnb2,* or the region of the mRNA encoding the *Efnb2,* comprises the sequence CTGAATTTTGCAATGT (Seq. ID No. 3), and the antisense-oligonucleotides comprise a sequence capable of hybridizing with said sequence CTGAATTTTGCAATGT (Seq. ID No. 3), and salts and optical isomers of said antisense-oligonucleotide.

Preferably, the antisense-oligonucleotide hybridizes selectively only with the sequence CTGAATTTTGCAATGT (Seq. ID No. 3) of the region of the gene encoding *Efnb2* or of the region of the mRNA encoding the *Efnb2.*

The complementary sequence to the sequence CTGAATTTTGCAATGT (Seq. ID No. 3) is ACATTGCAAAATTCAG (**5'-3'**) (Seq. ID No. 6). The sequence CTGAATTTTGCAATGT (Seq. ID No. 3) is also written in **5'-3'** direction.

Preferred antisense-oligonucleotides of the present invention comprise a sequence that overlaps or corresponds to the sequence ACATTGCAAAATTCAG (**5'-3'**) (Seq. ID No. 4). According to the present invention the antisense-oligonucleotides consist of 10 to 28 nucleotides and preferably comprise a sequence of at least 10 consecutive nucleotides that corresponds to a sequence of 10 consecutive nucleotides of the sequence ACATTGCAAAATTCAG (**5'-3'**) (Seq. ID No. 6).

Preferably, the present invention relates to antisense-oligonucleotides consisting of 10 to 28 nucleotides, wherein at least two of the 10 to 28 nucleotides are LNAs, wherein the antisense-oligonucleotides are capable of hybridizing with a region of the gene encoding *Efnb2,* or with a region of the mRNA encoding *Efnb2,* wherein the region of the gene encoding *Efnb2,* or the region of the mRNA encoding the *Efnb2,* comprises the sequence AATTCAGCCCTAACCT (Seq. ID No. 1), AAATGCCTTGCTTGTA (Seq. ID No. 2), or CTGAATTTTGCAATGT (Seq. ID No. 3), and the antisense-oligonucleotides comprise a sequence capable of hybridizing with said sequence AATTCAGCCCTAACCT (Seq. ID No. 1), AAATGCCTTGCTTGTA (Seq. ID No. 2), or CTGAATTTTGCAATGT (Seq. ID No. 3), and salts and optical isomers of said antisense-oligonucleotides.

Slightly reworded the present invention preferably relates to antisense-oligonucleotides consisting of 10 to 28 nucleotides, wherein at least two of the 10 to 28 nucleotides are LNAs, wherein the antisense-oligonucleotides are capable of hybridizing with a region of the open reading frame of the gene encoding *Efnb2,* or with a region of the mRNA encoding *Efnb2,* wherein the region of the open reading frame of the gene encoding *Efnb2,* or the region of the mRNA encoding the *Efnb2,* comprises the sequence AATTCAGCCCTAACCT (Seq. ID No. 1), AAATGCCTTGCTTGTA (Seq. ID No. 2), or CTGAATTTTGCAATGT (Seq. ID No. 3), and the antisense-oligonucleotides comprise a sequence capable of hybridizing with said sequence AATTCAGCCCTAACCT (Seq. ID No. 1), AAATGCCTTGCTTGTA (Seq. ID No. 2), or CTGAATTTTGCAATGT (Seq. ID No. 3), and salts and optical isomers of said antisense-oligonucleotides.

The present invention preferably relates to an antisense-oligonucleotide consisting of 10 to 28 nucleotides, wherein at least two of the 10 to 28 nucleotides are LNAs, wherein the antisense-oligonucleotides is capable of hybridizing with a region of the gene encoding *Efnb2,* or with a region of the mRNA encoding *Efnb2,* wherein the region of the gene encoding *Efnb2,* or the region of the mRNA encoding the *Efnb2,* comprises the sequence AATTCAGCCCTAACCT (Seq. ID No. 1), and the antisense-oligonucleotides comprise a sequence capable of hybridizing with said sequence AATTCAGCCCTAACCT (Seq. ID No. 1), and salts and optical isomers of said antisense-oligonucleotide.

Preferably, the antisense-oligonucleotide oligonucleotide consisting of 10 to 28 nucleotides, wherein at least two of the 10 to 28 nucleotides are LNAs, hybridizes selectively only with the sequence AATTCAGCCCTAACCT (Seq. ID No. 1) of the region of the gene encoding *Efnb2,* or of the region of the mRNA encoding the *Efnb2.*

The present invention preferably relates to an antisense-oligonucleotide consisting of 10 to 28 nucleotides, wherein at least two of the 10 to 28 nucleotides are LNAs, wherein the antisense-oligonucleotide is capable of hybridizing with a region of the gene encoding *Efnb2,* or with a region of the mRNA encoding *Efnb2,* wherein the region of the gene encoding *Efnb2,* or the region of the mRNA encoding the *Efnb2,* comprises the sequence AAATGCCTTGCTTGTA (Seq. ID No. 2), , and the antisense-oligonucleotides comprise a sequence capable of hybridizing with said sequence AAATGCCTTGCTTGTA (Seq. ID No. 2), and salts and optical isomers of said antisense-oligonucleotide.

Preferably, the antisense-oligonucleotide oligonucleotide consisting of 10 to 28 nucleotides, wherein at least two of the 10 to 28 nucleotides are LNAs, hybridizes selectively only with the sequence AAATGCCTTGCTTGTA (Seq. ID No. 2) of the region of the gene encoding *Efnb2,* or of the region of the mRNA encoding the *Efnb2.*

The present invention preferably relates to an antisense-oligonucleotide consisting of 10 to 28 nucleotides, wherein at least two of the 10 to 28 nucleotides are LNAs, wherein the antisense-oligonucleotides is capable of hybridizing with a region of the gene encoding *Efnb2,* or with a region of the mRNA encoding *Efnb2,* wherein the region of the gene encoding *Efnb2,* or the region of the mRNA encoding the *Efnb2,* comprises the sequence CTGAATTTTGCAATGT (Seq. ID No. 3), and the antisense-oligonucleotides comprise a sequence capable of hybridizing with said sequence CTGAATTTTGCAATGT (Seq. ID No. 3), and salts and optical isomers of said antisense-oligonucleotide.

Preferably, the antisense-oligonucleotide oligonucleotide consisting of 10 to 28 nucleotides, wherein at least two of the 10 to 28 nucleotides are LNAs, hybridizes selectively only with the sequence CTGAATTTTGCAATGT (Seq. ID No. 3) of the region of the gene encoding *Efnb2* or of the region of the mRNA encoding the *Efnb2.*

While the role of ephrin-B2 as a regulator of VEGF receptor signaling has been established during angiogenesis, the role of ephrin-B2 in mature endothelial cells (ECs) is largely unknown. Previous work has shown that VEGF signaling in GECs regulates endothelial fenestration, a critical characteristic of GEC.

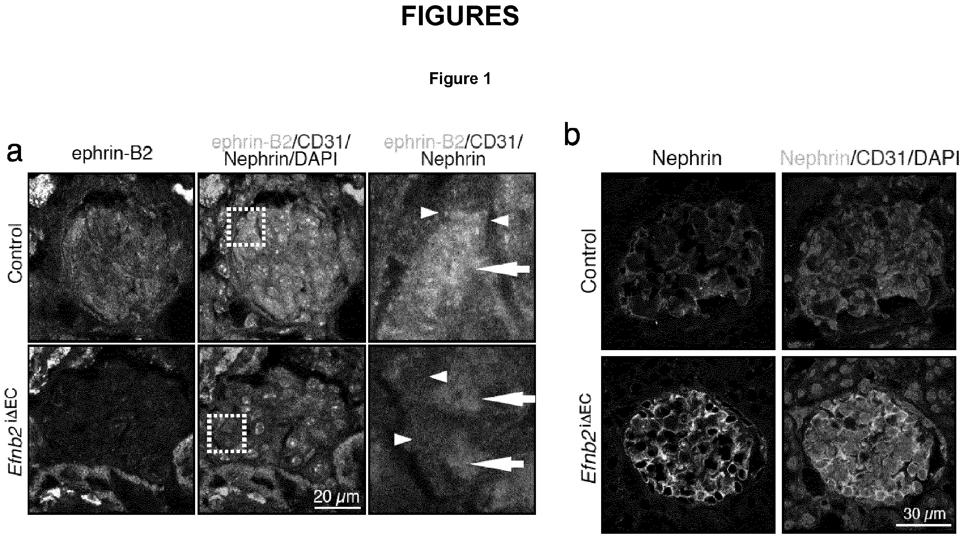

To gain further insight into the role of endothelial ephrin-B2 in mature GECs, the inventors examined the expression of ephrin-B2 in the glomerulus. The signal corresponding to the immunoreactivity of an ephrin-B2 antibody in the glomerulus was observed not only in CD31, a marker of endothelial cells (ECs), positive structures but also in the nephrin positive podocytes (Fig. 1a). To confirm the specificity of the ephrin-B2 antibody, the kidney from *Efnb2*, the gene encoding ephrin-B2, floxed mice bred with endothelial specific tamoxifen inducible Cre driver line, *Cdh5-CreErt2* mice was examined. To induce effective gene deletion after the completion of developmental angiogenesis, tamoxifen was injected into the mice from postnatal day (P) 21 to P23, and kidneys were collected at P35. Interestingly, ephrin-B2 expression was decreased not only in CD31 positive ECs but also in podocytes in ephrin-B2 endothelial inducible knockout (*Efnb2*^{iΔEC}) mice (Fig. 1a).

Since ephrin-B2 expression was reduced in the podocytes in the EC specific KO mice, the inventors have examined the phenotype of podocytes in the *Efnb2*^{iΔEC} mice. Surprisingly, significantly increased nephrin expression was observed in the *Efnb2*^{iΔEC} mice compared to control mice using immunostaining and western blotting analysis (Fig. 1b-d). These results indicate that ephrin-B2 is important for nephrin expression in the podocytes. Thus, it has been surprisingly found that Ephrin-B2 in the glomerular endothelial cells (GEC) controls nephrin expression in the podocyte.

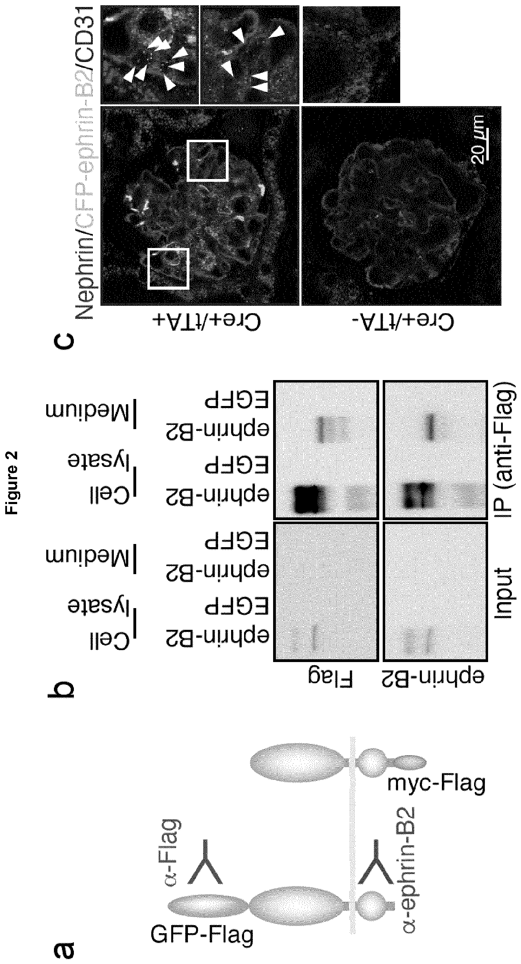

Podocytes and ECs in the glomerulus are known to be physically segregated by the glomerular basement membrane (GBM). To examine whether ephrin-B2 in ECs is able to activate EphB4 on podocytes without cell-to-cell contact formation, the inventors first ectopically expressed N-terminal GFP-flag-tagged ephrin-B2 in cultured cells (Fig. 2a). Ephrin-B2 was immunoprecipitated with the Flag M2 antibody from HEK293 cell and Human Umbilical Vein Endothelial cell (HUVEC) lysates and their cell culture conditioned medium (Fig. 2a). While immunoprecipitation of ephrin-B2 was confirmed in both cell lysates and culture conditioned medium (Fig. 2b).

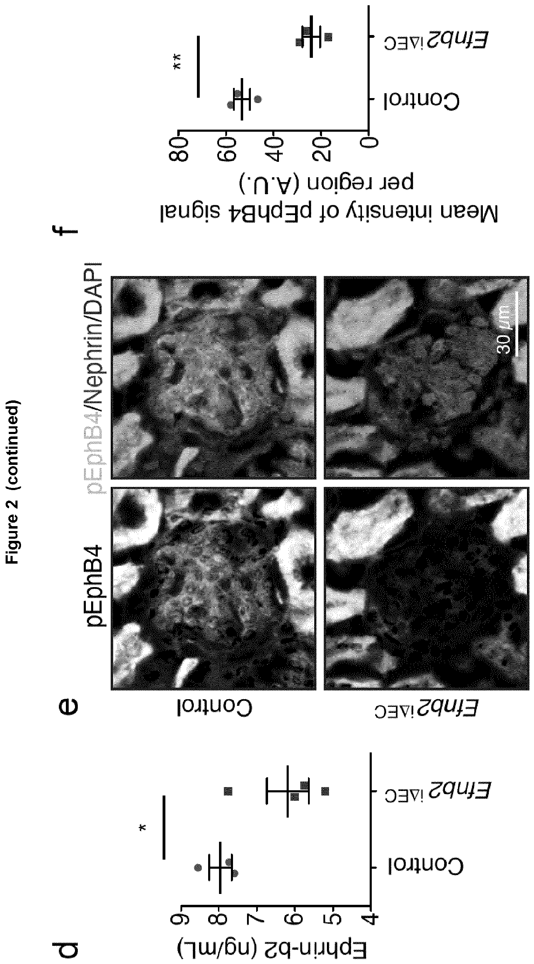

To confirm ephrin-B2 secretion *in vivo,* the inventors analyzed mice expressing CFP-ephrin-B2 in ECs. Overexpression of ephrin-B2 in endothelial cells causes toxicity in mice. In *VE-cadherin-tTA*/*tetO-CFP-Efnb2* mice, the administration of tetracycline to pregnant female mice circumvents the embryonic lethality of ephrin-B2 overexpression. After birth, mosaic expression of CFP-ephrin-B2 in ECs was induced by withdrawal of tetracycline treatment. Only mice displaying low levels of ephrin-B2 overexpression reached adulthood. Staining with an anti-GFP antibody (recognizing CFP) revealed the mosaic expression of CFP in CD31 positive ECs. Although the low levels of ephrin-B2 overexpression might not be enough to affect nephrin expression in these mice, a specific signal corresponding to CFP-ephrin-B2 was also observed in nephrin positive podocytes (Fig. 2c). In addtion, eprhin-B2 was detected in the blood plasma of control mice, which was significantly decreased in that of *Efnb2*^{iΔEC} mice (Fig. 2d). To investigate the functional action of endothelial ephrin-B2 on EphB4 in podocytes, phosphorylation of EphB4 in the *Efnb2*^{iΔEC} mice kidney was examined and decreased EphB4 phosphorylation in the glomerulus was confirmed (Fig. 2e, f). These observations suggest that ephrin-B2 is cleaved within the cytoplasmic tail in ECs, crosses the GBM from ECs and reaches the podocytes to stimulate EphB4 forward signaling in podocytes. Thus, it has been found that Ephrin-B2 is secreted from endothelial cell (EC) to podocyte by extracellular vesicles such as exosomes.

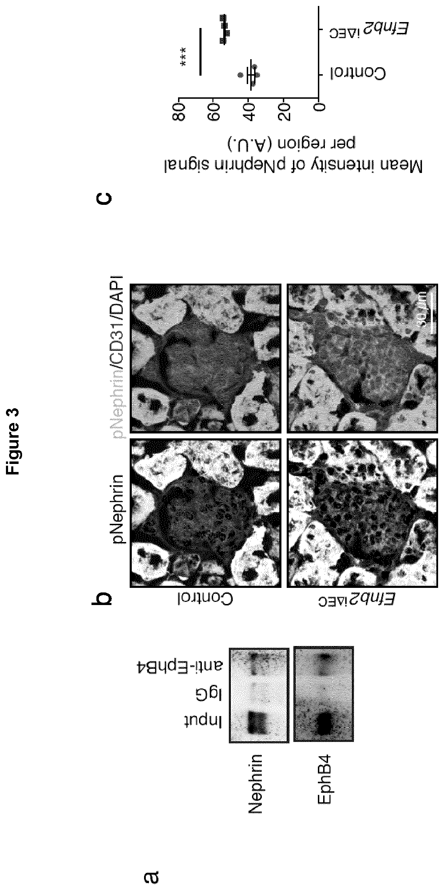

To investigate the link between ephrin-B2/EphB4 forward signaling and nephrin, the inventors next carried out immunoprecipitation of EphB4 from kidney tissue and confirmed nephrin/EphB4 complex formation (Fig. 3a). Nephrin tyrosine phosphorylation is required for its function and its localization and thereby important for stabilizing foot process architecture in podocytes. Phosphorylation of nephrin in the *Efnb2*^{iΔEC} mice kidney was confirmed with immunostaining using a phospho-Tyr1193/1176 nephrin antibody. The signal corresponding to phosphonephrin was increased in these mutant mice glomeruli, suggesting a suppressing effect of ephrin-B2/EphB4 forward signaling on nephrin phosphorylation (Fig. 3b-c). Thus, EphB4 forms a protein complex with nephrin controlling nephrin phosphorylation.

In agreement with an inhibitory role of ephrin-B2 on nephrin phosphorylation, mRNA of ephrin-B2 is increased in patients suffering from type 2 diabetic nephropathy compared to those suffering from type 2 diabetes. Additionally, EphB4 is shown to be a marker for advanced type 1 diabetic state presenting retinopathy and nephropathy.

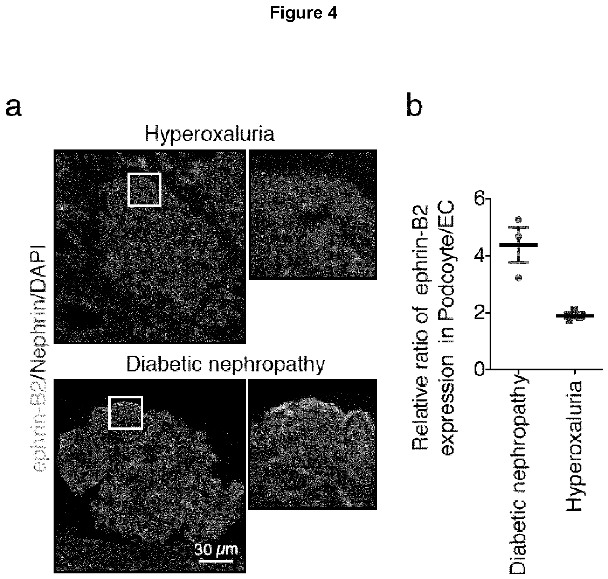

To gain further insight into the role of ephrin-B2 on diabetic nephropathy, the inventors examined immunostaining of ephrin-B2 in renal biopsy in diabetic patients. While three patients were diagnosed with hyperoxaluria, other three were diagnosed with diabetic nephropathy (DN). In the DN patient tissues, signal against ephrin-B2 co-localized with nephrin in those glomeruli was stronger than that in biopsy from the hyperoxaluria patients (Fig. 4a, b).

The inventors next examined if suppressing ephrin-B2/EphB4 forward signaling in mice with diabetic nephropathy would restore nephrin function and podocyte foot process architecture and would be beneficial for preventing diabetic kidney failure.

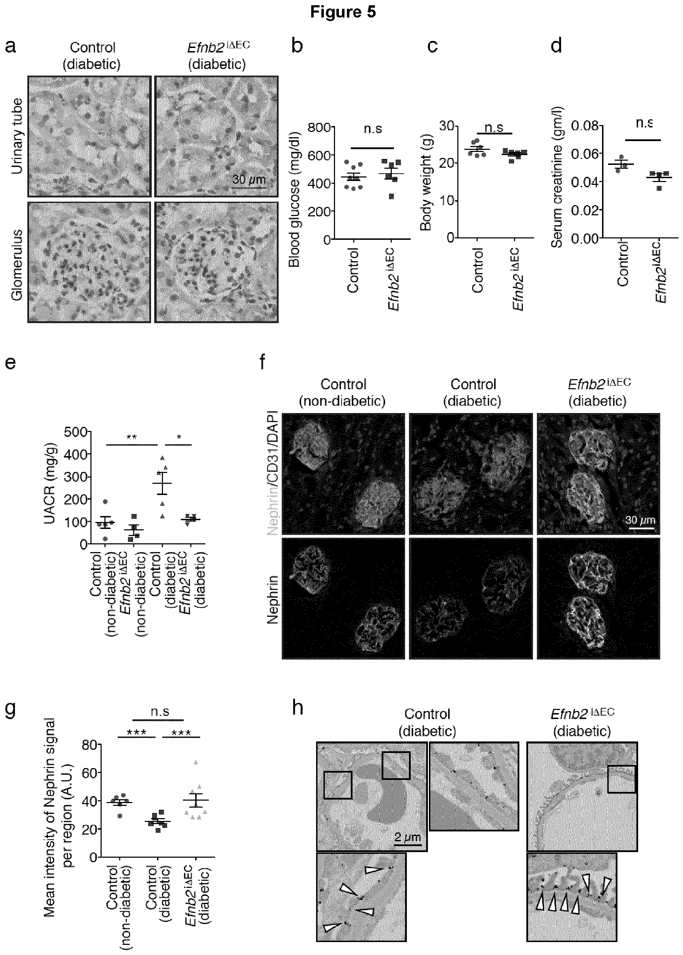

To address this, the inventors employed diabetic mice models with both the ephrin-B2 or EphB4 mutants. To examine the effect of ephrin-B2 loss of function in diabetes, control and *Efnb2*^{iΔEC} mice on the C57BL/6 background were injected with streptozotocin at 5 weeks of age and fed with high fat diet. C57BL/6 control and *Efnb2*^{iΔEC} mice developed a mild diabetic phenotype after 18 weeks. HE staining of the kidneys, blood glucose level, and body weight between control and *Efnb2*^{iΔEC} diabetic mice were not different. Serum creatinine levels were decreased in *Efnb2*^{iΔEC} mice although not at significant levels (Fig. 5a-d). Although the urinary albumin to creatinine ration (UACR) was significantly increased in the diabetic condition compared to control mice (Fig. 5e), UACR was not affected in *Efnb2*^{iΔEC} mice even after induction of diabetes (Fig. 5e). Also, the expression level of nephrin was reduced in the diabetic compared to non-diabetic control mice, but interestingly was not decreased in *Efnb2*^{iΔEC} mice compared to non-diabetic control (Fig. 5f, g). Moreover, at the ultrastructural level, podocyte effacement was frequently seen in diabetic control mice glomeruli, while it was less frequently observed in *Efnb2*^{iΔEC} mice (Fig. 5h). In agreement with a restored nephrin function, strong immunogold signal corresponding to an anti-nephrin antibody was observed at the podocyte slit diaphragm in *Efnb2*^{iΔEC} mice (Fig. 5h). Thus, suppression of ephrin-B2/EphB4 forward signaling recovers proteinuria in the diabetic condition.

Taken together, these results indicate that the suppression of ephrin-B2/EphB4 forward signaling across the GBM has renal protective effects by controlling nephrin function in the podocytes.

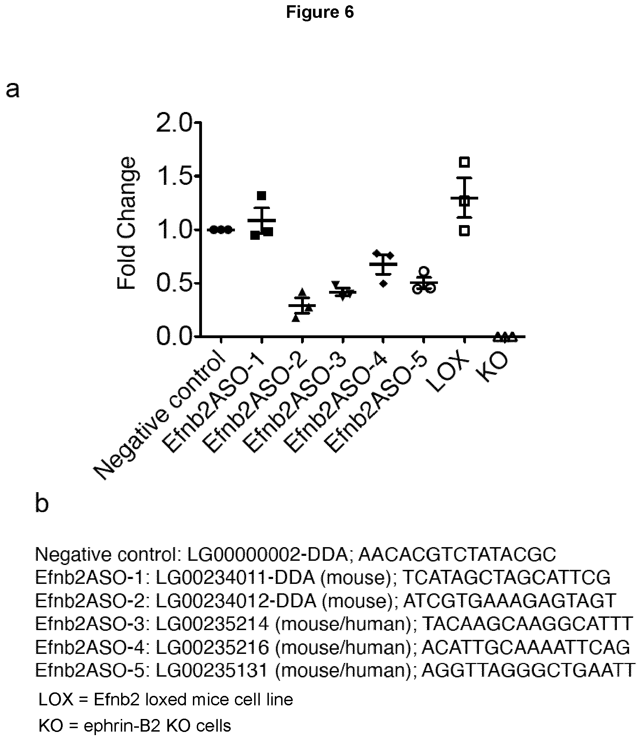

The inventors examined the effect of antisense oligonucleotides (ASOs) on ephrin-B2 expression. Different sequences against ephrin-B2 (Efnb2ASOs) were examined with the cultured ECs. Among them, treatment of ECs with 4 different ASOs resulted in effective knockdown of ephrin-B2 (Fig. 6). Among them, three are conserved between human and mouse. Thus, suppression of ephrin-B2 function or secretion improved proteinuria in diabetes and is therefore a novel and promising target for diabetic nephropathy patients.

Preferably, the antisense-oligonucleotide consists of 10 to 28 nucleotides, wherein at least two of the 10 to 28 nucleotides are LNAs. Thus, the antisense-oligonucleotides of the present invention preferably comprise 2 to 10 LNA units, more preferably 3 to 9 LNA units and still more preferably 4 to 8 LNA units and also preferably at least 6 non-LNA units, more preferably at least 7 non-LNA units and most preferably at least 8 non-LNA units. The non-LNA units are preferably DNA units. The LNA units are preferably positioned at the 3' terminal end (also named 3' terminus) and the 5' terminal end (also named 5' terminus). Preferably at least one and more preferably at least two LNA units are present at the 3' terminal end and/or at the 5' terminal end.

Thus, preferred are antisense-oligonucleotides of the present invention which contain 3 to 10 LNA units and which especially contain 1 to 5 LNA units at the 5' terminal end and 1 to 5 LNA units at the 3' terminal end of the antisense-oligonucleotide and between the LNA units at least 7 and more preferably at least 8 DNA units.

Thus, in preferred embodiments the antisense-oligonucleotides have a gapmer structure with 1 to 5 LNA units at the 3' terminal end and 1 to 5 LNA units at the 5' terminal end.

Moreover, the antisense-oligonucleotides may contain common nucleobases such as adenine, guanine, cytosine, thymine and uracil as well as common derivatives thereof. The antisense-oligonucleotides of the present invention may also contain modified internucleotide bridges such as phosphorothioate or phosphorodithioate instead of phosphate bridges. Such modifications may be present only in the LNA segments or only in the non-LNA segment of the antisense-oligonucleotide or both.

Preferred are the following antisense-oligonucleotides (Table 1) consisting of 10 to 16 nucleotides and preferably comprise a sequence of at least 10 consecutive nucleotides that corresponds to a sequence of 10 consecutive nucleotides of the sequence AGGTTAGGGCTGAATT (Seq. ID No. 4):

| **Seq ID No.** | **L** | **Sequence, 5'-3'** | **Seq ID No.** | **L** | **Sequence, 5'-3'** |
|---|---|---|---|---|---|
| 8 | 10 | AGGTT AGGGC | 33 | 11 | AGGTT AGGGCT |
| 9 | 10 | GGTTAGGGCT | 21 | 11 | GGTT AGGGCTG |
| 10 | 10 | GTTAGGGCTG | 22 | 11 | GTTAGGGCTGA |
| 11 | 10 | TTAGGGCTGA | 23 | 11 | TTAGGGCTGAA |
| 12 | 10 | TAGGGCTGAA | 24 | 11 | TAGGGCTGAAT |
| 13 | 10 | AGGGCTGAA T | 25 | 11 | AGGGCTGAA TT |
| 14 | 10 | GGGCTGAA TT | 26 | 13 | AGGTT AGGGCTGA |
| 15 | 12 | AGGTT AGGGCTG | 27 | 13 | GGTT AGGGCTGAA |
| 16 | 12 | GGTT AGGGCTGA | 28 | 13 | GTTAGGGCTGAA T |
| 17 | 12 | GTTAGGGCTGAA | 29 | 13 | TTAGGGCTGAATT |
| 18 | 12 | TTAGGGCTGAAT | 30 | 14 | AGGTT AGGGCTGAA |
| 36 | 12 | TAGGGCTGAATT | 31 | 14 | GGTT AGGGCTGAA T |
| 19 | 15 | AGGTTAGGGCTGAA T | 32 | 14 | GTT AGGGCTGAA TT |
| 20 | 15 | GGTT AGGGCTGAA TT | 4 | 16 | AGGTTAGGGCTGAATT |

More preferred are the following antisense-oligonucleotides (Table 2) consisting of 10 to 16 nucleotides and preferably comprise a sequence of at least 10 consecutive nucleotides that corresponds to a sequence of 10 consecutive nucleotides of the sequence AGGTTAGGGCTGAATT (Seq. ID No. 4):

| **Seq ID No.** | **L** | **Sequence, 5'-3'** | **Seq ID No.** | **L** | **Sequence, 5'-3'** |
|---|---|---|---|---|---|
| 9 | 10 | GGTTAGGGCT | 22 | 11 | GTTAGGGCTGA |
| 10 | 10 | GTTAGGGCTG | 23 | 11 | TTAGGGCTGAA |
| 11 | 10 | TTAGGGCTGA | 26 | 13 | AGGTT AGGGCTGA |
| 15 | 12 | AGGTT AGGGCTG | 27 | 13 | GGTT AGGGCTGAA |
| 16 | 12 | GGTT AGGGCTGA | 28 | 13 | GTTAGGGCTGAA T |
| 17 | 12 | GTTAGGGCTGAA | 29 | 13 | TTAGGGCTGAATT |
| 18 | 12 | TTAGGGCTGAAT | 30 | 14 | AGGTT AGGGCTGAA |
| 19 | 15 | AGGTTAGGGCTGAA T | 31 | 14 | GGTT AGGGCTGAA T |
| 20 | 15 | GGTT AGGGCTGAA TT | 32 | 14 | GTT AGGGCTGAA TT |
| 21 | 11 | GGTT AGGGCTG | 4 | 16 | AGGTTAGGGCTGAATT |

Preferred are antisense-oligonucleotides of general formula (S1) represented by the following sequence:
5'-N¹-**TAGGGCTG**-N²-3' (Seq. ID No. 7) wherein
N¹ represents: AATTCTAGACCCCAGAGGT-, ATTCTAGACCCCAGAGGT-, TTCTAGACCCCAGAGGT-, TCTAGACCCCAGAGGT-, CTAGACCCCAGAGGT-, TAGACCCCAGAGGT-, AGACCCCAGAGGT-, GACCCCAGAGGT-, ACCCCAGAGGT-, CCCCAGAGGT-, CCCAGAGGT-, CCAGAGGT-, CAGAGGT-, AGAGGT-, GAGGT-, AGGT-, GGT-, GT-, or T-; and
N² represents: -AATTCTTGAAACTTGATGG, -AATTCTTGAAACTTGATG, -AATTCTTGAAACTTGAT, -AATTCTTGAAACTTGA, -AATTCTTGAAACTTG, -AATTCTTGAAACTT, -AATTCTTGAAACT, -AATTCTTGAAAC, -AATTCTTGAAA, -AATTCTTGAA, -AATTCTTGA, -AATTCTTG, -AATTCTT, -AATTCT, -AATTC, -AATT, -AAT, -AA, or -A.

Preferably the antisense-oligonucleotide of general formula (S1) has between 10 and 28 nucleotides and at least one LNA nucleotide at the 3' terminus and at least one LNA nucleotide at the 5' terminus. Preferred are also antisense-oligonucleotides having between 10 and 28 nucleotides and two LNA nucleotides at the 3' terminus and two LNA nucleotides at the 5' terminus. As LNA nucleotides (LNA units) especially these disclosed in the chapter "Locked Nucleic Acids (LNA^{®})" and preferably in the chapter "Preferred LNAs" are suitable, but not limited to said LNA nucleotides (LNA units), and as internucleotides bridges especially these disclosed in the chapter "Internucleotide Linkages (IL)" are suitable, but are not limited to said "Internucleotide Linkages (IL)".

More preferably the antisense-oligonucleotide of general formula (S1) has between 11 and 24 nucleotides and at least two LNA nucleotides at the 3' terminus and at least two LNA nucleotides at the 5' terminus. Still more preferably the antisense-oligonucleotide has between 10 and 16, more preferably between 12 and 14 nucleotides and between 1 and 5, preferably 2 and 4 and more preferably between 2 and 3 LNA units at the 3' terminal end and between 1 and 5, preferably 2 and 4 and more preferably between 2 and 3 LNA units at the 5' terminal end. Preferably the antisense-oligonucleotides are gapmers of the form LNA segment A - DNA segment - LNA segment B. Preferably the antisense-oligonucleotides contain at least 6, more preferably at least 7 and most preferably at least 8 non-LNA units such as DNA units in between the two LNA segments. Suitable nucleobases for the non-LNA units and the LNA units are disclosed in the chapter "Nucleobases".

Further preferred are antisense-oligonucleotides of the formula (S1):
5'-N¹-**TAGGGCTG**-N²-3' (Seq. ID No. 7) wherein
N¹ represents: CCAGAGGT-, CAGAGGT-, AGAGGT-, GAGGT-, AGGT-, GGT-, GT-, or T-; and
N² represents: -AATTCTTG, -AATTCTT, -AATTCT, -AATTC, -AATT, -AAT, -AA, or -A.

Further preferred are antisense-oligonucleotides of the formula (S1):
5'-N¹-**TAGGGCTG**-N²-3' (Seq. ID No. 7) wherein
N¹ represents: AGGT-, GGT-, GT-, or T-; and
N² represents: -AATT, -AAT, -AA, or -A.

In preferred embodiments N¹ represents GGT- and N² represents -A. In another preferred embodiments N¹ represents GT- and N² represents -AA. In another preferred embodiments N¹ represents T- and N² represents -AAT.

Preferably, the present invention relates to antisense-oligonucleotides of the formula (S1):
5'-N¹-**TAGGGCTG**-N²-3' (Seq. ID No. 7) wherein
N¹ represents GGT- and N² represents -A.

Preferred are the following antisense-oligonucleotides (Table 3):

| **Seq ID No.** | **L** | **Sequence, 5'-3'** | **Seq ID No.** | **L** | **Sequence, 5'-3'** |
|---|---|---|---|---|---|
| 11 | 10 | TTAGGGCTGA | 22 | 11 | GTTAGGGCTGA |
| 16 | 12 | GGTT AGGGCTGA | 23 | 11 | TTAGGGCTGAA |
| 17 | 12 | GTTAGGGCTGAA | 26 | 13 | AGGTT AGGGCTGA |

| | | | | | |
|---|---|---|---|---|---|
| 18 | 12 | TTAGGGCTGAAT | 27 | 13 | GGTT AGGGCTGAA |
| 19 | 15 | AGGTTAGGGCTGAA T | 28 | 13 | GTT AGGGCTGAA T |
| 20 | 15 | GGTT AGGGCTGAA TT | 29 | 13 | TTAGGGCTGAATT |
| 4 | 16 | AGGTTAGGGCTGAATT | 30 | 14 | AGGTT AGGGCTGAA |
| 32 | 14 | GTT AGGGCTGAA TT | 31 | 14 | GGTT AGGGCTGAA T |

Preferred are antisense-oligonucleotides of general formula (S1A) represented by the following sequence:
5'-N^{1A}-**TTAGGGCT**-N^{2A}-3' (Seq. ID No. 34) wherein
N^{1A} represents: AAATTCTAGACCCCAGAGG-, AATTCTAGACCCCAGAGG-, ATTCTAGACCCCAGAGG-, TTCTAGACCCCAGAGG-, TCTAGACCCCAGAGG-, CTAGACCCCAGAGG-, TAGACCCCAGAGG-, AGACCCCAGAGG-, GACCCCAGAGG-, ACCCCAGAGG-, CCCCAGAGG-, CCCAGAGG-, CCAGAGG-, CAGAGG-, AGAGGT-, GAGG-, AGG-, GG-, or G-; and
N^{2A} represents: -GAATTCTTGAAACTTGATG, -GAATTCTTGAAACTTGAT, -GAATTCTTGAAACTTGA, -GAATTCTTGAAACTTG, -GAATTCTTGAAACTT, -GAATTCTTGAAACT, -GAATTCTTGAAAC, -GAATTCTTGAAA, -GAATTCTTGAA, -GAATTCTTGA, -GAATTCTTG, -GAATTCTT, -GAATTCT, -GAATTC, -GAATT, -GAAT, -GAA, -GA, or -G.

Preferably the antisense-oligonucleotide of general formula (S1A) has between 10 and 28 nucleotides and at least one LNA nucleotide at the 3' terminus and at least one LNA nucleotide at the 5' terminus. Preferred are also antisense-oligonucleotides having between 10 and 28 nucleotides and two LNA nucleotides at the 3' terminus and two LNA nucleotides at the 5' terminus. As LNA nucleotides (LNA units) especially these disclosed in the chapter "Locked Nucleic Acids (LNA^{®})" and preferably in the chapter "Preferred LNAs" are suitable, but not limited to said LNA nucleotides (LNA units), and as internucleotides bridges especially these disclosed in the chapter "Internucleotide Linkages (IL)" are suitable, but are not limited to said "Internucleotide Linkages (IL)".

More preferably the antisense-oligonucleotide of general formula (S1A) has between 11 and 24 nucleotides and at least two LNA nucleotides at the 3' terminus and at least two LNA nucleotides at the 5' terminus. Still more preferably the antisense-oligonucleotide has between 10 and 16, more preferably between 12 and 14 nucleotides and between 1 and 5, preferably 2 and 4 and more preferably between 2 and 3 LNA units at the 3' terminal end and between 1 and 5, preferably 2 and 4 and more preferably between 2 and 3 LNA units at the 5' terminal end. Preferably the antisense-oligonucleotides are gapmers of the form LNA segment A - DNA segment - LNA segment B. Preferably the antisense-oligonucleotides contain at least 6, more preferably at least 7 and most preferably at least 8 non-LNA units such as DNA units in between the two LNA segments. Suitable nucleobases for the non-LNA units and the LNA units are disclosed in the chapter "Nucleobases".

Further preferred are antisense-oligonucleotides of the formula (S1A):
5'-N^{1A}-**TTAGGGCT**-N^{2A}-3' (Seq. ID No. 34) wherein
N^{1A} represents: CCCAGAGG-, CCAGAGG-, CAGAGG-, AGAGG-, GAGG-, AGG-, GG-, or G-; and
N^{2A} represents: -GAATTCTT, -GAATTCT, -GAATTC, -GAATT, -GAAT, -GAA, -GA, or -G.

Further preferred are antisense-oligonucleotides of the formula (S1A):
5'-N^{1A}-**TTAGGGCT**-N^{2A}-3' (Seq. ID No. 34) wherein
N^{1A} represents: GAGG-, AGG-, GG-, or G-; and
N^{2A} represents: ―GAAT, ―GAA, ―GA, or ―G.

In preferred embodiments N^{1A} represents AGG- and N^{2A} represents ―G. In another preferred embodiments N^{1A} represents GG- and N^{2A} represents ―GA. In another preferred embodiments N^{1A} represents G- and N^{2A} represents ―GAA.

Preferably, the present invention relates to antisense-oligonucleotides of the formula (S1A):
5'-N^{1A}-**TTAGGGCT**-N^{2A}-3' (Seq. ID No. 34)
wherein N^{1A} represents: GG- and N^{2A} represents: ―GA.

Preferred are the following antisense-oligonucleotides (Table 4):

| **Seq ID No.** | **L** | **Sequence, 5'-3'** | **Seq ID No.** | **L** | **Sequence, 5'-3'** |
|---|---|---|---|---|---|
| 10 | 10 | GTTAGGGCTG | 21 | 11 | GGTTAGGGCTG |
| 15 | 12 | AGGTTAGGGCTG | 22 | 11 | GTTAGGGCTGA |
| 16 | 12 | GGTTAGGGCTGA | 26 | 13 | AGGTTAGGGCTGA |
| 17 | 12 | GTTAGGGCTGAA | 27 | 13 | GGTTAGGGCTGAA |
| 19 | 15 | AGGTTAGGGCTGAAT | 28 | 13 | GTTAGGGCTGAAT |
| 20 | 15 | GGTTAGGGCTGAATT | 30 | 14 | AGGTTAGGGCTGAA |
| 4 | 16 | AGGTTAGGGCTGAATT | 31 | 14 | GGTTAGGGCTGAAT |
| | | | 32 | 14 | GTTAGGGCTGAATT |

Preferred are antisense-oligonucleotides of general formula (S1B) represented by the following sequence:
5'-N^{1B}-**GTTAGGGC**-N^{2B}-3' (Seq. ID No. 35) wherein
N^{1B} represents: GAAATTCTAGACCCCAGAG-, AAATTCTAGACCCCAGAG-, AATTCTAGACCCCAGAG-, ATTCTAGACCCCAGAG-, TTCTAGACCCCAGAG-, TCTAGACCCCAGAG-, CTAGACCCCAGAG-, TAGACCCCAGAG-, AGACCCCAGAG-, GACCCCAGAG-, ACCCCAGAG-, CCCCAGAG-, CCCAGAG-, CCAGAG-, CAGAG-, AGAG-, GAG-, AG-, or G-; and
N^{2B} represents: -TGAATTCTTGAAACTTGAT, -TGAATTCTTGAAACTTGA, -TGAATTCTTGAAACTTG, -TGAATTCTTGAAACTT, -TGAATTCTTGAAACT, -TGAATTCTTGAAAC, -TGAATTCTTGAAA, -TGAATTCTTGAA, -TGAATTCTTGA, -TGAATTCTTG, -TGAATTCTT, -TGAATTCT, -TGAATTC, -TGAATT, -TGAAT, -TGAA, -TGA, -TG or -T.

Preferably the antisense-oligonucleotide of general formula (S1B) has between 10 and 28 nucleotides and at least one LNA nucleotide at the 3' terminus and at least one LNA nucleotide at the 5' terminus. Preferred are also antisense-oligonucleotides having between 10 and 28 nucleotides and two LNA nucleotides at the 3' terminus and two LNA nucleotides at the 5' terminus. As LNA nucleotides (LNA units) especially these disclosed in the chapter "Locked Nucleic Acids (LNA^{®})" and preferably in the chapter "Preferred LNAs" are suitable, but not limited to said LNA nucleotides (LNA units), and as internucleotides bridges especially these disclosed in the chapter "Internucleotide Linkages (IL)" are suitable, but are not limited to said "Internucleotide Linkages (IL)".

More preferably the antisense-oligonucleotide of general formula (S1B) has between 11 and 24 nucleotides and at least two LNA nucleotides at the 3' terminus and at least two LNA nucleotides at the 5' terminus. Still more preferably the antisense-oligonucleotide has between 10 and 16, more preferably between 12 and 14 nucleotides and between 1 and 5, preferably 2 and 4 and more preferably between 2 and 3 LNA units at the 3' terminal end and between 1 and 5, preferably 2 and 4 and more preferably between 2 and 3 LNA units at the 5' terminal end. Preferably the antisense-oligonucleotides are gapmers of the form LNA segment A - DNA segment - LNA segment B. Preferably the antisense-oligonucleotides contain at least 6, more preferably at least 7 and most preferably at least 8 non-LNA units such as DNA units in between the two LNA segments. Suitable nucleobases for the non-LNA units and the LNA units are disclosed in the chapter "Nucleobases".

Further preferred are antisense-oligonucleotides of the formula (S1B):
5'-N^{1B}-**GTTAGGGC**-N^{2B}-3' (Seq. ID No. 35) wherein
N^{1B} represents: CCCCAGAG-, CCCAGAG-, CCAGAG-, CAGAG-, AGAG-, GAG-, AG-, or G-; and
N^{2B} represents: -TGAATTCT, ―TGAATTC, -TGAATT, ―TGAAT, ―TGAA, ―TGA, ―TG or ―T.

Further preferred are antisense-oligonucleotides of the formula (S1B):
5'-N^{1B}-**GTTAGGGC**-N^{2B}-3' (Seq. ID No. 35) wherein
N^{1B} represents: AGAG-, GAG-, AG-, or G-; and
N^{2B} represents: ―TGAA, ―TGA, ―TG or ―T.

In preferred embodiments N^{1B} represents GAG- and N^{2B} represents ―T. In another preferred embodiments N^{1B} represents AG- and N^{2B} represents ―TG. In another preferred embodiments N^{1B} represents G- and N^{2B} represents ―TGA.

Preferably, the present invention relates to antisense-oligonucleotides of the formula (S1B):
5'-N^{1B}-**GTTAGGGC**-N^{2B}-3' (Seq. ID No. 35)
wherein N^{1B} represents G- and N^{2B} represents ―TGA.

Preferred are the following antisense-oligonucleotides (Table 5):

| **Seq ID No.** | **L** | **Sequence, 5'-3'** | **Seq ID No.** | **L** | **Sequence, 5'-3'** |
|---|---|---|---|---|---|
| 9 | 10 | GGTTAGGGCT | 21 | 11 | GGTT AGGGCTG |
| 15 | 12 | AGGTT AGGGCTG | 26 | 13 | AGGTT AGGGCTGA |
| 16 | 12 | GGTTAGGGCTGA | 27 | 13 | GGTT AGGGCTGAA |
| 19 | 15 | AGGTTAGGGCTGAAT | 30 | 14 | AGGTT AGGGCTGAA |
| 20 | 15 | GGTTAGGGCTGAA TT | 31 | 14 | GGTT AGGGCTGAA T |
| 4 | 16 | AGGTTAGGGCTGAATT | | | |

The present invention preferably relates to an antisense-oligonucleotide consisting of 10 to 28 nucleotides, wherein at least two of the 10 to 28 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence 5'-N¹-**TAGGGCTG**-N²-3' (Seq. ID No. 7) or 5'-N^{1A}-**TTAGGGCT**-N^{2A}-3' (Seq. ID No. 34) or 5'-N^{1B}-**GTTAGGGC**-N^{2B}-3' (Seq. ID No. 35), wherein
N¹ represents: AATTCTAGACCCCAGAGGT-, ATTCTAGACCCCAGAGGT-, TTCTAGACCCCAGAGGT-, TCTAGACCCCAGAGGT-, CTAGACCCCAGAGGT-, TAGACCCCAGAGGT-, AGACCCCAGAGGT-, GACCCCAGAGGT-, ACCCCAGAGGT-, CCCCAGAGGT-, CCCAGAGGT-, CCAGAGGT-, CAGAGGT-, AGAGGT-, GAGGT-, AGGT-, GGT-, GT-, or T-;
N² represents: -AATTCTTGAAACTTGATGG, -AATTCTTGAAACTTGATG, -AATTCTTGAAACTTGAT, -AATTCTTGAAACTTGA, -AATTCTTGAAACTTG, -AATTCTTGAAACTT, -AATTCTTGAAACT, -AATTCTTGAAAC, -AATTCTTGAAA, -AATTCTTGAA, -AATTCTTGA, -AATTCTTG, -AATTCTT, -AATTCT, -AATTC, -AATT, -AAT, -AA, or -A;
N^{1A} represents: AAATTCTAGACCCCAGAGG-, AATTCTAGACCCCAGAGG-, ATTCTAGACCCCAGAGG-, TTCTAGACCCCAGAGG-, TCTAGACCCCAGAGG-, CTAGACCCCAGAGG-, TAGACCCCAGAGG-, AGACCCCAGAGG-, GACCCCAGAGG-, ACCCCAGAGG-, CCCCAGAGG-, CCCAGAGG-, CCAGAGG-, CAGAGG-, AGAGGT-, GAGG-, AGG-, GG-, or G-;
N^{2A} represents: -GAATTCTTGAAACTTGATG, -GAATTCTTGAAACTTGAT, -GAATTCTTGAAACTTGA, -GAATTCTTGAAACTTG, -GAATTCTTGAAACTT, -GAATTCTTGAAACT, -GAATTCTTGAAAC, -GAATTCTTGAAA, -GAATTCTTGAA, -GAATTCTTGA, -GAATTCTTG, -GAATTCTT, -GAATTCT, -GAATTC, -GAATT, -GAAT, -GAA, -GA, or -G;
N^{1B} represents: GAAATTCTAGACCCCAGAG-, AAATTCTAGACCCCAGAG-, AATTCTAGACCCCAGAG-, ATTCTAGACCCCAGAG-, TTCTAGACCCCAGAG-, TCTAGACCCCAGAG-, CTAGACCCCAGAG-, TAGACCCCAGAG-, AGACCCCAGAG-, GACCCCAGAG-, ACCCCAGAG-, CCCCAGAG-, CCCAGAG-, CCAGAG-, CAGAG-, AGAG-, GAG-, AG-, or G-;
N^{2B} represents: -TGAATTCTTGAAACTTGAT, -TGAATTCTTGAAACTTGA, -TGAATTCTTGAAACTTG, -TGAATTCTTGAAACTT, -TGAATTCTTGAAACT, -TGAATTCTTGAAAC, -TGAATTCTTGAAA, -TGAATTCTTGAA, -TGAATTCTTGA, -TGAATTCTTG, -TGAATTCTT, -TGAATTCT, -TGAATTC, -TGAATT, -TGAAT, -TGAA, -TGA, -TG or -T;
and salts and optical isomers of the antisense-oligonucleotide.

The present invention preferably relates to an antisense-oligonucleotide consisting of 10 to 28 nucleotides, wherein at least two of the 10 to 28 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence 5'-N¹-**TAGGGCTG**-N²-3' (Seq. ID No. 7) or 5'-N^{1A}-**TTAGGGCT**-N^{2A}-3' (Seq. ID No. 34) or 5'-N^{1B}-**GTTAGGGC**-N^{2B}-3' (Seq. ID No. 35), wherein the residues N¹, N², N^{1A}, N^{2A}, N^{1b} and N^{2B} have the meanings especially the further limited meanings as disclosed herein and salts and optical isomers of said antisense-oligonucleotide.

The present invention preferably relates to an antisense-oligonucleotide consisting of 10 to 16 nucleotides, wherein at least two of the 10 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence GGTTAGGGCT(Seq. ID No. 9), GTTAGGGCTG (Seq. ID No. 10), TTAGGGCTGA (Seq. ID No. 11), AGGTTAGGGCTG (Seq. ID No. 15), GGTTAGGGCTGA (Seq. ID No. 16), GTTAGGGCTGAA (Seq. ID No. 17), TTAGGGCTGAAT (Seq. ID No. 18), AGGTTAGGGCTGAAT (Seq. ID No. 19), GGTTAGGGCTGAATT (Seq. ID No. 20), GGTTAGGGCTG (Seq. ID No. 21), GTTAGGGCTGA (Seq. ID No. 22), TTAGGGCTGAA (Seq. ID No. 23), AGGTTAGGGCTGA (Seq. ID No. 26), GGTTAGGGCTGAA (Seq. ID No. 27), GTTAGGGCTGAAT (Seq. ID No. 28), TTAGGGCTGAATT (Seq. ID No. 29), AGGTTAGGGCTGAA (Seq. ID No. 30), GGTTAGGGCTGAAT (Seq. ID No. 31), GTTAGGGCTGAATT (Seq. ID No. 32), AGGTTAGGGCTGAATT (Seq. ID No. 4) and salts and optical isomers of said antisense-oligonucleotide.

More preferably, the present invention relates to an antisense-oligonucleotide consisting of 10 to 16 nucleotides, wherein at least two of the 10 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence GGTTAGGGCT(Seq. ID No. 9), GTTAGGGCTG (Seq. ID No. 10), TTAGGGCTGA (Seq. ID No. 11), GGTTAGGGCTGA (Seq. ID No. 16), AGGTTAGGGCTGAAT (Seq. ID No. 19), GGTTAGGGCTGAATT (Seq. ID No. 20), GGTTAGGGCTG (Seq. ID No. 21), GTTAGGGCTGA (Seq. ID No. 22), AGGTTAGGGCTGA (Seq. ID No. 26), GGTTAGGGCTGAA (Seq. ID No. 27), AGGTTAGGGCTGAA (Seq. ID No. 30), GGTTAGGGCTGAAT (Seq. ID No. 31), AGGTTAGGGCTGAATT (Seq. ID No. 4) and salts and optical isomers of said antisense-oligonucleotide.

More preferably, the present invention relates to an antisense-oligonucleotide consisting of 12 to 16 nucleotides, wherein at least two of the 12 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence AGGTTAGGGCTG (Seq. ID No. 15), GGTTAGGGCTGA (Seq. ID No. 16), GTTAGGGCTGAA (Seq. ID No. 17), TTAGGGCTGAAT (Seq. ID No. 18), AGGTTAGGGCTGAAT (Seq. ID No. 19), GGTTAGGGCTGAATT (Seq. ID No. 20), AGGTTAGGGCTGA (Seq. ID No. 26), GGTTAGGGCTGAA (Seq. ID No. 27), GTTAGGGCTGAAT (Seq. ID No. 28), TTAGGGCTGAATT (Seq. ID No. 29), AGGTTAGGGCTGAA (Seq. ID No. 30), GGTTAGGGCTGAAT (Seq. ID No. 31), GTTAGGGCTGAATT (Seq. ID No. 32), AGGTTAGGGCTGAATT (Seq. ID No. 4) and salts and optical isomers of said antisense-oligonucleotide.

Still more preferably, the present invention relates to an antisense-oligonucleotide consisting of 12 to 16 nucleotides, wherein at least two of the 12 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence GGTTAGGGCTGA (Seq. ID No. 16), AGGTTAGGGCTGAAT (Seq. ID No. 19), GGTTAGGGCTGAATT (Seq. ID No. 20), AGGTTAGGGCTGA (Seq. ID No. 26), GGTTAGGGCTGAA (Seq. ID No. 27), AGGTTAGGGCTGAA (Seq. ID No. 30), GGTTAGGGCTGAAT (Seq. ID No. 31), AGGTTAGGGCTGAATT (Seq. ID No. 4) and salts and optical isomers of said antisense-oligonucleotide.

More preferably, the present invention relates to an antisense-oligonucleotide consisting of 12 to 14 nucleotides, wherein at least two of the 12 to 14 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence AGGTTAGGGCTG (Seq. ID No. 15), GGTTAGGGCTGA (Seq. ID No. 16), GTTAGGGCTGAA (Seq. ID No. 17), TTAGGGCTGAAT (Seq. ID No. 18), AGGTTAGGGCTGA (Seq. ID No. 26), GGTTAGGGCTGAA (Seq. ID No. 27), GTTAGGGCTGAAT (Seq. ID No. 28), TTAGGGCTGAATT (Seq. ID No. 29), AGGTTAGGGCTGAA (Seq. ID No. 30), GGTTAGGGCTGAAT (Seq. ID No. 31), GTTAGGGCTGAATT (Seq. ID No. 32), and salts and optical isomers of said antisense-oligonucleotide.

Still more preferably, the present invention relates to an antisense-oligonucleotide consisting of 12 to 14 nucleotides, wherein at least two of the 12 to 14 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence GGTTAGGGCTGA (Seq. ID No. 16), AGGTTAGGGCTGA (Seq. ID No. 26), GGTTAGGGCTGAA (Seq. ID No. 27), AGGTTAGGGCTGAA (Seq. ID No. 30), GGTTAGGGCTGAAT (Seq. ID No. 31), and salts and optical isomers of said antisense-oligonucleotide.

The antisense-oligonucleotides of formula **S1** ,**S1A** or **S1B** in form of gapmers (LNA segment 1 - DNA segment - LNA segment 2) contain an LNA segment at the 5' terminal end consisting of 2 to 5, preferably 2 to 4 LNA units and contain an LNA segment at the 3' terminal end consisting of 2 to 5, preferably 2 to 4 LNA units and between the two LNA segments one DNA segment consisting of 6 to 14, preferably 7 to 12 and more preferably 8 to 11 DNA units.

The antisense-oligonucleotides of formula **S1, S1A** or **S1B** contain the LNA nucleotides (LNA units) as disclosed herein, especially these disclosed in the chapter "Locked Nucleic Acids (LNA^{®})" and preferably these disclosed in the chapter "Preferred LNAs". The LNA units and the DNA units may comprise standard nucleobases such as adenine (A), cytosine (C), guanine (G), thymine (T) and uracil (U), but may also contain modified nucleobases as disclosed in the chapter "Nucleobases". The antisense-oligonucleotides of formula **S1**, **S1A** or **S1B** or the LNA segments and the DNA segment of the antisense-oligonucleotide may contain any internucleotide linkage as disclosed herein and especially these disclosed in the chapter "Internucleotide Linkages (IL)". The antisense-oligonucleotides of formula **S1**, **S1A** or **S1B** may optionally also contain endgroups at the 3' terminal end and/or the 5' terminal end and especially these disclosed in the chapter "Terminal groups".

Experiments have shown that modified nucleobases do not considerably increase or change the activity of the inventive antisense-oligonucleotides in regard to tested neurological and oncological indications. The modified nucleobases 5-methylcytosine or 2-aminoadenine have been demonstrated to further increase the activity of the antisense-oligonucleotides of formula **S1, S1A** or **S1B** especially if 5-methylcytosine is used in the LNA nucleotides only or in the LNA nucleotides and in the DNA nucleotides and/or if 2-aminoadenine is used in the DNA nucleotides and not in the LNA nucleotides.

As LNA units for the antisense-oligonucleotides of formula **S1, S1A** or **S1B** especially β-D-oxy-LNA (**b¹**), β-D-thio-LNA (**b²**), α-L-oxy-LNA (**b⁴**), β-D-ENA (**b⁵**), β-D-(NH)-LNA (**b⁶**), β-D-(NCH₃)-LNA (**b⁷**), β-D-(ONH)-LNA (**b⁸**) and (β-D-(ONCH₃)-LNA (**b⁹**) are preferred. Experiments have been shown that all of these LNA units b¹, b², b⁴, b⁵, b⁶, b⁷, b⁸, and b⁹ can be synthesized with the required effort and lead to antisense-oligonucleotides of comparable stability and activity. However based on the experiments the LNA units b¹, b², b⁴, b⁵, b⁶, and b⁷ are further preferred. Still further preferred are the LNA units b¹, b², b⁴, b⁶, and b⁷, and even more preferred are the LNA units b¹ and b⁴ and most preferred also in regard to the complexity of the chemical synthesis is the β-D-oxy-LNA (**b¹**).

So far no special 3' terminal group or 5' terminal group could be found which remarkably had changed or increased the stability or activity for oncological or neurological indications, so that 3' and 5' end groups are possible but not explicitly preferred.

Various internucleotide bridges or internucleotide linkages are possible. In the formulae disclosed herein the internucleotide linkage IL is represented by ―IL'―Y―. Thus, IL = ―IL'―Y― = ―X"―P(=X')(X⁻)―Y―, wherein IL is preferably selected form the group consisting of:
―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, ―O―P(O)(CH₃)―O―, ―O―P(O)(OCH₃)―O―, ―O―P(O)(NH(CH₃))―O―, ―O―P(O)[N(CH₃)₂]―O―, ―O―P(O)(BH₃⁻)―O―, ―O―P(O)(OCH₂CH₂OCH₃)―O―, ―O―P(O)(OCH₂CH₂SCH₃)―O―, ―O―P(O)(O⁻)―N(CH₃)―, ―N(CH₃)―P(O)(O⁻)―O―. Preferred are the internucleotide linkages IL selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, ―O―P(O)(OCH₃)―O―, ―O―P(O)(NH(CH₃))―O―, ―O―P(O)[N(CH₃)₂]―O―, ―O―P(O)(OCH₂CH₂OCH₃)―O―, and more preferred selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, and still more preferred selected from ―O―P(O)(O⁻)―O―, ―O―P(O)(S⁻)―O―, ―O―P(S)(S⁻)―O―, and most preferably selected from ―O―P(O)(O⁻)―O― and ―O―P(O)(S⁻)―O―.

Thus, the present invention preferably relates to an antisense-oligonucleotide in form of a gapmer consisting of 10 to 28 nucleotides, preferably 10 to 18 nucleotides, more preferably 10 to 16, and still more preferably 12 to 16 or 12 to 14 nucleotides and 1 to 5 of these nucleotides at the 5' terminal end and 1 to 5 nucleotides at the 3' terminal end of the antisense-oligonucleotide are LNA nucleotides and between the LNA nucleotides at the 5' terminal end and the 3' terminal end a sequence of at least 6, preferably 7 and more preferably 8 DNA nucleotides is present, and the antisense-oligonucleotides is capable of hybridizing with a region of the gene encoding *Efnb2,* or with a region of the mRNA encoding *Efnb2,* wherein the antisense-oligonucleotide is represented by the following sequence 5'-N¹-**TAGGGCTG**-N²-3' (Seq. ID No. 7) or 5'-N^{1A}-**TTAGGGCT**-N^{2A}-3' (Seq. ID No. 34) or 5'-N^{1B}-**GTTAGGGC**-N^{2B}-3' (Seq. ID No. 35), wherein the residues N¹, N², N^{1A}, N^{2A}, N^{1B} and N^{2B} have the meanings especially the further limited meanings as disclosed herein, and
the LNA nucleotides are selected from β-D-oxy-LNA (**b¹**), β-D-thio-LNA (**b²**), α-L-oxy-LNA (**b⁴**), β-D-ENA (**b⁵**), β-D-(NH)-LNA (**b⁶**), β-D-(NCH₃)-LNA (**b⁷**), β-D-(ONH)-LNA (**b⁸**) and β-D-(ONCH₃)-LNA (**b⁹**); and preferably from β-D-oxy-LNA (**b¹**), β-D-thio-LNA (**b²**), α-L-oxy-LNA (**b⁴**), β-D-(NH)-LNA (**b⁶**), and β-D-(NCH₃)-LNA (**b⁷**); and
the internucleotide linkages are selected from
   ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, ―O―P(O)(CH₃)―O―, ―O―P(O)(OCH₃)―O―, ―O―P(O)(NH(CH₃))―O―, ―O―P(O)[N(CH₃)₂]―O―, ―O―P(O)(BH₃⁻)―O―, ―O―P(O)(OCH₂CH₂OCH₃)―O―, ―O―P(O)(OCH₂CH₂SCH₃)―O―, ―O―P(O)(O⁻)―N(CH₃)―, ―N(CH₃)―P(O)(O⁻)―O―;
and preferably from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―; and salts and optical isomers of said antisense-oligonucleotide.
Such preferred antisense-oligonucleotides may not contain any modified 3' and 5' terminal end or may not contain any 3' and 5' terminal group and may as modified nucleobase contain 5-methylcytosine and/or 2-aminoadenine, and

Still further preferred, the present invention relates to an antisense-oligonucleotide in form of a gapmer consisting of 10 to 28 nucleotides, preferably 10 to 18 nucleotides, more preferably 10 to 16, and still more preferably 12 to 16 or 12 to 14 nucleotides and 1 to 5 of these nucleotides at the 5' terminal end and 1 to 5 nucleotides at the 3' terminal end of the antisense-oligonucleotide are LNA nucleotides and between the LNA nucleotides at the 5' terminal end and the 3' terminal end a sequence of at least 6, preferably 7 and more preferably 8 DNA nucleotides is present, and the antisense-oligonucleotides is capable of hybridizing with a region of the gene encoding *Efnb2,* or with a region of the mRNA encoding *Efnb2,* wherein the antisense-oligonucleotide is represented by the following sequence 5'-N¹-**TAGGGCTG**-N²-3' (Seq. ID No. 7) or 5'-N^{1A}-**TTAGGGCT**-N^{2A}-3' (Seq. ID No. 34) or 5'-N^{1B}-**GTTAGGGC**-N^{2B}-3' (Seq. ID No. 35), wherein the residues N¹, N², N^{1A}, N^{2A}, N^{1B} and N^{2B} have the meanings especially the further limited meanings as disclosed herein, and and the LNA nucleotides are selected from β-D-oxy-LNA (**b¹**), β-D-thio-LNA (**b²**), α-L-oxy-LNA (**b⁴**), β-D-(NH)-LNA (**b⁶**), and β-D-(NCH₃)-LNA (**b⁷**); and
the internucleotide linkages are selected from
   ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―; and
preferably selected from phosphate, phosphorothioate and phosphorodithioate; and salts and optical isomers of the antisense-oligonucleotide.

Especially preferred are the gapmer antisense-oligonucleotides of Table 1 or Table 2 or Tables 3 - 5 containing a segment of 2 to 5, preferably 2 to 4 and more preferably 2 to 3 LNA units at the 3' terminus and a segment of 2 to 5, preferably 2 to 4 and more preferably 2 to 3 LNA units at the 5' terminus and a segment of at least 6, preferably 7 and more preferably 8 DNA units between the two segments of LNA units, wherein the LNA units are selected from β-D-oxy-LNA (**b¹**), β-D-thio-LNA (**b²**), α-L-oxy-LNA (**b⁴**), β-D-(NH)-LNA (**b⁶**), and β-D-(NCH₃)-LNA (**b⁷**) and the internucleotide linkages are selected from phosphate, phosphorothioate and phosphorodithioate. Such preferred antisense-oligonucleotides may not contain any modified 3' and 5' terminal end or may not contain any 3' and 5' terminal group and may as modified nucleobase contain 5-methylcytosine in the LNA units, preferably all the LNA units and/or 2-aminoadenine in some or all DNA units and/or 5-methylcytosine in some or all DNA units.

Preferred are the following antisense-oligonucleotides (Table 6) consisting of 10 to 16 nucleotides and preferably comprise a sequence of at least 10 consecutive nucleotides that corresponds to a sequence of 10 consecutive nucleotides of the sequence TACAAGCAAGGCATTT (Seq. ID No. 5):

| **Seq ID No.** | **L** | **Sequence, 5'-3'** | **Seq ID No.** | **L** | **Sequence, 5'-3'** |
|---|---|---|---|---|---|
| 37 | 10 | TACAAGCAAG | 51 | 11 | TACAAGCAAGG |
| 38 | 10 | ACAAGCAAGG | 52 | 11 | ACAAGCAAGGC |
| 39 | 10 | CAAGCAAGGC | 53 | 11 | CAAGCAAGGCA |
| 40 | 10 | AAGCAAGGCA | 54 | 11 | AAGCAAGGCAT |
| 41 | 10 | AGCAAGGCAT | 55 | 11 | AGCAAGGCA TT |
| 42 | 10 | GCAAGGCA TT | 56 | 11 | GCAAGGCA TTT |
| 43 | 10 | CAAGGCA TTT | 57 | 13 | TACAAGCAAGGCA |
| 44 | 12 | TACAAGCAAGGC | 58 | 13 | ACAAGCAAGGCAT |
| 45 | 12 | ACAAGCAAGGCA | 59 | 13 | CAAGCAAGGCA TT |
| 46 | 12 | CAAGCAAGGCAT | 60 | 13 | AAGCAAGGCA TTT |
| 47 | 12 | AAGCAAGGCA TT | 61 | 14 | TACAAGCAAGGCAT |
| 48 | 12 | AGCAAGGCA TTT | 62 | 14 | ACAAGCAAGGCA TT |
| 49 | 15 | TACAAGCAAGGCATT | 63 | 14 | CAAGCAAGGCA TTT |
| 50 | 15 | ACAAGCAAGGCA TTT | 5 | 16 | TACAAGCAAGGCATTT |

More preferred are the following antisense-oligonucleotides (Table 7) consisting of 10 to 16 nucleotides and preferably comprise a sequence of at least 10 consecutive nucleotides that corresponds to a sequence of 10 consecutive nucleotides of the sequence TACAAGCAAGGCATTT (Seq. ID No. 5):

| **Seq ID No.** | **L** | **Sequence, 5'-3'** | **Seq ID No.** | **L** | **Sequence, 5'-3'** |
|---|---|---|---|---|---|
| 39 | 10 | CAAGCAAGGC | 52 | 11 | ACAAGCAAGGC |
| 40 | 10 | AAGCAAGGCA | 53 | 11 | CAAGCAAGGCA |
| 41 | 10 | AGCAAGGCAT | 54 | 11 | AAGCAAGGCAT |
| 44 | 12 | TACAAGCAAGGC | 55 | 11 | AGCAAGGCA TT |
| 45 | 12 | ACAAGCAAGGCA | 57 | 13 | TACAAGCAAGGCA |
| 46 | 12 | CAAGCAAGGCAT | 58 | 13 | ACAAGCAAGGCAT |
| 47 | 12 | AAGCAAGGCA TT | 59 | 13 | CAAGCAAGGCA TT |
| 48 | 12 | AGCAAGGCA TTT | 60 | 13 | AAGCAAGGCA TTT |
| 49 | 15 | TACAAGCAAGGCATT | 61 | 14 | TACAAGCAAGGCAT |
| 50 | 15 | ACAAGCAAGGCA TTT | 62 | 14 | ACAAGCAAGGCA TT |
| 5 | 16 | TACAAGCAAGGCATTT | 63 | 14 | CAAGCAAGGCA TTT |

Preferred are also antisense-oligonucleotides of general formula (S2) represented by the following sequence:
5'-N³- **AGCAAGGC** ―N⁴-3' (Seq. ID No. 64) wherein
N³ represents: GACCAGGGACGATCATACA-, ACCAGGGACGATCATACA-, CCAGGGACGATCATACA-, CAGGGACGATCATACA-, AGGGACGATCATACA-, GGGACGATCATACA-, GGACGATCATACA-, GACGATCATACA-, ACGATCATACA-, CGATCATACA-, GATCATACA-, ATCATACA-, TCATACA-, CATACA-, ATACA-, TACA-, ACA-, CA-, or A-; and
N⁴ represents: -ATTTACAGTAACTTTACAA, -ATTTACAGTAACTTTACA, -ATTTACAGTAACTTTAC, -ATTTACAGTAACTTTA, -ATTTACAGTAACTTT, -ATTTACAGTAACTT, -ATTTACAGTAACT, -ATTTACAGTAAC, -ATTTACAGTAA, -ATTTACAGTA, -ATTTACAGT, -ATTTACAGT, -ATTTACAG, -ATTTACA, -ATTTAC, -ATTTA, -ATTT, -ATT, -AT, or -A.

Preferably the antisense-oligonucleotide of general formula (S2) has between 10 and 28 nucleotides and at least one LNA nucleotide at the 3' terminus and at least one LNA nucleotide at the 5' terminus. Preferred are also antisense-oligonucleotides having between 10 and 28 nucleotides and two LNA nucleotides at the 3' terminus and two LNA nucleotides at the 5' terminus. As LNA nucleotides (LNA units) especially these disclosed in the chapter "Locked Nucleic Acids (LNA^{®})" and preferably in the chapter "Preferred LNAs" are suitable and as internucleotides bridges especially these disclosed in the chapter "Internucleotide Linkages (IL)" are suitable.

More preferably the antisense-oligonucleotide has between 11 and 24 nucleotides and at least two LNA nucleotides at the 3' terminus and at least two LNA nucleotides at the 5' terminus.

Still more preferably the antisense-oligonucleotide has between 10 and 16, more preferably between 12 and 14 nucleotides and between 1 and 5, preferably 2 and 4 and more preferably between 2 and 3 LNA units at the 3' terminal end and between 1 and 5, preferably 2 and 4 and more preferably between 2 and 3 LNA units at the 5' terminal end. Preferably the antisense-oligonucleotides are GapmeRs of the form LNA segment A - DNA segment - LNA segment B. Preferably the antisense-oligonucleotides contain at least 6, more preferably at least 7 and most preferably at least 8 non-LNA units such as DNA units in between the two LNA segments. Suitable nucleobases for the non-LNA units and the LNA units are disclosed in the chapter "Nucleobases".

Thus, preferred are also antisense-oligonucleotides of general formula (S2)
5'-N³- **AGCAAGGC** ―N⁴-3' (Seq. ID No. 64) wherein
N³ represents: ATCATACA-, TCATACA-, CATACA-, ATACA-, TACA-, ACA-, CA-, or A-; and
N⁴ represents: ―ATTTACAG, ―ATTTACA, -ATTTAC, ―ATTTA, ―ATTT, ―ATT, ―AT, or -A.

Further preferred are antisense-oligonucleotides of the formula (S2):
5'-N³- **AGCAAGGC** ―N⁴-3' (Seq. ID No. 64) wherein
N³ represents: TACA-, ACA-, CA-, or A-; and
N⁴ represents: ―ATTT, ―ATT, ―AT, or -A.

In preferred embodiments N³ represents ACA- and N⁴ represents ―A. In another preferred embodiments N³ represents CA- and N⁴ represents ―AT. In another preferred embodiments N³ represents A- and N⁴ represents ―ATT.

Preferably, the present invention relates to antisense-oligonucleotides of the formula (S2):
5'-N³- **AGCAAGGC** ―N⁴-3' (Seq. ID No. 64) wherein
N³ represents CA-, and N⁴ represents ―AT.

Preferred are the following antisense-oligonucleotides (Table 8):

| **Seq ID No.** | **L** | **Sequence, 5'-3'** | **Seq ID No.** | **L** | **Sequence, 5'-3'** |
|---|---|---|---|---|---|
| 40 | 10 | AAGCAAGGCA | 53 | 11 | CAAGCAAGGCA |
| 45 | 12 | ACAAGCAAGGCA | 54 | 11 | AAGCAAGGCAT |
| 46 | 12 | CAAGCAAGGCAT | 57 | 13 | TACAAGCAAGGCA |
| 47 | 12 | AAGCAAGGCA TT | 58 | 13 | ACAAGCAAGGCAT |
| 49 | 15 | TACAAGCAAGGCATT | 59 | 13 | CAAGCAAGGCA TT |
| 50 | 15 | ACAAGCAAGGCA TTT | 60 | 13 | AAGCAAGGCA TTT |
| 63 | 14 | CAAGCAAGGCA TTT | 61 | 14 | TACAAGCAAGGCAT |
| 5 | 16 | TACAAGCAAGGCATTT | 62 | 14 | ACAAGCAAGGCA TT |

Preferred are also antisense-oligonucleotides of general formula (S2A) represented by the following sequence:
5'-N^{3A}- **AAGCAAGG** ―N^{4A}-3' (Seq. ID No. 65) wherein
N^{3A} represents: TGACCAGGGACGATCATAC-, GACCAGGGACGATCATAC-, ACCAGGGACGATCATAC-, CCAGGGACGATCATAC-, CAGGGACGATCATAC-, AGGGACGATCATAC-, GGGACGATCATAC-, GGACGATCATAC-, GACGATCATAC-, ACGATCATAC-, CGATCATAC-, GATCATAC-, ATCATAC-, TCATAC-, CATAC-, ATAC-, TAC-, AC-, or C-; and
N^{4A} represents: -CATTTACAGTAACTTTACA, -CATTTACAGTAACTTTAC, -CATTTACAGTAACTTTA, -CATTTACAGTAACTTT, -CATTTACAGTAACTT, -CATTTACAGTAACT, -CATTTACAGTAAC, -CATTTACAGTAA, -CATTTACAGTA, -CATTTACAGT, -CATTTACAGT, -CATTTACAG, -CATTTACA, -CATTTAC, -CATTTA, -CATTT, -CATT, -CAT, -CA or -C.

Preferably the antisense-oligonucleotide of general formula (S2A) has between 10 and 28 nucleotides and at least one LNA nucleotide at the 3' terminus and at least one LNA nucleotide at the 5' terminus. Preferred are also antisense-oligonucleotides having between 10 and 28 nucleotides and two LNA nucleotides at the 3' terminus and two LNA nucleotides at the 5' terminus. As LNA nucleotides (LNA units) especially these disclosed in the chapter "Locked Nucleic Acids (LNA^{®})" and preferably in the chapter "Preferred LNAs" are suitable and as internucleotides bridges especially these disclosed in the chapter "Internucleotide Linkages (IL)" are suitable.

More preferably the antisense-oligonucleotide has between 11 and 24 nucleotides and at least two LNA nucleotides at the 3' terminus and at least two LNA nucleotides at the 5' terminus.

Still more preferably the antisense-oligonucleotide has between 10 and 16, more preferably between 12 and 14 nucleotides and between 1 and 5, preferably 2 and 4 and more preferably between 2 and 3 LNA units at the 3' terminal end and between 1 and 5, preferably 2 and 4 and more preferably between 2 and 3 LNA units at the 5' terminal end. Preferably the antisense-oligonucleotides are GapmeRs of the form LNA segment A - DNA segment - LNA segment B. Preferably the antisense-oligonucleotides contain at least 6, more preferably at least 7 and most preferably at least 8 non-LNA units such as DNA units in between the two LNA segments. Suitable nucleobases for the non-LNA units and the LNA units are disclosed in the chapter "Nucleobases".

Thus, preferred are also antisense-oligonucleotides of general formula (S2A):
5'-N^{3A}- **AAGCAAGG** ―N^{4A}-3' (Seq. ID No. 65) wherein
N^{3A} represents: GATCATAC-, ATCATAC-, TCATAC-, CATAC-, ATAC-, TAC-, AC-, or C-; and
N^{4A} represents: ―CATTTACA, -CATTTAC, ―CATTTA, ―CATTT, ―CATT, ―CAT, -CA or -C.

Further preferred are also antisense-oligonucleotides of general formula (S2A):
5'-N^{3A}- **AAGCAAGG** ―N^{4A}-3' (Seq. ID No. 65) wherein
N^{3A} represents: ATAC-, TAC-, AC-, or C-; and
N^{4A} represents: ―CATT, ―CAT, -CA or -C.

In preferred embodiments N^{3A} represents TAC- and N^{4A} represents ―C. In another preferred embodiments N^{3A} represents AC- and N^{4A} represents ―CA. In another preferred embodiments N^{3A} represents C- and N^{4A} represents ―CAT.

Preferably, the present invention relates to antisense-oligonucleotides of the formula (S2A):
5'-N^{3A}- **AAGCAAGG** ―N^{4A}-3' (Seq. ID No. 65) wherein
N^{3A} represents C- and N^{4A} represents ―CAT.

Preferred are the following antisense-oligonucleotides (Table 9):

| **Seq ID No.** | **L** | **Sequence, 5'-3'** | **Seq ID No.** | **L** | **Sequence, 5'-3'** |
|---|---|---|---|---|---|
| 39 | 10 | CAAGCAAGGC | 52 | 11 | ACAAGCAAGGC |
| 44 | 12 | TACAAGCAAGGC | 53 | 11 | CAAGCAAGGCA |
| 45 | 12 | ACAAGCAAGGCA | 57 | 13 | TACAAGCAAGGCA |
| 46 | 12 | CAAGCAAGGCAT | 58 | 13 | ACAAGCAAGGCAT |
| 49 | 15 | TACAAGCAAGGCATT | 59 | 13 | CAAGCAAGGCA TT |
| 50 | 15 | ACAAGCAAGGCA TTT | 61 | 14 | TACAAGCAAGGCAT |
| 5 | 16 | TACAAGCAAGGCATTT | 62 | 14 | ACAAGCAAGGCA TT |
| | | | 63 | 14 | CAAGCAAGGCA TTT |

Preferred are also antisense-oligonucleotides of general formula (S2B) represented by the following sequence:
5'-N^{3B}-**GCAAGGCA**―N^{4B}-3' (Seq. ID No. 66) wherein
N^{3B} represents: ACCAGGGACGATCATACAA-, CCAGGGACGATCATACAA-, CAGGGACGATCATACAA-, AGGGACGATCATACAA-, GGGACGATCATACAA-, GGACGATCATACAA-, GACGATCATACAA-, ACGATCATACAA-, CGATCATACAA-, GATCATACAA-, ATCATACAA-, TCATACAA-, CATACAA-, ATACAA-, TACAA-, ACAA-, CAA-, AA-, or A-; and
N^{4B} represents: ―TTTACAGTAACTTTACAAA, -TTTACAGTAACTTTACAA, -TTTACAGTAACTTTACA, -TTTACAGTAACTTTAC, ―TTTACAGTAACTTTA, -TTTACAGTAACTTT, -TTTACAGTAACTT, ―TTTACAGTAACT, -TTTACAGTAAC, -TTTACAGTAA, ―TTTACAGTA, ―TTTACAGT, -TTTACAGT, ―TTTACAG, ―TTTACA, -TTTAC, ―TTTA, ―TTT, ―TT, or ―T.

Preferably the antisense-oligonucleotide of general formula (S2B) has between 10 and 28 nucleotides and at least one LNA nucleotide at the 3' terminus and at least one LNA nucleotide at the 5' terminus. Preferred are also antisense-oligonucleotides having between 10 and 28 nucleotides and two LNA nucleotides at the 3' terminus and two LNA nucleotides at the 5' terminus. As LNA nucleotides (LNA units) especially these disclosed in the chapter "Locked Nucleic Acids (LNA^{®})" and preferably in the chapter "Preferred LNAs" are suitable and as internucleotides bridges especially these disclosed in the chapter "Internucleotide Linkages (IL)" are suitable.

More preferably the antisense-oligonucleotide has between 11 and 24 nucleotides and at least two LNA nucleotides at the 3' terminus and at least two LNA nucleotides at the 5' terminus.

Still more preferably the antisense-oligonucleotide has between 10 and 16, more preferably between 12 and 14 nucleotides and between 1 and 5, preferably 2 and 4 and more preferably between 2 and 3 LNA units at the 3' terminal end and between 1 and 5, preferably 2 and 4 and more preferably between 2 and 3 LNA units at the 5' terminal end. Preferably the antisense-oligonucleotides are GapmeRs of the form LNA segment A - DNA segment - LNA segment B. Preferably the antisense-oligonucleotides contain at least 6, more preferably at least 7 and most preferably at least 8 non-LNA units such as DNA units in between the two LNA segments. Suitable nucleobases for the non-LNA units and the LNA units are disclosed in the chapter "Nucleobases".

Thus, preferred are also antisense-oligonucleotides of general formula (S2B):
5'-N^{3B}-**GCAAGGCA**―N^{4B}-3' (Seq. ID No. 66) wherein
N^{3B} represents: TCATACAA-, CATACAA-, ATACAA-, TACAA-, ACAA-, CAA-, AA-, or A-; and
N^{4B} represents: -TTTACAGT, ―TTTACAG, ―TTTACA, -TTTAC, ―TTTA, ―TTT, ―TT, or ―T.

Further preferred are also antisense-oligonucleotides of general formula (S2B):
5'-N^{3B}-**GCAAGGCA**―N^{4B}-3' (Seq. ID No. 66) wherein
N^{3B} represents: ACAA-, CAA-, AA-, or A-; and
N^{4B} represents: ―TTTA, ―TTT, ―TT, or ―T.

In preferred embodiments N^{3B} represents CAA- and N^{4B} represents ―T. In another preferred embodiments N^{3B} represents AA- and N^{4B} represents ―TT. In another preferred embodiments N^{3B} represents A- and N^{4B} represents ―T.

Preferably, the present invention relates to antisense-oligonucleotides of the formula (S2B):
5'-N^{3B}-**GCAAGGCA**―N^{4B}-3' (Seq. ID No. 66) wherein
N^{3B} represents CAA- and N^{4B} represents ―T.

Preferred are the following antisense-oligonucleotides (Table 10):

| **Seq ID No.** | **L** | **Sequence, 5'-3'** | **Seq ID No.** | **L** | **Sequence, 5'-3'** |
|---|---|---|---|---|---|
| 41 | 10 | AGCAAGGCAT | 54 | 11 | AAGCAAGGCAT |
| 46 | 12 | CAAGCAAGGCAT | 55 | 11 | AGCAAGGCA TT |
| 47 | 12 | AAGCAAGGCA TT | 58 | 13 | ACAAGCAAGGCAT |
| 48 | 12 | AGCAAGGCA TTT | 59 | 13 | CAAGCAAGGCA TT |
| 49 | 15 | TACAAGCAAGGCATT | 60 | 13 | AAGCAAGGCA TTT |
| 50 | 15 | ACAAGCAAGGCA TTT | 61 | 14 | TACAAGCAAGGCAT |
| 5 | 16 | TACAAGCAAGGCATTT | 62 | 14 | ACAAGCAAGGCA TT |
| | | | 63 | 14 | CAAGCAAGGCA TTT |

The present invention preferably relates to an antisense-oligonucleotide consisting of 10 to 28 nucleotides, wherein at least two of the 10 to 28 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence 5'-N³- **AGCAAGGC** ―N⁴-3' (Seq. ID No. 64) or 5'-N^{3A}- **AAGCAAGG** ―N^{4A}-3' (Seq. ID No. 65) or 5'-N^{3B}-**GCAAGGCA**―N^{4B}-3' (Seq. ID No. 66), wherein
N³ represents: GACCAGGGACGATCATACA-, ACCAGGGACGATCATACA-, CCAGGGACGATCATACA-, CAGGGACGATCATACA-, AGGGACGATCATACA-, GGGACGATCATACA-, GGACGATCATACA-, GACGATCATACA-, ACGATCATACA-, CGATCATACA-, GATCATACA-, ATCATACA-, TCATACA-, CATACA-, ATACA-, TACA-, ACA-, CA-, or A-;
N⁴ represents: -ATTTACAGTAACTTTACAA, -ATTTACAGTAACTTTACA, -ATTTACAGTAACTTTAC, -ATTTACAGTAACTTTA, -ATTTACAGTAACTTT, -ATTTACAGTAACTT, -ATTTACAGTAACT, -ATTTACAGTAAC, -ATTTACAGTAA, -ATTTACAGTA, -ATTTACAGT, -ATTTACAGT, -ATTTACAG, -ATTTACA, -ATTTAC, -ATTTA, -ATTT, -ATT, -AT, or -A;
N^{3A} represents: TGACCAGGGACGATCATAC-, GACCAGGGACGATCATAC-, ACCAGGGACGATCATAC-, CCAGGGACGATCATAC-, CAGGGACGATCATAC-, AGGGACGATCATAC-, GGGACGATCATAC-, GGACGATCATAC-, GACGATCATAC-, ACGATCATAC-, CGATCATAC-, GATCATAC-, ATCATAC-, TCATAC-, CATAC-, ATAC-, TAC-, AC-, or C-;
N^{4A} represents: -CATTTACAGTAACTTTACA, -CATTTACAGTAACTTTAC, ―CATTTACAGTAACTTTA, -CATTTACAGTAACTTT, -CATTTACAGTAACTT, ―CATTTACAGTAACT, -CATTTACAGTAAC, -CATTTACAGTAA, ―CATTTACAGTA, ―CATTTACAGT, -CATTTACAGT, ―CATTTACAG, ―CATTTACA, -CATTTAC, ―CATTTA, ―CATTT, ―CATT, ―CAT, -CA or ―C;
N^{3B} represents: ACCAGGGACGATCATACAA-, CCAGGGACGATCATACAA-, CAGGGACGATCATACAA-, AGGGACGATCATACAA-, GGGACGATCATACAA-, GGACGATCATACAA-, GACGATCATACAA-, ACGATCATACAA-, CGATCATACAA-, GATCATACAA-, ATCATACAA-, TCATACAA-, CATACAA-, ATACAA-, TACAA-, ACAA-, CAA-, AA-, or A-;
N^{4B} represents: ―TTTACAGTAACTTTACAAA, -TTTACAGTAACTTTACAA, -TTTACAGTAACTTTACA, -TTTACAGTAACTTTAC, ―TTTACAGTAACTTTA, -TTTACAGTAACTTT, -TTTACAGTAACTT, ―TTTACAGTAACT, -TTTACAGTAAC, -TTTACAGTAA, ―TTTACAGTA, ―TTTACAGT, -TTTACAGT, ―TTTACAG, ―TTTACA, -TTTAC, ―TTTA, ―TTT, ―TT, or ―T;
and salts and optical isomers of the antisense-oligonucleotide.

The present invention preferably relates to an antisense-oligonucleotide consisting of 10 to 28 nucleotides, wherein at least two of the 10 to 28 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence 5'-N³- **AGCAAGGC** ―N⁴-3' (Seq. ID No. 64) or 5'-N^{3A}- **AAGCAAGG** ―N^{4A}-3' (Seq. ID No. 65) or 5'-N^{3B}-**GCAAGGCA**―N^{4B}-3' (Seq. ID No. 66), wherein the residues N³, N⁴, N^{3A}, N^{4A}, N^{3B} and N^{4B} have the meanings especially the further limited meanings as disclosed herein and salts and optical isomers of said antisense-oligonucleotide.

The present invention preferably relates to an antisense-oligonucleotide consisting of 10 to 16 nucleotides, wherein at least two of the 10 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence CAAGCAAGGC (Seq. ID No. 39), AAGCAAGGCA (Seq. ID No. 40), AGCAAGGCAT (Seq. ID No. 41), TACAAGCAAGGC (Seq. ID No. 44), ACAAGCAAGGCA (Seq. ID No. 45), CAAGCAAGGCAT (Seq. ID No. 46), AAGCAAGGCATT (Seq. ID No. 47), AGCAAGGCATTT (Seq. ID No. 48), TACAAGCAAGGCATT (Seq. ID No. 49), ACAAGCAAGGCATTT (Seq. ID No. 50), ACAAGCAAGGC (Seq. ID No. 52), CAAGCAAGGCA (Seq. ID No. 53), AAGCAAGGCAT (Seq. ID No. 54), AGCAAGGCATT (Seq. ID No. 55), TACAAGCAAGGCA (Seq. ID No. 57), ACAAGCAAGGCAT (Seq. ID No. 58), CAAGCAAGGCATT (Seq. ID No. 59), AAGCAAGGCATTT (Seq. ID No. 60), TACAAGCAAGGCAT (Seq. ID No. 61), ACAAGCAAGGCATT (Seq. ID No. 62) CAAGCAAGGCATTT (Seq. ID No. 63) TACAAGCAAGGCATTT (Seq. ID No. 5) and salts and optical isomers of said antisense-oligonucleotide.

More preferably, the present invention relates to an antisense-oligonucleotide consisting of 10 to 16 nucleotides, wherein at least two of the 10 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence CAAGCAAGGC (Seq. ID No. 39), AAGCAAGGCA (Seq. ID No. 40), AGCAAGGCAT (Seq. ID No. 41), CAAGCAAGGCAT (Seq. ID No. 46), TACAAGCAAGGCATT (Seq. ID No. 49), ACAAGCAAGGCATTT (Seq. ID No. 50),CAAGCAAGGCA (Seq. ID No. 53), AAGCAAGGCAT (Seq. ID No. 54), ACAAGCAAGGCAT (Seq. ID No. 58), CAAGCAAGGCATT (Seq. ID No. 59), TACAAGCAAGGCAT (Seq. ID No. 61), ACAAGCAAGGCATT (Seq. ID No. 62) CAAGCAAGGCATTT (Seq. ID No. 63) TACAAGCAAGGCATTT (Seq. ID No. 5) and salts and optical isomers of said antisense-oligonucleotide.

More preferably, the present invention relates to an antisense-oligonucleotide consisting of 12 to 16 nucleotides, wherein at least two of the 12 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence TACAAGCAAGGC (Seq. ID No. 44), ACAAGCAAGGCA (Seq. ID No. 45), CAAGCAAGGCAT (Seq. ID No. 46), AAGCAAGGCATT (Seq. ID No. 47), AGCAAGGCATTT (Seq. ID No. 48), TACAAGCAAGGCATT (Seq. ID No. 49), ACAAGCAAGGCATTT (Seq. ID No. 50), TACAAGCAAGGCA (Seq. ID No. 57), ACAAGCAAGGCAT (Seq. ID No. 58), CAAGCAAGGCATT (Seq. ID No. 59), AAGCAAGGCATTT (Seq. ID No. 60), TACAAGCAAGGCAT (Seq. ID No. 61), ACAAGCAAGGCATT (Seq. ID No. 62), CAAGCAAGGCATTT (Seq. ID No. 63), TACAAGCAAGGCATTT (Seq. ID No. 5) and salts and optical isomers of said antisense-oligonucleotide.

Still more preferably, the present invention relates to an antisense-oligonucleotide consisting of 12 to 16 nucleotides, wherein at least two of the 12 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence CAAGCAAGGCAT (Seq. ID No. 46), TACAAGCAAGGCATT (Seq. ID No. 49), ACAAGCAAGGCATTT (Seq. ID No. 50), ACAAGCAAGGCAT (Seq. ID No. 58), CAAGCAAGGCATT (Seq. ID No. 59), TACAAGCAAGGCAT (Seq. ID No. 61), ACAAGCAAGGCATT (Seq. ID No. 62), CAAGCAAGGCATTT (Seq. ID No. 63), TACAAGCAAGGCATTT (Seq. ID No. 5) and salts and optical isomers of said antisense-oligonucleotide.

More preferably, the present invention relates to an antisense-oligonucleotide consisting of 12 to 14 nucleotides, wherein at least two of the 12 to 14 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence TACAAGCAAGGC (Seq. ID No. 44), ACAAGCAAGGCA (Seq. ID No. 45), CAAGCAAGGCAT (Seq. ID No. 46), AAGCAAGGCATT (Seq. ID No. 47), AGCAAGGCATTT (Seq. ID No. 48), TACAAGCAAGGCA (Seq. ID No. 57), ACAAGCAAGGCAT (Seq. ID No. 58), CAAGCAAGGCATT (Seq. ID No. 59), AAGCAAGGCATTT (Seq. ID No. 60), TACAAGCAAGGCAT (Seq. ID No. 61), ACAAGCAAGGCATT (Seq. ID No. 62), CAAGCAAGGCATTT (Seq. ID No. 63) and salts and optical isomers of said antisense-oligonucleotide.

Still more preferably, the present invention relates to an antisense-oligonucleotide consisting of 12 to 14 nucleotides, wherein at least two of the 12 to 14 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence CAAGCAAGGCAT (Seq. ID No. 46), ACAAGCAAGGCAT (Seq. ID No. 58), CAAGCAAGGCATT (Seq. ID No. 59), TACAAGCAAGGCAT (Seq. ID No. 61), ACAAGCAAGGCATT (Seq. ID No. 62), CAAGCAAGGCATTT (Seq. ID No. 63) and salts and optical isomers of said antisense-oligonucleotide.

The antisense-oligonucleotides of formula **S2, S2A** or **S2B** in form of gapmers (LNA segment 1 - DNA segment - LNA segment 2) contain an LNA segment at the 5' terminal end consisting of 2 to 5, preferably 2 to 4 LNA units and contain an LNA segment at the 3' terminal end consisting of 2 to 5, preferably 2 to 4 LNA units and between the two LNA segments one DNA segment consisting of 6 to 14, preferably 7 to 12 and more preferably 8 to 11 DNA units.

The antisense-oligonucleotides of formula **S2, S2A** or **S2B** contain the LNA nucleotides (LNA units) as disclosed herein, especially these disclosed in the chapter "Locked Nucleic Acids (LNA^{®})" and preferably these disclosed in the chapter "Preferred LNAs". The LNA units and the DNA units may comprise standard nucleobases such as adenine (A), cytosine (C), guanine (G), thymine (T) and uracil (U), but may also contain modified nucleobases as disclosed in the chapter "Nucleobases". The antisense-oligonucleotides of formula **S2, S2A** or **S2B** or the LNA segments and the DNA segment of the antisense-oligonucleotide may contain any internucleotide linkage as disclosed herein and especially these disclosed in the chapter "Internucleotide Linkages (IL)". The antisense-oligonucleotides of formula **S2, S2A** or **S2B** may optionally also contain endgroups at the 3' terminal end and/or the 5' terminal end and especially these disclosed in the chapter "Terminal groups".

Experiments have shown that modified nucleobases do not considerably increase or change the activity of the inventive antisense-oligonucleotides in regard to tested neurological and oncological indications. The modified nucleobases 5-methylcytosine or 2-aminoadenine have been demonstrated to further increase the activity of the antisense-oligonucleotides of formula **S2, S2A** or **S2B** especially if 5-methylcytosine is used in the LNA nucleotides only or in the LNA nucleotides and in the DNA nucleotides and/or if 2-aminoadenine is used in the DNA nucleotides and not in the LNA nucleotides.

As LNA units for the antisense-oligonucleotides of formula **S2, S2A** or **S2B** especially β-D-oxy-LNA (**b¹**), β-D-thio-LNA (**b²**), α-L-oxy-LNA (**b⁴**), β-D-ENA (**b⁵**), β-D-(NH)-LNA (**b⁶**), β-D-(NCH₃)-LNA (**b⁷**), β-D-(ONH)-LNA (**b⁸**) and β-D-(ONCH₃)-LNA (**b⁹**) are preferred. Experiments have been shown that all of these LNA units b¹, b², b⁴, b⁵, b⁶, b⁷, b⁸, and b⁹ can be synthesized with the required effort and lead to antisense-oligonucleotides of comparable stability and activity. However based on the expermients the LNA units b¹, b², b⁴, b⁵, b⁶, and b⁷ are further preferred. Still further preferred are the LNA units b¹, b², b⁴, b⁶, and b⁷, and even more preferred are the LNA units b¹ and b⁴ and most preferred also in regard to the complexity of the chemical synthesis is the β-D-oxy-LNA (**b¹**).

So far no special 3' terminal group or 5' terminal group could be found which remarkably had changed or increased the stability or activity for oncological or neurological indications, so that 3' and 5' end groups are possible but not explicitly preferred.

Various internucleotide bridges or internucleotide linkages are possible. In the formulae disclosed herein the internucleotide linkage IL is represented by ―IL'―Y―. Thus, IL = ―IL'―Y― = ―X"―P(=X')(X⁻)―Y―, wherein IL is preferably selected form the group consisting of: ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, ―O―P(O)(CH₃)―O―, ―O―P(O)(OCH₃)―O―, ―O―P(O)(NH(CH₃))―O―, ―O―P(O)[N(CH₃)₂]―O―, ―O―P(O)(BH₃⁻)―O―, ―O―P(O)(OCH₂CH₂OCH₃)―O―, ―O―P(O)(OCH₂CH₂SCH₃)―O―, ―O―P(O)(O⁻)―N(CH₃)―, ―N(CH₃)―P(O)(O⁻)―O―. Preferred are the internucleotide linkages IL selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, ―O―P(O)(OCH₃)―O―, ―O―P(O)(NH(CH₃))―O―, ―O―P(O)[N(CH₃)₂]―O―, ―O―P(O)(OCH₂CH₂OCH₃)―O―, and more preferred selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, and still more preferred selected from ―O―P(O)(O⁻)―O―, ―O―P(O)(S⁻)―O―, ―O―P(S)(S⁻)―O―, and most preferably selected from ―O―P(O)(O⁻)―O― and ―O―P(O)(S⁻)―O―.

Thus, the present invention preferably relates to an antisense-oligonucleotide in form of a gapmer consisting of 10 to 28 nucleotides, preferably 10 to 18 nucleotides, more preferably 10 to 16, and still more preferably 12 to 16 or 12 to 14 nucleotides and 1 to 5 of these nucleotides at the 5' terminal end and 1 to 5 nucleotides at the 3' terminal end of the antisense-oligonucleotide are LNA nucleotides and between the LNA nucleotides at the 5' terminal end and the 3' terminal end a sequence of at least 6, preferably 7 and more preferably 8 DNA nucleotides is present, and the antisense-oligonucleotides is capable of hybridizing with a region of the gene encoding *Efnb2,* or with a region of the mRNA encoding *Efnb2,* wherein the antisense-oligonucleotide is represented by the following sequence 5'-N³- **AGCAAGGC** ―N⁴-3' (Seq. ID No. 64) or 5'-N^{3A}- **AAGCAAGG** - N^{4A}-3' (Seq. ID No. 65) or 5'-N^{3B}-**GCAAGGCA**―N^{4B}-3' (Seq. ID No. 66), wherein the residues N³, N⁴, N^{3A}, N^{4A}, N^{3B} and N^{4B} have the meanings especially the further limited meanings as disclosed herein, and
the LNA nucleotides are selected from β-D-oxy-LNA (b¹), β-D-thio-LNA (**b²**), α-L-oxy-LNA (**b⁴**), β-D-ENA (**b⁵**), β-D-(NH)-LNA (**b⁶**), β-D-(NCH₃)-LNA (**b⁷**), β-D-(ONH)-LNA (**b⁸**) and β-D-(ONCH₃)-LNA (**b⁹**); and preferably from β-D-oxy-LNA (**b¹**), β-D-thio-LNA (**b²**), α-L-oxy-LNA (**b⁴**), β-D-(NH)-LNA (**b⁶**), and β-D-(NCH₃)-LNA (**b⁷**); and
the internucleotide linkages are selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, ―O―P(O)(CH₃)―O―, ―O―P(O)(OCH₃)―O―, ―O―P(O)(NH(CH₃))―O―, ―O―P(O)[N(CH₃)₂]―O―, ―O―P(O)(BH₃⁻)―O―, ―O―P(O)(OCH₂CH₂OCH₃)―O―, ―O―P(O)(OCH₂CH₂SCH₃)―O―, ―O―P(O)(O⁻)―N(CH₃)―, ―N(CH₃)―P(O)(O⁻)―O―;
and preferably from ―O―P(O)(O⁻)―O―, ―O―P(O)(S⁻)―O―, ―O―P(S)(S⁻)―O―_{,} ―S―P(O)(O⁻)―O―, ―S―P(O)(S⁻)―O―, ―O―P(O)(O⁻)―S―, ―O―P(O)(S⁻)―S―, ―S―P(O)(O⁻)―S―; and salts and optical isomers of said antisense-oligonucleotide. Such preferred antisense-oligonucleotides may not contain any modified 3' and 5' terminal end or may not contain any 3' and 5' terminal group and may as modified nucleobase contain 5-methylcytosine and/or 2-aminoadenine, and

Still further preferred, the present invention relates to an antisense-oligonucleotide in form of a gapmer consisting of 10 to 28 nucleotides, preferably 10 to 18 nucleotides, more preferably 10 to 16, and still more preferably 12 to 16 or 12 to 14 nucleotides and 1 to 5 of these nucleotides at the 5' terminal end and 1 to 5 nucleotides at the 3' terminal end of the antisense-oligonucleotide are LNA nucleotides and between the LNA nucleotides at the 5' terminal end and the 3' terminal end a sequence of at least 6, preferably 7 and more preferably 8 DNA nucleotides is present, and the antisense-oligonucleotides is capable of hybridizing with a region of the gene encoding *Efnb2,* or with a region of the mRNA encoding *Efnb2,* wherein the antisense-oligonucleotide is represented by the following sequence 5'-N³- **AGCAAGGC** ―N⁴-3' (Seq. ID No. 64) or 5'-N^{3A}- **AAGCAAGG** - N^{4A}-3' (Seq. ID No. 65) or 5'-N^{3B}-**GCAAGGCA**―N^{4B}-3' (Seq. ID No. 66), wherein the residues N³, N⁴, N^{3A}, N^{4A}, N^{3B} and N^{4B} have the meanings especially the further limited meanings as disclosed herein, and and the LNA nucleotides are selected from β-D-oxy-LNA (**b¹**), β-D-thio-LNA (**b²**), α-L-oxy-LNA (**b⁴**), β-D-(NH)-LNA (**b⁶**), and β-D-(NCH₃)-LNA (**b⁷**); and
the internucleotide linkages are selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―; and
preferably selected from phosphate, phosphorothioate and phosphorodithioate; and salts and optical isomers of the antisense-oligonucleotide.

Especially preferred are the gapmer antisense-oligonucleotides of Table 6 or Table 7 or Tables 8 - 10 containing a segment of 2 to 5, preferably 2 to 4 and more preferably 2 to 3 LNA units at the 3' terminus and a segment of 2 to 5, preferably 2 to 4 and more preferably 2 to 3 LNA units at the 5' terminus and a segment of at least 6, preferably 7 and more preferably 8 DNA units between the two segments of LNA units, wherein the LNA units are selected from β-D-oxy-LNA (**b¹**), β-D-thio-LNA (**b²**), α-L-oxy-LNA (**b⁴**), β-D-(NH)-LNA (**b⁶**), and β-D-(NCH₃)-LNA (**b⁷**) and the internucleotide linkages are selected from phosphate, phosphorothioate and phosphorodithioate. Such preferred antisense-oligonucleotides may not contain any modified 3' and 5' terminal end or may not contain any 3' and 5' terminal group and may as modified nucleobase contain 5-methylcytosine in the LNA units, preferably all the LNA units and/or 2-aminoadenine in some or all DNA units and/or 5-methylcytosine in some or all DNA units.

Preferred are the following antisense-oligonucleotides (Table 11) consisting of 10 to 16 nucleotides and preferably comprise a sequence of at least 10 consecutive nucleotides that corresponds to a sequence of 10 consecutive nucleotides of the sequence ACATTGCAAAATTCAG (Seq. ID No. 6):

| **Seq ID No.** | **L** | **Sequence, 5'-3'** | **Seq ID No.** | **L** | **Sequence, 5'-3'** |
|---|---|---|---|---|---|
| 67 | 10 | ACATTGCAAA | 81 | 11 | ACATTGCAAAA |
| 68 | 10 | CATTGCAAAA | 82 | 11 | CATTGCAAAAT |
| 69 | 10 | ATTGCAAAAT | 83 | 11 | ATTGCAAAATT |
| 70 | 10 | TTGCAAAATT | 84 | 11 | TTGCAAAATTC |
| 71 | 10 | TGCAAAATTC | 85 | 11 | TGCAAAATTCA |
| 72 | 10 | GCAAAATTCA | 86 | 11 | GCAAAATTCAG |
| 73 | 10 | CAAAATTCAG | 87 | 13 | ACATTGCAAAATT |
| 74 | 12 | TGCAAAATTCAG | 88 | 13 | CATTGCAAAATTC |
| 75 | 12 | ACATTGCAAAAT | 89 | 13 | ATTGCAAAATTCA |
| 76 | 12 | CATTGCAAAATT | 90 | 13 | TTGCAAAATTCAG |

| | | | | | |
|---|---|---|---|---|---|
| 77 | 12 | ATTGCAAAATTC | 91 | 14 | ACATTGCAAAATTC |
| 78 | 12 | TTGCAAAATTCA | 92 | 14 | CATTGCAAAATTCA |
| 79 | 15 | ACATTGCAAAATTCA | 93 | 14 | ATTGCAAAATTCAG |
| 80 | 15 | CATTGCAAAATTCAG | 6 | 16 | ACATTGCAAAATTCAG |

More preferred are the following antisense-oligonucleotides (Table 12) consisting of 10 to 16 nucleotides and preferably comprise a sequence of at least 10 consecutive nucleotides that corresponds to a sequence of 10 consecutive nucleotides of the sequence ACATTGCAAAATTCAG (Seq. ID No. 6):

| **Seq ID No.** | **L** | **Sequence, 5'-3'** | **Seq ID No.** | **L** | **Sequence, 5'-3'** |
|---|---|---|---|---|---|
| 71 | 10 | TGCAAAATTC | 84 | 11 | TTGCAAAATTC |
| 72 | 10 | GCAAAATTCA | 85 | 11 | TGCAAAATTCA |
| 73 | 10 | CAAAATTCAG | 86 | 11 | GCAAAATTCAG |
| 74 | 12 | TGCAAAATTCAG | 88 | 13 | CATTGCAAAATTC |
| 77 | 12 | ATTGCAAAATTC | 89 | 13 | ATTGCAAAATTCA |
| 78 | 12 | TTGCAAAATTCA | 90 | 13 | TTGCAAAATTCAG |
| 79 | 15 | ACATTGCAAAATTCA | 91 | 14 | ACATTGCAAAATTC |
| 80 | 15 | CATTGCAAAATTCAG | 92 | 14 | CATTGCAAAATTCA |
| 6 | 16 | ACATTGCAAAATTCAG | 93 | 14 | ATTGCAAAATTCAG |

Preferred are also antisense-oligonucleotides of the general formula (S3) represented by the following sequence:
5'-N⁵-**GCAAAATT**-N⁶-3' (Seq. ID No. 94) wherein
N⁵ represents: AGCTGTAGCTAAATACATT-, GCTGTAGCTAAATACATT-, CTGTAGCTAAATACATT-, TGTAGCTAAATACATT-, GTAGCTAAATACATT-, TAGCTAAATACATT-, AGCTAAATACAT-, GCTAAATACATT-, GCTAAATACATT-, CTAAATACATT-, TAAATACATT-, AAATACAT-, AATACAT-, ATACATT-, TACATT-, ACATT-, CATT-, ATT-, TT- or T-; and
N⁶ represents: -CAGATTTTATACAAAACAT, -CAGATTTTATACAAAACA, -CAGATTTTATACAAAAC, -CAGATTTTATACAAAA, -CAGATTTTATACAAA, -CAGATTTTATACAA, -CAGATTTTATACA, -CAGATTTTATAC, -CAGATTTTATA, -CAGATTTTAT, -CAGATTTTA, -CAGATTTT, -CAGATTT, -CAGATT, -CAGAT, -CAGA, -CAG, -CA, or -C.

Preferably the antisense-oligonucleotide of general formula (S3) has between 10 and 28 nucleotides and at least one LNA nucleotide at the 3' terminus and at least one LNA nucleotide at the 5' terminus. Preferred are also antisense-oligonucleotides having between 10 and 28 nucleotides and two LNA nucleotides at the 3' terminus and two LNA nucleotides at the 5' terminus. As LNA nucleotides (LNA units) especially these disclosed in the chapter "Locked Nucleic Acids (LNA^{®})" and preferably in the chapter "Preferred LNAs" are suitable and as internucleotides bridges especially these disclosed in the chapter "Internucleotide Linkages (IL)" are suitable.

More preferably the antisense-oligonucleotide has between 11 and 24 nucleotides and at least two LNA nucleotides at the 3' terminus and at least two LNA nucleotides at the 5' terminus.

Still more preferably the antisense-oligonucleotide has between 10 and 16, more preferably between 12 and 14 and still more preferable 12 nucleotides and between 1 and 5, preferably 2 and 4 and more preferably between 2 and 3 LNA units at the 3' terminal end and between 1 and 5, preferably 2 and 4 and more preferably between 2 and 3 LNA units at the 5' terminal end. Preferably the antisense-oligonucleotides are GapmeRs of the form LNA segment A - DNA segment - LNA segment B. Preferably the antisense-oligonucleotides contain at least 6, more preferably at least 7 and most preferably at least 8 non-LNA units such as DNA units in between the two LNA segments. Suitable nucleobases for the non-LNA units and the LNA units are disclosed in the chapter "Nucleobases".

Thus, preferred are also antisense-oligonucleotides of general formula (S3)
5'-N⁵-**GCAAAATT**-N⁶-3' (Seq. ID No. 94) wherein
N⁵ represents: AAATACAT-, AATACAT-, ATACATT-, TACATT-, ACATT-, CATT-, ATT-, TT- or T-; and
N⁶ represents: -CAGATTTT, -CAGATTT, -CAGATT, -CAGAT, -CAGA, -CAG, -CA, or -C.

Further preferred are antisense-oligonucleotides of general formula (S3)
5'-N⁵-**GCAAAATT**-N⁶-3' (Seq. ID No. 94) wherein
N⁵ represents: CATT-, ATT-, TT- or T-; and
N⁶ represents: -CAGA, -CAG, -CA, or -C.

In preferred embodiments N⁵ represents ATT- and N⁶ represents -C. In another preferred embodiments N⁵ represents TT-and N⁶ represents -CA. In another preferred embodiments N⁵ represents T- and N⁶ represents -CAG.

Preferably, the present invention relates to antisense-oligonucleotides of the formula (S3):
5'-N⁵-**GCAAAATT**-N⁶-3' (Seq. ID No. 64) wherein
N⁵ represents T- and N⁶ represents ―CAG.

Preferred are the following antisense-oligonucleotides (Table 13):

| **Seq ID No.** | **L** | **Sequence, 5'-3'** | **Seq ID No.** | **L** | **Sequence, 5'-3'** |
|---|---|---|---|---|---|
| 71 | 10 | TGCAAAATTC | 84 | 11 | TTGCAAAATTC |
| 74 | 12 | TGCAAAATTCAG | 85 | 11 | TGCAAAATTCA |
| 77 | 12 | ATTGCAAAATTC | 88 | 13 | CATTGCAAAATTC |
| 78 | 12 | TTGCAAAATTCA | 89 | 13 | ATTGCAAAATTCA |
| 79 | 15 | ACATTGCAAAATTCA | 90 | 13 | TTGCAAAATTCAG |
| 80 | 15 | CATTGCAAAATTCAG | 91 | 14 | ACATTGCAAAATTC |
| 6 | 16 | ACATTGCAAAATTCAG | 92 | 14 | CATTGCAAAATTCA |
| | | | 93 | 14 | ATTGCAAAATTCAG |

Preferred are also antisense-oligonucleotides of the general formula (S3A) represented by the following sequence:
5'-N^{5A}-**CAAAATTC** -N^{6A}-3' (Seq. ID No. 95) wherein
N^{5A} represents: GCTGTAGCTAAATACATTG-, CTGTAGCTAAATACATTG-, TGTAGCTAAATACATTG-, GTAGCTAAATACATTG-, TAGCTAAATACATTG-, AGCTAAATACATG-, GCTAAATACATTG-, GCTAAATACATTG-, CTAAATACATTG-, TAAATACATTG-, AAATACATG-, AATACATG-, ATACATTG-, TACATTG-, ACATTG-, CATTG-, ATTG-, TTG-, TG-, or G-; and
N^{6A} represents: ―AGATTTTATACAAAACATC, ―AGATTTTATACAAAACAT, ―AGATTTTATACAAAACA, ―AGATTTTATACAAAAC, ―AGATTTTATACAAAA, -AGATTTTATACAAA, -AGATTTTATACAA, -AGATTTTATACA, -AGATTTTATAC, -AGATTTTATA, -AGATTTTAT, -AGATTTTA, -AGATTTT, -AGATTT, -AGATT, -AGAT, -AGA, -AG, or -A.

Preferably the antisense-oligonucleotide of general formula (S3A) has between 10 and 28 nucleotides and at least one LNA nucleotide at the 3' terminus and at least one LNA nucleotide at the 5' terminus. Preferred are also antisense-oligonucleotides having between 10 and 28 nucleotides and two LNA nucleotides at the 3' terminus and two LNA nucleotides at the 5' terminus. As LNA nucleotides (LNA units) especially these disclosed in the chapter "Locked Nucleic Acids (LNA^{®})" and preferably in the chapter "Preferred LNAs" are suitable and as internucleotides bridges especially these disclosed in the chapter "Internucleotide Linkages (IL)" are suitable.

More preferably the antisense-oligonucleotide has between 11 and 24 nucleotides and at least two LNA nucleotides at the 3' terminus and at least two LNA nucleotides at the 5' terminus.

Still more preferably the antisense-oligonucleotide has between 10 and 16, more preferably between 12 and 14 and still more preferable 12 nucleotides and between 1 and 5, preferably 2 and 4 and more preferably between 2 and 3 LNA units at the 3' terminal end and between 1 and 5, preferably 2 and 4 and more preferably between 2 and 3 LNA units at the 5' terminal end. Preferably the antisense-oligonucleotides are GapmeRs of the form LNA segment A - DNA segment - LNA segment B. Preferably the antisense-oligonucleotides contain at least 6, more preferably at least 7 and most preferably at least 8 non-LNA units such as DNA units in between the two LNA segments. Suitable nucleobases for the non-LNA units and the LNA units are disclosed in the chapter "Nucleobases".

Thus, preferred are also antisense-oligonucleotides of general formula (S3A)
5'-N^{5A}-**CAAAATTC** -N^{6A}-3' (Seq. ID No. 95) wherein
N^{5A} represents: AATACATG-, ATACATTG-, TACATTG-, ACATTG-, CATTG-, ATTG-, TTG-, TG-, or G-; and
N^{6A} represents: ―AGATTTTA, ―AGATTTT, ―AGATTT, ―AGATT, -AGAT, ―AGA, ―AG, or ―A.

Further preferred are antisense-oligonucleotides of general formula (S3A)
5'-N^{5A}-**CAAAATTC** -N^{6A}-3' (Seq. ID No. 95) wherein
N^{5A} represents: ATTG-, TTG-, TG-, or G-; and
N^{6A} represents: -AGAT, ―AGA, ―AG, or ―A.

In preferred embodiments N^{5A} represents TTG- and N^{6A} represents ―A. In another preferred embodiments N^{5A} represents TG- and N^{6A} represents ―AG. In another preferred embodiments N^{5A} represents G- and N^{6A} represents ―AGA.

Preferably, the present invention relates to antisense-oligonucleotides of the formula (S3A):
5'-N^{5A}-**CAAAATTC** -N^{6A}-3' (Seq. ID No. 95) Wherein N^{5A} represents TG- and N^{6A} represents ―AG.

Preferred are the following antisense-oligonucleotides (Table 14):

| **Seq ID No.** | **L** | **Sequence, 5'-3'** | **Seq ID No.** | **L** | **Sequence, 5'-3'** |
|---|---|---|---|---|---|
| 72 | 10 | GCAAAATTCA | 85 | 11 | TGCAAAATTCA |
| 74 | 12 | TGCAAAATTCAG | 86 | 11 | GCAAAATTCAG |
| 78 | 12 | TTGCAAAATTCA | 89 | 13 | ATTGCAAAATTCA |
| 79 | 15 | ACATTGCAAAATTCA | 90 | 13 | TTGCAAAATTCAG |
| 80 | 15 | CATTGCAAAATTCAG | 92 | 14 | CATTGCAAAATTCA |
| 6 | 16 | ACATTGCAAAATTCAG | 93 | 14 | ATTGCAAAATTCAG |

Preferred are also antisense-oligonucleotides of the general formula (S3B) represented by the following sequence:
5'-N^{5B}-**AAAATTCA**-N^{6B}-3' (Seq. ID No. 96) wherein
N^{5B} represents: CTGTAGCTAAATACATTGC-, TGTAGCTAAATACATTGC-, GTAGCTAAATACATTGC-, TAGCTAAATACATTGC-, AGCTAAATACATGC-, GCTAAATACATTGC-, GCTAAATACATTGC-, CTAAATACATTGC-, TAAATACATTGC-, AAATACATGC-, AATACATGC-, ATACATTGC-, TACATTGC-, ACATTGC-, CATTGC-, ATTGC-, TTGC-, TGC-, GC- or C-; and
N^{6B} represents: -GATTTTATACAAAACATCT, -GATTTTATACAAAACATC -GATTTTATACAAAACAT, -GATTTTATACAAAACA, -GATTTTATACAAAAC, -GATTTTATACAAAA, -GATTTTATACAAA, -GATTTTATACAA, -GATTTTATACA, -GATTTTATAC, -GATTTTATA, -GATTTTAT, -GATTTTA, -GATTTT, -GATTT, -GATT, -GAT, -GA, or -G.

Preferably the antisense-oligonucleotide of general formula (S3A) has between 10 and 28 nucleotides and at least one LNA nucleotide at the 3' terminus and at least one LNA nucleotide at the 5' terminus. Preferred are also antisense-oligonucleotides having between 10 and 28 nucleotides and two LNA nucleotides at the 3' terminus and two LNA nucleotides at the 5' terminus. As LNA nucleotides (LNA units) especially these disclosed in the chapter "Locked Nucleic Acids (LNA^{®})" and preferably in the chapter "Preferred LNAs" are suitable and as internucleotides bridges especially these disclosed in the chapter "Internucleotide Linkages (IL)" are suitable.

More preferably the antisense-oligonucleotide has between 11 and 24 nucleotides and at least two LNA nucleotides at the 3' terminus and at least two LNA nucleotides at the 5' terminus.

Still more preferably the antisense-oligonucleotide has between 10 and 16, more preferably between 12 and 14 and still more preferable 12 nucleotides and between 1 and 5, preferably 2 and 4 and more preferably between 2 and 3 LNA units at the 3' terminal end and between 1 and 5, preferably 2 and 4 and more preferably between 2 and 3 LNA units at the 5' terminal end. Preferably the antisense-oligonucleotides are GapmeRs of the form LNA segment A - DNA segment - LNA segment B. Preferably the antisense-oligonucleotides contain at least 6, more preferably at least 7 and most preferably at least 8 non-LNA units such as DNA units in between the two LNA segments. Suitable nucleobases for the non-LNA units and the LNA units are disclosed in the chapter "Nucleobases".

Thus, preferred are also antisense-oligonucleotides of general formula (S3B)
5'-N^{5B}-**AAAATTCA**-N^{6B}-3' (Seq. ID No. 96) wherein
N^{5B} represents: TACATTGC-, ACATTGC-, CATTGC-, ATTGC-, TTGC-, TGC-, GC- or C-; and
N^{6B} represents: ―GATTTTAT, ―GATTTTA, ―GATTTT, ―GATTT, ―GATT, ―GAT, ―GA, or ―G.

Further preferred are antisense-oligonucleotides of general formula (S3B)
5'-N^{5B}-**AAAATTCA**-N^{6B}-3' (Seq. ID No. 96) wherein
N^{5B} represents: TTGC-, TGC-, GC- or C-; and
N^{6B} represents: ―GATT, -GAT, ―GA, or ―G.

In preferred embodiments N^{5B} represents TGC- and N^{6B} represents ―G. In another preferred embodiments N^{5B} represents GC- and N^{6B} represents ―GA. In another preferred embodiments N^{5B} represents C- and N^{6B} represents ―GAT.

Preferably, the present invention relates to antisense-oligonucleotides of the formula (S3B):
5'-N^{5B}-**AAAATTCA**-N^{6B}-3' (Seq. ID No. 96) wherein N^{5B} represents: TGC- and N^{6B} represents ―G.

Preferred are the following antisense-oligonucleotides (Table 15):

| **Seq ID No.** | **L** | **Sequence, 5'-3'** | **Seq ID No.** | **L** | **Sequence, 5'-3'** |
|---|---|---|---|---|---|
| 73 | 10 | CAAAATTCAG | 86 | 11 | GCAAAATTCAG |
| 74 | 12 | TGCAAAATTCAG | 90 | 13 | TTGCAAAATTCAG |
| 80 | 15 | CATTGCAAAATTCAG | 93 | 14 | ATTGCAAAATTCAG |
| | | | 6 | 16 | ACATTGCAAAATTCAG |

The present invention preferably relates to an antisense-oligonucleotide consisting of 10 to 28 nucleotides, wherein at least two of the 10 to 28 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence 5'-N⁵-**GCAAAATT**-N⁶-3' (Seq. ID No. 94) or 5'-N^{5A}-**CAAAATTC** -N^{6A}-3' (Seq. ID No. 95) or 5'-N^{5B}-**AAAATTCA**-N^{6B}-3' (Seq. ID No. 96), wherein
N⁵ represents: AGCTGTAGCTAAATACATT-, GCTGTAGCTAAATACATT-, CTGTAGCTAAATACATT-, TGTAGCTAAATACATT-, GTAGCTAAATACATT-, TAGCTAAATACATT-, AGCTAAATACAT-, GCTAAATACATT-, GCTAAATACATT-, CTAAATACATT-, TAAATACATT-, AAATACAT-, AATACAT-, ATACATT-, TACATT-, ACATT-, CATT-, ATT-, TT- or T-;
N⁶ represents: ―CAGATTTTATACAAAACAT, ―CAGATTTTATACAAAACA, ―CAGATTTTATACAAAAC, ―CAGATTTTATACAAAA, -CAGATTTTATACAAA, ―CAGATTTTATACAA, ―CAGATTTTATACA, ―CAGATTTTATAC, -CAGATTTTATA, ―CAGATTTTAT, ―CAGATTTTA, ―CAGATTTT, ―CAGATTT, ―CAGATT, -CAGAT, ―CAGA, ―CAG, ―CA, or ―C;
N^{5A} represents: GCTGTAGCTAAATACATTG-, CTGTAGCTAAATACATTG-, TGTAGCTAAATACATTG-, GTAGCTAAATACATTG-, TAGCTAAATACATTG-, AGCTAAATACATG-, GCTAAATACATTG-, GCTAAATACATTG-, CTAAATACATTG-, TAAATACATTG-, AAATACATG-, AATACATG-, ATACATTG-, TACATTG-, ACATTG-, CATTG-, ATTG-, TTG-, TG-, or G-;
N^{6A} represents: ―AGATTTTATACAAAACATC, ―AGATTTTATACAAAACAT, ―AGATTTTATACAAAACA, ―AGATTTTATACAAAAC, ―AGATTTTATACAAAA, -AGATTTTATACAAA, ―AGATTTTATACAA, ―AGATTTTATACA, ―AGATTTTATAC, -AGATTTTATA, ―AGATTTTAT, ―AGATTTTA, ―AGATTTT, ―AGATTT, ―AGATT, -AGAT, ―AGA, ―AG, or ―A;
N^{5B} represents: CTGTAGCTAAATACATTGC-, TGTAGCTAAATACATTGC-, GTAGCTAAATACATTGC-, TAGCTAAATACATTGC-, AGCTAAATACATGC-, GCTAAATACATTGC-, GCTAAATACATTGC-, CTAAATACATTGC-, TAAATACATTGC-, AAATACATGC-, AATACATGC-, ATACATTGC-, TACATTGC-, ACATTGC-, CATTGC-, ATTGC-, TTGC-, TGC-, GC- or C-;
N^{6B} represents: ―GATTTTATACAAAACATCT, ―GATTTTATACAAAACATC ―GATTTTATACAAAACAT, ―GATTTTATACAAAACA, ―GATTTTATACAAAAC, ―GATTTTATACAAAA, -GATTTTATACAAA, ―GATTTTATACAA, ―GATTTTATACA, ―GATTTTATAC, -GATTTTATA, ―GATTTTAT, ―GATTTTA, ―GATTTT, ―GATTT, ―GATT, -GAT, ―GA, or ―G;
and salts and optical isomers of the antisense-oligonucleotide.

The present invention preferably relates to an antisense-oligonucleotide consisting of 10 to 28 nucleotides, wherein at least two of the 10 to 28 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence 5'-N⁵-**GCAAAATT**-N⁶-3' (Seq. ID No. 94) or 5'-N^{5A}-**CAAAATTC** -N^{6A}-3' (Seq. ID No. 95) or 5'-N^{5B}-**AAAATTCA**-N^{6B}-3' (Seq. ID No. 96), wherein the residues N⁵, N⁶, N^{5A}, N^{6A}, N^{5B} and N^{6B} have the meanings especially the further limited meanings as disclosed herein and salts and optical isomers of said antisense-oligonucleotide.

The present invention preferably relates to an antisense-oligonucleotide consisting of 10 to 16 nucleotides, wherein at least two of the 10 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence TGCAAAATTC (Seq. ID No. 71), GCAAAATTCA (Seq. ID No. 72), CAAAATTCAG (Seq. ID No. 73), TGCAAAATTCAG (Seq. ID No. 74), ATTGCAAAATTC (Seq. ID No. 77), TTGCAAAATTCA (Seq. ID No. 78), ACATTGCAAAATTCA (Seq. ID No. 79), CATTGCAAAATTCAG (Seq. ID No. 80), ACATTGCAAAATTCAG (Seq. ID No. 6), TTGCAAAATTC (Seq. ID No. 84), TGCAAAATTCA (Seq. ID No. 85), GCAAAATTCAG (Seq. ID No. 86), CATTGCAAAATTC (Seq. ID No. 88), ATTGCAAAATTCA (Seq. ID No. 89), TTGCAAAATTCAG (Seq. ID No. 90), ACATTGCAAAATTC (Seq. ID No. 91), CATTGCAAAATTCA (Seq. ID No. 92), ATTGCAAAATTCAG (Seq. ID No. 93), and salts and optical isomers of said antisense-oligonucleotide.

More preferably, the present invention relates to an antisense-oligonucleotide consisting of 10 to 16 nucleotides, wherein at least two of the 10 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence TGCAAAATTC (Seq. ID No. 71), GCAAAATTCA (Seq. ID No. 72), CAAAATTCAG (Seq. ID No. 73), TGCAAAATTCAG (Seq. ID No. 74), CATTGCAAAATTCAG (Seq. ID No. 80), ACATTGCAAAATTCAG (Seq. ID No. 6), TGCAAAATTCA (Seq. ID No. 85), GCAAAATTCAG (Seq. ID No. 86), TTGCAAAATTCAG (Seq. ID No. 90), ATTGCAAAATTCAG (Seq. ID No. 93), and salts and optical isomers of said antisense-oligonucleotide.

More preferably, the present invention relates to an antisense-oligonucleotide consisting of 12 to 16 nucleotides, wherein at least two of the 12 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence TGCAAAATTCAG (Seq. ID No. 74), ATTGCAAAATTC (Seq. ID No. 77), TTGCAAAATTCA (Seq. ID No. 78), ACATTGCAAAATTCA (Seq. ID No. 79), CATTGCAAAATTCAG (Seq. ID No. 80), ACATTGCAAAATTCAG (Seq. ID No. 6), CATTGCAAAATTC (Seq. ID No. 88), ATTGCAAAATTCA (Seq. ID No. 89), TTGCAAAATTCAG (Seq. ID No. 90), ACATTGCAAAATTC (Seq. ID No. 91), CATTGCAAAATTCA (Seq. ID No. 92), ATTGCAAAATTCAG (Seq. ID No. 93), and salts and optical isomers of said antisense-oligonucleotide.

Still more preferably, the present invention relates to an antisense-oligonucleotide consisting of 12 to 16 nucleotides, wherein at least two of the 12 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence TGCAAAATTCAG (Seq. ID No. 74), CATTGCAAAATTCAG (Seq. ID No. 80), ACATTGCAAAATTCAG (Seq. ID No. 6), TTGCAAAATTCAG (Seq. ID No. 90), ATTGCAAAATTCAG (Seq. ID No. 93), and salts and optical isomers of said antisense-oligonucleotide.

More preferably, the present invention relates to an antisense-oligonucleotide consisting of 12 to 14 nucleotides, wherein at least two of the 12 to 14 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence TGCAAAATTCAG (Seq. ID No. 74), ATTGCAAAATTC (Seq. ID No. 77), TTGCAAAATTCA (Seq. ID No. 78), CATTGCAAAATTC (Seq. ID No. 88), ATTGCAAAATTCA (Seq. ID No. 89), TTGCAAAATTCAG (Seq. ID No. 90), ACATTGCAAAATTC (Seq. ID No. 91), CATTGCAAAATTCA (Seq. ID No. 92), ATTGCAAAATTCAG (Seq. ID No. 93), and salts and optical isomers of said antisense-oligonucleotide.

Still more preferably, the present invention relates to an antisense-oligonucleotide consisting of 12 to 14 nucleotides, wherein at least two of the 12 to 14 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence TGCAAAATTCAG (Seq. ID No. 74), TTGCAAAATTCAG (Seq. ID No. 90), ATTGCAAAATTCAG (Seq. ID No. 93), and salts and optical isomers of said antisense-oligonucleotide.

The antisense-oligonucleotides of formula **S3, S3A, S3B** in form of gapmers (LNA segment 1 - DNA segment - LNA segment 2) contain an LNA segment at the 5' terminal end consisting of 2 to 5, preferably 2 to 4 LNA units and contain an LNA segment at the 3' terminal end consisting of 2 to 5, preferably 2 to 4 LNA units and between the two LNA segments one DNA segment consisting of 6 to 14, preferably 7 to 12 and more preferably 8 to 11 DNA units.

The antisense-oligonucleotides of formula **S3, S3A, S3B** contain the LNA nucleotides (LNA units) as disclosed herein, especially these disclosed in the chapter "Locked Nucleic Acids (LNA^{®})" and preferably these disclosed in the chapter "Preferred LNAs". The LNA units and the DNA units may comprise standard nucleobases such as adenine (A), cytosine (C), guanine (G), thymine (T) and uracil (U), but may also contain modified nucleobases as disclosed in the chapter "Nucleobases". The antisense-oligonucleotides of formula **S3, S3A, S3B** or the LNA segments and the DNA segment of the antisense-oligonucleotide may contain any internucleotide linkage as disclosed herein and especially these disclosed in the chapter "Internucleotide Linkages (IL)". The antisense-oligonucleotides of formula **S3, S3A, S3B** may optionally also contain endgroups at the 3' terminal end and/or the 5' terminal end and especially these disclosed in the chapter "Terminal groups".

Experiments have shown that modified nucleobases do not considerably increase or change the activity of the inventive antisense-oligonucleotides in regard to tested neurological and oncological indications. The modified nucleobases 5-methylcytosine or 2-aminoadenine have been demonstrated to further increase the activity of the antisense-oligonucleotides of formula **S3, S3A, S3B** especially if 5-methylcytosine is used in the LNA nucleotides only or in the LNA nucleotides and in the DNA nucleotides and/or if 2-aminoadenine is used in the DNA nucleotides and not in the LNA nucleotides.

As LNA units for the antisense-oligonucleotides of formula **S3, S3A, S3B** especially β-D-oxy-LNA (**b¹**), β-D-thio-LNA (**b²**), α-L-oxy-LNA (**b4**), β-D-ENA (**b⁵**), β-D-(NH)-LNA (**b⁶**), β-D-(NCH₃)-LNA (**b⁷**), β-D-(ONH)-LNA (**b⁸**) and β-D-(ONCH₃)-LNA (**b⁹**) are preferred. Experiments have been shown that all of these LNA units b¹, b², b⁴, b⁵, b⁶, b⁷, b⁸, and b⁹ can be synthesized with the required effort and lead to antisense-oligonucleotides of comparable stability and activity. However based on the expermients the LNA units b¹, b², b⁴, b⁵, b⁶, and b⁷ are further preferred. Still further preferred are the LNA units b¹, b², b⁴, b⁶, and b⁷, and even more preferred are the LNA units b¹ and b⁴ and most preferred also in regard to the complexity of the chemical synthesis is the β-D-oxy-LNA (**b¹**).

So far no special 3' terminal group or 5' terminal group could be found which remarkably had changed or increased the stability or activity for oncological or neurological indications, so that 3' and 5' end groups are possible but not explicitly preferred.

Various internucleotide bridges or internucleotide linkages are possible. In the formulae disclosed herein the internucleotide linkage IL is represented by ―IL'―Y―. Thus, IL = ―IL'―Y― = ―X"―P(=X')(X⁻)―Y―, wherein IL is preferably selected form the group consisting of: ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, ―O―P(O)(CH₃)―O―, ―O―P(O)(OCH₃)―O―, ―O―P(O)(NH(CH₃))―O―, ―O―P(O)[N(CH₃)₂]―O―, ―O―P(O)(BH₃⁻)―O―, ―O―P(O)(OCH₂CH₂OCH₃)―O―, ―O―P(O)(OCH₂CH₂SCH₃)―O―, ―O―P(O)(O⁻)-N(CH₃)―, ―N(CH₃)―P(O)(O⁻)―O―. Preferred are the internucleotide linkages IL selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, ―O―P(O)(OCH₃)―O―, ―O―P(O)(NH(CH₃))―O―, ―O―P(O)[N(CH₃)₂]―O―, ―O―P(O)(OCH₂CH₂OCH₃)―O―, and more preferred selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, and still more preferred selected from ―O―P(O)(O⁻)―O―, ―O―P(O)(S⁻)―O―, ―O―P(S)(S⁻)―O―, and most preferably selected from ―O―P(O)(O⁻)―O― and ―O―P(O)(S⁻)―O―.

Thus, the present invention preferably relates to an antisense-oligonucleotide in form of a gapmer consisting of 10 to 28 nucleotides, preferably 10 to 18 nucleotides, more preferably 10 to 16, and still more preferably 12 to 16 or 12 to 14 nucleotides and 1 to 5 of these nucleotides at the 5' terminal end and 1 to 5 nucleotides at the 3' terminal end of the antisense-oligonucleotide are LNA nucleotides and between the LNA nucleotides at the 5' terminal end and the 3' terminal end a sequence of at least 6, preferably 7 and more preferably 8 DNA nucleotides is present, and the antisense-oligonucleotides is capable of hybridizing with a region of the gene encoding *Efnb2,* or with a region of the mRNA encoding *Efnb2,* wherein the antisense-oligonucleotide is represented by the following sequence 5'-N⁵-**GCAAAATT**-N⁶-3' (Seq. ID No. 94) or 5'-N^{5A}-**CAAAATTC** -N^{6A}-3' (Seq. ID No. 95) or 5'-N^{5B}-**AAAATTCA**-N^{6B}-3' (Seq. ID No. 96), wherein the residues N⁵, N⁶, N^{5A}, N^{6A}, N^{5B} and N^{6B} have the meanings especially the further limited meanings as disclosed herein, and
the LNA nucleotides are selected from β-D-oxy-LNA (**b¹**), β-D-thio-LNA (**b²**), α-L-oxy-LNA (**b⁴**), β-D-ENA (**b⁵**), β-D-(NH)-LNA (**b⁶**), β-D-(NCH₃)-LNA (**b⁷**), β-D-(ONH)-LNA (**b⁸**) and β-D-(ONCH₃)-LNA (**b⁹**); and preferably from β-D-oxy-LNA (**b¹**), β-D-thio-LNA (**b²**), α-L-oxy-LNA (**b⁴**), β-D-(NH)-LNA (**b⁶**), and β-D-(NCH₃)-LNA (**b⁷**); and
the internucleotide linkages are selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―, ―O―P(O)(CH₃)―O―, ―O―P(O)(OCH₃)―O―, ―O―P(O)(NH(CH₃))―O―, ―O―P(O)[N(CH₃)₂]―O―, ―O―P(O)(BH₃⁻)―O―, ―O―P(O)(OCH₂CH₂OCH₃)―O―, ―O―P(O)(OCH₂CH₂SCH₃)―O―, ―O―P(O)(O⁻)―N(CH₃)―, ―N(CH₃)―P(O)(O⁻)―O―;
and preferably from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―; and salts and optical isomers of said antisense-oligonucleotide.
Such preferred antisense-oligonucleotides may not contain any modified 3' and 5' terminal end or may not contain any 3' and 5' terminal group and may as modified nucleobase contain 5-methylcytosine and/or 2-aminoadenine, and

Still further preferred, the present invention relates to an antisense-oligonucleotide in form of a gapmer consisting of 10 to 28 nucleotides, preferably 10 to 18 nucleotides, more preferably 10 to 16, and still more preferably 12 to 16 or 12 to 14 nucleotides and 1 to 5 of these nucleotides at the 5' terminal end and 1 to 5 nucleotides at the 3' terminal end of the antisense-oligonucleotide are LNA nucleotides and between the LNA nucleotides at the 5' terminal end and the 3' terminal end a sequence of at least 6, preferably 7 and more preferably 8 DNA nucleotides is present, and the antisense-oligonucleotides is capable of hybridizing with a region of the gene encoding *Efnb2,* or with a region of the mRNA encoding *Efnb2,* wherein the antisense-oligonucleotide is represented by the following sequence 5'-N⁵-**GCAAAATT**-N**⁶**-3' (Seq. ID No. 94) or 5'-N^{5A}-**CAAAATTC** -N^{6A}-3' (Seq. ID No. 95) or 5'-N^{5B}-**AAAATTCA**-N^{6B}-3' (Seq. ID No. 96), wherein the residues N⁵, N⁶, N^{5A}, N^{6A}, N^{5B} and N^{6B} have the meanings especially the further limited meanings as disclosed herein, and and the LNA nucleotides are selected from β-D-oxy-LNA (**b¹**), β-D-thio-LNA (**b²**), α-L-oxy-LNA (**b⁴**), β-D-(NH)-LNA (**b⁶**), and β-D-(NCH₃)-LNA (**b⁷**); and
the internucleotide linkages are selected from ―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―O―P(O)(O⁻)―S―, ―O―P(O)(S⁻)―S―, ―S―P(O)(O⁻)―S―; and
preferably selected from phosphate, phosphorothioate and phosphorodithioate;
and salts and optical isomers of the antisense-oligonucleotide.

Especially preferred are the gapmer antisense-oligonucleotides of Table 11 or Table 12 or Tables 13 - 15 containing a segment of 2 to 5, preferably 2 to 4 and more preferably 2 to 3 LNA units at the 3' terminus and a segment of 2 to 5, preferably 2 to 4 and more preferably 2 to 3 LNA units at the 5' terminus and a segment of at least 6, preferably 7 and more preferably 8 DNA units between the two segments of LNA units, wherein the LNA units are selected from β-D-oxy-LNA (**b¹**), β-D-thio-LNA (**b²**), α-L-oxy-LNA (**b⁴**), β-D-(NH)-LNA (**b⁶**), and β-D-(NCH₃)-LNA (**b⁷**) and the internucleotide linkages are selected from phosphate, phosphorothioate and phosphorodithioate. Such preferred antisense-oligonucleotides may not contain any modified 3' and 5' terminal end or may not contain any 3' and 5' terminal group and may as modified nucleobase contain 5-methylcytosine in the LNA units, preferably all the LNA units and/or 2-aminoadenine in some or all DNA units and/or 5-methylcytosine in some or all DNA units.

Preferred are the following antisense-oligonucleotides (Table 16):

| **Seq ID No.** | **L** | **Sequence, 5'-3'** | **Seq ID No.** | **L** | **Sequence, 5'-3'** |
|---|---|---|---|---|---|
| 8 | 10 | AGGTTAGGGC | 33 | 11 | AGGTT AGGGCT |
| 9 | 10 | GGTTAGGGCT | 21 | 11 | GGTT AGGGCTG |
| 10 | 10 | GTTAGGGCTG | 22 | 11 | GTT AGGGCTGA |
| 11 | 10 | TTAGGGCTGA | 23 | 11 | TTAGGGCTGAA |
| 12 | 10 | TAGGGCTGAA | 24 | 11 | TAGGGCTGAAT |
| 13 | 10 | AGGGCTGAAT | 25 | 11 | AGGGCTGAA TT |
| 14 | 10 | GGGCTGAATT | 26 | 13 | AGGTT AGGGCTGA |
| 15 | 12 | AGGTTAGGGCTG | 27 | 13 | GGTT AGGGCTGAA |
| 16 | 12 | GGTTAGGGCTGA | 28 | 13 | GTT AGGGCTGAA T |
| 17 | 12 | GTTAGGGCTGAA | 29 | 13 | TTAGGGCTGAATT |
| 18 | 12 | TTAGGGCTGAAT | 30 | 14 | AGGTT AGGGCTGAA |
| 36 | 12 | TAGGGCTGAATT | 31 | 14 | GGTT AGGGCTGAA T |
| 19 | 15 | AGGTTAGGGCTGAAT | 32 | 14 | GTT AGGGCTGAA TT |
| 20 | 15 | GGTTAGGGCTGAATT | 4 | 16 | AGGTTAGGGCTGAATT |
| 37 | 10 | TACAAGCAAG | 51 | 11 | TACAAGCAAGG |
| 38 | 10 | ACAAGCAAGG | 52 | 11 | ACAAGCAAGGC |
| 39 | 10 | CAAGCAAGGC | 53 | 11 | CAAGCAAGGCA |

| | | | | | |
|---|---|---|---|---|---|
| 40 | 10 | AAGCAAGGCA | 54 | 11 | AAGCAAGGCAT |
| 41 | 10 | AGCAAGGCAT | 55 | 11 | AGCAAGGCATT |
| 42 | 10 | GCAAGGCATT | 56 | 11 | GCAAGGCATTT |
| 43 | 10 | CAAGGCATTT | 57 | 13 | TACAAGCAAGGCA |
| 44 | 12 | TACAAGCAAGGC | 58 | 13 | ACAAGCAAGGCAT |
| 45 | 12 | ACAAGCAAGGCA | 59 | 13 | CAAGCAAGGCATT |
| 46 | 12 | CAAGCAAGGCAT | 60 | 13 | AAGCAAGGCATTT |
| 47 | 12 | AAGCAAGGCATT | 61 | 14 | TACAAGCAAGGCAT |
| 48 | 12 | AGCAAGGCATTT | 62 | 14 | ACAAGCAAGGCATT |
| 49 | 15 | TACAAGCAAGGCATT | 63 | 14 | CAAGCAAGGCATTT |
| 50 | 15 | ACAAGCAAGGCATTT | 5 | 16 | TACAAGCAAGGCATTT |
| 67 | 10 | ACATTGCAAA | 81 | 11 | ACATTGCAAAA |
| 68 | 10 | CATTGCAAAA | 82 | 11 | CATTGCAAAAT |
| 69 | 10 | ATTGCAAAAT | 83 | 11 | ATTGCAAAATT |
| 70 | 10 | TTGCAAAATT | 84 | 11 | TTGCAAAATTC |
| 71 | 10 | TGCAAAATTC | 85 | 11 | TGCAAAATTCA |
| 72 | 10 | GCAAAATTCA | 86 | 11 | GCAAAATTCAG |
| 73 | 10 | CAAAATTCAG | 87 | 13 | ACATTGCAAAATT |
| 74 | 12 | TGCAAAATTCAG | 88 | 13 | CATTGCAAAATTC |
| 75 | 12 | ACATTGCAAAAT | 89 | 13 | ATTGCAAAATTCA |
| 76 | 12 | CATTGCAAAATT | 90 | 13 | TTGCAAAATTCAG |
| 77 | 12 | ATTGCAAAATTC | 91 | 14 | ACATTGCAAAATTC |
| 78 | 12 | TTGCAAAATTCA | 92 | 14 | CATTGCAAAATTCA |
| 79 | 15 | ACATTGCAAAATTCA | 93 | 14 | ATTGCAAAATTCAG |
| 80 | 15 | CATTGCAAAATTCAG | 6 | 16 | ACATTGCAAAATTCAG |

More preferred are the following antisense-oligonucleotides (Table 17):

| **Seq ID No.** | **L** | **Sequence, 5'-3'** | **Seq ID No.** | **L** | **Sequence, 5'-3'** |
|---|---|---|---|---|---|
| 9 | 10 | GGTTAGGGCT | 22 | 11 | GTTAGGGCTGA |
| 10 | 10 | GTTAGGGCTG | 23 | 11 | TTAGGGCTGAA |
| 11 | 10 | TTAGGGCTGA | 26 | 13 | AGGTTAGGGCTGA |
| 15 | 12 | AGGTTAGGGCTG | 27 | 13 | GGTTAGGGCTGAA |
| 16 | 12 | GGTTAGGGCTGA | 28 | 13 | GTTAGGGCTGAAT |
| 17 | 12 | GTTAGGGCTGAA | 29 | 13 | TTAGGGCTGAATT |
| 18 | 12 | TTAGGGCTGAAT | 30 | 14 | AGGTTAGGGCTGAA |
| 19 | 15 | AGGTT AGGGCTGAA T | 31 | 14 | GGTT AGGGCTGAA T |
| 20 | 15 | GGTT AGGGCTGAA TT | 32 | 14 | GTT AGGGCTGAA TT |
| 21 | 11 | GGTT AGGGCTG | 4 | 16 | AGGTTAGGGCTGAATT |
| 39 | 10 | CAAGCAAGGC | 52 | 11 | ACAAGCAAGGC |
| 40 | 10 | AAGCAAGGCA | 53 | 11 | CAAGCAAGGCA |
| 41 | 10 | AGCAAGGCAT | 54 | 11 | AAGCAAGGCAT |
| 44 | 12 | TACAAGCAAGGC | 55 | 11 | AGCAAGGCA TT |
| 45 | 12 | ACAAGCAAGGCA | 57 | 13 | TACAAGCAAGGCA |
| 46 | 12 | CAAGCAAGGCAT | 58 | 13 | ACAAGCAAGGCAT |
| 47 | 12 | AAGCAAGGCA TT | 59 | 13 | CAAGCAAGGCA TT |
| 48 | 12 | AGCAAGGCA TTT | 60 | 13 | AAGCAAGGCA TTT |
| 49 | 15 | TACAAGCAAGGCATT | 61 | 14 | TACAAGCAAGGCAT |
| 50 | 15 | ACAAGCAAGGCA TTT | 62 | 14 | ACAAGCAAGGCA TT |
| 5 | 16 | TACAAGCAAGGCATTT | 63 | 14 | CAAGCAAGGCA TTT |
| 71 | 10 | TGCAAAATTC | 84 | 11 | TTGCAAAATTC |
| 72 | 10 | GCAAAATTCA | 85 | 11 | TGCAAAATTCA |
| 73 | 10 | CAAAATTCAG | 86 | 11 | GCAAAATTCAG |
| 74 | 12 | TGCAAAATTCAG | 88 | 13 | CATTGCAAAATTC |
| 77 | 12 | ATTGCAAAATTC | 89 | 13 | ATTGCAAAATTCA |
| 78 | 12 | TTGCAAAATTCA | 90 | 13 | TTGCAAAATTCAG |
| 79 | 15 | ACATTGCAAAATTCA | 91 | 14 | ACATTGCAAAATTC |
| 80 | 15 | CATTGCAAAATTCAG | 92 | 14 | CATTGCAAAATTCA |
| 6 | 16 | ACATTGCAAAATTCAG | 93 | 14 | ATTGCAAAATTCAG |

The antisense-oligonucleotides as disclosed herein such as the antisense-oligonucleotides of **Tables 16 and 17** consist of nucleotides, preferably DNA nucleotides, which are non-LNA units (also named herein non-LNA nucleotides) as well as LNA units (also named herein LNA nucleotides). Although not explicitly indicated, the antisense-oligonucleotides of the sequences Seq. ID No.s 4-93 of Table 16 and 17 comprise 2 to 4 LNA nucleotides (LNA units) at the 3' terminus and 2 to 4 LNA nucleotides (LNA units) at the 5' terminus.

That means, as long as not explicitly indicated, the antisense-oligonucleotides of the present invention or as disclosed herein by the letter code A, C, G, T and U may contain any internucleotide linkage, any end group and any nucleobase as disclosed herein. Moreover the antisense-oligonucleotides of the present invention or as disclosed herein are gapmers of any gapmer structure as disclosed herein with at least one LNA unit at the 3' terminus and at least one LNA unit at the 5' terminus. Moreover any LNA unit as disclosed herein can be used within the antisense-oligonucleotides of the present invention or as disclosed herein. Thus, for instance, the antisense-oligonucleotide AGGT**TAGGGCTG**AATT (Seq. ID No. 4) or TACA**AGCAAGGC**ATTT (Seq. ID No. 5) or ACATT**GCAAAATT**CAG (Seq. ID No. 6) or GG**TTAGGGCT**GA (Seq. ID No. 16) or CA**AGCAAGGC**AT (Seq. ID No. 46) or TG**CAAAATTC**AG (Seq. ID No. 74) contains at least one LNA unit at the 5' terminus and at least one LNA unit at the 3' terminus, any nucleobase, any 3' end group, any 5' end group, any gapmer structure, and any internucleotide linkage as disclosed herein and covers also salts and optical isomers of that antisense-oligonucleotide.

The use of LNA units is preferred especially at the 3' terminal and the 5' terminal end. Thus it is preferred if the last 1 - 5 nucleotides at the 3' terminal end and also the last 1 - 5 nucleotides at the 5' terminal end especially of the sequences disclosed herein and particularly of Seq. ID No.s 4 - 93 of Table 16 and 17 are LNA units (also named LNA nucleotides) while in between the 1 - 5 LNA units at the 3' and 5' end 2 - 14, preferably 3 ― 12, more preferably 4 - 10, more preferably 5 ― 9, still more preferably 6 ― 8, non-LNA units (also named non-LNA nucleotides) are present. Such kinds of antisense-oligonucleotides are called gapmers and are disclosed in more detail below. More preferred are 2 ― 5 LNA nucleotides at the 3' end and 2 ― 5 LNA nucleotides at the 5' end or 1 - 4 LNA nucleotides at the 3' end and 1 - 4 LNA nucleotides at the 5' end and still more preferred are 2 ― 4 LNA nucleotides at the 3' end and 2 ― 4 LNA nucleotides at the 5' end of the antisense-oligonucleotides with a number of preferably 4 - 10, more preferably 5 ― 9, still more preferably 6 ― 8 non-LNA units in between the LNA units at the 3' and the 5' end.

Moreover as internucleotide linkages between the LNA units and between the LNA units and the non-LNA units, the use of phosphorothioates or phosphorodithioates and preferably phosphorothioates is preferred.

Thus further preferred are antisense-oligonucleotides wherein more than 50%, preferably more than 60%, more preferably more than 70%, still more preferably more than 80%, and most preferably more than 90% of the internucleotide linkages are phosphorothioates or phosphates and more preferably phosphorothioate linkages and wherein the last 1 - 4 or 2 - 5 nucleotides at the 3' end are LNA units and the last 1 - 4 or 2 - 5 nucleotides at the 5' end are LNA units and between the LNA units at the ends a sequence of 6 - 14 nucleotides, preferably 7 - 12, preferably 8 - 11, more preferably 8 ― 10 are present which are non-LNA units, preferably DNA units. Moreover it is preferred that these antisense-oligonucleotides in form of gapmers consist in total of 12 to 20, preferably 12 to 18 nucleotides, more preferably 12 to 16 nucleotides, more preferably 12 to 14 nucleotides.

The present invention therefore preferably relates to an antisense-oligonucleotide consisting of 10 to 16 nucleotides, wherein at least two of the 10 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence GGTTAGGGCT(Seq. ID No. 9), GTTAGGGCTG (Seq. ID No. 10), TTAGGGCTGA (Seq. ID No. 11), AGGTTAGGGCTG (Seq. ID No. 15), GGTTAGGGCTGA (Seq. ID No. 16), GTTAGGGCTGAA (Seq. ID No. 17), TTAGGGCTGAAT (Seq. ID No. 18), AGGTTAGGGCTGAAT (Seq. ID No. 19), GGTTAGGGCTGAATT (Seq. ID No. 20), GGTTAGGGCTG (Seq. ID No. 21), GTTAGGGCTGA (Seq. ID No. 22), TTAGGGCTGAA (Seq. ID No. 23), AGGTTAGGGCTGA (Seq. ID No. 26), GGTTAGGGCTGAA (Seq. ID No. 27), GTTAGGGCTGAAT (Seq. ID No. 28), TTAGGGCTGAATT (Seq. ID No. 29), AGGTTAGGGCTGAA (Seq. ID No. 30), GGTTAGGGCTGAAT (Seq. ID No. 31), GTTAGGGCTGAATT (Seq. ID No. 32), AGGTTAGGGCTGAATT (Seq. ID No. 4), CAAGCAAGGC (Seq. ID No. 39), AAGCAAGGCA (Seq. ID No. 40), AGCAAGGCAT (Seq. ID No. 41), TACAAGCAAGGC (Seq. ID No. 44), ACAAGCAAGGCA (Seq. ID No. 45), CAAGCAAGGCAT (Seq. ID No. 46), AAGCAAGGCATT (Seq. ID No. 47), AGCAAGGCATTT (Seq. ID No. 48), TACAAGCAAGGCATT (Seq. ID No. 49), ACAAGCAAGGCATTT (Seq. ID No. 50), ACAAGCAAGGC (Seq. ID No. 52), CAAGCAAGGCA (Seq. ID No. 53), AAGCAAGGCAT (Seq. ID No. 54), AGCAAGGCATT (Seq. ID No. 55), TACAAGCAAGGCA (Seq. ID No. 57), ACAAGCAAGGCAT (Seq. ID No. 58), CAAGCAAGGCATT (Seq. ID No. 59), AAGCAAGGCATTT (Seq. ID No. 60), TACAAGCAAGGCAT (Seq. ID No. 61), ACAAGCAAGGCATT (Seq. ID No. 62) CAAGCAAGGCATTT (Seq. ID No. 63) TACAAGCAAGGCATTT (Seq. ID No. 5), TGCAAAATTC (Seq. ID No. 71), GCAAAATTCA (Seq. ID No. 72), CAAAATTCAG (Seq. ID No. 73), TGCAAAATTCAG (Seq. ID No. 74), ATTGCAAAATTC (Seq. ID No. 77), TTGCAAAATTCA (Seq. ID No. 78), ACATTGCAAAATTCA (Seq. ID No. 79), CATTGCAAAATTCAG (Seq. ID No. 80), ACATTGCAAAATTCAG (Seq. ID No. 6), TTGCAAAATTC (Seq. ID No. 84), TGCAAAATTCA (Seq. ID No. 85), GCAAAATTCAG (Seq. ID No. 86), CATTGCAAAATTC (Seq. ID No. 88), ATTGCAAAATTCA (Seq. ID No. 89), TTGCAAAATTCAG (Seq. ID No. 90), ACATTGCAAAATTC (Seq. ID No. 91), CATTGCAAAATTCA (Seq. ID No. 92), ATTGCAAAATTCAG (Seq. ID No. 93), and salts and optical isomers of said antisense-oligonucleotide.

More preferably, the present invention relates to an antisense-oligonucleotide consisting of 10 to 16 nucleotides, wherein at least two of the 10 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence GGTTAGGGCT(Seq. ID No. 9), GTTAGGGCTG (Seq. ID No. 10), TTAGGGCTGA (Seq. ID No. 11), GGTTAGGGCTGA (Seq. ID No. 16), AGGTTAGGGCTGAAT (Seq. ID No. 19), GGTTAGGGCTGAATT (Seq. ID No. 20), GGTTAGGGCTG (Seq. ID No. 21), GTTAGGGCTGA (Seq. ID No. 22), AGGTTAGGGCTGA (Seq. ID No. 26), GGTTAGGGCTGAA (Seq. ID No. 27), AGGTTAGGGCTGAA (Seq. ID No. 30), GGTTAGGGCTGAAT (Seq. ID No. 31), AGGTTAGGGCTGAATT (Seq. ID No. 4), CAAGCAAGGC (Seq. ID No. 39), AAGCAAGGCA (Seq. ID No. 40), AGCAAGGCAT (Seq. ID No. 41), CAAGCAAGGCAT (Seq. ID No. 46), TACAAGCAAGGCATT (Seq. ID No. 49), ACAAGCAAGGCATTT (Seq. ID No. 50),CAAGCAAGGCA (Seq. ID No. 53), AAGCAAGGCAT (Seq. ID No. 54), ACAAGCAAGGCAT (Seq. ID No. 58), CAAGCAAGGCATT (Seq. ID No. 59), TACAAGCAAGGCAT (Seq. ID No. 61), ACAAGCAAGGCATT (Seq. ID No. 62) CAAGCAAGGCATTT (Seq. ID No. 63) TACAAGCAAGGCATTT (Seq. ID No. 5), TGCAAAATTC (Seq. ID No. 71), GCAAAATTCA (Seq. ID No. 72), CAAAATTCAG (Seq. ID No. 73), TGCAAAATTCAG (Seq. ID No. 74), CATTGCAAAATTCAG (Seq. ID No. 80), ACATTGCAAAATTCAG (Seq. ID No. 6), TGCAAAATTCA (Seq. ID No. 85), GCAAAATTCAG (Seq. ID No. 86), TTGCAAAATTCAG (Seq. ID No. 90), ATTGCAAAATTCAG (Seq. ID No. 93), and salts and optical isomers of said antisense-oligonucleotide.

More preferably, the present invention relates to an antisense-oligonucleotide consisting of 12 to 16 nucleotides, wherein at least two of the 12 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence AGGTTAGGGCTG (Seq. ID No. 15), GGTTAGGGCTGA (Seq. ID No. 16), GTTAGGGCTGAA (Seq. ID No. 17), TTAGGGCTGAAT (Seq. ID No. 18), AGGTTAGGGCTGAAT (Seq. ID No. 19), GGTTAGGGCTGAATT (Seq. ID No. 20), AGGTTAGGGCTGA (Seq. ID No. 26), GGTTAGGGCTGAA (Seq. ID No. 27), GTTAGGGCTGAAT (Seq. ID No. 28), TTAGGGCTGAATT (Seq. ID No. 29), AGGTTAGGGCTGAA (Seq. ID No. 30), GGTTAGGGCTGAAT (Seq. ID No. 31), GTTAGGGCTGAATT (Seq. ID No. 32), AGGTTAGGGCTGAATT (Seq. ID No. 4), TACAAGCAAGGC (Seq. ID No. 44), ACAAGCAAGGCA (Seq. ID No. 45), CAAGCAAGGCAT (Seq. ID No. 46), AAGCAAGGCATT (Seq. ID No. 47), AGCAAGGCATTT (Seq. ID No. 48), TACAAGCAAGGCATT (Seq. ID No. 49), ACAAGCAAGGCATTT (Seq. ID No. 50), TACAAGCAAGGCA (Seq. ID No. 57), ACAAGCAAGGCAT (Seq. ID No. 58), CAAGCAAGGCATT (Seq. ID No. 59), AAGCAAGGCATTT (Seq. ID No. 60), TACAAGCAAGGCAT (Seq. ID No. 61), ACAAGCAAGGCATT (Seq. ID No. 62), CAAGCAAGGCATTT (Seq. ID No. 63), TACAAGCAAGGCATTT (Seq. ID No. 5), TGCAAAATTCAG (Seq. ID No. 74), ATTGCAAAATTC (Seq. ID No. 77), TTGCAAAATTCA (Seq. ID No. 78), ACATTGCAAAATTCA (Seq. ID No. 79), CATTGCAAAATTCAG (Seq. ID No. 80), ACATTGCAAAATTCAG (Seq. ID No. 6), CATTGCAAAATTC (Seq. ID No. 88), ATTGCAAAATTCA (Seq. ID No. 89), TTGCAAAATTCAG (Seq. ID No. 90), ACATTGCAAAATTC (Seq. ID No. 91), CATTGCAAAATTCA (Seq. ID No. 92), ATTGCAAAATTCAG (Seq. ID No. 93), and salts and optical isomers of said antisense-oligonucleotide.

Still more preferably, the present invention relates to an antisense-oligonucleotide consisting of 12 to 16 nucleotides, wherein at least two of the 12 to 16 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence GGTTAGGGCTGA (Seq. ID No. 16), AGGTTAGGGCTGAAT (Seq. ID No. 19), GGTTAGGGCTGAATT (Seq. ID No. 20), AGGTTAGGGCTGA (Seq. ID No. 26), GGTTAGGGCTGAA (Seq. ID No. 27), AGGTTAGGGCTGAA (Seq. ID No. 30), GGTTAGGGCTGAAT (Seq. ID No. 31), AGGTTAGGGCTGAATT (Seq. ID No. 4), CAAGCAAGGCAT (Seq. ID No. 46), TACAAGCAAGGCATT (Seq. ID No. 49), ACAAGCAAGGCATTT (Seq. ID No. 50), ACAAGCAAGGCAT (Seq. ID No. 58), CAAGCAAGGCATT (Seq. ID No. 59), TACAAGCAAGGCAT (Seq. ID No. 61), ACAAGCAAGGCATT (Seq. ID No. 62), CAAGCAAGGCATTT (Seq. ID No. 63), TACAAGCAAGGCATTT (Seq. ID No. 5), TGCAAAATTCAG (Seq. ID No. 74), CATTGCAAAATTCAG (Seq. ID No. 80), ACATTGCAAAATTCAG (Seq. ID No. 6), TTGCAAAATTCAG (Seq. ID No. 90), ATTGCAAAATTCAG (Seq. ID No. 93), and salts and optical isomers of said antisense-oligonucleotide.

More preferably, the present invention relates to an antisense-oligonucleotide consisting of 12 to 14 nucleotides, wherein at least two of the 12 to 14 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence AGGTTAGGGCTG (Seq. ID No. 15), GGTTAGGGCTGA (Seq. ID No. 16), GTTAGGGCTGAA (Seq. ID No. 17), TTAGGGCTGAAT (Seq. ID No. 18), AGGTTAGGGCTGA (Seq. ID No. 26), GGTTAGGGCTGAA (Seq. ID No. 27), GTTAGGGCTGAAT (Seq. ID No. 28), TTAGGGCTGAATT (Seq. ID No. 29), AGGTTAGGGCTGAA (Seq. ID No. 30), GGTTAGGGCTGAAT (Seq. ID No. 31), GTTAGGGCTGAATT (Seq. ID No. 32), TACAAGCAAGGC (Seq. ID No. 44), ACAAGCAAGGCA (Seq. ID No. 45), CAAGCAAGGCAT (Seq. ID No. 46), AAGCAAGGCATT (Seq. ID No. 47), AGCAAGGCATTT (Seq. ID No. 48), TACAAGCAAGGCA (Seq. ID No. 57), ACAAGCAAGGCAT (Seq. ID No. 58), CAAGCAAGGCATT (Seq. ID No. 59), AAGCAAGGCATTT (Seq. ID No. 60), TACAAGCAAGGCAT (Seq. ID No. 61), ACAAGCAAGGCATT (Seq. ID No. 62), CAAGCAAGGCATTT (Seq. ID No. 63) TGCAAAATTCAG (Seq. ID No. 74), ATTGCAAAATTC (Seq. ID No. 77), TTGCAAAATTCA (Seq. ID No. 78), CATTGCAAAATTC (Seq. ID No. 88), ATTGCAAAATTCA (Seq. ID No. 89), TTGCAAAATTCAG (Seq. ID No. 90), ACATTGCAAAATTC (Seq. ID No. 91), CATTGCAAAATTCA (Seq. ID No. 92), ATTGCAAAATTCAG (Seq. ID No. 93), and salts and optical isomers of said antisense-oligonucleotide.

Still more preferably, the present invention relates to an antisense-oligonucleotide consisting of 12 to 14 nucleotides, wherein at least two of the 12 to 14 nucleotides are LNAs, wherein the antisense-oligonucleotides is capable of hybridizing with a region of the gene encoding *Efnb2,* or with a region of the mRNA encoding *Efnb2,* wherein the antisense-oligonucleotide is represented by the following sequence GGTTAGGGCTGA (Seq. ID No. 16), AGGTTAGGGCTGA (Seq. ID No. 26), GGTTAGGGCTGAA (Seq. ID No. 27), AGGTTAGGGCTGAA (Seq. ID No. 30), GGTTAGGGCTGAAT (Seq. ID No. 31), CAAGCAAGGCAT (Seq. ID No. 46), ACAAGCAAGGCAT (Seq. ID No. 58), CAAGCAAGGCATT (Seq. ID No. 59), TACAAGCAAGGCAT (Seq. ID No. 61), ACAAGCAAGGCATT (Seq. ID No. 62), CAAGCAAGGCATTT (Seq. ID No. 63) TGCAAAATTCAG (Seq. ID No. 74), TTGCAAAATTCAG (Seq. ID No. 90), ATTGCAAAATTCAG (Seq. ID No. 93), and salts and optical isomers of said antisense-oligonucleotide.

It shall be understood, that "**coding DNA strand**", as used herein, refers to the DNA strand that is identical to the mRNA (except that is written in the DNA code) and that encompasses the codons that used for protein translation. It is not used as template for the transcription into mRNA. Thus, the terms "coding DNA strand", "sense DNA strand" and "non-template DNA strand" can be used interchangeably.

Furthermore, "**non-coding DNA strand**", as used herein, refers to the DNA strand that is complementary to the "coding DNA strand" and serves as a template for the transcription of mRNA. Thus, the terms "non-coding DNA strand", "antisense DNA strand" and "template DNA strand" can be used interchangeably

The term "**antisense-oligonucleotide**" refers to an oligomer or polymer of ribonucleic acid (RNA) or deoxyribonucleic acid (DNA) or mimetics or variants thereof such. The term "antisense-oligonucleotide" includes oligonucleotides composed of naturally-occurring nucleobases, sugars and covalent internucleotide (backbone) linkages as well as oligonucleotides having non-naturally-occurring portions which function similarly. Such modified or substituted oligonucleotides are often preferred over native forms, because of desirable properties such as, for example, enhanced cellular uptake, enhanced affinity for nucleic acid target and increased stability in the presence of nucleases. The antisense-oligonucleotides are short catalytic RNAs or catalytic oligonucleotides which hybridize to the target nucleic acid and inhibit its expression.

The term "**nucleoside**" is well known to a skilled person and refers to a pentose sugar moiety like ribose, desoxyribose or a modified or locked ribose or a modified or locked desoxyribose like the LNAs which are below disclosed in detail. A nucleobase is linked to the glycosidic carbon atom (position 1' of the pentose) and an internucleotide linkage is formed between the 3' oxygen or sulfur atom and preferably the 3' oxygen atom of a nucleoside and the 5' oxygen or sulfur atom and preferably the 5' oxygen atom of the adjacent nucleoside, while the internucleotide linkage does not belong to the nucleoside.

The term "**nucleotide**" is well known to a skilled person and refers to a pentose sugar moiety like ribose, desoxyribose or a modified or locked ribose or a modified or locked desoxyribose like the LNAs which are below disclosed in detail. A nucleobase is linked to the glycosidic carbon atom (position 1' of the pentose) and an internucleotide linkage is formed between the 3' oxygen or sulfur atom and preferably the 3' oxygen atom of a nucleotide and the 5' oxygen or sulfur atom and preferably the 5' oxygen atom of the adjacent nucleotide, while the internucleotide linkage is a part of the nucleotide.

### Nucleobases

The term "**nucleobase**" is herein abbreviated with "B" and refers to the five standard nucleotide bases adenine (A), thymine (T), guanine (G), cytosine (C), and uracil (U) as well as to modifications or analogues thereof or analogues with ability to form Watson-Crick base pair with bases in the complimentary strand. Modified nucleobases include other synthetic and natural nucleobases such as 5-methylcytosine (C*), 5-hydroxymethyl cytosine, N⁴-methylcytosine, xanthine, hypoxanthine, 7-deazaxanthine, 2-aminoadenine, 6-methyladenine, 6-methylguanine, 6-ethyladenine, 6-ethylguanine, 2-propyladenine, 2-propylguanine, 6-carboxyuracil, 5-halouracil, 5,6-dihydrouracil, 5-halocytosine, 5-propynyl uracil, 5-propynyl cytosine, 6-aza uracil, 6-aza cytosine, 6-aza thymine, 5-uracil (pseudouracil), 4-thiouracil, 8-fluoroadenine, 8-chloroadenine, 8-bromoadenine, 8-iodoadenine, 8-aminoadenine, 8-thioladenine, 8-thioalkyladenine, 8-hydroxyladenine, 8-fluoroguanine, 8-chloroguanine, 8-bromoguanine, 8-iodoguanine, 8-aminoguanine, 8-thiolguanine, 8-thioalkylguanine, 8-hydroxylguanine, 5-fluorouracil, 5-bromouracil, 5-chlorouracil, 5-iodouracil, 5-trifluoromethyluracil, 5-fluorocytosine, 5-bromocytosine, 5-chlorocytosine, 5-iodocytosine, 5-trifluoromethylcytosine, 7-methylguanine, 7-methyladenine, 8-azaguanine, 8-azaadenine, 7-deazaguanine, 7-deazaadenine, 7-deaza-8-azaadenine, 3-deazaguanine, 3-deazaadenine, 2-thiouracil, 2-thiothymine and 2-thiocytosine etc., with 5-methylcytosine and/or 2-aminoadenine substitutions being preferred since these modifications have been shown to increase nucleic acid duplex stability.

Preferred antisense-oligonucleotides of the present invention can comprise analogues of nucleobases. The nucleobase of only one nucleotide unit of the antisense-oligonucleotide could be replaced by an analogue of a nucleobase or two, three, four, five or even all nucleobases in an antisense-oligonucleotide could be replaced by analogues of nucleobases.

It will be recognized that when referring to a sequence of nucleotides or monomers, what is referred to, is the sequence of bases, such as A, T, G, C or U. The representation of the antisense-oligonucleotides by the letter code A, T, G, C and U has to be understood that said antisense-oligonucleotide may contain any the nucleobases as disclosed herein, any of the 3' end groups as disclosed herein, any of the 5' end groups as disclosed herein, and any of the internucleotide linkages (also referred to as internucleotide bridges) as disclosed herein. The nucleotides A, T, G, C and U have also to be understood as being LNA nucleotides or non-LNA nucleotides such as preferably DNA nucleotides.

The antisense-oligonucleotides as well as the salts of the antisense-oligonucleotides as disclosed herein have been proven to be complementary to the target which is the gene encoding for *EfnB2* or the mRNA encoding the *EfnB2,* i.e., hybridize sufficiently well and with sufficient specificity and especially selectivity to give the desired inhibitory effect.

The term "**salt**" refers to physiologically and/or pharmaceutically acceptable salts of the antisense-oligonucleotides of the present invention. The antisense-oligonucleotides contain nucleobases like adenine, guanine, thymine, cytosine or derivatives thereof which are basic and which form a salt like a chloride or mesylate salt. The internucleotide linkage preferably contains a negatively charged oxygen or sulfur atom which form salts like the sodium, lithium or potassium salt. Thus, pharmaceutically acceptable base addition salts are formed with inorganic bases or organic bases. Examples for suitable organic and inorganic bases are bases derived from metal ions, e.g., aluminum, alkali metal ions, such as sodium or potassium, alkaline earth metal ions such as calcium or magnesium, or an amine salt ion or alkali- or alkaline-earth hydroxides, - carbonates or -bicarbonates. Examples include aqueous LiOH, NaOH, KOH, NH₄OH, potassium carbonate, ammonia and sodium bicarbonate, ammonium salts, primary, secondary and tertiary amines, such as, e.g., tetraalkylammonium hydroxide, lower alkylamines such as methylamine, t-butylamine, procaine, ethanolamine, arylalkylamines such as dibenzylamine and N,N-dibenzylethylenediamine, lower alkylpiperidines such as N-ethylpiperidine, cycloalkylamines such as cyclohexylamine or dicyclohexylamine, morpholine, glucamine, N-methyl- and N,N-dimethylglucamine, 1-adamantylamine, benzathine, or salts derived from amino acids like arginine, lysine, ornithine or amides of originally neutral or acidic amino acids, chloroprocaine, choline, procaine or the like.

Since the antisense-oligonucleotides are basic, they form pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogen-benzenesulfonic acid, picric acid, adipic acid, D-o-tolyltartaric acid, tartronic acid, toluic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

In the context of this invention, "hybridization" means nucleic acid hybridization, wherein a single-stranded nucleic acid (DNA or RNA) interacts with another single-stranded nucleic acid having a very similar or even complementary sequence. Thereby the interaction takes place by hydrogen bonds between specific nucleobases (base pairing).

As used herein, the term "complementarity" (DNA and RNA base pair complementarity) refers to the capacity for precise pairing between two nucleic acids. The nucleotides in a base pair are complementary when their shape allows them to bond together by hydrogen bonds. Thereby forms the pair of adenine and thymidine (or uracil) two hydrogen bonds and the cytosine-guanine pair forms three hydrogen bonds. "Complementary sequences" as used herein means DNA or RNA sequences, being such that when they are aligned antiparallel to each other, the nucleotide bases at each position in the sequences will be complementary, much like looking in the mirror and seeing the reverse of things.

The term "specifically hybridizable" as used herein indicates a sufficient degree of complementarity or precise base pairing of the antisense-oligonucleotide to the target sequence such that stable and specific binding occurs between the antisense-oligonucleotide and the DNA or RNA target. The sequence of an - oligonucleotide according to the invention does not need to be 100% complementary to that of its target nucleic acid to be specifically hybridizable, although a 100% complementarity is preferred. Thereby "100% complementarity" means that the antisense-oligonucleotide hybridizes with the target over its complete or full length without mismatch. In other words, within the present invention it is defined that an antisense compound is specifically hybridizable when binding of the compound to the target DNA or RNA molecule takes place under physiological or pathological conditions but non-specific binding of the antisense-oligonucleotide to non-target sequences is highly unlikely or even impossible.

Therefore, the present invention refers preferably to antisense oligonucleotides, wherein the antisense oligonucleotides bind with 100% complementarity to the mRNA encoding *EfnB2* and do not bind to any other region in the complete human transcriptome. Further preferred the present invention refers to antisense oligonucleotides, wherein the antisense oligonucleotides have 100% complementarity over their complete length to the mRNA encoding *EfnB2* and have no off-target effects. Alternatively, the present invention refers preferably to antisense oligonucleotides having 100% complementarity to the mRNA encoding *EfnB2* but no complementarity to another mRNA of the human transcriptome. Thereby the term "human transcriptome" refers to the total set of transcripts in the human organism, which means transcripts of all cell types and environmental conditions (at any given time).

The antisense-oligonucleotides of the present invention have in common that they are specific in regard to the region where they bind to the gene or to the mRNA encoding *EfnB2.* According to the present invention it is preferred that within the human transcriptome, the antisense-oligonucleotides have 100% complementarity over their full length only with the mRNA encoding *EfnB2.*

The term "**mRNA**", as used herein, may encompass both mRNA containing introns (also referred to as pre-mRNA) as well as mature mRNA which does not contain any introns.

The antisense-oligonucleotides of the present invention are able to bind or hybridize with the pre-mRNA and/or with the mature mRNA. That means the antisense-oligonucleotides can bind to or hybridize at an intron region or within an intron region of the Pre-mRNA or can bind to or hybridize at an overlapping intron - exon region of the Pre-mRNA or can bind to or hybridize at an exon region or within an exon region of the Pre-mRNA and the exon region of the mRNA. Preferred are antisense-oligonucleotides which are able to bind to or hybridize with Pre-mRNA and mRNA. Binding or hybridization of the antisense-oligonucleotides (ASO) to the Pre-mRNA inhibits the 5' cap formation, inhibits splicing of the Pre-mRNA in order to obtain the mRNA and activates RNase H which cleaves the Pre-mRNA. Binding or hybridization of the antisense-oligonucleotides (ASO) to the mRNA activates RNase H which cleaves the mRNA and inhibits binding of the ribosomal subunits.

Preferably, the antisense oligonucleotides according to the present invention bind to or hybridize at an exon region or within an exon region of the Pre-mRNA and the exon region of the mRNA.

The antisense-oligonucleotides of the present invention consist of at least 10 and no more than 28, preferably no more than 24 and more preferably no more than 20 nucleotides and consequently consist of 10, 11, 12, 13, 14, 15, 16, 17, 18, 19, or 20 nucleotides, preferably of 10 to 20, or 10 to 19, or 11 to 19, or 12 to 19, or 12 to 18 nucleotides and more preferably of 12 to 16 nucleotides. Preferably at least two, preferably three, more preferably four of these nucleotides are locked nucleic acids (LNA). Shorter antisense-oligonucleotides, i.e. antisense-oligonucleotides having less than 10 nucleotides, are also possible, but the shorter the antisense-oligonucleotides, the higher the risk that the hybridization is not sufficiently strong anymore and that selectivity will decrease or will get lost. Non-selective antisense-oligonucleotides bear the risk to bind to undesired regions in the human transcriptome and to undesired mRNAs coding for other proteins thereby causing undesired side effects. Longer antisense-oligonucleotides having more than 20 nucleotides are also possible but further increasing the length make the synthesis of such antisense-oligonucleotides even more complicated and expensive without any further benefit in increasing selectivity or strength of hybridization or better stability in regard to degradation.

Thus the present invention is directed to antisense-oligonucleotides consisting of 10 to 20 nucleotides. Preferably at least two nucleotides and preferably the 3' and 5' terminal nucleotides are LNAs. Thus, it is preferred that at least the terminal 3' nucleotide is an LNA and also at least the 5' terminal nucleotide is an LNA. In case more than 2 LNAs are present, it is preferred that the further LNAs are linked to the 3' or 5' terminal LNA like it is the case in gapmers as disclosed herein.

One nucleotide building block present in an antisense-oligonucleotide of the present invention can be represented by the following general formula (B1) and (B2): 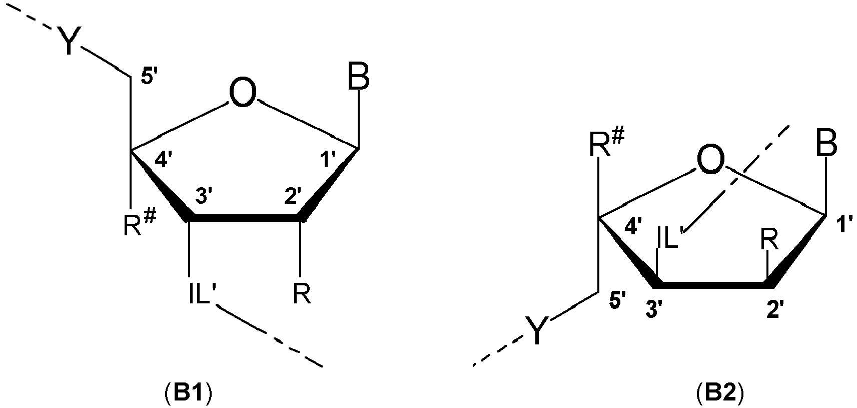 wherein
B represents a nucleobase;
IL' represents ―X"―P(=X')(X⁻)―;
R represents ―H, ―F, ―OH, ―NH₂, ―OCH₃, ―OCH₂CH₂OCH₃ and R^{#} represents -H;
or R and R^{#} form together the bridge ―R^{#}―R― which is selected from ―CH₂―O―, ―CH₂―S―, ―CH₂―NH―, ―CH₂―N(CH₃)―, ―CH₂―N(C₂H₅)―, ―CH₂―CH₂―O―, ―CH₂―CH₂―S―, ―CH₂―CH₂―NH―, ―CH₂―CH₂―N(CH₃)―, or ―CH―CH₂―N(C₂H₅)―;
X' represents =O or =S;
X⁻ represents ―O⁻, ―OH, ―OR^{H}, ―NHR^{H}, ―N(R^{H})₂, ―OCH₂CH₂OR^{H}, ―OCH₂CH₂SR^{H}, ―BH₃⁻, ―R^{H}, ―SH, ―SR^{H}, or ―S⁻;
X" represents ―O―, ―NH―, ―NR^{H}―, ―CH₂―, or ―S―;
Y is ―O―, ―NH―, ―NR^{H}―, ―CH₂― or ―S―;
R^{H} is selected from hydrogen and C₁₋₄-alkyl and preferably ―CH₃ or ―C₂H₅ and most preferably ―CH₃.

Preferably X⁻ represents ―O⁻, ―OH, ―OCH₃, ―NH(CH₃), ―N(CH₃)₂, ―OCH₂CH₂OCH₃, ―OCH₂CH₂SCH₃, ―BH₃⁻, ―CH₃, ―SH, ―SCH₃, or ―S⁻; and more preferably ―O⁻, ―OH, ―OCH₃, ―N(CH₃)₂, ―OCH₂CH₂OCH₃, ―BH₃⁻, ―SH, ―SCH₃, or ―S⁻.

IL' represents preferably ―O―P(O)(O⁻)―, ―O―P(O)(S⁻)―, ―O―P(S)(S⁻)―, ―S―P(O)(O⁻)―, ―S―P(O)(S⁻)―, ―S―P(S)(S⁻)―, ―O―P(O)(O⁻)―, ―O―P(O)(S⁻)―, ―S―P(O)(O⁻)―, ―O―P(O)(R^{H})―, ―O―P(O)(OR^{H})―, ―O―P(O)(NHR^{H})―, ―O―P(O)[N(R^{H})₂]―, ―O―P(O)(BH₃⁻)―, ―O―P(O)(OCH₂CH₂OR^{H})―, ―O―P(O)(OCH₂CH₂SR^{H})―, ―O―P(O)(O⁻)―, ―NR^{H}―P(O)(O⁻)―, wherein R^{H} is selected from hydrogen and C₁₋₄-alkyl.

The group ―O―P(O)(R^{H})―O― is preferably ―O―P(O)(CH₃)―O― or ―O―P(O)(C₂H₅)―O― and most preferably ―O―P(O)(CH₃)―O―. The group ―O―P(O)(OR^{H})―O― is preferably ―O―P(O)(OCH₃)―O― or ―O―P(O)(OC₂H₅)―O― and most preferably ―O―P(O)(OCH₃)―O―. The group ―O―P(O)(NHR^{H})―O― is preferably ―O―P(O)(NHCH₃)―O― or ―O―P(O)(NHC₂H₅)―O― and most preferably ―O―P(O)(NHCH₃)―O―. The group ―O―P(O)[N(R^{H})₂]―O― is preferably ―O―P(O)[N(CH₃)₂]―O― or ―O―P(O)[N(C₂H₅)₂]―O― and most preferably ―O―P(O)[N(CH₃)₂]―O―. The group ―O―P(O)(OCH₂CH₂OR^{H})―O― is preferably ―O―P(O)(OCH₂CH₂OCH₃)―O― or ―O―P(O)(OCH₂CH₂OC₂H₅)―O― and most preferably ―O―P(O)(OCH₂CH₂OCH₃)―O―. The group ―O―P(O)(OCH₂CH₂SR^{H})―O― is preferably ―O―P(O)(OCH₂CH₂SCH₃)―O― or ―O―P(O)(OCH₂CH₂SC₂H₅)―O― and most preferably ―O―P(O)(OCH₂CH₂SCH₃)―O―. The group ―O―P(O)(O⁻)―NR^{H}― is preferably ―O―P(O)(O⁻)―NH― or ―O―P(O)(O⁻)―N(CH₃)― and most preferably ―O―P(O)(O⁻)―NH―. The group ―NR^{H}―P(O)(O⁻)―O― is preferably ―NH―P(O)(O⁻)―O― or ―N(CH₃)―P(O)(O⁻)―O― and most preferably ―NH―P(O)(O⁻)―O―.

Even more preferably IL' represents ―O―P(O)(O⁻)―, ―O―P(O)(S⁻)―, ―O―P(S)(S⁻)―, ―O―P(O)(NHR^{H})―, or ―O―P(O)[N(R^{H})₂]―, and still more preferably IL' represents ―O―P(O)(O⁻)―, ―O―P(O)(S⁻)―, or ―O―P(S)(S⁻)―, and most preferably IL' represents ―O―P(O)(S⁻)―, or ―O―P(S)(S⁻)―.

Preferably Y represents ―O―.

Preferably B represents a standard nucleobase selected from A, T, G, C, U.

Preferably IL represents ―O―P(=O)(S⁻)― or ―O―P(=S)(S⁻)―.

Thus the following general formula (B3) to (B6) are preferred: 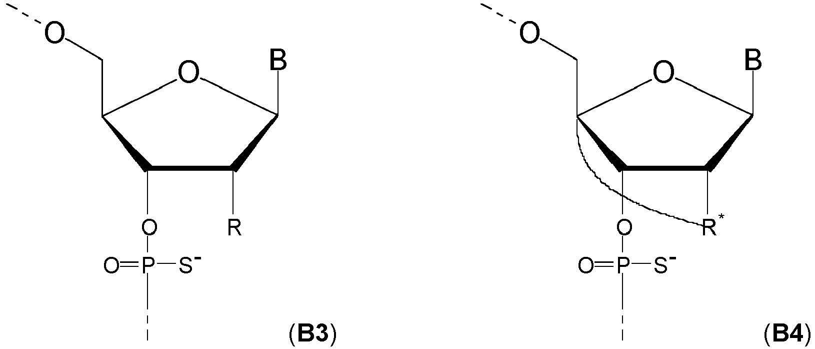 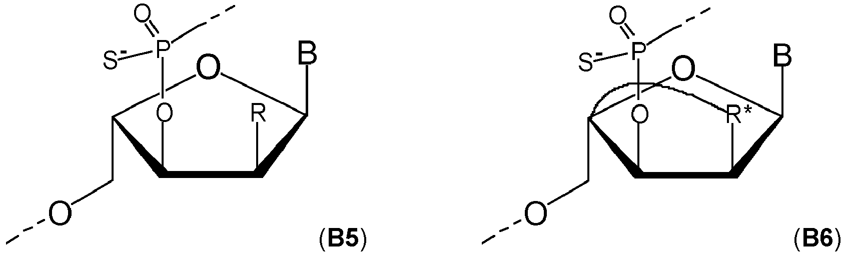 wherein
B represents a nucleobase and preferably A, T, G, C, U;
R represents ―H, ―F, ―OH, ―NH₂, ―N(CH₃)₂, ―OCH₃, ―OCH₂CH₂OCH₃, ―OCH₂CH₂CH₂OH, ―OCH₂CH₂CH₂NH₂ and preferably -H;
R^{*} represents the moiety ―R^{#}―R― as defined below and is, for instance, preferably selected from ―C(R^{a}R^{b})―O―, ―C(R^{a}R^{b})―NR^{c}―, ―C(R^{a}R^{b})―S―, and ―C(R^{a}R^{b})―C(R^{a}R^{b})―O―, wherein the substituents R^{a}, R^{b} and R^{c} have the meanings as defined herein. More preferably R^{*} is selected from ―CH₂―O―, ―CH₂―S―, ―CH₂―NH―, ―CH₂―N(CH₃)―, ―CH₂―CH₂―O―, or ―CH₂―CH₂―S―, and more preferably ―CH₂―O―, ―CH₂―S―, ―CH₂―CH₂―O―, or ―CH₂―CH₂―S―, and still more preferably ―CH₂―O―, ―CH₂―S―, or ―CH₂―CH₂―O―, and still more preferably ―CH₂―O― or ―CH₂―S―, and most preferably ―CH₂―O―.

Examples of preferred nucleotides which are non-LNA units are the following:

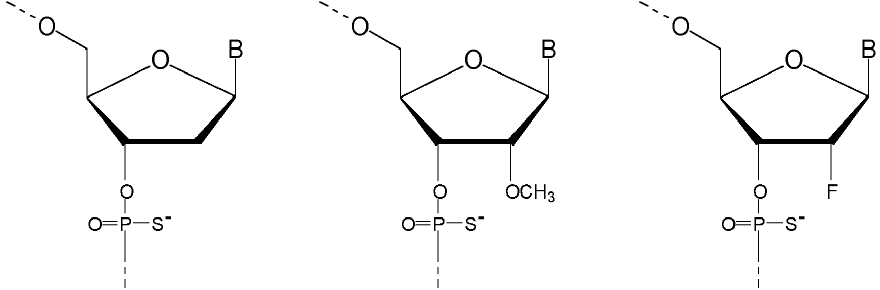

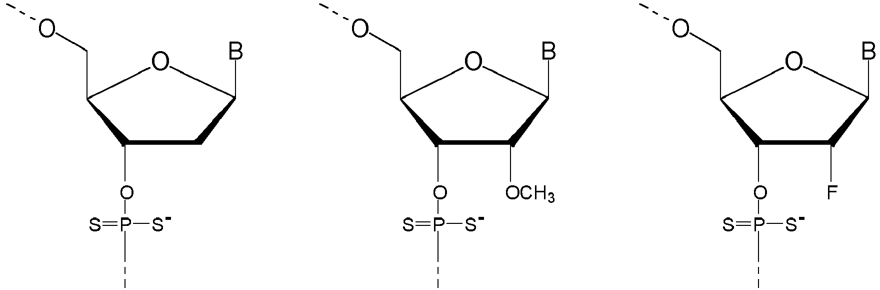

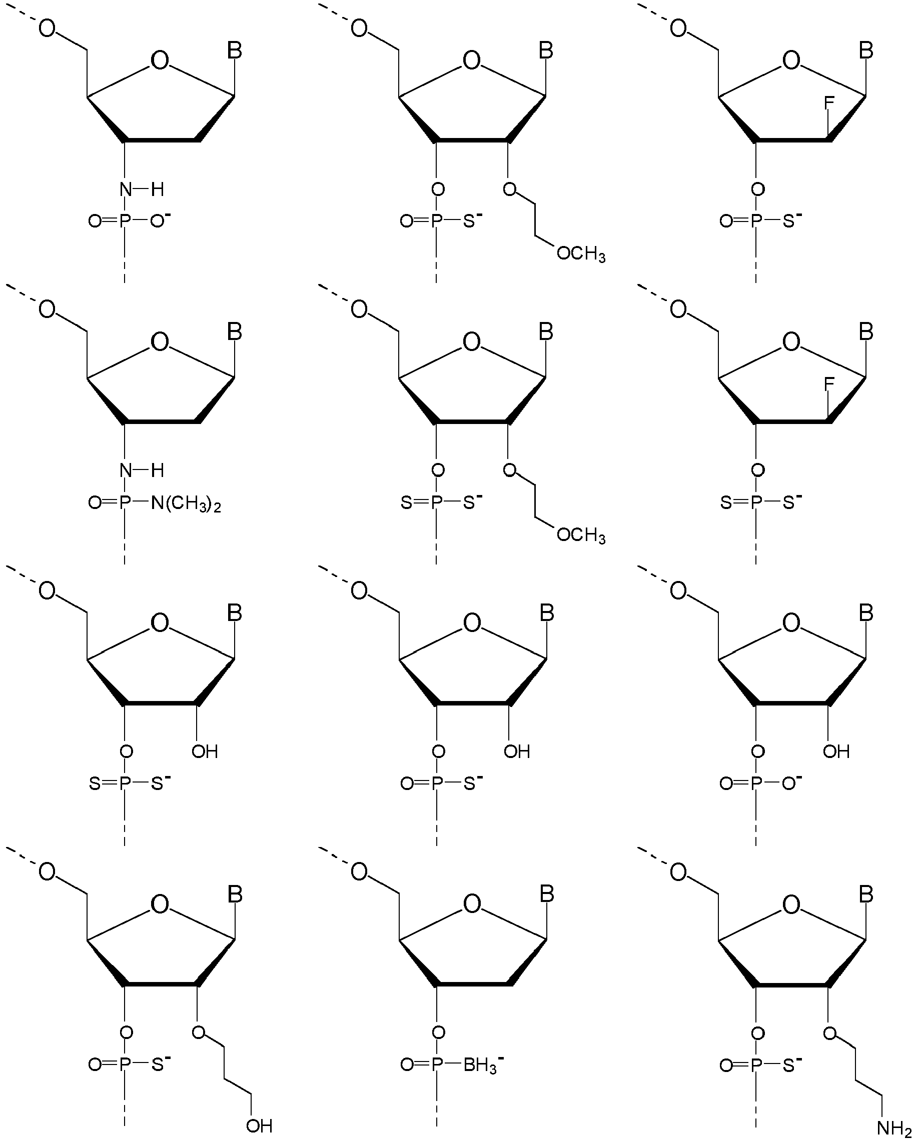

### Internucleotide Linkages (IL)

The monomers of the antisense-oligonucleotides described herein are coupled together via an internucleotide linkage. Suitably, each monomer is linked to the 3' adjacent monomer via an internucleotide linkage. The person having ordinary skill in the art would understand that, in the context of the present invention, the 5' monomer at the end of an oligomer does not comprise a 5' internucleotide linkage, although it may or may not comprise a 5' terminal group. The term "internucleotide linkage" is intended to mean a group capable of covalently coupling together two nucleotides, two nucleotide analogues like two LNAs, and a nucleotide and a nucleotide analogue like an LNA. Specific and preferred examples include phosphate groups and phosphorothioate groups.

The nucleotides of the antisense-oligonucleotides of the present invention or contiguous nucleotide sequences thereof are coupled together via internucleotide linkages. Suitably each nucleotide is linked through the 5' position to the 3' adjacent nucleotide via an internucleotide linkage.

The antisense-oligonucleotides can be modified by several different ways. Modifications within the backbone are possible and refer to antisense-oligonucleotides wherein the phosphate groups (also named phosphodiester groups) in their internucleotide backbone are partially or completely replaced by other groups. Preferred modified antisense-oligonucleotide backbones include, for instance, phosphorothioates, chiral phosphorothioates, phosphorodithioates, phosphotriester, aminoalkylphosphotriesters, methyl, ethyl and C₃―C₁₀―alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters and boranophosphates having normal 3'-5' linkages, 2'-5' linked analogues of these, and those having inverted polarity wherein the adjacent pairs of nucleotide units are linked 3'-5' to 5'-3' or 2'-5' to 5'-2'. Various salts, mixed salts and free acids forms thereof are also included and disclosed herein in further detail.

Suitable internucleotide linkages include those listed within WO2007/031091, for example the internucleotide linkages listed on the first paragraph of page 34 of WO2007/031091 (hereby incorporated by reference). It is, in some embodiments, preferred to modify the internucleotide linkage from its normal phosphodiester to one that is more resistant to nuclease attack, such as phosphorothioate or boranophosphate - these two, accepted by RNase H mediated cleavage, also allow that route of antisense inhibition in reducing the expression of the target gene.

The internucleotide linkage consists of the group IL' which is the group bound to the 3' carbon atom of the ribose moiety and the group Y which is the group bound to the 5' carbon atom of the contiguous ribose moiety as shown in the formula (IL'Y) below

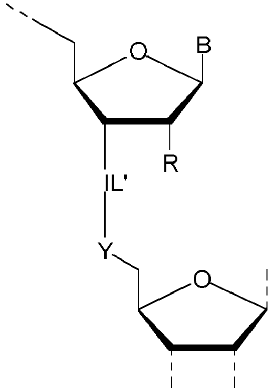

The internucleotide linkage IL is represented by ―IL'―Y―. IL' represents ―X"―P(=X')(X⁻)― so that IL is represented by ―X"-P(=X')(X⁻)―Y―, wherein the substituents X⁻, X', X" and Y have the meanings as disclosed herein.

The internucleotide linkage IL = ―X"―P(=X')(X⁻)―Y― is preferably selected form the group consisting of:
―O―P(O)(O⁻)―O―, ―O―P(O)(S⁻)―O―, ―O―P(S)(S⁻)―O―, ―S―P(O)(O⁻)―O―, ―S―P(O)(S⁻)―O―, ―S―P(S)(S⁻)―O―, ―O―P(O)(O⁻)―S―, ―O―P(O)(S⁻)―S―, ―S―P(O)(O⁻)―S―, ―O―P(O)(R^{H})―O―, ―O―P(O)(OR^{H})―O―, ―O―P(O)(NHR^{H})―O―, ―O―P(O)[N(R^{H})₂]―O―, ―O―P(O)(BH₃⁻)―O―, ―O―P(O)(OCH₂CH₂OR^{H})―O―, ―O―P(O)(OCH₂CH₂SR^{H})―O―, ―O―P(O)(O⁻)―NR^{H}―, ―NR^{H}―P(O)(O⁻)―O― where R^{H} is selected from hydrogen and C1-4-alkyl.

The group ―O―P(O)(R^{H})―O― is preferably ―O―P(O)(CH₃)―O― or ―O―P(O)(C₂H₅)―O― and most preferably ―O―P(O)(CH₃)―O―. The group ―O―P(O)(OR^{H})―O― is preferably ―O―P(O)(OCH₃)―O― or ―O―P(O)(OC₂H₅)―O― and most preferably ―O―P(O)(OCH₃)―O―. The group ―O―P(O)(NHR^{H})―O― is preferably ―O―P(O)(NHCH₃)―O― or ―O―P(O)(NHC₂H₅)―O― and most preferably ―O―P(O)(NHCH₃)―O―. The group ―O―P(O)[N(R^{H})₂]―O― is preferably ―O―P(O)[N(CH₃)₂]―O― or ―O―P(O)[N(C₂H₅)₂]―O― and most preferably ―O―P(O)[N(CH₃)₂]―O―. The group ―O―P(O)(OCH₂CH₂OR^{H})―O― is preferably ―O―P(O)(OCH₂CH₂OCH₃)―O― or ―O―P(O)(OCH₂CH₂OC₂H₅)―O― and most preferably ―O―P(O)(OCH₂CH₂OCH₃)―O―.

The group ―O―P(O)(OCH₂CH₂SR^{H})―O― is preferably ―O―P(O)(OCH₂CH₂SCH₃)―O― or ―O―P(O)(OCH₂CH₂SC₂H₅)―O― and most preferably ―O―P(O)(OCH₂CH₂SCH₃)―O―. The group ―O―P(O)(O⁻)―NR^{H}― is preferably ―O―P(O)(O⁻)―NH― or ―O―P(O)(O⁻)―N(CH₃)― and most preferably ―O―P(O)(O⁻)―NH―. The group ―NR^{H}―P(O)(O⁻)―O― is preferably ―NH―P(O)(O⁻)―O― or ―N(CH₃)―P(O)(O⁻)―O― and most preferably ―NH―P(O)(O⁻)―O―.

Even more preferably IL represents ―O―P(O)(O⁻)―O―, ―O―P(O)(S⁻)―O―, ―O―P(S)(S⁻)―O―, ―O―P(O)(NHR^{H})―O―, or ―O―P(O)[N(R^{H})₂]―O―, and still more preferably IL represents ―O―P(O)(O⁻)―O―, ―O―P(O)(S⁻)―O―, or ―O―P(S)(S⁻)―O―, and most preferably IL represents ―O―P(O)(S⁻)―O―, or ―O―P(O)(O⁻)―O―.
Thus IL is preferably a phosphate group (―O―P(O)(O⁻)―O―), a phosphorothioate group (―O―P(O)(S⁻)―O―) or a phosphorodithioate group (―O―P(S)(S⁻)―O―).

The nucleotide units or the nucleosides of the antisense-oligonucleotides are connected to each other by internucleotide linkages so that within one antisense-oligonucleotide different internucleotide linkages can be present. The LNA units are preferably linked by internucleotide linkages which are not phosphate groups. The LNA units are linked to each other by a group IL which is preferably selected from ―O―P(O)(S⁻)―O―, ―O―P(S)(S⁻)―O―, ―O―P(O)(NHR^{H})―O―, and ―O―P(O)[N(R^{H})₂]―O― and more preferably from ―O―P(O)(S⁻)―O― and ―O―P(S)(S⁻)―O―.

The non-LNA units are linked to each other by a group IL which is preferably selected from ―O―P(O)(O⁻)―O―, ―O―P(O)(S⁻)―O―, ―O―P(S)(S⁻)―O―, ―O―P(O)(NHR^{H})―O―, and ―O―P(O)[N(R^{H})₂]―O― and more preferably from ―O―P(O)(O⁻)―O―, ―O―P(O)(S⁻)―O― and ―O―P(S)(S⁻)―O―.

A non-LNA unit is linked to an LNA unit by a group IL which is preferably selected from ―O―P(O)(S⁻)―O―, ―O―P(S)(S⁻)―O―, ―O―P(O)(NHR^{H})―O―, and ―O―P(O)[N(R^{H})₂]―O― and more preferably from ―O―P(O)(S⁻)―O― and ―O―P(S)(S⁻)―O―.

The term "**LNA unit**" as used herein refers to a nucleotide which is locked, i.e. to a nucleotide which has a bicyclic structure and especially a bicyclic ribose structure and more especially a bicyclic ribose structure as shown in general formula (II). The bridge "locks" the ribose in the 3'-*endo* (North) conformation. The ribose moiety of an LNA nucleotide is modified with an extra bridge connecting the 2' oxygen and 4' carbon. Alternatively used terms for LNA are bicyclic nucleotides or bridged nucleotides, thus, an alternative term for LNA unit is bicyclic nucleotide unit or bridged nucleotide unit.

The term "**non-LNA unit**" as used herein refers to a nucleotide which is not locked, i.e. to a nucleotide which has no bicyclic sugar moiety and especially no bicyclic ribose structure and more especially no bicyclic ribose structure as shown in general formula (II). The non-LNA units are most preferably DNA units.

The term "**DNA unit**" as used herein refers to a nucleotide containing a 2-deoxyribose as sugar. Thus, the nucleotide is made of a nucleobase and a 2-deoxyribose.

The term "**unit**" as used herein refers to a part or a fragment or a moiety of an antisense-oligonucleotide of the present invention. Thus a "unit" is not a complete molecule, it is a part or a fragment or a moiety of an antisense-oligonucleotide which has at least one position for a covalent linkage to another part or fragment or moiety of the antisense-oligonucleotide. For example, the general structures (**B1**) to (**B6**) are units, because they can be covalently linked through the group Y and IL' or ―O― and ―O―P(O)(S⁻)― respectively. Preferably a unit is a moiety consisting of a pentose structure, a nucleobase connected to the pentose structure a 5' radical group and an IL' radical group.

The term "**building block**" or "**monomer**" as used herein refers to a molecule and especially to a nucleoside which is used in the synthesis of an antisense-oligonucleotide of the present invention.
Examples are the LNA molecules of general formula (I), wherein Y represents a 5'-terminal group and IL' represents a 3'-terminal group.

Furthermore, pure diastereomeric antisense-oligonucleotides are preferred. Preferred are Sp- and Rp-diastereomers as shown at hand-right side:

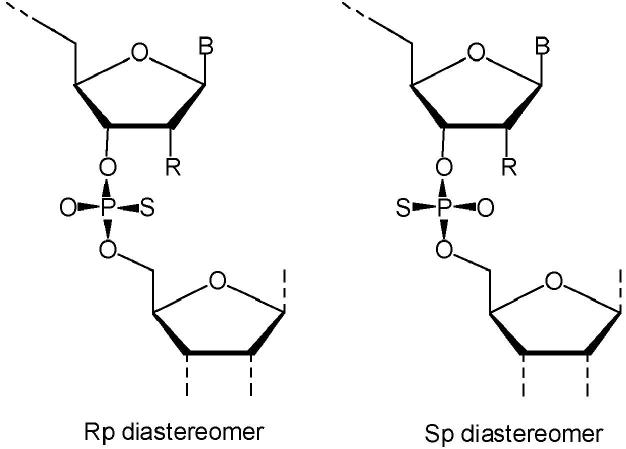

Suitable sulphur (S) containing internucleotide linkages as provided herein are preferred.

Preferred are phosphorothioate moieties in the backbone where at least 50% of the internucleotide linkages are phosphorothioate groups. Also preferred is that the LNA units, if present, are linked through phosphorothioates as internucleotide linkages. Most preferred is a complete phosphorothioate backbone, i.e. most preferred is when all nucleotide units and also the LNA units (if present) are linked to each other through phosphorothioate groups which are defined as follows: ―O―P(O)(S⁻)―O― which is synonymous to ―O―P(O,S)―O― or to ―O―P(O⁻)(S)―O―.

In case the antisense-oligonucleotide is a **gapmer**, it is preferred that the LNA regions have internucleotide linkages selected from ―O―P(O)(S⁻)―O― and ―O―P(S)(S⁻)―O― and that the non-LNA region, the middle part, has internucleotide linkages selected from ―O―P(O)(O⁻)―O―, ―O―P(O)(S⁻)―O― and ―O―P(S)(S⁻)―O― and that the LNA regions are connected to the non-LNA region through internucleotide linkages selected from ―O―P(O)(O⁻)―O―, ―O―P(O)(S⁻)―O― and ―O―P(S)(S⁻)―O―.

It is even more preferred if all internucleotide linkages which are 9 in a 10-mer and 19 in a 20-mer are selected from ―O―P(O)(S⁻)―O― and ―O―P(S)(S⁻)―O―. Still more preferred is that all internucleotide linkages are phosphorothioate groups (―O―P(O)(S⁻)―O―) or are phosphorodithioate groups (―O―P(S)(S⁻)―O―).

### Locked Nucleic Acids (LNA^{®})

It is especially preferred that some of the nucleotides of the general formula (**B1**) or (**B2**) in the antisense-oligonucleotides are replaced by so-called LNAs (Locked Nucleic Acids). The abbreviation LNA is a registered trademark, but herein the term "LNA" is solely used in a descriptive manner.

Preferably the terminal nucleotides are replaced by LNAs and more preferred the last 1 to 4 nucleotides at the 3' end and/or the last 1 to 4 nucleotides at the 5' end are replaced by LNAs. It is also preferred to have at least the terminal nucleotide at the 3' end and at the 5' end replaced by an LNA each.

The term "**LNA**" as used herein, refers to a bicyclic nucleotide analogue, known as "Locked Nucleic Acid". It may refer to an LNA monomer, or, when used in the context of an "LNA antisense-oligonucleotide" or an "antisense-oligonucleotide containing LNAs", LNA refers to an oligonucleotide containing one or more such bicyclic nucleotide analogues. LNA nucleotides are characterized by the presence of a linker group (such as a bridge) between C2' and C4' of the ribose sugar ring - for example as shown as the biradical R^{#} ― R as described below. The LNA used in the antisense-oligonucleotides of the present invention preferably has the structure of the general formula (I) 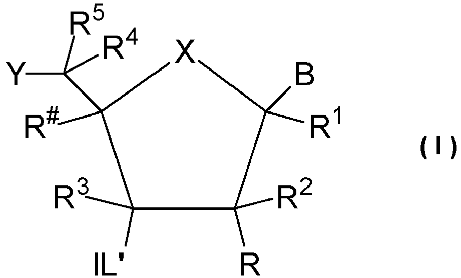
wherein for all chiral centers, asymmetric groups may be found in either R or S orientation;
wherein X is selected from ―O―, ―S―, ―N(R^{N})―, ―C(R⁶R⁷)―, and preferably X is ―O―;
B is selected from hydrogen, optionally substituted C₁₋₄-alkoxy, optionally substituted C₁₋₄-alkyl, optionally substituted C₁₋₄-acyloxy, nucleobases and nucleobase analogues, and preferably B is a nucleobase or a nucleobase analogue and most preferred a standard nucleobase;
Y represents a part of an internucleotide linkage to an adjacent nucleotide in case the moiety of general formula (I) is an LNA unit of an antisense-oligonucleotide of the present invention, or a **5'-terminal group** in case the moiety of general formula (I) is a monomer or building block for synthesizing an antisense-oligonucleotide of the present invention. The 5' carbon atom optionally includes the substituent R⁴ and R⁵;
IL' represents a part of an internucleotide linkage to an adjacent nucleotide in case the moiety of general formula (I) is an LNA unit of an antisense-oligonucleotide of the present invention, or a **3'-terminal group** in case the moiety of general formula (I) is a monomer or building block for synthesizing an antisense-oligonucleotide of the present invention.

R^{#} and R together represent a bivalent linker group consisting of 1 - 4 groups or atoms selected from ―C(R^{a}R^{b})―, ―C(R^{a})=C(R^{b})―, ―C(R^{a})=N―, ―O―, ―Si(R^{a})₂―, ―S―, ―SO_{2―}, ―N(R^{c})―, and >C=Z, wherein Z is selected from ―O―, ―S―, and ―N(R^{a})―, and R^{a}, R^{b} and R^{c} are independently of each other selected from hydrogen, optionally substituted C₁₋₁₂-alkyl, optionally substituted C₂₋₆-alkenyl, optionally substituted C₂₋₆-alkynyl, hydroxy, optionally substituted C₁₋₁₂-alkoxy, C₁₋₆-alkoxy-C₁₋₆-alkyl, C₂₋₆-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkylenyl-aminocarbonyl, mono- and di(C₁₋₆-alkyl)amino-C₁₋₆-alkylenyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, where aryl and heteroaryl may be optionally substituted and where two geminal substituents R^{a} and R^{b} together may represent optionally substituted methylene (=CH2), wherein for all chiral centers, asymmetric groups may be found in either R or S orientation, and;
each of the substituents R¹, R², R³, R⁴, R⁵, R⁶ and R⁷, which are present is independently selected from hydrogen, optionally substituted C₁₋₁₂-alkyl, optionally substituted C₂₋₆-alkenyl, optionally substituted C₂₋₆-alkynyl, hydroxy, C₁₋₁₂-alkoxy, C₁₋₆-alkoxy-C₁₋₆-alkyl, C₂₋₆-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, where aryl and heteroaryl may be optionally substituted, and where two geminal substituents together may designate oxo, thioxo, imino, or optionally substituted methylene;
wherein R^{N} is selected from hydrogen and C₁₋₄-alkyl, and where two adjacent (non-geminal) substituents may designate an additional bond resulting in a double bond; and R^{N}, when present and not involved in a biradical, is selected from hydrogen and C₁₋₄-alkyl; and basic salts and acid addition salts thereof. For all chiral centers, asymmetric groups may be found in either R or S orientation.

In preferred embodiments, R^{#} and R together represent a biradical consisting of a groups selected from the group consisting of ―C(R^{a}R^{b})―C(R^{a}R^{b})―, ―C(R^{a}R^{b})―O―, ―C(R^{a}R^{b})―NR^{c}―, ―C(R^{a}R^{b})―S―, and ―C(R^{a}R^{b})―C(R^{a}R^{b})―O―, wherein each R^{a}, R^{b} and R^{c} may optionally be independently selected.

In some embodiments, R^{a} and R^{b} may be, optionally independently selected from the group consisting of hydrogen and C₁₋₆-alkyl, such as methyl, and preferred is hydrogen.

In preferred embodiments, R¹, R², R³, R⁴, and R⁵ are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂₋₆-alkynyl, C₁₋₆-alkoxyl, substituted C₁₋₆-alkoxyl, acyl, substituted acyl, C₁₋₆-aminoalkyl or substituted C₁₋₆-aminoalkyl. For all chiral centers, asymmetric groups may be found in either R or S orientation.

In preferred embodiments R¹, R², R³, R⁴, and R⁵ are hydrogen.

In some embodiments, R¹, R², and R³, are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂₋₆-alkynyl, C₁₋₆-alkoxyl, substituted C₁₋₆-alkoxyl, acyl, substituted acyl, C₁₋₆-aminoalkyl or substituted C₁₋₆-aminoalkyl. For all chiral centers, asymmetric groups may be found in either R or S orientation. In preferred embodiments R¹, R², and R³ are hydrogen.

In preferred embodiments, R⁴ and R⁵ are each independently selected from the group consisting of ―H, ―CH₃, ―CH_{2―}CH₃, ―CH₂―O―CH₃, and ―CH=CH₂. Suitably in some embodiments, either R⁴ or R⁵ are hydrogen, whereas the other group (R⁴ or R⁵ respectively) is selected from the group consisting of C₁₋₆-alkyl, C₂₋₆-alkenyl, C₂₋₆-alkynyl, substituted C₁₋₆-alkyl, substituted C₂₋₆-alkenyl, substituted C₂₋₆-alkynyl or substituted acyl (―C(=O)―); wherein each substituted group is mono or poly substituted with substituent groups independently selected from halogen, C₁₋₆-alkyl, substituted C₁₋₆-alkyl, C₂₋₆-alkenyl, substituted C₂₋₆-alkenyl, C₂₋₆-alkynyl, substituted C₂₋₆-alkynyl, ―OJ₁, ―SJ₁, ―NJ₁J₂, ―N₃, ―COOJ₁, ―CN, ―O―C(=O)NJ₁J₂, ―N(H)C(=NH)NJ₁J₂ or ―N(H)C(=X)N(H)J₂, wherein X is O or S; and each J₁ and J₂ is, independently ―H, C₁₋₆-alkyl, substituted C₁₋₆-alkyl, C₂₋₆-alkenyl, substituted C₂₋₆-alkenyl, C₂₋₆-alkynyl, substituted C₂₋₆-alkynyl, C₁₋₆-aminoalkyl, substituted C₁₋₆-aminoalkyl or a protecting group. In some embodiments either R⁴ or R⁵ is substituted C₁₋₆-alkyl. In some embodiments either R⁴ or R⁵ is substituted methylene, wherein preferred substituent groups include one or more groups independently selected from ―F, ―NJ₁J₂, ―N₃, ―CN, ―OJ₁, ―SJ₁, ―O―C(=O)NJ₁J₂, ―N(H)C(=NH)NJ₁J₂ or ―N(H)C(=O)N(H)J₂. In some embodiments each J₁ and J₂ is, independently -H or C₁₋₆-alkyl. In some embodiments either R⁴ or R⁵ is methyl, ethyl or methoxymethyl. In some embodiments either R⁴ or R⁵ is methyl. In a further embodiment either R⁴ or R⁵ is ethylenyl. In some embodiments either R⁴ or R⁵ is substituted acyl. In some embodiments either R⁴ or R⁵ is ―O―C(=O)NJ₁J₂. For all chiral centers, asymmetric groups may be found in either R or S orientation. Such 5' modified bicyclic nucleotides are disclosed in WO 2007/134181 A, which is hereby incorporated by reference in its entirety.

In some embodiments B is a nucleobase, including nucleobase analogues and naturally occurring nucleobases, such as a purine or pyrimidine, or a substituted purine or substituted pyrimidine, such as a nucleobase referred to herein, such as a nucleobase selected from the group consisting of adenine, cytosine, thymine, adenine, uracil, and/or a modified or substituted nucleobase, such as 5-thiazolo-uracil, 2-thio-uracil, 5-propynyl-uracil, 2'thio-thymine, 5-methyl cytosine, 5-thiozolo-cytosine, 5-propynyl-cytosine, and 2,6- diaminopurine.

In preferred embodiments, R^{#} and R together represent a biradical selected from ―C(R^{a}R^{b})―O―, ―C(R^{a}R^{b})―C(R^{c}R^{d})―O―, ―C(R^{a}R^{b})―C(R^{c}R^{d})―C(R^{e}R^{f})―O―, ―C(R^{a}R^{b})―O―C(R^{d}R^{e})―, ―C(R^{a}R^{b})―O―C(R^{d}R^{e})―O―, ―C(R^{a}R^{b})―C(R^{d}R^{e})―, ―C(R^{a}R^{b})―C(R^{c}R^{d})―C(R^{e}R^{f})―, ―C(R^{a})=C(R^{b})―C(R^{d}R^{e})―, ―C(R^{a}R^{b})―N(R^{c})―, ―C(R^{a}R^{b})―C(R^{d}R^{e})―N(R^{c})―, ―C(R^{a}R^{b})―N(R^{c})―O―, ―C(R^{a}R^{b})―S―, and ―C(R^{a}R^{b})―C(R^{d}R^{e})―S―, wherein R^{a}, R^{b}, R^{c}, R^{d}, R^{e}, and R^{f} each is independently selected from hydrogen, optionally substituted C₁₋₁₂-alkyl, optionally substituted C₂₋₆-alkenyl, optionally substituted C₂₋₆-alkynyl, hydroxy, C₁₋₁₂-alkoxy, C₁₋₆-alkoxy-C₁₋₆-alkyl, C₂₋₆-alkenyloxy, carboxy, C₁₋₁₂-alkoxycarbonyl C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, where aryl and heteroaryl may be optionally substituted and where two geminal substituents R^{a} and R^{b} together may designate optionally substituted methylene (=CH₂). For all chiral centers, asymmetric groups may be found in either R or S orientation.

In a further embodiment R^{#} and R together designate a biradical (bivalent group) selected from ―CH₂―O―, ―CH₂―S―, ―CH₂―NH―, ―CH₂―N(CH₃)―, ―CH₂―CH₂―O―, ―CH₂―CH(CH₃)―, ―CH₂―CH₂―S―, ―CH_{2―}CH₂―NH―, ―CH₂―CH₂―CH₂―, ―CH_{2―}CH₂―CH₂―O―, ―CH₂―CH₂―CH(CH₃)―, ―CH=CH―CH₂―, ―CH₂―O―CH₂―O―, ―CH₂―NH―O―, ―CH₂―N(CH₃)―O―, ―CH₂―O―CH₂―, ―CH(CH₃)―O―, ―CH(CH₂―O―CH₃)―O―, ―CH₂―CH₂―, and ―CH=CH―. For all chiral centers, asymmetric groups may be found in either R or S orientation.

In some embodiments, R^{#} and R together designate the biradical ―C(R^{a}R^{b})―N(R^{c})―O―, wherein R^{a} and R^{b} are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂₋₆-alkynyl, C₁₋₆-alkoxyl, substituted C₁₋₆-alkoxyl, acyl, substituted acyl, C₁₋₆-aminoalkyl or substituted C₁₋₆-aminoalkyl, such as hydrogen, and; wherein R^{c} is selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂₋₆-alkynyl, C₁₋₆-alkoxyl, substituted C₁₋₆-alkoxyl, acyl, substituted acyl, C₁₋₆-aminoalkyl or substituted C₁₋₆-aminoalkyl, and preferably hydrogen.

In preferred embodiments, R^{#} and R together represent the biradical ―C(R^{a}R^{b})―O―C(R^{d}R^{e})―O―, wherein R^{a}, R^{b}, R^{d}, and R^{e} are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂₋₆-alkynyl, C₁₋₆-alkoxyl, substituted C₁₋₆-alkoxyl, acyl, substituted acyl, C₁₋₆-aminoalkyl or substituted C₁₋₆-aminoalkyl, and preferably hydrogen.

In preferred embodiments, R^{#} and R form the biradical ―CH(Z)―O―, wherein Z is selected from the group consisting of C₁₋₆-alkyl, C₂₋₆-alkenyl, C₂₋₆-alkynyl, substituted C₁₋₆-alkyl, substituted C₂₋₆-alkenyl, substituted C₂₋₆-alkynyl, acyl, substituted acyl, substituted amide, thiol or substituted thio; and wherein each of the substituted groups, is, independently, mono or poly substituted with optionally protected substituent groups independently selected from halogen, oxo, hydroxyl, ―OJ₁, ―NJ₁J₂, ―SJ₁, ―N₃, ―OC(=X)J₁, ―OC(=X)NJ₁J₂, ―NJ³C(=X)NJ₁J₂ and -CN, wherein each J₁, J₂ and J₃ is, independently, ―H or C₁₋₆-alkyl, and X is O, S or NJ₁. In preferred embodiments Z is C₁₋₆-alkyl or substituted C₁₋₆-alkyl. In further preferred embodiments Z is methyl. In preferred embodiments Z is substituted C₁₋₆-alkyl. In preferred embodiments said substituent group is C₁₋₆-alkoxy. In some embodiments Z is CH₃OCH₂―. For all chiral centers, asymmetric groups may be found in either R or S orientation. Such bicyclic nucleotides are disclosed in US 7,399,845 which is hereby incorporated by reference in its entirety. In preferred embodiments, R¹, R², R³, R⁴, and R⁵ are hydrogen. In preferred embodiments, R¹, R², and R³ are hydrogen, and one or both of R⁴, R⁵ may be other than hydrogen as referred to above and in WO 2007/134181.

In preferred embodiments, R^{#} and R together represent a biradical which comprise a substituted amino group in the bridge such as the biradical ―CH₂―N(R^{c})―, wherein R^{c} is C₁₋₁₂-alkyloxy. In preferred embodiments R^{#} and R together represent a biradical ―Cq₃q₄―NOR―, wherein q₃ and q₄ are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂₋₆-alkynyl, C₁₋₆-alkoxyl, substituted C₁₋₆-alkoxyl, acyl, substituted acyl, C₁₋₆-aminoalkyl or substituted C₁₋₆-aminoalkyl; wherein each substituted group is, independently, mono or poly substituted with substituent groups independently selected from halogen, ―OJ₁, ―SJ₁, ―NJ₁J₂, ―COOJ₁, -CN, ―OC(=O)NJ₁J₂,
―NH―C(=NH)NJ₁J₂ or ―NH―C(=X)NHJ₂, wherein X is O or S; and each of J₁ and J₂ is, independently, -H, C₁₋₆-alkyl, C₂₋₆-alkenyl, C₂₋₆-alkynyl, C₁₋₆-aminoalkyl or a protecting group. For all chiral centers, asymmetric groups may be found in either R or S orientation. Such bicyclic nucleotides are disclosed in WO2008/150729 which is hereby incorporated by reference in its entirety. In preferred embodiments, R¹, R², R³, R⁴, and R⁵ are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂₋₆-alkynyl, C₁₋₆-alkoxyl, substituted C₁₋₆-alkoxyl, acyl, substituted acyl, C₁₋₆-aminoalkyl or substituted C₁₋₆-aminoalkyl. In preferred embodiments, R¹, R², R³, R⁴, and R⁵ are hydrogen. In preferred embodiments, R¹, R², and R³ are hydrogen and one or both of R⁴, R⁵ may be other than hydrogen as referred to above and in WO 2007/134181.

In preferred embodiments R^{#} and R together represent a biradical (bivalent group) ―C(R^{a}R^{b})―O―, wherein R^{a} and R^{b} are each independently halogen, C₁₋₁₂-alkyl, substituted C₁₋₁₂-alkyl, C₂₋₆-alkenyl, substituted C₂₋₆-alkenyl, C₂₋₆-alkynyl, substituted C₂₋₆-alkynyl, C₁₋₁₂-alkoxy, substituted C₁₋₁₂-alkoxy, ―OJ₁, ―SJ₁, ―S(O)J₁, ―SO₂―J₁, ―NJ₁J₂, ―N₃, -CN, ―C(=O)OJ₁, ―C(=O)NJ₁J₂, ―C(=O)J₁, ―OC(=O)NJ₁J₂, ―NH―C(=NH)NJ₁J₂, ―NH―C(=O)NJ₁J₂, or , ―NH―C(=S)NJ₁J₂; or R^{a} and R^{b} together are =C(q₃)(q₄); q₃ and q₄ are each, independently, - H, halogen, C₁₋₁₂-alkyl or substituted C₁₋₁₂-alkyl; each substituted group is, independently, mono or poly substituted with substituent groups independently selected from halogen, C₁₋₆-alkyl, substituted C₁₋₆-alkyl, C₂₋₆-alkenyl, substituted C₂₋₆-alkenyl, C₂₋₆-alkynyl, substituted C₂₋₆-alkynyl, ―OJ₁, ―SJ₁, ―NJ₁J₂, ―N₃, ―CN, ―C(=O)OJ₁, ―C(=O)NJ₁J₂, ―C(=O)J₁, ―OC(=O)NJ₁J₂, ―NH―C(=O)NJ₁J₂, or ―NH―C(=S)NJ₁J₂ and; each J₁ and J₂ is independently, -H, C₁₋₆-alkyl, substituted C₁₋₆-alkyl, C₂₋₆-alkenyl, substituted C₂₋₆-alkenyl, C₂₋₆-alkynyl, substituted C₂₋₆-alkynyl, C₁₋₆-aminoalkyl, substituted C₁₋₆-aminoalkyl or a protecting group. Such compounds are disclosed in WO2009006478A, hereby incorporated in its entirety by reference.

In preferred embodiments, R^{#} and R form the biradical -Q-, wherein Q is ―C(q₁)(q₂)C(q₃)(q₄)―, ―C(q₁)=C(q₃)―, ―C[=C(q₁)(q₂)]―C(q₃)(q₄)― or ―C(q₁)(q₂)―C[=C(q₃)(q₄)]―; q₁, q₂, q₃, q₄ are each independently of each other -H, halogen, C₁₋₁₂-alkyl, substituted C₁₋₁₂-alkyl, C₂₋₆-alkenyl, substituted C₁₋₁₂-alkoxy, ―OJ₁, ―SJ₁, ―S(O)J₁ ―SO₂―J₁, ―NJ₁J₂, ―N₃, -CN, ―C(=O)OJ₁, ―C(=O)NJ₁J₂, ―C(=O)J₁, ―OC(=O)NJ₁J₂, ―NH―C(=NH)NJ₁J₂, ―NH―C(=O)NJ₁J₂, or ―NH―C(=S)NJ₁J₂; each J₁ and J₂ is independently of each other -H, C₁₋₆-alkyl, C₂₋₆-alkenyl, C₂₋₆-alkynyl, C₁₋₆-aminoalkyl or a protecting group; and optionally when Q is ―C(q₁)(q₂)C(q₃)(q₄)― and one of q₃ or q₄ is ―CH₃, then at least one of the other of q₃ or q₄ or one of q₁ and q₂ is other than -H. In preferred embodiments R¹, R², R³, R⁴, and R⁵ are hydrogen. For all chiral centers, asymmetric groups may be found in either R or S orientation. Such bicyclic nucleotides are disclosed in WO2008/154401 which is hereby incorporated by reference in its entirety. In preferred embodiments R¹, R², R³, R⁴, and R⁵ are independently of each other selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂₋₆-alkynyl, C₁₋₆-alkoxyl, substituted C₁₋₆-alkoxyl, acyl, substituted acyl, C₁₋₆-aminoalkyl or substituted C₁₋₆-aminoalkyl. In preferred embodiments R¹, R², R³, R⁴, and R⁵ are hydrogen. In preferred embodiments R¹, R², and R³ are hydrogen and one or both of R⁴, R⁵ may be other than hydrogen as referred to above and in WO 2007/134181 or WO2009/067647 (alpha-L-bicyclic nucleic acids analogues).

As used herein, the term "C₁-C₆-alkyl" refers to ―CH₃, ―C₂H₅, ―C₃H₇, ―CH(CH₃)₂, ―C₄H₉, ―CH₂―CH(CH₃)₂, ―CH(CH₃)―C₂H₅, ―C(CH₃)₃, ―C₅H₁₁, ―CH(CH₃)―C₃H₇, ―CH₂―CH(CH₃)―C₂H₅, ―CH(CH₃)―CH(CH₃)₂, ―C(CH₃)₂―C₂H₅, ―CH₂―C(CH₃)₃, ―CH(C₂H₅)₂, ―C₂H₄―CH(CH₃)₂, ―C₆H₁₃, ―C₃H₆―CH(CH₃)₂, ―C₂H₄―CH(CH₃)―C₂H₅, ―CH(CH₃)―C₄H₉, ―CH₂―CH(CH₃)―C₃H₇, ―CH(CH₃)―CH₂―CH(CH₃)₂, ―CH(CH₃)―CH(CH₃)―C₂H₅, ―CH₂―CH(CH₃)―CH(CH₃)₂, ―CH₂―C(CH₃)₂―C₂H₅, ―C(CH₃)₂―C₃H₇, ―C(CH₃)₂―CH(CH₃)₂, ―C₂H₄―C(CH₃)₃, ―CH₂―CH(C₂H₅)₂, and ―CH(CH₃)―C(CH₃)₃. The term "C₁-C₆-alkyl" shall also include "C₁-C₆-cycloalkyl" like cyclo-C₃H₅, cyclo-C₄H₇, cyclo-C₅H₉, and cyclo-C₆H₁₁.

Preferred are ―CH₃, ―C₂H₅, ―C₃H₇, ―CH(CH₃)₂, ―C₄H₉, ―CH₂―CH(CH₃)₂, ―CH(CH₃)―C₂H₅, ―C(CH₃)₃, and ―C₅H₁₁. Especially preferred are ―CH₃, ―C₂H₅, ―C₃H₇, and ―CH(CH₃)₂.

The term "C₁-C₆-alkyl" shall also include "C₁-C₆-cycloalkyl" like cyclo-C₃H₅, cyclo-C₄H₇, cyclo-C₅H₉, and cyclo-C₆H₁₁.

As used herein, the term "C₁-C₁₂-alkyl" refers to C₁-C₆-alkyl, ―C₇H₁₅, ―C₈H₁₇, ―C₉H₁₉, ―C₁₀H₂₁, ―C₁₁H₂₃, ―C₁₂H₂₅.

As used herein, the term "C₁-C₆-alkylenyl" refers to ―CH₂―, ―C₂H₄―, ―CH(CH₃)―, ―C₃H₆―, ―CH₂―CH(CH₃)―, ―CH(CH₃)―CH₂―, ―C(CH₃)₂―, ―C₄H₈―, ―CH₂―C(CH₃)₂―, ―C(CH₃)₂―CH₂―, ―C₂H₄―CH(CH₃)―, ―CH(CH₃)―C₂H₄―, ―CH₂―CH(CH₃)―CH₂―, ―CH(CH₃)―CH(CH₃)―, ―C₅H₁₀―, ―CH(CH₃)―C₃H₆―, ―CH₂―CH(CH₃)―C₂H₄―, ―C₂H₄―CH(CH₃)―CH₂―, ―C₃H₆―CH(CH₃)―, ―C₂H₄―C(CH₃)₂―, ―C(CH₃)₂―C₂H₄―, ―CH₂―C(CH₃)₂―CH₂―, ―CH₂―CH(CH₃)―CH(CH₃)―, ―CH(CH₃)―CH₂―CH(CH₃)―, ―CH(CH₃)―CH(CH₃)―CH₂―, ―CH(CH₃)―CH(CH₃)―CH(CH₃)―, ―C(CH₃)₂―C₃H₆―, ―CH₂―C(CH₃)₂―C₂H₄―, ―C₂H₄―C(CH₃)₂―CH₂―, ―C₃H₆―C(CH₃)₂―, ―CH(CH₃)―C₄H₈―, ―C₆H₁₂―, ―CH₂―CH(CH₃)―C₃H₆―, ―C₂H₄―CH(CH₃)―C₂H₄―, ―C₃H₆―CH(CH₃)―CH₂―, ―C₄H₈―CH(CH₃)―, ―C₂H₄―CH(CH₃)―CH(CH₃)―, ―CH₂―CH(CH₃)―CH(CH₃)―CH₂―, ―CH₂―CH(CH₃)―CH₂―CH(CH₃)―, ―CH(CH₃)―C₂H₄―CH(CH₃)―, ―CH(CH₃)―CH₂―CH(CH₃)―CH₂―, and ―CH(CH₃)―CH(CH₃)―C₂H₄―.

As used herein, the term "C₂-C₆-alkenyl" refers to ―CH=CH₂, ―CH₂―CH=CH₂, ―C(CH₃)=CH₂, ―CH=CH―CH₃, ―C₂H₄―CH=CH₂, ―CH₂―CH=CH―CH₃, ―CH=CH―C₂H₅, ―CH₂―C(CH₃)=CH₂, ―CH(CH₃)―CH=CH, ―CH=C(CH₃)₂, ―C(CH₃)=CH―CH₃, ―CH=CH―CH=CH₂, ―C₃H₆―CH=CH₂, ―C₂H₄―CH=CH―CH₃, ―CH₂―CH=CH―C₂H₅, ―CH=CH―C₃H₇, ―CH₂―CH=CH―CH=CH₂, ―CH=CH―CH=CH―CH₃, ―CH=CH―CH₂―CH=CH₂, ―C(CH₃)=CH―CH=CH₂, ―CH=C(CH₃)―CH=CH₂, ―CH=CH―C(CH₃)=CH₂, ―C₂H₄―C(CH₃)=CH₂, ―CH₂―CH(CH₃)―CH=CH₂, ―CH(CH₃)―CH₂―CH=CH₂, ―CH₂―CH=C(CH₃)₂, ―CH₂―C(CH₃)=CH―CH₃, ―CH(CH₃)―CH=CH―CH₃, ―CH=CH―CH(CH₃)₂, ―CH=C(CH₃)―C₂H₅, ―C(CH₃)=CH―C₂H₅, ―C(CH₃)=C(CH₃)₂, ―C(CH₃)₂―CH=CH₂, ―CH(CH₃)―C(CH₃)=CH₂, ―C(CH₃)=CH―CH=CH₂, ―CH=C(CH₃)―CH=CH₂, ―CH=CH―C(CH₃)=CH₂, ―C₄H₈―CH=CH₂, ―C₃H₆―CH=CH―CH₃, ―C₂H₄―CH=CH―C₂H₅, ―CH₂―CH=CH―C₃H₇, ―CH=CH―C₄H₉, ―C₃H₆―C(CH₃)=CH₂, ―C₂H₄―CH(CH₃)―CH=CH₂, ―CH₂―CH(CH₃)―CH₂―CH=CH₂, ―CH(CH₃)―C₂H₄―CH=CH₂, ―C₂H₄―CH=C(CH₃)₂, ―C₂H₄―C(CH₃)=CH―CH₃, ―CH₂―CH(CH₃)―CH=CH―CH₃, ―CH(CH₃)―CH₂―CH=CH―CH₃, ―CH₂―CH=CH―CH(CH₃)₂, ―CH₂―CH=C(CH₃)―C₂H₅, ―CH₂―C(CH₃)=CH―C₂H₅, ―CH(CH₃)―CH=CH―C₂H₅, ―CH=CH―CH₂―CH(CH₃)₂, ―CH=CH―CH(CH₃)―C₂H₅, ―CH=C(CH₃)―C₃H₇, ―C(CH₃)=CH―C₃H₇, ―CH₂―CH(CH₃)―C(CH₃)=CH₂, ―CH(CH₃)―CH₂―C(CH₃)=CH₂, ―CH(CH₃)―CH(CH₃)―CH=CH₂, ―CH₂―C(CH₃)₂―CH=CH₂, ―C(CH₃)₂―CH₂―CH=CH₂, ―CH₂―C(CH₃)=C(CH₃)₂, ―CH(CH₃)―CH=C(CH₃)₂, ―C(CH₃)₂―CH=CH―CH₃, ―CH(CH₃)―C(CH₃)=CH―CH₃, ―CH=C(CH₃)―CH(CH₃)₂, ―C(CH₃)=CH―CH(CH₃)₂, ―C(CH₃)=C(CH₃)―C₂H₅, ―CH=CH―C(CH₃)₃, ―C(CH₃)₂―C(CH₃)=CH₂, ―CH(C₂H₅)―C(CH₃)=CH₂, ―C(CH₃)(C₂H₅)―CH=CH₂, ―CH(CH₃)―C(C₂H₅)=CH₂, ―CH₂―C(C₃H₇)=CH₂, ―CH₂―C(C₂H₅)=CH―CH₃, ―CH(C₂H₅)―CH=CH―CH₃, ―C(C₄H₉)=CH₂, ―C(C₃H₇)=CH―CH₃, ―C(C₂H₅)=CH―C₂H₅, ―C(C₂H₅)=C(CH₃)₂, ―C[C(CH₃)_{3]}=CH₂, ―C[CH(CH₃)(C₂H₅)]=CH₂, ―C[CH₂―CH(CH₃)_{2]}=CH₂, ―C₂H₄―CH=CH―CH=CH₂, ―CH₂―CH=CH―CH₂―CH=CH₂, ―CH=CH―C₂H₄―CH=CH₂, ―CH₂―CH=CH―CH=CH―CH₃, ―CH=CH―CH₂―CH=CH―CH₃, ―CH=CH―CH=CH―C₂H₅, ―CH₂―CH=CH―C(CH₃)=CH₂, ―CH₂―CH=C(CH₃)―CH=CH₂, ―CH₂―C(CH₃)=CH―CH=CH₂, ―CH(CH₃)―CH=CH―CH=CH₂, ―CH=CH―CH₂―C(CH₃)=CH₂, ―CH=CH―CH(CH₃)―CH=CH₂, ―CH=C(CH₃)―CH₂―CH=CH₂, ―C(CH₃)=CH―CH₂―CH=CH₂, ―CH=CH―CH=C(CH₃)₂, ―CH=CH―C(CH₃)=CH―CH₃, ―CH=C(CH₃)―CH=CH―CH₃, ―C(CH₃)=CH―CH=CH―CH₃, ―CH=C(CH₃)―C(CH₃)=CH₂, ―C(CH₃)=CH―C(CH₃)=CH₂, ―C(CH₃)=C(CH₃)―CH=CH₂, and ―CH=CH―CH=CH―CH=CH₂.

Preferred are ―CH=CH₂, ―CH₂―CH=CH₂, ―C(CH₃)=CH₂, ―CH=CH―CH₃, ―C₂H₄―CH=CH₂, ―CH₂―CH=CH―CH₃. Especially preferred are ―CH=CH₂, ―CH₂―CH=CH₂, and ―CH=CH―CH₃.

As used herein, the term "C₂-C₆-alkynyl" refers to -C=CH, ―C≡C―CH₃, ―CH₂―C≡CH, ―C₂H₄―C≡CH, ―CH₂―C≡C―CH₃, ―C≡C―C₂H₅, ―C₃H₆―C≡CH, ―C₂H₄―C≡C―CH₃, ―CH₂―C≡C―C₂H₅, ―C≡C―C₃H₇, ―CH(CH₃)―C≡CH, ―CH₂―CH(CH₃)―C≡CH, ―CH(CH₃)―CH₂―C≡CH, -CH(CH₃)―C≡C―CH₃, ―C₄H₈―C≡CH, ―C₃H₆―C≡C―CH₃, ―C₂H₄―C≡C―C₂H₅, ―CH₂―C≡C―C₃H₇, ―C≡C―C₄H₉, ―C₂H₄―CH(CH₃)―C≡CH, ―CH₂―CH(CH₃)―CH₂―C≡CH, ―CH(CH₃)―C₂H₄―C≡CH, ―CH₂―CH(CH₃)―C≡C―CH₃, ―CH(CH₃)―CH₂―C≡C―CH₃, ―CH(CH₃)―C≡C―C₂H₅, ―CH₂―C≡C―CH(CH₃)₂, ―C≡C―CH(CH₃)―C₂H₅, ―C≡C―CH₂―CH(CH₃)₂, ―C≡C―C(CH₃)₃, ―CH(C₂H₅)―C≡C―CH₃, ―C(CH₃)₂―C≡C―CH₃, ―CH(C₂H₅)―CH₂―C≡CH, ―CH₂―CH(C₂H₅)―C≡CH, ―C(CH₃)₂―CH₂―C≡CH, ―CH₂―C(CH₃)₂―C≡CH, ―CH(CH₃)―CH(CH₃)―C≡CH, ―CH(C₃H₇)―C≡CH, ―C(CH₃)(C₂H₅)―C≡CH, -C=C-C=CH, ―CH₂―C≡C―C≡CH, ―C≡C―C≡C―CH₃, ―CH(C≡CH)₂, ―C₂H₄―C≡C―C≡CH, ―CH₂―C≡C―CH₂―C≡CH, ―C=C―C₂H₄―C≡CH, ―CH₂―C≡C―C≡C―CH₃, ―C≡C―CH₂―C≡C―CH₃, ―C≡C―C≡C―C₂H₅, ―C≡C―CH(CH₃)―C≡CH, ―CH(CH₃)―C≡C―C≡CH, ―CH(C≡CH)―CH₂―C≡CH, ―C(C≡CH)₂―CH₃, ―CH₂―CH(C≡CH)₂, ―CH(C≡CH)―C≡C―CH₃. Preferred are -C=CH and ―C≡C―CH₃.

The term "C₁₋₆-alkoxyl" refers to "C₁-C₆-alkyl-O―". The term "C₁₋₁₂-alkoxyl" refers to "C₁-C₁₂-alkyl-O―". The term "C₁₋₆-aminoalkyl" refers to "H₂N―C₁-C₆-alkyl―". The term "C₂-C₆-alkenyloxy" refers to "C₂-C₆-alkenyl―O―". The term "C₁₋₆-alkylcarbonyl" refers to "C₁-C₆-alkyl-CO―". Also referred to as "acyl". The term "C₁₋₁₂-alkylcarbonyl" refers to "C₁-C₁₂-alkyl-CO―". Also referred to as "acyl". The term "C₁₋₆-alkoxycarbonyl" refers to "C₁-C₆-alkyl-O―CO―". The term "C₁₋₁₂-alkoxycarbonyl" refers to "C₁-C₁₂-alkyl-O―CO―". The term "C₁-C₆-alkanoyloxy" refers to "C₁-C₆-alkyl-CO―O―". The term "C₁₋₆-alkylthio" refers to "C₁-C₆-alkyl―S―". The term "C₁₋₆-alkylsulphonyloxy" refers to "C₁-C₆-alkyl―SO₂―O―". The term "C₁₋₆-alkylcarbonylamino" refers to "C₁-C₆-alkyl―CO―NH―". The term "C₁₋₆-alkylamino" refers to "C₁-C₆-alkyl―NH―". The term "(C₁₋₆-)₂alkylamino" refers to a dialkylamino group like "[C₁-C₆-alkyl][C₁-C₆-alkyl]N―". The term "C₁₋₆-alkylaminocarbonyl" refers to "C₁-C₆-alkyl―NH―CO―" The term "(C₁₋₆-)₂alkylaminocarbonyl" refers to a dialkylaminocarbonyl group like "[C₁-C₆-alkyl][C₁-C₆-alkyl]N―CO―". The term "amino-C₁₋₆-alkylaminocarbonyl" refers to "H₂N―[C₁-C₆-alkylenyl]―NH―CO―". The term "C₁₋₆-alkyl-amino-C₁₋₆-alkylaminocarbonyl" refers to "C₁₋₆-alkyl―HN―[C₁-C₆-alkylenyl]―NH―CO―". The term "(C₁₋₆-)₂alkyl-amino-C₁₋₆-alkylaminocarbonyl" refers to "[C₁-C₆-alkyl][C₁-C₆-alkyl]N―[C₁-C₆-alkylenyl]―NH―CO―". The term "aryl" refers to phenyl, toluyl, substituted phenyl and substituted toluyl. The term "aryloxy" refers to "aryl―O―". The term "arylcarbonyl" refers to "aryl-CO-". The term "aryloxycarbonyl" refers to "aryl-O-CO-".

The term "heteroaryl" refers to substituted or not substituted heteroaromatic groups which have from 4 to 9 ring atoms, from 1 to 4 of which are selected from O, N and/or S. Preferred "heteroaryl" groups have 1 or 2 heteroatoms in a 5- or 6-membered aromatic ring. Mono and bicyclic ring systems are included. Typical "heteroaryl" groups are pyridyl, furyl, thienyl, pyrrolyl, oxazolyl, thiazolyl, imidazolyl, isoxazolyl, isothiazolyl, oxadiazolyl, pyridazinyl, pyrimidyl, pyrazinyl, 1,3,5-triazinyl, 1,2,3-triazolyl, 1,3,4-thiadiazolyl, indolizinyl, indolyl, isoindolyl, benzo[b]furyl, benzo[b]thienyl, indazolyl, benzimidazolyl, benzthiazolyl, purinyl, quinolizinyl, quinolyl, isoquinolyl, quinazolinyl, quinoxalinyl, 1,8-naphthyridinyl, tetrahydroquinolyl, benzooxazolyl, chrom-2-onyl, indazolyl, and the like.

The term "heteroaryloxy" refers to "heteroaryl-O-". The term "heteroarylcarbonyl" refers to "heteroaryl-CO-". The term "heteroaryloxycarbonyl" refers to "heteroaryl―O―CO―".

The term "substituted" refers to groups wherein one or more hydrogen atoms are replaced by one or more of the following substituents: -OH, ―OCH₃, ―OC₂H₅, ―OC₃H₇, ―O―cyclo-C₃H₅, ―OCH(CH₃)₂, ―OCH₂Ph, -F, -CI, ―COCH₃, ―COC₂H₅, ―COC₃H₇, ―CO―cyclo-C₃H₅, ―COCH(CH₃)₂, -COOH, ―CONH₂, ―NH₂, ―NHCH₃, ―NHC₂H₅, ―NHC₃H₇, ―NH―cyclo-C₃H₅, ―NHCH(CH₃)₂, ―N(CH₃)₂, ―N(C₂H₅)₂, ―N(C₃H₇)₂, ―N(cyclo-C₃H₅)₂, ―N[CH(CH₃)_{2]2}, ―SO₃H, ―OCF₃, ―OC₂F₅, cyclo-C₃H₅, ―CH₃, ―C₂H₅, ―C₃H₇, ―CH(CH₃)₂, ―CH=CH₂, ―CH₂―CH=CH₂, -C=CH and/or ―C≡C―CH₃.

In case the general structure (I) represents monomers or building blocks for synthesizing the antisense-oligonucleotides of the present invention, the terminal groups Y and IL' are selected independently of each other from hydrogen, azido, halogen, cyano, nitro, hydroxy, PG-O-, AG-O-, mercapto, PG-S-, AG-S-, C₁₋₆-alkylthio, amino, PG-N(R^{H})-, AG-N(R^{H})-, mono- or di(C₁₋₆-alkyl)amino, optionally substituted C₁₋₆-alkoxy, optionally substituted C₁₋₆-alkyl, optionally substituted C₂₋₆-alkenyl, optionally substituted C₂₋₆-alkenyloxy, optionally substituted C₂₋₆-alkynyl, optionally substituted C₂₋₆-alkynyloxy, monophosphate, monothiophosphate, diphosphate, dithiophosphate triphosphate, trithiophosphate, carboxy, sulphono, hydroxymethyl, PG-O-CH₂-, AG-O-CH₂-, aminomethyl, PG-N(R^{H})-CH₂-, AG-N(R^{H})-CH₂-, carboxymethyl, sulphonomethyl, where PG is a protection group for -OH, -SH, and -NH(R^{H}), respectively, AG is an activation group for -OH, -SH, and -NH(R^{H}), respectively, and R^{H} is selected from hydrogen and C₁₋₆- alkyl.

The protection groups PG of hydroxy substituents comprise substituted trityl, such as 4,4'-dimethoxytrityl (DMT), 4-monomethoxytrityl (MMT), optionally substituted 9-(9-phenyl)xanthenyl (pixyl), optionally substituted methoxytetrahydropyranyl (mthp), silyl such as trimethylsilyl (TMS), triisopropylsilyl (TIPS), tert-butyldimethylsilyl (TBDMS), triethylsilyl, and phenyldimethylsilyl, tert-butylethers, acetals (including two hydroxy groups), acyl such as acetyl or halogen substituted acetyls, e.g. chloroacetyl or fluoroacetyl, isobutyryl, pivaloyl, benzoyl and substituted benzoyls, methoxymethyl (MOM), benzyl ethers or substituted benzyl ethers such as 2,6-dichlorobenzyl (2,6-Cl₂Bzl). Alternatively when Y or IL' is hydroxyl they may be protected by attachment to a solid support optionally through a linker.

When Y or IL' is an amino group, illustrative examples of the amino protection groups are fluorenylmethoxycarbonyl (Fmoc), tert-butyloxycarbonyl (BOC), trifluoroacetyl, allyloxycarbonyl (alloc or AOC), benzyloxycarbonyl (Z or Cbz), substituted benzyloxycarbonyls such as 2-chloro benzyloxycarbonyl (2-CIZ), monomethoxytrityl (MMT), dimethoxytrityl (DMT), phthaloyl, and 9-(9-phenyl)xanthenyl (pixyl).

Act represents an activation group for -OH, -SH, and -NH(R^{H}), respectively. Such activation groups are, for instance, selected from optionally substituted O-phosphoramidite, optionally substituted O-phosphortriester, optionally substituted O-phosphordiester, optionally substituted H-phosphonate, and optionally substituted O-phosphonate.

In the present context, the term "phosphoramidite" means a group of the formula -P(OR^{x})-N(R^{y})₂, wherein R^{x} designates an optionally substituted alkyl group, e.g. methyl, 2-cyanoethyl, or benzyl, and each of R^{y} designate optionally substituted alkyl groups, e.g. ethyl or isopropyl, or the group -N(R^{y})₂ forms a morpholino group (-N(CH₂CH₂)₂O). R^{x} preferably designates 2-cyanoethyl and the two R^{y} are preferably identical and designate isopropyl. Thus, an especially relevant phosphoramidite is N,N-diisopropyl-O-(2-cyanoethyl)-phosphoramidite.

### LNA monomers or LNA building blocks

The LNA monomers or LNA building blocks used as starting materials in the synthesis of the antisense-oligonucleotides of the present invention are preferably LNA nucleosides of the following general formulae:

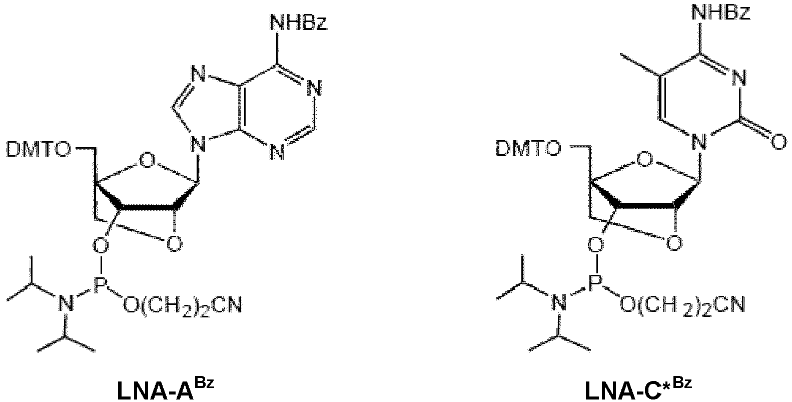

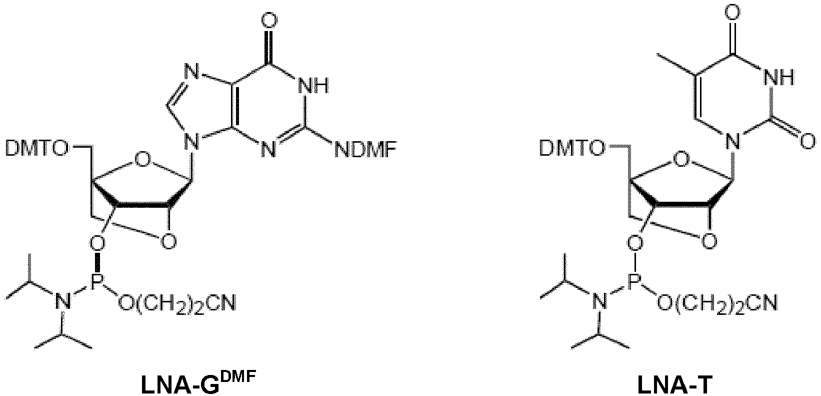

The LNA building blocks are normally provided as LNA phosphoramidites with the four different nucleobases: adenine (A), guanine (G), 5-methyl-cytosine (C*) and thymine (T). The antisense-oligonucleotides of the present invention containing LNA units are synthesized by standard phosphoramidite chemistry. In the LNA building blocks the nucleobases are protected. A preferred protecting group for the amino group of the purin base is a benzoyl group (Bz), indicated as A^{Bz}. A preferred protecting group for the amino group of the 5-methylpyrimidinone base is a benzoyl group (Bz), indicated as C*^{Bz}. A preferred protecting group for the amino group of the purinone base is a dimethylformamidine (DMF) group, a diethylformamidine (DEF), a dipropylformamidine (DPF), a dibutylformamidine (DBF), or a iso-butyryl (-CO-CH(CH₃)₂) group, indicated as G^{DMF}, G^{DEF}, G^{DPF}, G^{DBF}, or G^{iBu}. Thus the group -NDMF refers to ―N=CH―N(CH₃)₂. DMT refers to 4,4'-dimethoxytrityl.

Thus, **LNA-T** refers to 5'-O-(4,4'-dimethoxytrityl)-3'-O-(2-cyanoethyl-N,N-diisopropyl)-phosphoramidite-thymidine LNA. **LNA-C*^{Bz}** refers to 5'-O-(4,4'-dimethoxytrityl)-3'- O-(2-cyanoethyl-N,N-diisopropyl)phosphoramidite-4-N-benzoyl-5-methyl-2'-cytidine LNA. **LNA-A^{Bz}** refers to 5'-O-(4,4'-dimethoxytrityl)-3'-O-(2-cyanoethyl-N,N-diisopropyl)phosphoramidite-6-N-benzoyl-2'-adenosine LNA. **LNA-G^{DMF}** refers to 5'-O-(4,4'-dimethoxytrityl)-3'-O-(2-cyanoethyl-N,N-diisopropyl)-phosphoramidite-2-N-dimethylformamidine-2'-guanosine LNA. **LNA-G^{iBu}** refers to 5'-O-(4,4'-dimethoxytrityl)-3'-O-(2-cyanoethyl-N,N-diisopropyl)phosphoramidite-2-N-butyryl-2'-guanosin e LNA.

### Terminal groups

In case Y represents the 5'-terminal group of an antisense-oligonucleotide of the present invention, the residue Y is also named Y^{5'} and represents:
-OH, ―O-C₁₋₆-alkyl, ―S-C₁₋₆-alkyl, ―O-C₆₋₉-phenyl, ―O-C₇₋₁₀-benzyl, ―NH-C₁₋₆-alkyl, ―N(C₁₋₆-alkyl)₂, ―O-C₂₋₆-alkenyl, ―S-C₂₋₆-alkenyl, ―NH-C₂₋₆-alkenyl, ―N(C₂₋₆-alkenyl)₂, ―O-C₂₋₆-alkynyl, ―S-C₂₋₆-alkynyl, ―NH-C₂₋₆-alkynyl, ―N(C₂₋₆-alkynyl)₂, ―O-C₁₋₆-alkylenyl―O-C₁₋₆-alkyl, ―O-[C₁₋₆-alkylenyl―O]ₘ-C₁₋₆-alkyl, ―O-CO-C-₁₋₆-alkyl, ―O-CO-C₂₋₆-alkenyl, ―O-CO-C₂₋₆-alkynyl, ―O-S(O)-C₁₋₆-alkyl, ―O-SO₂-C₁₋₆-alkyl, ―O-SO₂-O-C₁₋₆-alkyl, ―O-P(O)(O⁻)₂, ―O-P(O)(O⁻)(O-C₁₋₆-alkyl), ―O-P(O)(O-C₁₋₆-alkyl)₂, ―O-P(O)(S⁻)₂, ―O-P(O)(S-C₁₋₆-alkyl)₂, ―O-P(O)(S⁻)(O-C₁₋₆-alkyl), ―O-P(O)(O⁻)(NH-C₁₋₆-alkyl), -O-P(O)(O-C₁₋₆-alkyl)(NH-C₁₋₆-alkyl), ―O-P(O)(O⁻)[N(C₁₋₆-alkyl)₂], -O-P(O)(O-C₁₋₆-alkyl)[N(C₁₋₆-alkyl)₂], ―O-P(O)(O⁻)(BH₃⁻), -O-P(O)(O-C₁₋₆-alkyl)(BH₃⁻), ―O-P(O)(O⁻)(O-C₁₋₆-alkylenyl-O-C₁₋₆-alkyl), ―O-P(O)(O-C₁₋₆-alkylenyl-O-C₁₋₆-alkyl)₂, ―O-P(O)(O⁻)(O-C₁₋₆-alkylenyl-S-C₁₋₆-alkyl), -O-P(O)(O-C₁₋₆-alkylenyl-S-C₁₋₆-alkyl)₂, ―O-P(O)(O⁻)(OCH₂CH₂O-C₁₋₆-alkyl), -O-P(O)(OCH₂CH₂O-C₁₋₆-alkyl)₂, ―O-P(O)(O⁻)(OCH₂CH₂S-C₁₋₆-alkyl), ―O-P(O)(OCH₂CH₂S-C₁₋₆-alkyl)₂, ―O-P(O)(O⁻)OC₃H₆OH, ―O-P(O)(S⁻)OC₃H₆OH, ―O-P(S)(S⁻)OC₃H₆OH, wherein the C₁₋₆-alkyl, C₂₋₆-alkenyl, C₂₋₆-alkynyl, ―O-C₆₋₉-phenyl or ―O-C₇₋₁₀-benzyl may be further substituted by -F, -OH, C₁₋₄-alkyl, C₂₋₄-alkenyl and/or C₂₋₄-alkynyl where m is selected from 1, 2, 3, 4, 5, 6, 7, 8, 9 or 10.

More preferred are: ―OCH₃, ―OC₂H₅, ―OC₃H₇, ―O-cyclo-C₃H₅, ―OCH(CH₃)₂, ―OC(CH₃)₃, ―OC₄H₉, -OPh, ―OCH₂―Ph, ―O-COCH₃, ―O-COC₂H₅, ―O-COC₃H₇, ―O-CO―cyclo-C₃H₅, ―O-COCH(CH₃)₂, ―OCF₃, ―O-S(O)CH₃, ―O-S(O)C₂H₅, ―O-S(O)C₃H₇, ―O-S(O)-cyclo-C₃H₅, ―O-SO₂CH₃, ―O-SO₂C₂H₅, ―O-SO₂C₃H₇, ―O-SO₂-cyc!o-C₃H₅, ―O-SO₂-OCH₃, ―O-SO₂-OC₂H₅, ―O-SO₂-OC₃H₇, ―O-SO₂-O-cyclo-C₃H₅, ―O(CH₂)ₙN[(CH₂)ₙOH], ―O(CH₂)ₙN[(CH₂)ₙ-H], ―O-P(O)(O⁻)OC₃H₆OH, ―O-P(O)(S⁻)O₃H₆OH,
even more preferred are:
   ―OCH₃, ―OC₂H₅, ―OCH₂CH₂OCH₃ (also known as MOE), ―OCH₂CH₂-N(CH₃)₂ (also known as DMAOE), ―O[(CH₂)ₙO]ₘCH₃, ―O(CH₂)ₙOCH₃, ―O(CH₂)ₙNH₂, ―O(CH₂)ₙN(CH₃)₂, , ―O-P(O)(O⁻)OC₃H₆OH, ―O-P(O)(S⁻)O₃H₆OH,
where n is selected from 1, 2, 3, 4, 5, or 6; and
where m is selected from 1, 2, 3, 4, 5, 6, 7, 8, 9 or 10.

In case IL' represents the 3'-terminal group of an antisense-oligonucleotide of the present invention, the residue IL' is also named IL^{,3'} and represents:
-OH, ―O-C₁₋₆-alkyl, ―S-C₁₋₆-alkyl, ―O-C₆₋₉-phenyl, ―O-C₇₋₁₀-benzyl, ―NH-C₁₋₆-alkyl, ―N(C₁₋₆-alkyl)₂, -O-C₂₋₆-alkenyl, ―S-C₂₋₆-alkenyl, ―NH-C₂₋₆-alkenyl, ―N(C₂₋₆-alkenyl)₂, ―O-C₂₋₆-alkynyl, ―S-C₂₋₆-alkynyl, ―NH-C₂₋₆-alkynyl, ―N(C₂₋₆-alkynyl)₂, ―O-C₁₋₆-alkylenyl―O-C₁₋₆-alkyl, ―O-[C₁₋₆-alkylenyl―O]ₘ-C₁₋₆-alkyl, ―O-CO-C₁₋₆-alkyl, ―O-CO-C₂₋₆-alkenyl, ―O-CO-C₂₋₆-alkynyl, ―O-S(O)-C₁₋₆-alkyl, ―O-SO₂-C₁₋₆-alkyl, ―O-SO₂-O-C₁₋₆-alkyl, ―O-P(O)(O⁻)₂, ―O-P(O)(O⁻)(O-C₁₋₆-alkyl), ―O-P(O)(O-C₁₋₆-alkyl)₂, ―O-P(O)(S⁻)₂, ―O-P(O)(S-C₁₋₆-alkyl)₂, ―O-P(O)(S⁻)(O-C₁₋₆-alkyl), ―O-P(O)(O⁻)(NH-C₁₋₆-alkyl), ―O-P(O)(O-C₁₋₆-alkyl)(NH-C₁₋₆-alkyl), ―O-P(O)(O⁻)[N(C₁₋₆-alkyl)₂], ―O-P(O)(O-C₁₋₆-alkyl)[N(C₁₋₆-alkyl)₂], ―O-P(O)(O⁻)(BH₃⁻), ―O-P(O)(O-C₁₋₆-alkyl)(BH₃⁻), ―O-P(O)(O⁻)(O-C₁₋₆-alkylenyl-O-C₁₋₆-alkyl), ―O-P(O)(O-C₁₋₆-alkylenyl-O-C₁₋₆-alkyl)₂, ―O-P(O)(O⁻)(O-C₁₋₆-alkylenyl-S-C₁₋₆-alkyl), -O-P(O)(O-C₁₋₆-alkylenyl-S-C₁₋₆-alkyl)₂, ―O-P(O)(O⁻)(OCH₂CH₂O-C₁₋₆-alkyl), -O-P(O)(OCH₂CH₂O-C₁₋₆-alkyl)₂, ―O-P(O)(O⁻)(OCH₂CH₂S-C₁₋₆-alkyl), ―O-P(O)(OCH₂CH₂S-C₁₋₆-alkyl)₂, ―O-P(O)(O⁻)OC₃H₆OH, ―O-P(O)(S⁻)OC₃H₆OH, wherein the C₁₋₆-alkyl, C₂₋₆-alkenyl, C₂₋₆-alkynyl, ―O-C₆₋₉-phenyl or ―O-C₇₋₁₀-benzyl may be further substituted by -F, -OH, C₁₋₄-alkyl, C₂₋₄-alkenyl and/or C₂₋₄-alkynyl where m is selected from 1, 2, 3, 4, 5, 6, 7, 8, 9 or 10.

More preferred are: ―OCH₃, ―OC₂H₅, ―OC₃H₇, ―O-cyclo-C₃H₅, ―OCH(CH₃)₂, ―OC(CH₃)₃, ―OC₄H₉, -OPh, ―OCH₂―Ph, ―O-COCH₃, ―O-COC₂H₅, ―O-COC₃H₇, ―O-CO-cyclo-C₃H₅, ―O-COCH(CH₃)₂, ―OCF₃, ―O-S(O)CH₃, ―O-S(O)C₂H₅, ―O-S(O)C₃H₇, ―O-S(O)―cyclo-C₃H₅, ―O-SO₂CH₃, ―O-SO₂C₂H₅, ―O-SO₂C₃H₇, ―O-SO₂―cyclo-C₃H₅, ―O-SO₂-OCH₃, ―O-SO₂-OC₂H₅, ―O-SO₂-OC₃H₇, ―O-SO₂-O-cyclo-C₃H₅, ―O(CH₂)ₙN[(CH₂)ₙOH], ―O(CH₂)ₙN[(CH₂)ₙ-H], , ―O-P(O)(O⁻)OC₃H₆OH, ―O-P(O)(S⁻)O₃H₆OH,
even more preferred are:
   ―OCH₃, ―OC₂H₅, ―OCH₂CH₂OCH₃ (also known as MOE), ―OCH₂CH₂-N(CH₃)₂ (also known as DMAOE), ―O[(CH₂)ₙO]ₘCH₃, ―O(CH₂)ₙOCH₃, ―O(CH₂)ₙNH₂, ―O(CH₂)ₙN(CH₃)₂, ―O-P(O)(O⁻)OC₃H₆OH, ―O-P(O)(S⁻)OC₃H₆OH,
where n is selected from 1, 2, 3, 4, 5, or 6; and
where m is selected from 1, 2, 3, 4, 5, 6, 7, 8, 9 or 10.

### Preferred LNAs

In preferred embodiments LNA units used in the antisense-oligonucleotides of the present invention preferably have the structure of general formula (II):

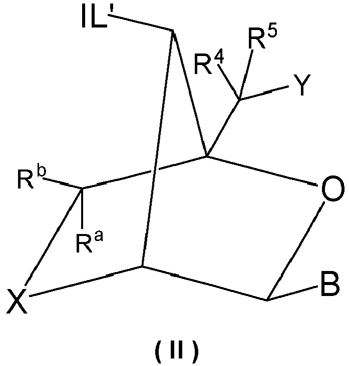

The moiety ―C(R^{a}R^{b})―X― represents preferably ―C(R^{a}R^{b})―O―, ―C(R^{a}R^{b})―NR^{c}―, ―C(R^{a}R^{b})―S―, and ―C(R^{a}R^{b})―C(R^{a}R^{b})―O―, wherein the substituents R^{a}, R^{b} and R^{c} have the meanings as defined herein and are preferably C₁₋₆-alkyl and more preferably C₁₋₄-alkyl. More preferably ―C(R^{a}R^{b})―X― is selected from ―CH₂―O―, ―CH₂―S―, ―CH₂―NH―, ―CH₂―N(CH₃)―, ―CH₂―CH₂―O―, or ―CH₂―CH₂―S―, and more preferably ―CH₂―O―, ―CH₂―S―, ―CH₂―CH₂―O―, or ―CH₂―CH₂―S―, and still more preferably ―CH₂―O―, ―CH₂―S―, or ―CH₂―CH₂―O―, and still more preferably ―CH₂―O― or ―CH₂―S―, and most preferably ―CH₂―O―.

All chiral centers and asymmetric substituents (if any) can be either in R or in S orientation. For example, two exemplary stereochemical isomers are the beta-D and alpha-L isoforms as shown below:

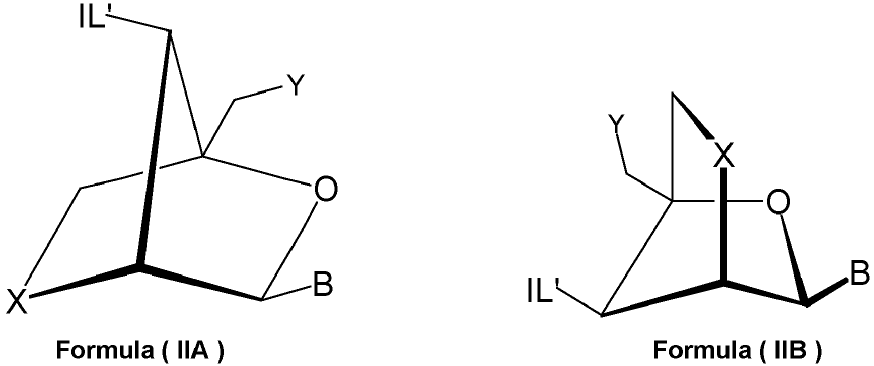

Preferred LNA units are selected from general formula (b¹) to (b⁹):

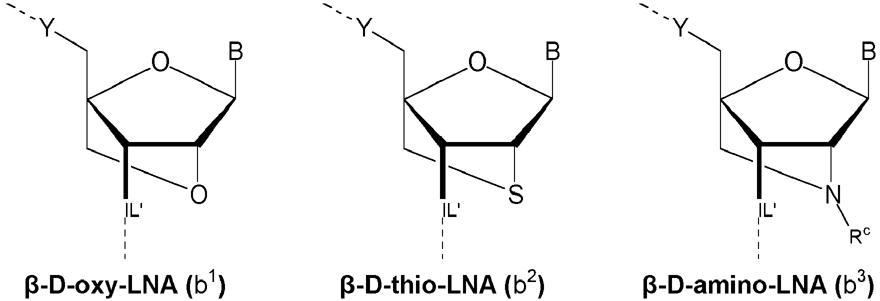

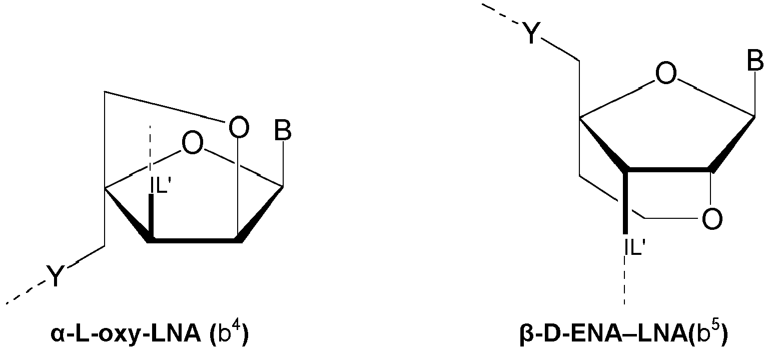

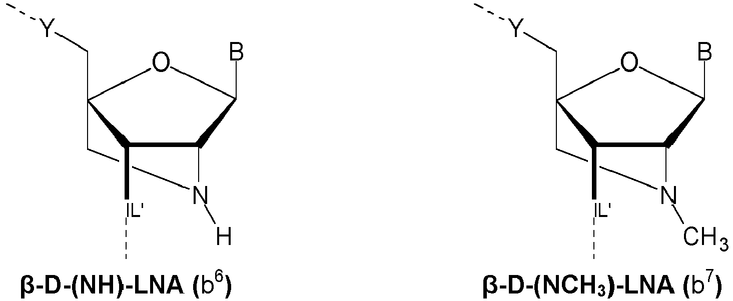

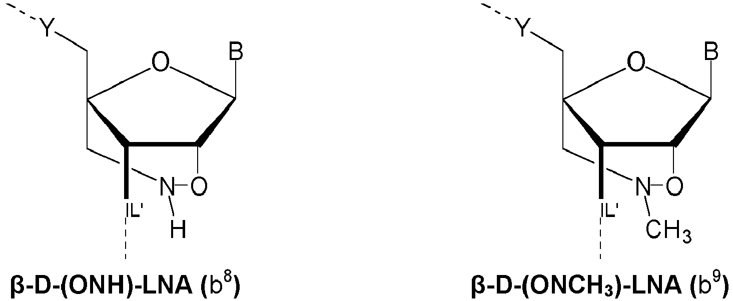

The term "thio-LNA" comprises a locked nucleotide in which X in the general formula (II) is selected from -S- or ―CH₂―S―. Thio-LNA can be in both beta-D and alpha-L-configuration.

The term "amino-LNA" comprises a locked nucleotide in which X in the general formula (II) is selected from -NH-, -N(R)-, ―CH₂―NH―, and ―CH₂―N(R)―, where R is selected from hydrogen and C₁₋₄-alkyl. Amino-LNA can be in both beta-D and alpha-L-configuration.

The term "oxy-LNA" comprises a locked nucleotide in which X in the general formula (II) is ―O―. Oxy-LNA can be in both beta-D and alpha-L-configuration.

The term "ENA" comprises a locked nucleotide in which X in the general formula (II) is ―CH₂―O― (where the oxygen atom of ―CH₂―O― is attached to the 2'-position relative to the base B). R^{a} and R^{b} are independently of each other hydrogen or methyl.

In preferred exemplary embodiments LNA is selected from beta-D-oxy-LNA, alpha-L-oxy-LNA, beta-D-amino-LNA and beta-D-thio-LNA, in particular beta-D-oxy-LNA.

### Gapmers

The antisense-oligonucleotides of the invention may consist of nucleotide sequences which comprise both DNA nucleotides which are non-LNA units as well as LNA nucleotides, and may be arranged in the form of a gapmer. Herein preferred are LNA gapmers having fully phosphorothioated backbones. In general phosphorothioated backbones ensure exceptional resistance to enzymatic degradation.

Thus, the antisense-oligonucleotides of the present invention are preferably gapmers. A gapmer consists of a middle part of DNA nucleotide units which are not locked, thus which are non-LNA units. The DNA nucleotides of this middle part could be linked to each other by the internucleotide linkages (IL) as disclosed herein which preferably may be phosphate groups, phosphorothioate groups or phosphorodithioate groups and which may contain nucleobase analogues such as 5-propynyl cytosine, 7-methylguanine, 7-methyladenine, 2-aminoadenine, 2-thiothymine, 2-thiocytosine, or 5-methylcytosine. That DNA units or DNA nucleotides are not bicyclic pentose structures. The middle part of non-LNA units is flanked at the 3' end and the 5' end by sequences consisting of LNA units. Thus gapmers have the general formula:

### LNA sequence 1 - non-LNA sequence ― LNA sequence 2 or region A ― region B - region C

The middle part of the antisense-oligonucleotide which consists of DNA nucleotide units which are non-LNA units is, when formed in a duplex with the complementary target RNA, capable of recruiting RNase. The 3' and 5' terminal nucleotide units are LNA units which are preferably in alpha-L configuration, particularly preferred being beta-D-oxy-LNA and alpha-L-oxy LNAs.

Thus, a gapmer is an antisense-oligonucleotide which comprises a contiguous stretch of DNA nucleotides which is capable of recruiting an RNase, such as RNaseH, such as a region of at least 6 or 7 DNA nucleotides which are non-LNA units, referred to herein as middle part or region B, wherein region B is flanked both 5' and 3' by regions of affinity enhancing nucleotide analogues which are LNA units, such as between 1 - 6 LNA units 5' and 3' to the contiguous stretch of DNA nucleotides which is capable of recruiting RNase - these flanking regions are referred to as regions A and C respectively.

Preferably the gapmers comprises a (poly)nucleotide sequence of formula (5' to 3'), A-B-C, or optionally A-B-C-D or D-A-B-C, wherein; region A (5' region) consists of at least one nucleotide analogue, such as at least one LNA unit, such as between 1-6 LNA units, and region B consists of at least five consecutive DNA nucleotides which are non-LNA units and which are capable of recruiting RNase (when formed in a duplex with a complementary RNA molecule, such as the mRNA target), and region C (3'region) consists of at least one nucleotide analogue, such as at least one LNA unit, such as between 1-6 LNA units, and region D, when present consists of 1, 2 or 3 DNA nucleotide units which are non-LNA units.

In some embodiments, region A consists of 1, 2, 3, 4, 5 or 6 LNA units, such as between 2-5 LNA units, such as 2 or 3 LNA units; and/or region C consists of 1, 2, 3, 4, 5 or 6 LNA units, such as between 2-5 LNA units, such as 2 or 3 LNA units.

In some embodiments B consists of 5, 6, 7, 8, 9, 10, 11 or 12 consecutive DNA nucleotides which are capable of recruiting RNase, or between 6-10, or between 7-9, such as 8 consecutive nucleotides which are capable of recruiting RNase. In some embodiments region B consists of at least one DNA nucleotide unit, such as 1-12 DNA nucleotide units, preferably between 4-12 DNA nucleotide units, more preferably between 6-10 DNA nucleotide units, still more preferred such as between 7-10 DNA nucleotide units, and most preferably 8, 9 or 10 DNA nucleotide units which are non-LNA units.

In some embodiments region A consist of 1 to 4 LNA, region B consists of 7, 8, 9, 10 or 11 DNA nucleotide units, and region C consists of 1 to 4 LNA units. Such designs include (A-B-C): 1-7-2, 2-7-1, 2-7-2, 3-7-1, 3-7-2, 1-7-3, 2-7-3, 3-7-3, 2-7-4, 3-7-4, 4-7-2, 4-7-3, 4-7-4, 1-8-1, 1-8-2, 2-8-1, 2-8-2, 1-8-3, 3-8-1, 3-8-3, 2-8-3, 3-8-2, 4-8-1, 4-8-2, 1-8-4, 2-8-4, 3-8-4, 4-8-3, 4-8-4, 1-9-1, 1-9-2, 2-9-1, 2-9-2, 2-9-3, 3-9-2, 3-9-3, 1-9-3, 3-9-1, 4-9-1, 1-9-4, 4-9-2, 2-9-4, 4-9-3, 3-9-4, 4-9-4, 1-10-1, 1-10-2, 2-10-1, 2-10-2, 1-10-3, 3-10-1, 2-10-2, 2-10-3, 3-10-2, 3-10-3, 2-10-4, 4-10-2, 3-10-4, 4-10-3, 4-10-4, 1-11-1, 1-11-2, 2-11-1, 2-11-2, 1-11-3, 3-11-1, 2-11-2, 2-11-3, 3-11-2, 3-11-3, 2-11-4, 4-11-2, 3-11-4, 4-11-3, 4-11-4, and may further include region D, which may have one or 2 DNA nucleotide units, which are non-LNA units. Further gapmer designs are disclosed in WO2004/046160A and are hereby incorporated by reference.

In some embodiments the antisense-oligonucleotide consists of a contiguous nucleotide sequence of a total of 10, 11, 12, 13 or 14 nucleotide units (LNA units and non-LNA units together), wherein the contiguous nucleotide sequence is of formula (5' - 3'), A-B-C, or optionally A-B-C-D or D-A-B-C, wherein A consists of 1, 2 or 3 LNA units, and B consists of 7, 8 or 9 contiguous DNA nucleotide units which are non-LNA units and which are capable of recruiting RNase when formed in a duplex with a complementary RNA molecule (such as a mRNA target), and C consists of 1, 2 or 3 LNA units. When present, D consists of a single DNA nucleotide unit which is a non-LNA unit.

In some embodiments A consists of 1 LNA unit. In some embodiments A consists of 2 LNA units. In some embodiments A consists of 3 LNA units. In some embodiments C consists of 1 LNA unit. In some embodiments C consists of 2 LNA units. In some embodiments C consists of 3 LNA units. In some embodiments B consists of 7 DNA nucleotide units which are non-LNA units. In some embodiments B consists of 8 DNA nucleotide units which are non-LNA units. In some embodiments B consists of 9 DNA nucleotide units which are non-LNA units. In some embodiments B consists of 1 - 9 DNA nucleotide units which are non-LNA units, such as 2, 3, 4, 5, 6, 7 or 8 DNA nucleotide units. The DNA nucleotide units are always non-LNA units. In some embodiments B comprises 1, 2 or 3 LNA units which are preferably in the alpha-L configuration and which are more preferably alpha-L-oxy LNA units. In some embodiments the number of nucleotides present in A-B-C are selected from the group consisting of (LNA units - region B - LNA units and more preferably alpha-L-oxy LNA units (region A) - region B - (region C) alpha-L-oxy LNA units): 1-7-2, 2-7-1, 2-7-2, 3-7-1, 3-7-2, 1-7-3, 2-7-3, 3-7-3, 2-7-4, 3-7-4, 4-7-2, 4-7-3, 4-7-4, 1-8-1, 1-8-2, 2-8-1, 2-8-2, 1-8-3, 3-8-1, 3-8-3, 2-8-3, 3-8-2, 4-8-1, 4-8-2, 1-8-4, 2-8-4, 3-8-4, 4-8-3, 4-8-4, 1-9-1, 1-9-2, 2-9-1, 2-9-2, 2-9-3, 3-9-2, 3-9-3, 1-9-3, 3-9-1, 4-9-1, 1-9-4, 4-9-2, 2-9-4, 4-9-3, 3-9-4, 4-9-4, 1-10-1, 1-10-2, 2-10-1, 2-10-2, 1-10-3, 3-10-1, 2-10-2, 2-10-3, 3-10-2, 3-10-3, 2-10-4, 4-10-2, 3-10-4, 4-10-3, 4-10-4, 1-11-1, 1-11-2, 2-11-1, 2-11-2, 1-11-3, 3-11-1, 2-11-2, 2-11-3, 3-11-2, 3-11-3, 2-11-4, 4-11-2, 3-11-4, 4-11-3, 4-11-4. In further preferred embodiments the number of nucleotides in A-B-C are selected from the group consisting of: 2-8-2, 3-8-3, 4-8-2, 2-8-4, 3-8-4, 4-8-3, 4-8-4, 2-9-2, 3-9-3, 4-9-2, 2-9-4, 4-9-3, 3-9-4, 4-9-4, 2-10-2, 3-10-3, 2-10-4, 4-10-2, 3-10-4, 4-10-3, 4-10-4, 2-11-2, 2-11-4, 4-11-2, 3-11-4, 4-11-3 and still more preferred are: 2-8-2, 3-8-3, 3-8-4, 4-8-3, 4-8-4, 2-9-2, 3-9-3, 4-9-3, 3-9-4, 4-9-4, 2-10-2, 3-10-3, 3-10-4, 4-10-3, 4-10-4, 2-11-2, 3-11-4, and 4-11-3.

Phosphorothioate, phosphate or phosphorodithioate and especially phosphorothioate internucleotide linkages are also preferred, particularly for the gapmer region B. Phosphorothioate, phosphate or phosphorodithioate linkages and especially phosphorothioate internucleotide linkages may also be used for the flanking regions (A and C, and for linking A or C to D, and within region D, if present).

Regions A, B and C, may however comprise internucleotide linkages other than phosphorothioate or phosphorodithioate, such as phosphodiester linkages, particularly, for instance when the use of nucleotide analogues protects the internucleotide linkages within regions A and C from endo-nuclease degradation - such as when regions A and C consist of LNA units.

The internucleotide linkages in the antisense-oligonucleotide may be phosphodiester, phosphorothioate, phosphorodithioate or boranophosphate so as to allow RNase H cleavage of targeted RNA. Phosphorothioate or phosphorodithioate is preferred, for improved nuclease resistance and other reasons, such as ease of manufacture. In one aspect of the oligomer of the invention, the LNA units and/or the non-LNA units are linked together by means of phosphorothioate groups.

It is recognized that the inclusion of phosphodiester linkages, such as one or two linkages, into an otherwise phosphorothioate antisense-oligonucleotide, particularly between or adjacent to LNA units (typically in region A and or C) can modify the bioavailability and/or bio-distribution of an antisense-oligonucleotide (see WO2008/053314A which is hereby incorporated by reference).

In some embodiments, such as in the sequences of the antisense-oligonucleotides disclosed herein and where suitable and not specifically indicated, all remaining internucleotide linkage groups are either phosphodiester groups or phosphorothioate groups, or a mixture thereof.

In some embodiments all the internucleotide linkage groups are phosphorothioate groups. When referring to specific gapmer antisense-oligonucleotide sequences, such as those provided herein, it will be understood that, in various embodiments, when the linkages are phosphorothioate linkages, alternative linkages, such as those disclosed herein may be used, for example phosphate (also named phosphodiester) linkages may be used, particularly for linkages between nucleotide analogues, such as LNA units. Likewise, when referring to specific gapmer antisense-oligonucleotide sequences, such as those provided herein, when the C residues are annotated as 5'-methyl modified cytosine, in various embodiments, one or more of the Cs present in the oligomer may be unmodified C residues.

### Legend

As used herein the abbreviations b, d, s, ss have the following meaning:
- b: LNA unit or LNA nucleotide (any one selected from b¹ ― b⁷)
- b¹: β-D-oxy-LNA
- b²: β-D-thio-LNA
- b³: β-D-amino-LNA
- b⁴: α-L-oxy-LNA
- b⁵: β-D-ENA
- b⁶: β-D-(NH)-LNA
- b⁷: β-D-(NCH₃)-LNA
- d: 2-deoxy, that means 2-deoxyribose units
- C*: methyl-C (5-methylcytosine); [consequently dC* is 5-methyl-2'-deoxycytidine]
- A*: 2-aminoadenine [consequently dA* is 2-amino-2'-deoxyadenosine]
- s: the internucleotide linkage is a phosphorothioate group (-O-P(O)(S⁻)-O-)
- ss: the internucleotide linkage is a phosphorodithioate group (-O-P(S)(S⁻)-O-)
- /5SpC3/: ―O-P(O)(O⁻)OC₃H₆OH at 5'-terminal group of an antisense-oligonucleotide
- /3SpC3/: ―O-P(O)(O⁻)OC₃H₆OH at 3'-terminal group of an antisense-oligonucleotide
- /5SpC3s/: ―O-P(O)(S⁻)OC₃H₆OH at 5'-terminal group of an antisense-oligonucleotide
- /3SpC3s/: ―O-P(O)(S⁻)OC₃H₆OH at 3'-terminal group of an antisense-oligonucleotide

nucleotides in **bold** are LNA nucleotides
nucleotides not in bold are non-LNA nucleotides

### Gapmer Sequences

The following antisense-oligonucleotides in form of gapmers as listed in Table 18 to Table 29 are preferred. The antisense-oligonucleotides as disclosed herein such as the antisense-oligonucleotides of Tables 18 to 29 consist of nucleotides, preferably DNA nucleotides, which are non-LNA units (also named herein non-LNA nucleotides) as well as LNA units (also named herein LNA nucleotides). Although not explicitly indicated, the antisense-oligonucleotides of the sequences Although not explicitly indicated, the "C" in Tables 18 to 29 which refer to LNA units preferably contain 5-methylcytosine (C*) as nucleobase.

**Table 18**

| **L** | **Seq ID No.** | **Sequence, 5'-3'** |
|---|---|---|
| 12 | 16a | **GbsGbs**dTsdTsdAsdGsdGsdGsdCsdTs**GbsAb** |
| 12 | 15a | **AbsGbs**dGsdTsdTsdAsdGsdGsdGsdCsd**TbsGb** |
| 12 | 17a | **GbsTbs**dTsdAsdGsdGsdGsdCsdTsdGs**AbsAb** |
| 12 | 18a | **TbsTbs**dAsdGsdGsdGsdCsdTsdGsdAs**AbsTb** |
| 12 | 36a | **TbsAbs**dGsdGsdGsdCsdTsdGsdAsdAs**TbsTb** |
| 12 | 16b | **Gbs**dGsdTsdTsdAsdGsdGsdGsdCsdTs**GbsAb** |
| 12 | 15b | **Abs**dGsdGsdT sdT sdAsdGsdGsdGsdCsd**TbsGb** |
| 12 | 17b | **Gbs**dTsdTsdAsdGsdGsdGsdCsdTsdGs**AbsAb** |
| 12 | 18b | **Tbs**dTsdAsdGsdGsdGsdCsdTsdGsdAs**AbsTb** |
| 12 | 36b | **Tbs**dAsdGsdGsdGsdCsdTsdGsdAsdAs**TbsTb** |
| 12 | 16c | **GbsGbs**dTsdTsdAsdGsdGsdGsdCsdTsdGs**Ab** |
| 12 | 15c | **AbsGbs**dGsdTsdTsdAsdGsdGsdGsdCsddTs**Gb** |
| 12 | 17c | **GbsTbs**dTsdAsdGsdGsdGsdCsdTsdGsdAs**Ab** |
| 12 | 18c | **TbsTbs**dAsdGsdGsdGsdCsdTsdGsdAsdAs**Tb** |
| 12 | 36c | **TbsAbs**dGsdGsdGsdCsdTsdGsdAsdAsdTs**Tb** |
| 11 | 33a | **AbsGbs**dGsdTsdTsdAsdGsdGsdGsdCs**Tb** |
| 11 | 33b | **AbsdGsd**GsdTsdTsdAsdGsdGsdGsdC**bsTb** |
| 11 | 21a | **GbsGbs**dTsdTsdAsdGsdGsdGsdCsdT**sGb** |
| 11 | 21b | **Gbs**dGsdTsdTsdAsdGsdGsdGsdCs**TbsGb** |
| 11 | 22a | **Gbs**dTsdTsdAsdGsdGsdGsdCsdTs**GbsAb** |
| 11 | 22b | **GbsTbs**dTsdAsdGsdGsdGsdCsdTsdGs**Ab** |
| 11 | 23a | **Tbs**TbsdAsdGsdGsdGsdCsdTsdGsdAs**Ab** |
| 11 | 23b | **Tbs**dTsdAsdGsdGsdGsdCsdTsdGs**AbsAb** |
| 11 | 24a | **TbsAbs**dGsdGsdGsdCsdTsdGsdAsdAs**Tb** |
| 11 | 24b | **Tbs**dAsdGsdGsdGsdCsdTsdGsdAs**AbsTb** |
| 11 | 25a | **Abs**dGsdGsdGsdCsdTsdGsdAsdAs**TbsTb** |
| 11 | 25b | **AbsGbs**dGsdGsdCsdTsdGsdAsdAsdTs**Tb** |
| 10 | 8a | **Abs**dGsdGsdTsdTsdAsdGsdGsdGsd**Cb** |
| 10 | 9a | **Gbs**dGsdTsdTsdAsdGsdGsdGsdCs**Tb** |
| 10 | 10a | **Gbs**dTsdTsdAsdGsdGsdGsdCsdTs**Gb** |
| 10 | 11a | **Tbs**dTsdAsdGsdGsdGsdCsdTsdGs**Ab** |
| 10 | 12a | **Tbs**dAsdGsdGsdGsdCsdTsdGsdAs**Ab** |
| 10 | 13a | **Abs**dGsdGsdGsdCsdTsdGsdAsdAs**Tb** |
| 10 | 14a | **Gbs**dGsdGsdCsdTsdGsdAsdAsdTs**Tb** |
| 13 | 26a | **AbsGbs**dGsdTsdTsdAsdGsdGsdGsdCsdTs**GbsAb** |
| 13 | 27a | **GbsGbs**dTsdTsdAsdGsdGsdGsdCsdTsdGs**AbsAb** |
| 13 | 28a | **GbsTbs**dTsdAsdGsdGsdGsdCsdTsdGsdAs**AbsTb** |
| 13 | 29a | **TbsTbs**dAsdGsdGsdGsdCsdTsdGsdAsdAs**TbsTb** |
| 13 | 26b | **AbsGbsGbs**dTsdTsdAsdGsdGsdGsdCsdTs**GbsAb** |
| 13 | 27b | **GbsGbsTbs**dTsdAsdGsdGsdGsdCsdTsdGs**AbsAb** |
| 13 | 28b | **GbsTbsTbs**dAsdGsdGsdGsdCsdTsdGsdAs**AbsTb** |
| 13 | 29b | **TbsTbsAbs**dGsdGsdGsdCsdTsdGsdAsdAs**TbsTb** |
| 13 | 26c | **AbsGbs**dGsdTsdTsdAsdGsdGsdGs**CbsTbsGbsAb** |
| 13 | 27c | **GbsGbs**dTsdTsdAsdGsdGsdGsdCsdTs**GbsAbsAb** |
| 13 | 28c | **GbsTbs**dTsdAsdGsdGsdGsdCsdTsdGs**AbsAbsTb** |
| 13 | 29c | **TbsTbs**dAsdGsdGsdGsdCsdTsdGsdAs**AbsTbsTb** |
| 14 | 30a | **AbsGbs**dGsdTsdTsdAsdGsdGsdGsdCsdTs**GbsAbsAb** |
| 14 | 31a | **GbsGb**sdTsdTsdAsdGsdGsdGsdCsdTsdGs**AbsAbsTb** |
| 14 | 32a | **GbsTbs**dTsdAsdGsdGsdGsdCsdTsdGsdAs**AbsTbsTb** |
| 14 | 30b | **AbsGbsGbs**dTsdTsdAsdGsdGsdGsdCsdTs**GbsAbsAb** |
| 14 | 31b | **GbsGbsTbs**dTsdAsdGsdGsdGsdCsdTsdGs**AbsAbsTb** |
| 14 | 32b | **GbsTbsTbs**dAsdGsdGsdGsdCsdTsdGsdAs**AbsTbsTb** |
| 14 | 30c | **AbsGbs**dGsdTsdTsdAsdGsdGsdGsdCsdTs**GbsAbsAb** |
| 14 | 31c | **GbsGbs**dTsdTsdAsdGsdGsdGsdCsdTsdGs**AbsAbsTb** |
| 14 | 32c | **GbsTbs**dTsdAsdGsdGsdGsdCsdTsdGsdAs**AbsTbsTb** |
| 14 | 30d | **AbsGbs**dGsdTsdTsdAsdGsdGsdGsdCsdTsdGs**AbsAb** |
| 14 | 31d | **GbsGbs**dTsdTsdAsdGsdGsdGsdCsdTsdGsdAs**AbsTb** |
| 14 | 32d | **GbsTbs**dTsdAsdGsdGsdGsdCsdTsdGsdAsdAs**TbsTb** |
| 15 | 19a | **AbsGbs**dGsdTsdTsdAsdGsdGsdGsdCsdTs**GbsAbsAbsTb** |
| 15 | 20a | **GbsGbs**dTsdTsdAsdGsdGsdGsdCsdTsdGs**AbsAbsTbsTb** |
| 15 | 19b | **AbsGbs**dGsdTsdTsdAsdGsdGsdGsdCsdTsdGsdAs**AbsTb** |
| 15 | 20b | **GbsGbs**dTsdTsdAsdGsdGsdGsdCsdTsdGsdAsdAs**TbsTb** |
| 15 | 19c | **AbsGbsGbs**dTsdTsdAsdGsdGsdGsdCsdTsdGs**AbsAbsTb** |
| 15 | 20c | **GbsGbsTb**sdTsdAsdGsdGsdGsdCsdTsdGsdAs**AbsTbsTb** |
| 15 | 19d | **AbsGbsGbsTbs**dTsdAsdGsdGsdGsdCsdTsdGs**AbsAbsTb** |
| 15 | 20d | **GbsGbsTbsTbs**dAsdGsdGsdGsdCsdTsdGsdAsAbsTbsTb |
| 16 | 4a | **AbsGbs**dGsdTsdTsdAsdGsdGsdGsdCsdTsdGsdAs**AbsTbsTb** |
| 16 | 4b | **AbsGbs**dGsdTsdTsdAsdGsdGsdGsdCsdTsdGsdAsdAs**TbsTb** |
| 16 | 4c | **AbsGbsGbs**dTsdTsdAsdGsdGsdGsdCsdTsdGsdAsdAs**TbsTb** |
| 16 | 4d | **AbsGbsGbsTbs**dTsdAsdGsdGsdGsdCsdTsdGsdAsdAs**TbsTb** |
| 16 | 4e | **AbsGbs**dGsdTsdTsdAsdGsdGsdGsdCsdTsdGs**AbsAbsTbsTb** |
| 16 | 4f | **AbsGbsGbs**dTsdTsdAsdGsdGsdGsdCsdTsdGsdAs**AbsTbsTb** |
| 16 | 4g | **AbsGbsGbsTbs**dTsdAsdGsdGsdGsdCsdTsdGs**AbsAbsTbsTb** |
| 16 | 4h | **AbsGbsGbsTbsTbs**dAsdGsdGsdGsdCsdTsdGsdAs**AbsTbsTb** |
| 16 | 4i | **AbsGbsGbs**dTsdTsdAsdGsdGsdGsdCsdTs**GbsAbsAbsTbsTb** |
| 16 | 4j | **AbsGbsGbs**dTsdTsdAsdGsdGsdGsdCsdTsdGs**AbsAbsTbsTb** |
| 16 | 4k | **AbsGbsGbsTbs**dTsdAsdGsdGsdGsdCsdTsdGs**dAsAbsTbsTb** |

**Table 19**

| **L** | **Seq ID No.** | **Sequence, 5'-3'** |
|---|---|---|
| 12 | 46a | **CbsAbs**dAsdGsdCsdAsdAsdGsdGsdCs**AbsTb** |
| 12 | 45a | **AbsCbs**dAsdAsdGsdCsdAsdAsdGsdGs**CbsAb** |
| 12 | 44a | **TbsAbs**dCsdAsdAsdGsdCsdAsdAsdGs**GbsCb** |
| 12 | 47a | **AbsAbs**dGsdCsdAsdAsdGsdGsdCsdAs**TbsTb** |
| 12 | 48a | **AbsGbs**dCsdAsdAsdGsdGsdCsdAsdTs**TbsTb** |
| 12 | 46b | **Cbs**dAsdAsdGsdCsdAsdAsdGsdGsdCs**AbsTb** |
| 12 | 45b | **Abs**dCsdAsdAsdGsdCsdAsdAsdGsdGs**CbsAb** |
| 12 | 44b | **Tbs**dAsdCsdAsdAsdGsdCsdAsdAsdGs**GbsCb** |
| 12 | 47b | **Abs**dAsdGsdCsdAsdAsdGsdGsdCsdAs**TbsTb** |
| 12 | 48b | **Abs**dGsdCsdAsdAsdGsdGsdCsdAsdTs**TbsTb** |
| 12 | 46c | **CbsAbs**dAsdGsdCsdAsdAsdGsdGsdCsdAsT**b** |
| 12 | 45c | **AbsCbs**dAsdAsdGsdCsdAsdAsdGsdGsdCs**Ab** |
| 12 | 44c | **TbsAbs**dCsdAsdAsdGsdCsdAsdAsdGsdGs**Cb** |
| 12 | 47c | **AbsAbs**dGsdCsdAsdAsdGsdGsdCsdAsdTs**Tb** |
| 12 | 48c | **AbsGbs**dCsdAsdAsdGsdGsdCsdAsdTsdTs**Tb** |
| 11 | 51a | **Tbs**dAsdCsdAsdAsdGsdCsdAsdAs**GbsGb** |
| 11 | 52a | **Abs**dCsdAsdAsdGsdCsdAsdAsdGs**GbsCb** |
| 11 | 53a | **Cbs**dAsdAsdGsdCsdAsdAsdGsdGs**CbsAb** |
| 11 | 54a | **Abs**dAsdGsdCsdAsdAsdGsdGsdCs**AbsTb** |
| 11 | 55a | **Abs**dGsdCsdAsdAsdGsdGsdCsdAsd**TbsTb** |
| 11 | 56a | **Gbs**dCsdAsdAsdGsdGsdCsdAsdTs**TbsTb** |
| 11 | 51b | **TbsAbs**dCsdAsdAsdGsdCsdAsdAsdGs**Gb** |
| 11 | 52b | **AbsCbs**dAsdAsdGsdCsdAsdAsdGsdGs**Cb** |
| 11 | 53b | **CbsAbs**dAsdGsdCsdAsdAsdGsdGsdCs**Ab** |
| 11 | 54b | **AbsAbs**dGsdCsdAsdAsdGsdGsdCsdAs**Tb** |
| 11 | 55b | **AbsGbs**dCsdAsdAsdGsdGsdCsdAsddTs**Tb** |
| 11 | 56b | **GbsCbs**dAsdAsdGsdGsdCsdAsdTsdTs**Tb** |
| 10 | 37a | **Tbs**dAsdCsdAsdAsdGsdCsdAsdAs**Gb** |
| 10 | 38a | **Abs**dCsdAsdAsdGsdCsdAsdAsdGs**Gb** |
| 10 | 39a | **Cbs**dAsdAsdGsdCsdAsdAsdGsdGs**Cb** |
| 10 | 40a | **Abs**dAsdGsdCsdAsdAsdGsdGsdCs**Ab** |
| 10 | 41a | **Abs**dGsdCsdAsdAsdGsdGsdCsdAsd**Tb** |
| 10 | 42a | **Gbs**dCsdAsdAsdGsdGsdCsdAsdTs**Tb** |
| 10 | 43a | **Cbs**dAsdAsdGsdGsdCsdAsdTsdTs**Tb** |
| 13 | 57a | **TbsAbs**dCsdAsdAsdGsdCsdAsdAsdGsdGs**CbsAb** |
| 13 | 58a | **AbsCbs**dAsdAsdGsdCsdAsdAsdGsdGsdCs**AbsTb** |
| 13 | 59a | **CbsAbs**dAsdGsdCsdAsdAsdGsdGsdCsdAs**TbsTb** |
| 13 | 60a | **AbsAbs**dGsdCsdAsdAsdGsdGsdCsdAsdTs**TbsTb** |
| 13 | 57b | **TbsAbsCbs**dAsdAsdGsdCsdAsdAsdGsdGs**CbsAb** |
| 13 | 58b | **AbsCbsAbs**dAsdGsdCsdAsdAsdGsdGsdCs**AbsTb** |
| 13 | 59b | **CbsAbsAbs**dGsdCsdAsdAsdGsdGsdCsdAs**TbsTb** |
| 13 | 60b | **AbsAbsGbs**dCsdAsdAsdGsdGsdCsdAsdTs**TbsTb** |
| 13 | 57c | **TbsAbs**dCsdAsdAsdGsdCsdAsdAsdGs**GbsCbsAb** |
| 13 | 58c | **AbsCbs**dAsdAsdGsdCsdAsdAsdGsdGs**CbsAbsTb** |
| 13 | 59c | **CbsAbs**dAsdGsdCsdAsdAsdGsdGsdCs**AbsTbsTb** |
| 13 | 60c | **AbsAbs**dGsdCsdAsdAsdGsdGsdCsdAs**TbsTbsTb** |
| 14 | 61a | **TbsAbs**dCsdAsdAsdGsdCsdAsdAsdGsdGsdCs**AbsTb** |
| 14 | 62a | **AbsCbs**dAsdAsdGsdCsdAsdAsdGsdGsdCsdAs**TbsTb** |
| 14 | 63a | **CbsAbs**dAsdGsdCsdAsdAsdGsdGsdCsdAsdTs**TbsTb** |
| 14 | 61b | **TbsAbsCbs**dAsdAsdGsdCsdAsdAsdGsdGsdCs**AbsTb** |
| 14 | 62b | **AbsCbsAbs**dAsdGsdCsdAsdAsdGsdGsdCsdAs**TbsTb** |
| 14 | 63b | **CbsAbsAbs**dGsdCsdAsdAsdGsdGsdCsdAsdTs**TbsTb** |
| 14 | 61c | **TbsAbs**dCsdAsdAsdGsdCsdAsdAsdGsdGs**CbsAbsTb** |
| 14 | 62c | **AbsCbs**dAsdAsdGsdCsdAsdAsdGsdGsdCs**AbsTbsTb** |
| 14 | 63c | **CbsAbs**dAsdGsdCsdAsdAsdGsdGsdCsdAs**TbsTbsTb** |
| 14 | 61d | **TbsAbsCbs**dAsdAsdGsdCsdAsdAsdGsdGs**CbsAbsTb** |
| 14 | 62d | **AbsCbsAbs**dAsdGsdCsdAsdAsdGsdGsdCs**AbsTbsTb** |
| 14 | 63d | **CbsAbsAbs**dGsdCsdAsdAsdGsdGsdCsdAs**TbsTbsTb** |
| 15 | 49a | **TbsAbs**dCsdAsdAsdGsdCsdAsdAsdGsdGsdCsdAs**TbsTbs** |
| 15 | 50a | **AbsCbs**dAsdAsdGsdCsdAsdAsdGsdGsdCsdAsdTs**TbsTb** |
| 15 | 49b | **TbsAbsCbs**dAsdAsdGsdCsdAsdAsdGsdGsdCsdAs**TbsTbs** |
| 15 | 50b | **AbsCbsAbs**dAsdGsdCsdAsdAsdGsdGsdCsdAsdTs**TbsTb** |
| 15 | 49c | **TbsAbs**dCsdAsdAsdGsdCsdAsdAsdGsdGsdCs**AbsTbsTbs** |
| 15 | 50c | **AbsCbs**dAsdAsdGsdCsdAsdAsdGsdGsdCsdAs**TbsTbsTb** |
| 15 | 49d | **TbsAbsCbs**dAsdAsdGsdCsdAsdAsdGsdGsdCs**AbsTbsTbs** |
| 15 | 50d | **AbsCbsAbs**dAsdGsdCsdAsdAsdGsdGsdCsdAs**TbsTbsTb** |
| 16 | 5a | **TbsAbs**dCsdAsdAsdGsdCsdAsdAsdGsdGsdCsdAsdTs**TbsTb** |
| 16 | 5b | **TbsAbsCbs**dAsdAsdGsdCsdAsdAsdGsdGsdCsdAsdTs**TbsTb** |
| 16 | 5c | **TbsAbsCbsAbs**dAsdGsdCsdAsdAsdGsdGsdCsdAsdTs**TbsTb** |
| 16 | 5d | **TbsAbs**dCsdAsdAsdGsdCsdAsdAsdGsdGsdCsdAs**TbsTbsTb** |
| 16 | 5e | **TbsAbs**dCsdAsdAsdGsdCsdAsdAsdGsdGsdCs**AbsTbsTbsTb** |
| 16 | 5f | **TbsAbsCbs**dAsdAsdGsdCsdAsdAsdGsdGsdCsdAs**TbsTbsTb** |
| 16 | 5g | **TbsAbsCbsAbs**dAsdGsdCsdAsdAsdGsdGsdCsdAs**TbsTbsTb** |
| 16 | 5h | **TbsAbsCbs**dAsdAsdGsdCsdAsdAsdGsdGsdCs**AbsTbsTbsTb** |
| 16 | 5i | **TbsAbsCbsAbs**dAsdGsdCsdAsdAsdGsdGsdCs**AbsTbsTbsTb** |
| 16 | 5j | **TbsAbsCbsAbsd**AsdGsdCsdAsdAsdGsdGsdCsdAs**TbsTbsTb** |
| 16 | 5k | **TbsAbsCbs**dAsdAsdGsdCsdAsdAsdGsdGsdCs**AbsTbsTbsTb** |

**Table 20**

| **L** | **Seq ID No.** | **Sequence, 5'-3'** |
|---|---|---|
| 12 | 74a | **TbsGbs**dCsdAsdAsdAsdAsdT sdT sdCs**AbsGb** |
| 12 | 75a | **AbsCbs**dAsdTsdTsdGsdCsdAsdAsdAs**AbsTb** |
| 12 | 76a | **CbsAbs**dTsdTsdGsdCsdAsdAsdAsdAs**TbsTb** |
| 12 | 77a | **AbsTbs**dTsdGsdCsdAsdAsdAsdAsdTs**TbsCb** |
| 12 | 78a | **TbsTbs**dGsdCsdAsdAsdAsdAsdTsdTs**CbsAb** |
| 12 | 74b | **TbsGbsCbs**dAsdAsdAsdAsdTsdTsdCs**AbsGb** |
| 12 | 75b | **AbsCbsAbs**dTsdTsdGsdCsdAsdAsdAs**AbsTb** |
| 12 | 76b | **CbsAbsTbs**dTsdGsdCsdAsdAsdAsdAs**TbsTb** |
| 12 | 77b | **AbsTbsTbs**dGsdCsdAsdAsdAsdAsdTs**TbsCb** |
| 12 | 78b | **TbsTbsGbs**dCsdAsdAsdAsdAsdTsdTs**CbsAb** |
| 12 | 74c | **TbsGbs**dCsdAsdAsdAsdAsdTsdTs**CbsAbsGb** |
| 12 | 75c | **AbsCbs**dAsdTsdTsdGsdCsdAsdAs**AbsAbsTb** |
| 12 | 76c | **CbsAbs**dTsdTsdGsdCsdAsdAsdAs**AbsTbsTb** |
| 12 | 77c | **AbsTbs**dTsdGsdCsdAsdAsdAsdAs**TbsTbsCb** |
| 12 | 78c | **TbsTbs**dGsdCsdAsdAsdAsdAsdTs**TbsCbsAb** |
| 11 | 81a | **Abs**dCsdAsdTsdTsdGsdCsdAsdAs**AbsAb** |
| 11 | 82a | **Cbs**dAsdTsdTsdGsdCsdAsdAsdAs**AbsTb** |
| 11 | 83a | **Abs**dTsdTsdGsdCsdAsdAsdAsdAs**TbsTb** |
| 11 | 84a | **Tbs**dTsdGsdCsdAsdAsdAsdAsdTs**TbsCb** |
| 11 | 85a | **Tbs**dGsdCsdAsdAsdAsdAsdTsdTs**CbsAb** |
| 11 | 86a | **Gbs**dCsdAsdAsdAsdAsdTsdTsdCs**AbsGb** |
| 11 | 81b | **AbsCbs**dAsdTsdTsdGsdCsdAsdAsdAs**Ab** |
| 11 | 82b | **CbsAbs**dTsdTsdGsdCsdAsdAsdAsdAs**Tb** |
| 11 | 83b | **AbsTbs**dTsdGsdCsdAsdAsdAsdAsdTs**Tb** |
| 11 | 84b | **TbsTbs**dGsdCsdAsdAsdAsdAsdTsdTs**Cb** |
| 11 | 85b | **TbsGbs**dCsdAsdAsdAsdAsdTsdTsdCs**Ab** |
| 11 | 86b | **GbsCbs**dAsdAsdAsdAsdTsdTsdCsdAs**Gb** |
| 10 | 67a | **Abs**dCsdAsdTsdTsdGsdCsdAsdAs**Ab** |
| 10 | 68a | **Cbs**dAsdTsdTsdGsdCsdAsdAsdAs**Ab** |
| 10 | 69a | **Abs**dTsdTsdGsdCsdAsdAsdAsdAs**Tb** |
| 10 | 70a | **Tbs**dTsdGsdCsdAsdAsdAsdAsdTs**Tb** |
| 10 | 71a | **Tbs**dGsdCsdAsdAsdAsdAsdTsdTs**Cb** |
| 10 | 72a | **Gbs**dCsdAsdAsdAsdAsdTsdTsdCs**Ab** |
| 10 | 73a | **Cbs**dAsdAsdAsdAsdTsdTsdCsdAs**Gb** |
| 13 | 87a | **AbsCbs**dAsdTsdTsdGsdCsdAsdAsdAsdAs**TbsTb** |
| 13 | 88a | **CbsAbs**dTsdTsdGsdCsdAsdAsdAsdAsdTs**TbsCb** |
| 13 | 89a | **AbsTbs**dTsdGsdCsdAsdAsdAsdAsdTsdTs**CbsAb** |
| 13 | 90a | **TbsTbs**dGsdCsdAsdAsdAsdAsdTsdTsdCs**AbsGb** |
| 13 | 87b | **AbsCbsAbs**dTsdTsdGsdCsdAsdAsdAsdAs**TbsTb** |
| 13 | 88b | **CbsAbsTbs**dTsdGsdCsdAsdAsdAsdAsdTs**TbsCb** |
| 13 | 89b | **AbsTbsTbs**dGsdCsdAsdAsdAsdAsdTsdTs**CbsAb** |
| 13 | 90b | **TbsTbsGbs**dCsdAsdAsdAsdAsdTsdTsdCs**AbsGb** |
| 13 | 87c | **AbsCbs**dAsdTsdTsdGsdCsdAsdAsdAs**AbsTbsTb** |
| 13 | 88c | **CbsAbs**dTsdTsdGsdCsdAsdAsdAsdAs**TbsTbsCb** |
| 13 | 89c | **AbsTbs**dTsdGsdCsdAsdAsdAsdAsdTs**TbsCbsAb** |
| 13 | 90c | **TbsTbs**dGsdCsdAsdAsdAsdAsdTsdTs**CbsAbsGb** |
| 14 | 91a | **AbsCbs**dAsdTsdTsdGsdCsdAsdAsdAsdAsdTs**TbsCb** |
| 14 | 92a | **CbsAbs**dTsdTsdGsdCsdAsdAsdAsdAsdTsdTs**CbsAb** |
| 14 | 93a | **AbsTbs**dTsdGsdCsdAsdAsdAsdAsdTsdTsdCs**AbsGb** |
| 14 | 91b | **AbsCbsAbs**dTsdTsdGsdCsdAsdAsdAsdAsdTs**TbsCb** |
| 14 | 92b | **CbsAbsTbs**dTsdGsdCsdAsdAsdAsdAsdTsdTs**CbsAb** |
| 14 | 93b | **AbsTbsTb**sdGsdCsdAsdAsdAsdAsdTsdTsdCs**AbsGb** |
| 14 | 91c | **AbsCbs**dAsdTsdTsdGsdCsdAsdAsdAsdAs**TbsTbsCb** |
| 14 | 92c | **CbsAbs**dTsdTsdGsdCsdAsdAsdAsdAsdTs**TbsCbsAb** |
| 14 | 93c | **AbsTbs**dTsdGsdCsdAsdAsdAsdAsdTsdTs**CbsAbsGb** |
| 14 | 91d | **AbsCbsAbs**dTsdTsdGsdCsdAsdAsdAsdAs**TbsTbsCb** |
| 14 | 92d | **CbsAbsTbs**dTsdGsdCsdAsdAsdAsdAsdTs**TbsCbsAb** |
| 14 | 93d | **AbsTbsTbs**dGsdCsdAsdAsdAsdAsdTsdTs**CbsAbsGb** |
| 15 | 79a | **AbsCbs**dAsdTsdTsdGsdCsdAsdAsdAsdAsdTsdTs**CbsAb** |
| 15 | 80a | **CbsAbs**dTsdTsdGsdCsdAsdAsdAsdAsdTsdTsdCs**AbsGb** |
| 15 | 79b | **AbsCbsAbs**dTsdTsdGsdCsdAsdAsdAsdAsdTsdTs**CbsAb** |
| 15 | 80b | **CbsAbsTbs**dTsdGsdCsdAsdAsdAsdAsdTsdTsdCs**AbsGb** |
| 15 | 79c | **AbsCbsAbs**dTsdTsdGsdCsdAsdAsdAsdAsdTs**TbsCbsAb** |
| 15 | 80c | **CbsAbsTbs**dTsdGsdCsdAsdAsdAsdAsdTsdTs**CbsAbsGb** |
| 15 | 79d | **AbsCbs**dAsdTsdTsdGsdCsdAsdAsdAsdAsdTs**TbsCbsAb** |
| 15 | 80d | **CbsAbs**dTsdTsdGsdCsdAsdAsdAsdAsdTsdTs**CbsAbsGb** |
| 16 | 6a | **AbsCbs**dAsdTsdTsdGsdCsdAsdAsdAsdAsdTsdTsdCs**AbsGb** |
| 16 | 6b | **AbsCbsAbs**dTsdTsdGsdCsdAsdAsdAsdAsdTsdTsdCs**AbsGb** |
| 16 | 6c | **AbsCbsAbsTbs**dTsdGsdCsdAsdAsdAsdAsdTsdTsdCs**AbsGb** |
| 16 | 6d | **AbsCbsAbs**dTsdTsdGsdCsdAsdAsdAsdAsdTsdTs**CbsAbsGb** |
| 16 | 6e | **AbsCbsAbsTbs**dTsdGsdCsdAsdAsdAsdAsdTsdTs**CbsAbsGb** |
| 16 | 6f | **AbsCbs**dAsdTsdTsdGsdCsdAsdAsdAsdAsdTsdTs**CbsAbsGb** |
| 16 | 6g | **AbsCbs**dAsdTsdTsdGsdCsdAsdAsdAsdAsdTs**TbsCbsAbsGb** |
| 16 | 6h | **AbsCbsAbs**dTsdTsdGsdCsdAsdAsdAsdAsdTs**TbsCbsAbsGb** |
| 16 | 6i | **AbsCbsAbsTbs**dTsdGsdCsdAsdAsdAsdAsdTs**TbsCbsAbsGb** |
| 16 | 6j | **AbsCbsAbs**dTsdTsdGsdCsdAsdAsdAsdAs**TbsTbsCbsAbsGb** |
| 16 | 6k | **AbsCbsAbsTbsTbs**dGsdCsdAsdAsdAsdAsdTsdTs**CbsAbsGb** |

**Table 21**

| **L** | **Seq ID No**. | **Sequence, 5'-3'** |
|---|---|---|
| 12 | 16d | **GbsGbs**dT sdT sdAsdGsdGsdGsdC*sdTs**GbsAb** |
| 12 | 15d | **AbsGbs**dGsdT sdT sdAsdGsdGsdGsdC*sd**TbsGb** |
| 12 | 17d | **GbsTbs**dTsdAsdGsdGsdGsdC*sdTsdGs**AbsAb** |
| 12 | 18d | **TbsTbs**dAsdGsdGsdGsdC*sdTsdGsdAs**AbsTb** |
| 12 | 36d | **TbsAbs**dGsdGsdGsdC*sdTsdGsdAsdAs**TbsTb** |
| 12 | 16e | **Gbs**dGsdT sdT sdAsdGsdGsdGsdC*sdTs**GbsAb** |
| 12 | 15e | **Abs**dGsdGsdT sdT sdAsdGsdGsdGsdC*sd**TbsGb** |
| 12 | 17e | **Gbs**dTsdTsdAsdGsdGsdGsdC*sdTsdGs**AbsAb** |
| 12 | 18e | **Tbs**dTsdAsdGsdGsdGsdC*sdTsdGsdAs**AbsTb** |
| 12 | 36e | **Tbs**dAsdGsdGsdGsdC*sdTsdGsdAsdAs**TbsTb** |
| 12 | 16f | **GbsGbs**dTsdTsdAsdGsdGsdGsdC*sdTsdGs**Ab** |
| 12 | 15f | **AbsGbs**dGsdTsdTsdAsdGsdGsdGsdC*sddTs**Gb** |
| 12 | 17f | **GbsTbs**dTsdAsdGsdGsdGsdC*sdTsdGsdAs**Ab** |
| 12 | 18f | **TbsTbs**dAsdGsdGsdGsdC*sdTsdGsdAsdAs**Tb** |
| 12 | 36f | **TbsAbs**dGsdGsdGsdC*sdTsdGsdAsdAsdTs**Tb** |
| 11 | 33c | AbsGbsdGsdT sdT sdAsdGsdGsdGsdC*s**Tb** |
| 11 | 33d | **Abs**dGsdGsdTsdTsdAsdGsdGsdGsdC***bsTb** |
| 11 | 21c | GbsGbsdT sdT sdAsdGsdGsdGsdC*sdTsGb |
| 11 | 21d | GbsdGsdT sdT sdAsdGsdGsdGsdC*s**TbsGb** |
| 11 | 22c | GbsdT sdT sdAsdGsdGsdGsdC*sdTsGbsAb |
| 11 | 22d | Gbs TbsdT sdAsdGsdGsdGsdC*sdTsdGsAb |
| 11 | 23c | **TbsTbs**dAsdGsdGsdGsdC*sdTsdGsdAs**Ab** |
| 11 | 23d | TbsdT sdAsdGsdGsdGsdC*sdTsdGs**AbsAb** |
| 11 | 24c | **TbsAbs**dGsdGsdGsdC*sdTsdGsdAsdAs**Tb** |
| 11 | 24d | **Tbs**dAsdGsdGsdGsdC*sdTsdGsdAs**AbsTb** |
| 11 | 25c | **Abs**dGsdGsdGsdC*sdTsdGsdAsdAs**TbsTb** |
| 11 | 25d | **Abs**GbsdGsdGsdC*sdTsdGsdAsdAsdTs**T**b |
| 10 | 8b | **Abs**dGsdGsdTsdTsdAsdGsdGsdGsd**C*b** |
| 10 | 9b | **Gbs**dGsdTsdTsdAsdGsdGsdGsdC*s**Tb** |
| 10 | 10b | **Gbs**dTsdTsdAsdGsdGsdGsdC*sdTs**Gb** |
| 10 | 11b | TbsdT sdAsdGsdGsdGsdC*sdTsdGs**Ab** |
| 10 | 12b | **Tbs**dAsdGsdGsdGsdC*sdTsdGsdAs**Ab** |
| 10 | 13b | **Abs**dGsdGsdGsdC*sdTsdGsdAsdAs**Tb** |
| 10 | 14b | **Gbs**dGsdGsdC*sdTsdGsdAsdAsdTs**Tb** |
| 13 | 26d | **AbsGbs**dGsdTsdTsdAsdGsdGsdGsdC*sdTs**GbsAb** |
| 13 | 27d | **GbsGbs**dTsdTsdAsdGsdGsdGsdC*sdTsdGs**AbsAb** |
| 13 | 28d | **GbsTbs**dTsdAsdGsdGsdGsdC*sdTsdGsdAs**AbsTb** |
| 13 | 29d | **TbsTbs**dAsdGsdGsdGsdC*sdTsdGsdAsdAs**TbsTb** |
| 13 | 26e | **AbsGbsGbs**dT sdT sdAsdGsdGsdGsdC*sdTs**GbsAb** |
| 13 | 27e | **GbsGbsTbs**dTsdAsdGsdGsdGsdC*sdTsdGs**AbsAb** |
| 13 | 28e | **GbsTbsTbs**dAsdGsdGsdGsdC*sdTsdGsdAs**AbsTb** |
| 13 | 29e | **TbsTbs**AbsdGsdGsdGsdC*sdTsdGsdAsdAs**TbsTb** |
| 13 | 26f | **AbsGbs**dGsdTsdTsdAsdGsdGsdGs**C*bsTbsGbsAb** |
| 13 | 27f | **GbsGbsdTs**dTsdAsdGsdGsdGsdC*sdTsGbsAbsAb |
| 13 | 28f | GbsTbsdTsdAsdGsdGsdGsdC*sdTsdGsAbsAbs Tb |
| 13 | 29f | **TbsTbs**dAsdGsdGsdGsdC*sdTsdGsdAs**AbsTbsTb** |
| 14 | 30e | AbsGbsdGsdT sdT sdAsdGsdGsdGsdC*sdTsGbsAbsAb |
| 14 | 31e | GbsGbsdT sdT sdAsdGsdGsdGsdC*sdTsdGsAbsAbs Tb |
| 14 | 32e | **GbsTbs**dTsdAsdGsdGsdGsdC*sdTsdGsdAs**AbsTbsTb** |
| 14 | 30f | AbsGbsGbsdT sdT sdAsdGsdGsdGsdC*sdTsGbsAbsAb |
| 14 | 31f | GbsGbs TbsdT sdAsdGsdGsdGsdC*sdTsdGsAbsAbs Tb |
| 14 | 32f | **GbsTbsTbs**dAsdGsdGsdGsdC*sdTsdGsdAs**AbsTbsTb** |
| 14 | 30g | **AbsGbs**dGsdTsdTsdAsdGsdGsdGsdC*sdTs**GbsAbsAb** |
| 14 | 31g | **GbsGbs**dTsdTsdAsdGsdGsdGsdC*sdTsdGs**AbsAbsTb** |
| 14 | 32g | **GbsTbs**dTsdAsdGsdGsdGsdC*sdTsdGsdAs**AbsTbsTb** |
| 14 | 30h | **AbsGbs**dGsdT sdT sdAsdGsdGsdGsdC*sdTsdGs**AbsAb** |
| 14 | 31h | **GbsGbs**dTsdTsdAsdGsdGsdGsdC*sdTsdGsdAs**AbsTb** |
| 14 | 32h | **GbsTbs**dTsdAsdGsdGsdGsdC*sdTsdGsdAsdAs**TbsTb** |
| 15 | 19e | **AbsGbs**dGsdTsdTsdAsdGsdGsdGsdC*sdTs**GbsAbsAbsTb** |
| 15 | 20e | **GbsGbs**dTsdTsdAsdGsdGsdGsdC*sdTsdGs**AbsAbsTbsTb** |
| 15 | 19f | **AbsGbs**dGsdTsdTsdAsdGsdGsdGsdC*sdTsdGsdAs**AbsTb** |
| 15 | 20f | **GbsGbs**dTsdTsdAsdGsdGsdGsdC*sdTsdGsdAsdAs**TbsTb** |
| 15 | 19g | **AbsGbsGbs**dTsdTsdAsdGsdGsdGsdC*sdTsdGs**AbsAbsTb** |
| 15 | 20g | **GbsGbsTbs**dTsdAsdGsdGsdGsdC*sdTsdGsdAs**AbsTbsTb** |
| 15 | 19h | **AbsGbsGbsTbs**dTsdAsdGsdGsdGsdC*sdTsdGs**AbsAbsTb** |
| 15 | 20h | **GbsGbsTbsTbs**dAsdGsdGsdGsdC*sdTsdGsdAs**AbsTbsTb** |
| 16 | 4l | **AbsGbs**dGsdTsdTsdAsdGsdGsdGsdC*sdTsdGsdAs**AbsTbsTb** |
| 16 | 4m | **AbsGbs**dGsdT sdTsdAsdGsdGsdGsdC*sdTsdGsdAsdAs**TbsTb** |
| 16 | 4n | **AbsGbsGbs**dTsdTsdAsdGsdGsdGsdC*sdTsdGsdAsdAs**TbsTb** |
| 16 | 4o | **AbsGbsGbsTbs**dTsdAsdGsdGsdGsdC*sdTsdGsdAsdAs**TbsTb** |
| 16 | 4p | **AbsGbs**dGsdTsdTsdAsdGsdGsdGsdC*sdTsdGs**AbsAbsTbsTb** |
| 16 | 4q | **AbsGbsGbs**dTsdTsdAsdGsdGsdGsdC*sdTsdGsdAs**AbsTbsTb** |
| 16 | 4r | **AbsGbsGbsTbs**dTsdAsdGsdGsdGsdC*sdTsdGs**AbsAbsTbsTb** |
| 16 | 4s | **AbsGbsGbsTbsTbs**dAsdGsdGsdGsdC*sdTsdGsdAs**AbsTbsTb** |
| 16 | 4t | AbsGbsGbsdT sdT sdAsdGsdGsdGsdC*sdTsGbsAbsAbs Tbs Tb |
| 16 | 4u | AbsGbsGbsdT sdT sdAsdGsdGsdGsdC*sdTsdGsAbsAbs Tbs Tb |
| 16 | 4 | **AbsGbsGbsTbs**dTsdAsdGsdGsdGsdC*sdTsdGs**dAsAbsTbsTb** |

**Table 22**

| **L** | **Seq ID No.** | **Sequence, 5'-3'** |
|---|---|---|
| 12 | 46d | **C*bsAbs**dAsdGsdC*sdAsdAsdGsdGsdC*s**Abs**Tb |
| 12 | 45d | **AbsC*bs**dAsdAsdGsdC*sdAsdAsdGsdGs**C*bsAb** |
| 12 | 44d | **TbsAbs**dC*sdAsdAsdGsdC*sdAsdAsdGs**GbsC*b** |
| 12 | 47d | **AbsAbs**dGsdC*sdAsdAsdGsdGsdC*sdAs**Tbs**Tb |
| 12 | 48d | **AbsGbs**dC*sdAsdAsdGsdGsdC*sdAsdTsTbs Tb |
| 12 | 46e | **C*bs**dAsdAsdGsdC*sdAsdAsdGsdGsdC*s**Abs**Tb |
| 12 | 45e | **Abs**dC*sdAsdAsdGsdC*sdAsdAsdGsdGs**C*bsAb** |
| 12 | 44e | **Tbs**dAsdC*sdAsdAsdGsdC*sdAsdAsdGs**GbsC*b** |
| 12 | 47e | **Abs**dAsdGsdC*sdAsdAsdGsdGsdC*sdAs**TbsTb** |
| 12 | 48e | **Abs**dGsdC*sdAsdAsdGsdGsdC*sdAsdTs**TbsTb** |
| 12 | 46f | **C*bsAbs**dAsdGsdC*sdAsdAsdGsdGsdC*sdAs**Tb** |
| 12 | 45f | **AbsC*bs**dAsdAsdGsdC*sdAsdAsdGsdGsdC*s**Ab** |
| 12 | 44f | **TbsAbs**dC*sdAsdAsdGsdC*sdAsdAsdGsdGs**C*b** |
| 12 | 47f | **AbsAbs**dGsdC*sdAsdAsdGsdGsdC*sdAsdTs**Tb** |
| 12 | 48f | **AbsGbs**dC*sdAsdAsdGsdGsdC*sdAsdTsdTs**Tb** |
| 11 | 51c | **Tbs**dAsdC*sdAsdAsdGsdC*sdAsdAs**GbsGb** |
| 11 | 52c | **Abs**dC*sdAsdAsdGsdC*sdAsdAsdGs**GbsC*b** |
| 11 | 53c | **C*bs**dAsdAsdGsdC*sdAsdAsdGsdGs**C*bsAb** |
| 11 | 54c | **Abs**dAsdGsdC*sdAsdAsdGsdGsdC*s**AbsTb** |
| 11 | 55c | **Abs**dGsdC*sdAsdAsdGsdGsdC*sdAsd**TbsTb** |
| 11 | 56c | **Gbs**dC*sdAsdAsdGsdGsdC*sdAsdTs**TbsTb** |
| 11 | 51d | **TbsAbs**dC*sdAsdAsdGsdC*sdAsdAsdGs**Gb** |
| 11 | 52d | **AbsC*bs**dAsdAsdGsdC*****sdAsdAsdGsdGs**C*****b** |
| 11 | 53d | **C*bsAbs**dAsdGsdC*sdAsdAsdGsdGsdC*s**Ab** |
| 11 | 54d | **AbsAbs**dGsdC*sdAsdAsdGsdGsdC*sdAs**Tb** |
| 11 | 55d | **AbsGbs**dC*sdAsdAsdGsdGsdC*sdAsddTs**Tb** |
| 11 | 56d | **GbsC*bs**dAsdAsdGsdGsdC*sdAsdTsdTs**Tb** |
| 10 | 37b | **Tbs**dAsdC*sdAsdAsdGsdC*sdAsdAs**Gb** |
| 10 | 38b | **Abs**dC*sdAsdAsdGsdC*sdAsdAsdGs**Gb** |
| 10 | 39b | **C*bs**dAsdAsdGsdC*sdAsdAsdGsdGs**C*b** |
| 10 | 40b | **Abs**dAsdGsdC*sdAsdAsdGsdGsdC*s**Ab** |
| 10 | 41b | **Abs**dGsdC*sdAsdAsdGsdGsdC*sdAsd**Tb** |
| 10 | 42b | **Gbs**dC*sdAsdAsdGsdGsdC*sdAsdTs**Tb** |
| 10 | 43b | **C*bs**dAsdAsdGsdGsdC*sdAsdTsdTs**Tb** |
| 13 | 57d | **TbsAbs**dC*sdAsdAsdGsdC*sdAsdAsdGsdGs**C*bsAb** |
| 13 | 58d | **AbsC*bs**dAsdAsdGsdC*sdAsdAsdGsdGsdC*s**AbsTb** |
| 13 | 59d | **C*bsAbs**dAsdGsdC*sdAsdAsdGsdGsdC*sdAs**TbsTb** |
| 13 | 60d | **AbsAbs**dGsdC*sdAsdAsdGsdGsdC*sdAsdTs**TbsTb** |
| 13 | 57e | **TbsAbsC*bs**dAsdAsdGsdC*sdAsdAsdGsdGs**C*bsAb** |
| 13 | 58e | **AbsC*bsAbs**dAsdGsdC*sdAsdAsdGsdGsdC*s**AbsTb** |
| 13 | 59e | **C*bsAbsAbs**dGsdC*sdAsdAsdGsdGsdC*sdAs**TbsTb** |
| 13 | 60e | **AbsAbsGbs**dC*sdAsdAsdGsdGsdC*sdAsdTs**TbsTb** |
| 13 | 57f | **TbsAbs**dC*sdAsdAsdGsdC*sdAsdAsdGs**GbsC*bsAb** |
| 13 | 58f | **AbsC*bs**dAsdAsdGsdC*sdAsdAsdGsdGs**C*bsAbsTb** |
| 13 | 59f | **C*bsAbs**dAsdGsdC*sdAsdAsdGsdGsdC*s**AbsTbsTb** |
| 13 | 60f | **AbsAbs**dGsdC*sdAsdAsdGsdGsdC*sdAs**TbsTbsTb** |
| 14 | 61e | **TbsAbs**dC*sdAsdAsdGsdC*sdAsdAsdGsdGsdC*s**AbsTb** |
| 14 | 62e | **AbsC*bs**dAsdAsdGsdC*sdAsdAsdGsdGsdC*sdAs**TbsTb** |
| 14 | 63e | **C*bsAbs**dAsdGsdC*sdAsdAsdGsdGsdC*sdAsdTs**TbsTb** |
| 14 | 61f | **TbsAbsC*bs**dAsdAsdGsdC*sdAsdAsdGsdGsdC*s**AbsTb** |
| 14 | 62f | **AbsC*bsAbs**dAsdGsdC*sdAsdAsdGsdGsdC*sdAs**TbsTb** |
| 14 | 63f | **C*bsAbsAbs**dGsdC*sdAsdAsdGsdGsdC*sdAsdTs**TbsTb** |
| 14 | 61g | **TbsAbs**dC*sdAsdAsdGsdC*sdAsdAsdGsdGs**C*bsAbsTb** |
| 14 | 62g | **AbsC*bs**dAsdAsdGsdC*sdAsdAsdGsdGsdC*s**AbsTbsTb** |
| 14 | 63g | **C*bsAbs**dAsdGsdC*sdAsdAsdGsdGsdC*sdAs**TbsTbsTb** |
| 14 | 61h | **TbsAbsC*bs**dAsdAsdGsdC*sdAsdAsdGsdGs**C*****bsAbsTb** |
| 14 | 62h | **AbsC*bsAbs**dAsdGsdC*sdAsdAsdGsdGsdC*s**AbsTbsTb** |
| 14 | 63h | **C*bsAbsAbs**dGsdC*sdAsdAsdGsdGsdC*sdAs**TbsTbsTb** |
| 15 | 49e | **TbsAbs**dC*sdAsdAsdGsdC*sdAsdAsdGsdGsdC*sdAs**TbsTbs** |
| 15 | 50e | **AbsC*bs**dAsdAsdGsdC*sdAsdAsdGsdGsdC*sdAsdTs**TbsTb** |
| 15 | 49f | **TbsAbsC*bs**dAsdAsdGsdC*sdAsdAsdGsdGsdC*sdAs**TbsTbs** |
| 15 | 50f | **AbsC*bsAbs**dAsdGsdC*sdAsdAsdGsdGsdC*sdAsdTs**TbsTb** |
| 15 | 49g | **TbsAbs**dC*sdAsdAsdGsdC*sdAsdAsdGsdGsdC*s**AbsTbsTbs** |
| 15 | 50g | **AbsC*bs**dAsdAsdGsdC*sdAsdAsdGsdGsdC*sdAs**TbsTbsTb** |
| 15 | 49h | **TbsAbsC*bs**dAsdAsdGsdC*sdAsdAsdGsdGsdC*s**AbsTbsTbs** |
| 15 | 50h | **AbsC*bsAbs**dAsdGsdC*sdAsdAsdGsdGsdC*sdAs**TbsTbsTb** |
| 16 | 5l | **TbsAbs**dC*sdAsdAsdGsdC*sdAsdAsdGsdGsdC*sdAsdTs**TbsTb** |
| 16 | 5m | **TbsAbsC*bs**dAsdAsdGsdC*sdAsdAsdGsdGsdC*sdAsdTs**TbsTb** |
| 16 | 5n | **TbsAbsC*bsAbs**dAsdGsdC*sdAsdAsdGsdGsdC*sdAsdTs**TbsTb** |
| 16 | 5ο | **TbsAbs**dC*sdAsdAsdGsdC*sdAsdAsdGsdGsdC*sdAs**TbsTbsTb** |
| 16 | 5p | **TbsAbs**dC*sdAsdAsdGsdC*sdAsdAsdGsdGsdC*s**AbsTbsTbsTb** |
| 16 | 5q | **TbsAbsC*bs**dAsdAsdGsdC*sdAsdAsdGsdGsdC*sdAs**TbsTbsTb** |
| 16 | 5r | **TbsAbsC*bsAbs**dAsdGsdC*sdAsdAsdGsdGsdC*sdAs**TbsTbsTb** |
| 16 | 5s | **TbsAbsC*bs**dAsdAsdGsdC*sdAsdAsdGsdGsdC*s**AbsTbsTbsTb** |
| 16 | 5t | **TbsAbsC*bsAbs**dAsdGsdC*sdAsdAsdGsdGsdC*s**AbsTbsTbsTb** |
| 16 | 5z | **TbsAbsC*bsAbs**dAsdGsdC*sdAsdAsdGsdGsdC*sdAs**TbsTbsTb** |
| 16 | 5v | **TbsAbsC*bs**dAsdAsdGsdC*sdAsdAsdGsdGsdC*s**AbsTbsTbsTb** |

**Table 23**

| **L** | **Seq ID No.** | **Sequence, 5'-3'** |
|---|---|---|
| 12 | 74d | **TbsGbs**dC*sdAsdAsdAsdAsdTsdTsdC*s**AbsGb** |
| 12 | 75d | **AbsC*bs**dAsdTsdTsdGsdC*****sdAsdAsdAs**AbsTb** |
| 12 | 76d | **C*bsAbs**dTsdTsdGsdC*sdAsdAsdAsdAs**TbsTb** |
| 12 | 77d | **AbsTbs**dTsdGsdC*sdAsdAsdAsdAsdTs**TbsC*b** |
| 12 | 78d | **TbsTbs**dGsdC*sdAsdAsdAsdAsdTsdTs**C*bsAb** |
| 12 | 74e | **TbsGbsC*bs**dAsdAsdAsdAsdTsdTsdC*s**AbsGb** |
| 12 | 75e | **AbsC*bsAbs**dTsdTsdGsdC*sdAsdAsdAs**AbsTb** |
| 12 | 76e | **C*bsAbsTbs**dTsdGsdC*sdAsdAsdAsdAs**TbsTb** |
| 12 | 77e | **AbsTbsTbs**dGsdC*sdAsdAsdAsdAsdTs**TbsC*b** |
| 12 | 78e | **TbsTbsGbs**dC*sdAsdAsdAsdAsdTsdTs**C*bsAb** |
| 12 | 74f | **TbsGbs**dC*sdAsdAsdAsdAsdTsdTs**C*bsAbsGb** |
| 12 | 75f | **AbsC*bs**dAsdTsdTsdGsdC*sdAsdAs**AbsAbsTb** |
| 12 | 76f | **C*bsAbs**dTsdTsdGsdC*sdAsdAsdAs**AbsTbsTb** |
| 12 | 77f | **AbsTbs**dTsdGsdC*sdAsdAsdAsdAs**TbsTbsC*b** |
| 12 | 78f | **TbsTbs**dGsdC*sdAsdAsdAsdAsdTs**TbsC*bsAb** |
| 11 | 81c | **Abs**dC*sdAsdTsdTsdGsdC*sdAsdAs**AbsAb** |
| 11 | 82c | **C*bs**dAsdTsdTsdGsdC*sdAsdAsdAs**AbsTb** |
| 11 | 83c | **Abs**dTsdTsdGsdC*sdAsdAsdAsdAs**TbsTb** |
| 11 | 84c | **Tbs**dTsdGsdC*sdAsdAsdAsdAsdTs**TbsC*b** |
| 11 | 85c | **Tbs**dGsdC*sdAsdAsdAsdAsdTsdTs**C*bsAb** |
| 11 | 86c | **Gbs**dC*sdAsdAsdAsdAsdTsdTsdC*s**AbsGb** |
| 11 | 81d | **AbsC*bs**dAsdTsdTsdGsdC*sdAsdAsdAs**Ab** |
| 11 | 82d | **C*bsAbs**dTsdTsdGsdC*sdAsdAsdAsdAs**Tb** |
| 11 | 83d | **AbsTbs**dTsdGsdC*sdAsdAsdAsdAsdTs**Tb** |
| 11 | 84d | **TbsTbs**dGsdC*sdAsdAsdAsdAsdTsdTs**C*b** |
| 11 | 85d | **TbsGbs**dC*sdAsdAsdAsdAsdTsdTsdC*s**Ab** |
| 11 | 86d | **GbsC*bs**dAsdAsdAsdAsdTsdTsdC*sdAs**Gb** |
| 10 | 67b | **Abs**dC*sdAsdTsdTsdGsdC*sdAsdAs**Ab** |
| 10 | 68b | **C*bs**dAsdTsdTsdGsdC*sdAsdAsdAs**Ab** |
| 10 | 69b | **Abs**dTsdTsdGsdC*sdAsdAsdAsdAs**Tb** |
| 10 | 70b | **Tbs**dTsdGsdC*sdAsdAsdAsdAsdTs**Tb** |
| 10 | 71b | **Tbs**dGsdC*sdAsdAsdAsdAsdTsdTs**C*b** |
| 10 | 72b | **Gbs**dC*sdAsdAsdAsdAsdTsdTsdC*s**Ab** |
| 10 | 73b | **C*bs**dAsdAsdAsdAsdTsdTsdC*sdAs**Gb** |
| 13 | 87d | **AbsC*bs**dAsdTsdTsdGsdC*sdAsdAsdAsdAs**TbsTb** |
| 13 | 88d | **C*bsAbs**dTsdTsdGsdC*sdAsdAsdAsdAsdTs**TbsC*b** |
| 13 | 89d | **AbsTbs**dTsdGsdC*sdAsdAsdAsdAsdTsdTs**C*bsAb** |
| 13 | 90d | **TbsTbs**dGsdC*sdAsdAsdAsdAsdTsdTsdC*s**AbsGb** |
| 13 | 87e | **AbsC*****bsAbs**dTsdTsdGsdC*sdAsdAsdAsdAs**TbsTb** |
| 13 | 88e | **C*bsAbsTbs**dTsdGsdC*sdAsdAsdAsdAsdTs**TbsC*b** |
| 13 | 89e | **AbsTbsTbs**dGsdC*sdAsdAsdAsdAsdTsdTs**C*bsAb** |
| 13 | 90e | **TbsTbsGbs**dC*sdAsdAsdAsdAsdTsdTsdC*s**AbsGb** |
| 13 | 87f | **AbsC*bs**dAsdTsdTsdGsdC*sdAsdAsdAs**AbsTbsTb** |
| 13 | 88f | **C*bsAbs**dTsdTsdGsdC*sdAsdAsdAsdAs**TbsTbsC*b** |
| 13 | 89f | **AbsTbs**dTsdGsdC*sdAsdAsdAsdAsdTs**TbsC*bsAb** |
| 13 | 90f | **TbsTbs**dGsdC*sdAsdAsdAsdAsdTsdTs**C*bsAbsGb** |
| 14 | 91e | **AbsC*bs**dAsdTsdTsdGsdC*sdAsdAsdAsdAsdTs**TbsC*b** |
| 14 | 92e | **C*bsAbs**dTsdTsdGsdC*sdAsdAsdAsdAsdTsdTs**C*bsAb** |
| 14 | 93e | **AbsTbs**dTsdGsdC*sdAsdAsdAsdAsdTsdTsdC*s**AbsGb** |
| 14 | 91f | **AbsC*bsAbs**dTsdTsdGsdC*sdAsdAsdAsdAsdTs**TbsC*b** |
| 14 | 92f | **C*bsAbsTbs**dTsdGsdC*sdAsdAsdAsdAsdTsdTs**C*bsAb** |
| 14 | 93f | **AbsTbsTbs**dGsdC*sdAsdAsdAsdAsdTsdTsdC*s**AbsGb** |
| 14 | 91g | **AbsC*bs**dAsdTsdTsdGsdC*sdAsdAsdAsdAs**TbsTbsC*b** |
| 14 | 92g | **C*bsAbs**dTsdTsdGsdC*sdAsdAsdAsdAsdTs**TbsC*bsAb** |
| 14 | 93g | **AbsTbs**dTsdGsdC*sdAsdAsdAsdAsdTsdTs**C*bsAbsGb** |
| 14 | 91h | **AbsC*bsAbs**dTsdTsdGsdC*sdAsdAsdAsdAs**TbsTbsC*b** |
| 14 | 92h | **C*bsAbsTbs**dTsdGsdC*sdAsdAsdAsdAsdTs**TbsC*bsAb** |
| 14 | 93h | **AbsTbsTbs**dGsdC*sdAsdAsdAsdAsdTsdTs**C*bsAbsGb** |
| 15 | 79e | **AbsC*bs**dAsdTsdTsdGsdC*sdAsdAsdAsdAsdTsdTs**C*bsAb** |
| 15 | 80e | **C*bsAbs**dTsdTsdGsdC*sdAsdAsdAsdAsdTsdTsdC*s**AbsGb** |
| 15 | 79f | **AbsC*bsAbs**dTsdTsdGsdC*sdAsdAsdAsdAsdTsdTs**C*bsAb** |
| 15 | 80f | **C*bsAbsTbs**dTsdGsdC*sdAsdAsdAsdAsdTsdTsdC*s**AbsGb** |
| 15 | 79g | **AbsC*bsAbs**dTsdTsdGsdC*sdAsdAsdAsdAsdTs**TbsC*bsAb** |
| 15 | 80g | **C*bsAbsTbs**dTsdGsdC*sdAsdAsdAsdAsdTsdTs**C*bsAbsGb** |
| 15 | 79h | **AbsC*bs**dAsdTsdTsdGsdC*sdAsdAsdAsdAsdTs**TbsC*bsAb** |
| 15 | 80h | **C*bsAbs**dTsdTsdGsdC*sdAsdAsdAsdAsdTsdTs**C*bsAbsGb** |
| 16 | 6l | **AbsC*bs**dAsdTsdTsdGsdC*sdAsdAsdAsdAsdTsdTsdC*s**AbsGb** |
| 16 | 6m | **AbsC*bsAbs**dTsdTsdGsdC*sdAsdAsdAsdAsdTsdTsdC*s**AbsGb** |
| 16 | 6n | **AbsC*bsAbsTbs**dTsdGsdC*sdAsdAsdAsdAsdTsdTsdC*s**AbsGb** |
| 16 | 6o | **AbsC*bsAbs**dTsdTsdGsdC*sdAsdAsdAsdAsdTsdTs**C*bsAbsGb** |
| 16 | 6p | **AbsC*bsAbsTbs**dTsdGsdC*sdAsdAsdAsdAsdTsdTs**C*bsAbsGb** |
| 16 | 6q | **AbsC*bs**dAsdTsdTsdGsdC*sdAsdAsdAsdAsdTsdTs**C*bsAbsGb** |
| 16 | 6r | **AbsC*bs**dAsdTsdTsdGsdC*sdAsdAsdAsdAsdTs**TbsC*bsAbsGb** |
| 16 | 6s | **AbsC*bsAbs**dTsdTsdGsdC*sdAsdAsdAsdAsdTs**TbsC*bsAbsGb** |
| 16 | 6t | **AbsC*bsAbsTbs**dTsdGsdC*sdAsdAsdAsdAsdTs**TbsC*bsAbsGb** |
| 16 | 6u | **AbsC*bsAbs**dTsdTsdGsdC*sdAsdAsdAsdAs**TbsTbsC*bsAbsGb** |
| 16 | 6v | **AbsC*bsAbsTbsTbs**dGsdC*sdAsdAsdAsdAsdTsdTs**C*bsAbsGb** |

**Table 24**

| **L** | **Seq ID No.** | **Sequence, 5'-3'** |
|---|---|---|
| 12 | 16g | **GbGb**dTsdTsdAsdGsdGsdGsdCsdTs**GbAb** |
| 12 | 15g | **AbGb**dGsdTsdTsdAsdGsdGsdGsdCsd**TbGb** |
| 12 | 17g | **GbTb**dTsdAsdGsdGsdGsdCsdTsdGs**AbAb** |
| 12 | 18g | **TbTb**dAsdGsdGsdGsdCsdTsdGsdAs**AbTb** |
| 12 | 36g | **TbAb**dGsdGsdGsdCsdTsdGsdAsdAs**TbTb** |
| 12 | 16h | **Gb**dGsdTsdTsdAsdGsdGsdGsdCsdTs**GbAb** |
| 12 | 15h | **Ab**dGsdGsdTsdTsdAsdGsdGsdGsdCsd**TbGb** |
| 12 | 17h | **Gb**dTsdTsdAsdGsdGsdGsdCsdTsdGs**AbAb** |
| 12 | 18h | **Tb**dTsdAsdGsdGsdGsdCsdTsdGsdAs**AbTb** |
| 12 | 36h | **Tb**dAsdGsdGsdGsdCsdTsdGsdAsdAs**TbTb** |
| 12 | 16i | **GbGb**dTsdTsdAsdGsdGsdGsdCsdTsdGs**Ab** |
| 12 | 15i | **AbGb**dGsdTsdTsdAsdGsdGsdGsdCsddTs**Gb** |
| 12 | 17i | **GbTb**dTsdAsdGsdGsdGsdCsdTsdGsdAs**Ab** |
| 12 | 18i | **TbTb**dAsdGsdGsdGsdCsdTsdGsdAsdAs**Tb** |
| 12 | 36i | **TbAb**dGsdGsdGsdCsdTsdGsdAsdAsdTs**Tb** |
| 11 | 33e | **AbGb**dGsdTsdTsdAsdGsdGsdGsdCs**Tb** |
| 11 | 33f | **Ab**dGsdGsdTsdTsdAsdGsdGsdGsd**CbTb** |
| 11 | 21e | **GbGb**dTsdTsdAsdGsdGsdGsdCsdTs**Gb** |
| 11 | 21f | **Gb**dGsdTsdTsdAsdGsdGsdGsdCs**TbGb** |
| 11 | 22e | **Gb**dTsdTsdAsdGsdGsdGsdCsdTs**GbAb** |
| 11 | 22f | **GbTb**dTsdAsdGsdGsdGsdCsdTsdGs**Ab** |
| 11 | 23e | **TbTb**dAsdGsdGsdGsdCsdTsdGsdAs**Ab** |
| 11 | 23f | **Tb**dTsdAsdGsdGsdGsdCsdTsdGs**AbAb** |
| 11 | 24e | **TbAb**dGsdGsdGsdCsdTsdGsdAsdAs**Tb** |
| 11 | 24f | **Tb**dAsdGsdGsdGsdCsdTsdGsdAs**AbTb** |
| 11 | 25e | **Ab**dGsdGsdGsdCsdTsdGsdAsdAs**TbTb** |
| 11 | 25f | **AbGb**dGsdGsdCsdTsdGsdAsdAsdTs**Tb** |
| 10 | 8c | **Ab**dGsdGsdTsdTsdAsdGsdGsdGsd**Cb** |
| 10 | 9c | **Gb**dGsdTsdTsdAsdGsdGsdGsdCs**Tb** |
| 10 | 10c | **Gb**dTsdTsdAsdGsdGsdGsdCsdTs**Gb** |
| 10 | 11c | **Tb**dTsdAsdGsdGsdGsdCsdTsdGs**Ab** |
| 10 | 12c | **Tb**dAsdGsdGsdGsdCsdTsdGsdAs**Ab** |
| 10 | 13c | **Ab**dGsdGsdGsdCsdTsdGsdAsdAs**Tb** |
| 10 | 14c | **Gb**dGsdGsdCsdTsdGsdAsdAsdTs**Tb** |
| 13 | 26g | **AbGb**dGsdTsdTsdAsdGsdGsdGsdCsdTs**GbAb** |
| 13 | 27g | **GbGb**dTsdTsdAsdGsdGsdGsdCsdTsdGs**AbAb** |
| 13 | 28g | **GbTb**dTsdAsdGsdGsdGsdCsdTsdGsdAs**AbTb** |
| 13 | 29g | **TbTb**dAsdGsdGsdGsdCsdTsdGsdAsdAs**TbTb** |
| 13 | 26h | **AbGbGb**dTsdTsdAsdGsdGsdGsdCsdTs**GbAb** |
| 13 | 27h | **GbGbTb**dTsdAsdGsdGsdGsdCsdTsdGs**AbAb** |
| 13 | 28h | **GbTbTb**dAsdGsdGsdGsdCsdTsdGsdAs**AbTb** |
| 13 | 29h | **TbTbAb**dGsdGsdGsdCsdTsdGsdAsdAs**TbTb** |
| 13 | 26i | **AbGb**dGsdTsdTsdAsdGsdGsdGs**CbTbGbAb** |
| 13 | 27i | **GbGb**dTsdTsdAsdGsdGsdGsdCsdTs**GbAbAb** |
| 13 | 28i | **GbTb**dTsdAsdGsdGsdGsdCsdTsdGs**AbAbTb** |
| 13 | 29i | **TbTb**dAsdGsdGsdGsdCsdTsdGsdAs**AbTbTb** |
| 14 | 30i | **AbGb**dGsdTsdTsdAsdGsdGsdGsdCsdTs**GbAbAb** |
| 14 | 31i | **GbGb**dTsdTsdAsdGsdGsdGsdCsdTsdGs**AbAbTb** |
| 14 | 32i | **GbTb**dTsdAsdGsdGsdGsdCsdTsdGsdAs**AbTbTb** |
| 14 | 30j | **AbGbGb**dTsdTsdAsdGsdGsdGsdCsdTs**GbAbAb** |
| 14 | 31j | **GbGbTb**dTsdAsdGsdGsdGsdCsdTsdGs**AbAbTb** |
| 14 | 32j | **GbTbTb**dAsdGsdGsdGsdCsdTsdGsdAs**AbTbTb** |
| 14 | 30k | **AbGb**dGsdTsdTsdAsdGsdGsdGsdCsdTs**GbAbAb** |
| 14 | 31k | **GbGb**dTsdTsdAsdGsdGsdGsdCsdTsdGs**AbAbTb** |
| 14 | 32k | **GbTb**dTsdAsdGsdGsdGsdCsdTsdGsdAs**AbTbTb** |
| 14 | 30l | **AbGb**dGsdTsdTsdAsdGsdGsdGsdCsdTsdGs**AbAb** |
| 14 | 311 | **GbGb**dTsdTsdAsdGsdGsdGsdCsdTsdGsdAs**AbTb** |
| 14 | 321 | **GbTb**dTsdAsdGsdGsdGsdCsdTsdGsdAsdAs**TbTb** |
| 15 | 19i | **AbGb**dGsdTsdTsdAsdGsdGsdGsdCsdTs**GbAbAbTb** |
| 15 | 20i | **GbGb**dTsdTsdAsdGsdGsdGsdCsdTsdGs**AbAbTbTb** |
| 15 | 19j | **AbGb**dGsdTsdTsdAsdGsdGsdGsdCsdTsdGsdAs**AbTb** |
| 15 | 20j | **GbGb**dTsdTsdAsdGsdGsdGsdCsdTsdGsdAsdAs**TbTb** |
| 15 | 19k | **AbGbGb**dTsdTsdAsdGsdGsdGsdCsdTsdGs**AbAbTb** |
| 15 | 20k | **GbGbTb**dTsdAsdGsdGsdGsdCsdTsdGsdAs**AbTbTb** |
| 15 | 191 | **AbGbGbTb**dTsdAsdGsdGsdGsdCsdTsdGs**AbAbTb** |
| 15 | 20l | **GbGbTbTb**dAsdGsdGsdGsdCsdTsdGsdAs**AbTbTb** |
| 16 | 4w | **AbGb**dGsdTsdTsdAsdGsdGsdGsdCsdTsdGsdAs**AbTbTb** |
| 16 | 4x | **AbGb**dGsdTsdTsdAsdGsdGsdGsdCsdTsdGsdAsdAs**TbTb** |
| 16 | 4y | **AbGbGb**dTsdTsdAsdGsdGsdGsdCsdTsdGsdAsdAs**TbTb** |
| 16 | 4z | **AbGbGbTb**dTsdAsdGsdGsdGsdCsdTsdGsdAsdAs**TbTb** |
| 16 | 4aa | **AbGb**dGsdTsdTsdAsdGsdGsdGsdCsdTsdGs**AbAbTbTb** |
| 16 | 4ab | **AbGbGb**dTsdTsdAsdGsdGsdGsdCsdTsdGsdAs**AbTbTb** |
| 16 | 4ac | **AbGbGbTb**dTsdAsdGsdGsdGsdCsdTsdGs**AbAbTbTb** |
| 16 | 4ad | **AbGbGbTbTb**dAsdGsdGsdGsdCsdTsdGsdAs**AbTbTb** |
| 16 | 4ae | **AbGbGb**dTsdTsdAsdGsdGsdGsdCsdTs**GbAbAbTbTb** |
| 16 | 4af | **AbGbGb**dTsdTsdAsdGsdGsdGsdCsdTsdGs**AbAbTbTb** |
| 16 | 4ag | **AbGbGbTb**dTsdAsdGsdGsdGsdCsdTsdGs**dAsAbTbTb** |

**Table 25**

| **L** | **Seq ID No.** | **Sequence, 5'-3'** |
|---|---|---|
| 12 | 46g | **CbAb**dAsdGsdCsdAsdAsdGsdGsdCs**AbTb** |
| 12 | 45g | **AbCb**dAsdAsdGsdCsdAsdAsdGsdGs**CbAb** |
| 12 | 44g | **TbAb**dCsdAsdAsdGsdCsdAsdAsdGs**GbCb** |
| 12 | 47g | **AbAb**dGsdCsdAsdAsdGsdGsdCsdAs**TbTb** |
| 12 | 48g | **AbGb**dCsdAsdAsdGsdGsdCsdAsdTs**TbTb** |
| 12 | 46h | **Cb**dAsdAsdGsdCsdAsdAsdGsdGsdCs**AbTb** |
| 12 | 45h | **Ab**dCsdAsdAsdGsdCsdAsdAsdGsdGs**CbAb** |
| 12 | 44h | **Tb**dAsdCsdAsdAsdGsdCsdAsdAsdGs**GbCb** |
| 12 | 47h | **Ab**dAsdGsdCsdAsdAsdGsdGsdCsdAs**TbTb** |
| 12 | 48h | **Ab**dGsdCsdAsdAsdGsdGsdCsdAsdTs**TbTb** |
| 12 | 46i | **CbAb**dAsdGsdCsdAsdAsdGsdGsdCsdAs**Tb** |
| 12 | 45i | **AbCb**dAsdAsdGsdCsdAsdAsdGsdGsdCs**Ab** |
| 12 | 44i | **TbAb**dCsdAsdAsdGsdCsdAsdAsdGsdGs**Cb** |
| 12 | 47i | **AbAb**dGsdCsdAsdAsdGsdGsdCsdAsdTs**Tb** |
| 12 | 48i | **AbGb**dCsdAsdAsdGsdGsdCsdAsdTsdTs**Tb** |
| 11 | 51e | **Tb**dAsdCsdAsdAsdGsdCsdAsdAs**GbGb** |
| 11 | 52e | **Ab**dCsdAsdAsdGsdCsdAsdAsdGs**GbCb** |
| 11 | 53e | **Cb**dAsdAsdGsdCsdAsdAsdGsdGs**CbAb** |
| 11 | 54e | **Ab**dAsdGsdCsdAsdAsdGsdGsdCs**AbTb** |
| 11 | 55e | **Ab**dGsdCsdAsdAsdGsdGsdCsdAsd**TbTb** |
| 11 | 56e | **Gb**dCsdAsdAsdGsdGsdCsdAsdTs**TbTb** |
| 11 | 51f | **TbAb**dCsdAsdAsdGsdCsdAsdAsdGs**Gb** |
| 11 | 52f | **AbCb**dAsdAsdGsdCsdAsdAsdGsdGs**Cb** |
| 11 | 53f | **CbAb**dAsdGsdCsdAsdAsdGsdGsdCs**Ab** |
| 11 | 54f | **AbAb**dGsdCsdAsdAsdGsdGsdCsdAs**Tb** |
| 11 | 55f | **AbGb**dCsdAsdAsdGsdGsdCsdAsddTs**Tb** |
| 11 | 56f | **GbCb**dAsdAsdGsdGsdCsdAsdTsdTs**Tb** |
| 10 | 37c | **Tb**dAsdCsdAsdAsdGsdCsdAsdAs**Gb** |
| 10 | 38c | **Ab**dCsdAsdAsdGsdCsdAsdAsdGs**Gb** |
| 10 | 39c | **Cb**dAsdAsdGsdCsdAsdAsdGsdGs**Cb** |
| 10 | 40c | **Ab**dAsdGsdCsdAsdAsdGsdGsdCs**Ab** |
| 10 | 41c | **Ab**dGsdCsdAsdAsdGsdGsdCsdAsd**Tb** |
| 10 | 42c | **Gb**dCsdAsdAsdGsdGsdCsdAsdTs**Tb** |
| 10 | 43c | **Cb**dAsdAsdGsdGsdCsdAsdTsdTs**Tb** |
| 13 | 57g | **TbAb**dCsdAsdAsdGsdCsdAsdAsdGsdGs**CbAb** |
| 13 | 58g | **AbCb**dAsdAsdGsdCsdAsdAsdGsdGsdCs**AbTb** |
| 13 | 59g | **CbAb**dAsdGsdCsdAsdAsdGsdGsdCsdAs**TbTb** |
| 13 | 60g | **AbAb**dGsdCsdAsdAsdGsdGsdCsdAsdTs**TbTb** |
| 13 | 57h | **TbAbCb**dAsdAsdGsdCsdAsdAsdGsdGs**CbAb** |
| 13 | 58h | **AbCbAb**dAsdGsdCsdAsdAsdGsdGsdCs**AbTb** |
| 13 | 59h | **CbAbAb**dGsdCsdAsdAsdGsdGsdCsdAs**TbTb** |
| 13 | 60h | **AbAbGb**dCsdAsdAsdGsdGsdCsdAsdTs**TbTb** |
| 13 | 57i | **TbAb**dCsdAsdAsdGsdCsdAsdAsdGs**GbCbAb** |
| 13 | 58i | **AbCb**dAsdAsdGsdCsdAsdAsdGsdGs**CbAbTb** |
| 13 | 59i | **CbAb**dAsdGsdCsdAsdAsdGsdGsdCs**AbTbTb** |
| 13 | 60i | **AbAb**dGsdCsdAsdAsdGsdGsdCsdAs**TbTbTb** |
| 14 | 61i | **TbAb**dCsdAsdAsdGsdCsdAsdAsdGsdGsdCs**AbTb** |
| 14 | 62i | **AbCb**dAsdAsdGsdCsdAsdAsdGsdGsdCsdAs**TbTb** |
| 14 | 63i | **CbAb**dAsdGsdCsdAsdAsdGsdGsdCsdAsdTs**TbTb** |
| 14 | 61j | **TbAbCb**dAsdAsdGsdCsdAsdAsdGsdGsdCs**AbTb** |
| 14 | 62j | **AbCbAb**dAsdGsdCsdAsdAsdGsdGsdCsdAs**TbTb** |
| 14 | 63j | **CbAbAb**dGsdCsdAsdAsdGsdGsdCsdAsdTs**TbTb** |
| 14 | 61k | **TbAb**dCsdAsdAsdGsdCsdAsdAsdGsdGs**CbAbTb** |
| 14 | 62k | **AbCb**dAsdAsdGsdCsdAsdAsdGsdGsdCs**AbTbTb** |
| 14 | 63k | **CbAb**dAsdGsdCsdAsdAsdGsdGsdCsdAs**TbTbTb** |
| 14 | 611 | **TbAbCb**dAsdAsdGsdCsdAsdAsdGsdGs**CbAbTb** |
| 14 | 621 | **AbCbAb**dAsdGsdCsdAsdAsdGsdGsdCs**AbTbTb** |
| 14 | 63l | **CbAbAb**dGsdCsdAsdAsdGsdGsdCsdAs**TbTbTb** |
| 15 | 49i | **TbAb**dCsdAsdAsdGsdCsdAsdAsdGsdGsdCsdAs**TbTb** |
| 15 | 50i | **AbCb**dAsdAsdGsdCsdAsdAsdGsdGsdCsdAsdTs**TbTb** |
| 15 | 49j | **TbAbCb**dAsdAsdGsdCsdAsdAsdGsdGsdCsdAs**TbTb** |
| 15 | 50j | **AbCbAb**dAsdGsdCsdAsdAsdGsdGsdCsdAsdTs**TbTb** |
| 15 | 49k | **TbAb**dCsdAsdAsdGsdCsdAsdAsdGsdGsdCs**AbTbTb** |
| 15 | 50k | **AbCb**dAsdAsdGsdCsdAsdAsdGsdGsdCsdAs**TbTbTb** |
| 15 | 49l | **TbAbCb**dAsdAsdGsdCsdAsdAsdGsdGsdCs**AbTbTb** |
| 15 | 50l | **AbCbAb**dAsdGsdCsdAsdAsdGsdGsdCsdAs**TbTbTb** |
| 16 | 5w | **TbAb**dCsdAsdAsdGsdCsdAsdAsdGsdGsdCsdAsdTs**TbTb** |
| 16 | 5x | **TbAbCb**dAsdAsdGsdCsdAsdAsdGsdGsdCsdAsdTs**TbTb** |
| 16 | 5y | **TbAbCbAb**dAsdGsdCsdAsdAsdGsdGsdCsdAsdTs**TbTb** |
| 16 | 5z | **TbAb**dCsdAsdAsdGsdCsdAsdAsdGsdGsdCsdAs**TbTbTb** |
| 16 | 5aa | **TbAb**dCsdAsdAsdGsdCsdAsdAsdGsdGsdCs**AbTbTbTb** |
| 16 | 5ab | **TbAbCb**dAsdAsdGsdCsdAsdAsdGsdGsdCsdAs**TbTbTb** |
| 16 | 5ac | **TbAbCbAb**dAsdGsdCsdAsdAsdGsdGsdCsdAs**TbTbTb** |
| 16 | 5ad | **TbAbCb**dAsdAsdGsdCsdAsdAsdGsdGsdCs**AbTbTbTb** |
| 16 | 5ae | **TbAbCbAb**dAsdGsdCsdAsdAsdGsdGsdCs**AbTbTbTb** |
| 16 | 5af | **TbAbCbAb**dAsdGsdCsdAsdAsdGsdGsdCsdAs**TbTbTb** |
| 16 | 5ag | **TbAbCb**dAsdAsdGsdCsdAsdAsdGsdGsdCs**AbTbTbTb** |

**Table 26**

| **L** | **Seq ID No.** | **Sequence, 5'-3'** |
|---|---|---|
| 12 | 74g | **TbGb**dCsdAsdAsdAsdAsdTsdTsdCs**AbGb** |
| 12 | 75g | **AbCb**dAsdTsdTsdGsdCsdAsdAsdAs**AbTb** |
| 12 | 76g | **CbAb**dTsdTsdGsdCsdAsdAsdAsdAs**TbTb** |
| 12 | 77g | **AbTb**dTsdGsdCsdAsdAsdAsdAsdTs**TbCb** |
| 12 | 78g | **TbTb**dGsdCsdAsdAsdAsdAsdTsdTs**CbAb** |
| 12 | 74h | **TbGbCb**dAsdAsdAsdAsdTsdTsdCs**AbGb** |
| 12 | 75h | **AbCbAb**dTsdTsdGsdCsdAsdAsdAs**AbTb** |
| 12 | 76h | **CbAbTb**dTsdGsdCsdAsdAsdAsdAs**TbTb** |
| 12 | 77h | **AbTbTb**dGsdCsdAsdAsdAsdAsdTs**TbCb** |
| 12 | 78h | **TbTbGb**dCsdAsdAsdAsdAsdTsdTs**CbAb** |
| 12 | 74i | **TbGb**dCsdAsdAsdAsdAsdTsdTs**CbAbGb** |
| 12 | 75i | **AbCb**dAsdTsdTsdGsdCsdAsdAs**AbAbTb** |
| 12 | 76i | **CbAb**dTsdTsdGsdCsdAsdAsdAs**AbTbTb** |
| 12 | 77i | **AbTb**dTsdGsdCsdAsdAsdAsdAs**TbTbCb** |
| 12 | 78i | **TbTb**dGsdCsdAsdAsdAsdAsdTs**TbCbAb** |
| 11 | 81e | **Ab**dCsdAsdTsdTsdGsdCsdAsdAs**AbAb** |
| 11 | 82e | **Cb**dAsdTsdTsdGsdCsdAsdAsdAs**AbTb** |
| 11 | 83e | **Ab**dTsdTsdGsdCsdAsdAsdAsdAs**TbTb** |
| 11 | 84e | **Tb**dTsdGsdCsdAsdAsdAsdAsdTs**TbCb** |
| 11 | 85e | **Tb**dGsdCsdAsdAsdAsdAsdTsdTs**CbAb** |
| 11 | 86e | **Gb**dCsdAsdAsdAsdAsdTsdTsdCs**AbGb** |
| 11 | 81f | **AbCb**dAsdTsdTsdGsdCsdAsdAsdAs**Ab** |
| 11 | 82f | **CbAb**dTsdTsdGsdCsdAsdAsdAsdAs**Tb** |
| 11 | 83f | **AbTb**dTsdGsdCsdAsdAsdAsdAsdTs**Tb** |
| 11 | 84f | **TbTb**dGsdCsdAsdAsdAsdAsdTsdTs**Cb** |
| 11 | 85f | **TbGb**dCsdAsdAsdAsdAsdTsdTsdCs**Ab** |
| 11 | 86f | **GbCb**dAsdAsdAsdAsdTsdTsdCsdAs**Gb** |
| 10 | 67c | **Ab**dCsdAsdTsdTsdGsdCsdAsdAs**Ab** |
| 10 | 68c | **Cb**dAsdTsdTsdGsdCsdAsdAsdAs**Ab** |
| 10 | 69c | **Ab**dTsdTsdGsdCsdAsdAsdAsdAs**Tb** |
| 10 | 70c | **Tb**dTsdGsdCsdAsdAsdAsdAsdTs**Tb** |
| 10 | 71c | **Tb**dGsdCsdAsdAsdAsdAsdTsdTs**Cb** |
| 10 | 72c | **Gb**dCsdAsdAsdAsdAsdTsdTsdCs**Ab** |
| 10 | 73c | **Cb**dAsdAsdAsdAsdTsdTsdCsdAs**Gb** |
| 13 | 87g | **AbCb**dAsdTsdTsdGsdCsdAsdAsdAsdAs**TbTb** |
| 13 | 88g | **CbAb**dTsdTsdGsdCsdAsdAsdAsdAsdTs**TbCb** |
| 13 | 89g | **AbTb**dTsdGsdCsdAsdAsdAsdAsdTsdTs**CbAb** |
| 13 | 90g | **TbTb**dGsdCsdAsdAsdAsdAsdTsdTsdCs**AbGb** |
| 13 | 87h | **AbCbAb**dTsdTsdGsdCsdAsdAsdAsdAs**TbTb** |
| 13 | 88h | **CbAbTb**dTsdGsdCsdAsdAsdAsdAsdTs**TbCb** |
| 13 | 89h | **AbTbTb**dGsdCsdAsdAsdAsdAsdTsdTs**CbAb** |
| 13 | 90h | **TbTbGb**dCsdAsdAsdAsdAsdTsdTsdCs**AbGb** |
| 13 | 87i | **AbCb**dAsdTsdTsdGsdCsdAsdAsdAs**AbTbTb** |
| 13 | 88i | **CbAb**dTsdTsdGsdCsdAsdAsdAsdAs**TbTbCb** |
| 13 | 89i | **AbTb**dTsdGsdCsdAsdAsdAsdAsdTs**TbCbAb** |
| 13 | 90i | **TbTb**dGsdCsdAsdAsdAsdAsdTsdTs**CbAbGb** |
| 14 | 91i | **AbCb**dAsdTsdTsdGsdCsdAsdAsdAsdAsdTs**TbCb** |
| 14 | 92i | **CbAb**dTsdTsdGsdCsdAsdAsdAsdAsdTsdTs**CbAb** |
| 14 | 93i | **AbTb**dTsdGsdCsdAsdAsdAsdAsdTsdTsdCs**AbGb** |
| 14 | 91j | **AbCbAb**dTsdTsdGsdCsdAsdAsdAsdAsdTs**TbCb** |
| 14 | 92j | **CbAbTb**dTsdGsdCsdAsdAsdAsdAsdTsdTs**CbAb** |
| 14 | 93j | **AbTbTb**dGsdCsdAsdAsdAsdAsdTsdTsdCs**AbGb** |
| 14 | 91k | **AbCb**dAsdTsdTsdGsdCsdAsdAsdAsdAs**TbTbCb** |
| 14 | 92k | **CbAb**dTsdTsdGsdCsdAsdAsdAsdAsdTs**TbCbAb** |
| 14 | 93k | **AbTb**dTsdGsdCsdAsdAsdAsdAsdTsdTs**CbAbGb** |
| 14 | 911 | **AbCbAb**dTsdTsdGsdCsdAsdAsdAsdAs**TbTbCb** |
| 14 | 921 | **CbAbTb**dTsdGsdCsdAsdAsdAsdAsdTs**TbCbAb** |
| 14 | 93l | **AbTbTb**dGsdCsdAsdAsdAsdAsdTsdTs**CbAbGb** |
| 15 | 79i | **AbCb**dAsdTsdTsdGsdCsdAsdAsdAsdAsdTsdTs**CbAb** |
| 15 | 80i | **CbAb**dTsdTsdGsdCsdAsdAsdAsdAsdTsdTsdCs**AbGb** |
| 15 | 79j | **AbCbAb**dTsdTsdGsdCsdAsdAsdAsdAsdTsdTs**CbAb** |
| 15 | 80j | **CbAbTb**dTsdGsdCsdAsdAsdAsdAsdTsdTsdCs**AbGb** |
| 15 | 79k | **AbCbAb**dTsdTsdGsdCsdAsdAsdAsdAsdTs**TbCbAb** |
| 15 | 80k | **CbAbTb**dTsdGsdCsdAsdAsdAsdAsdTsdTs**CbAbGb** |
| 15 | 79l | **AbCb**dAsdTsdTsdGsdCsdAsdAsdAsdAsdTs**TbCbAb** |
| 15 | 80l | **CbAb**dTsdTsdGsdCsdAsdAsdAsdAsdTsdTs**CbAbGb** |
| 16 | 6w | **AbCb**dAsdTsdTsdGsdCsdAsdAsdAsdAsdTsdTsdCs**AbGb** |
| 16 | 6x | **AbCbAb**dTsdTsdGsdCsdAsdAsdAsdAsdTsdTsdCs**AbGb** |
| 16 | 6y | **AbCbAbTb**dTsdGsdCsdAsdAsdAsdAsdTsdTsdCs**AbGb** |
| 16 | 6z | **AbCbAb**dTsdTsdGsdCsdAsdAsdAsdAsdTsdTs**CbAbGb** |
| 16 | 6aa | **AbCbAbTb**dTsdGsdCsdAsdAsdAsdAsdTsdTs**CbAbGb** |
| 16 | 6ab | **AbCb**dAsdTsdTsdGsdCsdAsdAsdAsdAsdTsdTs**CbAbGb** |
| 16 | 6ac | **AbCb**dAsdTsdTsdGsdCsdAsdAsdAsdAsdTs**TbCbAbGb** |
| 16 | 6ad | **AbCbAb**dTsdTsdGsdCsdAsdAsdAsdAsdTs**TbCbAbGb** |
| 16 | 6ae | **AbCbAbTb**dTsdGsdCsdAsdAsdAsdAsdTs**TbCbAbGb** |
| 16 | 6af | **AbCbAb**dTsdTsdGsdCsdAsdAsdAsdAs**TbTbCbAbGb** |
| 16 | 6ag | **AbCbAbTbTb**dGsdCsdAsdAsdAsdAsdTsdTs**CbAbGb** |

**Table 27**

| **L** | **Seq ID No.** | **Sequence, 5'-3'** |
|---|---|---|
| 12 | 16j | **GbssGbss**dTssdTssdAssdGssdGssdGssdCssdTss**GbssAb** |
| 12 | 15j | **AbssGbss**dGssdTssdTssdAssdGssdGssdGssdCssd**TbssGb** |
| 12 | 17j | **GbssTbss**dTssdAssdGssdGssdGssdCssdTssdGss**AbssAb** |
| 12 | 18j | **TbssTbss**dAssdGssdGssdGssdCssdTssdGssdAss**AbssTb** |
| 12 | 36j | **TbssAbss**dGssdGssdGssdCssdTssdGssdAssdAss**TbssTb** |
| 12 | 16k | **Gbss**dGssdTssdTssdAssdGssdGssdGssdCssdTss**GbssAb** |
| 12 | 15k | **Abss**dGssdGssdTssdTssdAssdGssdGssdGssdCssd**TbssGb** |
| 12 | 17k | **Gbss**dTssdTssdAssdGssdGssdGssdCssdTssdGss**AbssAb** |
| 12 | 18k | **Tbss**dTssdAssdGssdGssdGssdCssdTssdGssdAss**AbssTb** |
| 12 | 36k | **Tbss**dAssdGssdGssdGssdCssdTssdGssdAssdAss**TbssTb** |
| 12 | 161 | **GbssGbss**dTssdTssdAssdGssdGssdGssdCssdTssdGss**Ab** |
| 12 | 151 | **AbssGbss**dGssdTssdTssdAssdGssdGssdGssdCssddTss**Gb** |
| 12 | 171 | **GbssTbss**dTssdAssdGssdGssdGssdCssdTssdGssdAss**Ab** |
| 12 | 181 | **TbssTbss**dAssdGssdGssdGssdCssdTssdGssdAssdAss**Tb** |
| 12 | 36l | **TbssAbss**dGssdGssdGssdCssdTssdGssdAssdAssdTss**Tb** |
| 11 | 33g | **AbssGbss**dGssdTssdTssdAssdGssdGssdGssdCss**Tb** |
| 11 | 33h | **Abss**dGssdGssdTssdTssdAssdGssdGssdGssd**CbssTb** |
| 11 | 21g | **GbssGbss**dTssdTssdAssdGssdGssdGssdCssdTss**Gb** |
| 11 | 21h | **Gbss**dGssdTssdTssdAssdGssdGssdGssdCss**TbssGb** |
| 11 | 22g | **Gbss**dTssdTssdAssdGssdGssdGssdCssdTss**GbssAb** |
| 11 | 22h | **GbssTbss**dTssdAssdGssdGssdGssdCssdTssdGss**Ab** |
| 11 | 23g | **TbssTbss**dAssdGssdGssdGssdCssdTssdGssdAss**Ab** |
| 11 | 23h | **Tbss**dTssdAssdGssdGssdGssdCssdTssdGss**AbssAb** |
| 11 | 24g | **TbssAbss**dGssdGssdGssdCssdTssdGssdAssdAss**Tb** |
| 11 | 24h | **Tbss**dAssdGssdGssdGssdCssdTssdGssdAss**AbssTb** |
| 11 | 25g | **Abss**dGssdGssdGssdCssdTssdGssdAssdAss**TbssTb** |
| 11 | 25h | **AbssGbss**dGssdGssdCssdTssdGssdAssdAssdTss**Tb** |
| 10 | 8d | **Abss**dGssdGssdTssdTssdAssdGssdGssdGssd**Cb** |
| 10 | 9d | **Gbss**dGssdTssdTssdAssdGssdGssdGssdCss**Tb** |
| 10 | 10d | **Gbss**dTssdTssdAssdGssdGssdGssdCssdTss**Gb** |
| 10 | 11d | **Tbss**dTssdAssdGssdGssdGssdCssdTssdGss**Ab** |
| 10 | 12d | **Tbss**dAssdGssdGssdGssdCssdTssdGssdAss**Ab** |
| 10 | 13d | **Abss**dGssdGssdGssdCssdTssdGssdAssdAss**Tb** |
| 10 | 14d | **Gbss**dGssdGssdCssdTssdGssdAssdAssdTss**Tb** |
| 13 | 26j | **AbssGbss**dGssdTssdTssdAssdGssdGssdGssdCssdTss**GbssAb** |
| 13 | 27j | **GbssGbss**dTssdTssdAssdGssdGssdGssdCssdTssdGss**AbssAb** |
| 13 | 28j | **GbssTbss**dTssdAssdGssdGssdGssdCssdTssdGssdAss**AbssTb** |
| 13 | 29j | **TbssTbss**dAssdGssdGssdGssdCssdTssdGssdAssdAss**TbssTb** |
| 13 | 26k | **AbssGbssGbss**dTssdTssdAssdGssdGssdGssdCssdTss**GbssAb** |
| 13 | 27k | **GbssGbssTbss**dTssdAssdGssdGssdGssdCssdTssdGss**AbssAb** |
| 13 | 28k | **GbssTbssTbss**dAssdGssdGssdGssdCssdTssdGssdAss**AbssTb** |
| 13 | 29k | **TbssTbssAbss**dGssdGssdGssdCssdTssdGssdAssdAss**TbssTb** |
| 13 | 26l | **AbssGbss**dGssdTssdTssdAssdGssdGssdGss**CbssTbssGbssAb** |
| 13 | 27l | **GbssGbss**dTssdTssdAssdGssdGssdGssdCssdTss**GbssAbssAb** |
| 13 | 28l | **GbssTbss**dTssdAssdGssdGssdGssdCssdTssdGss**AbssAbssTb** |
| 13 | 29l | **TbssTbss**dAssdGssdGssdGssdCssdTssdGssdAss**AbssTbssTb** |
| 14 | 30m | **AbssGbss**dGssdTssdTssdAssdGssdGssdGssdCssdTss**GbssAbssAb** |
| 14 | 31m | **GbssGbss**dTssdTssdAssdGssdGssdGssdCssdTssdGss**AbssAbssTb** |
| 14 | 32m | **GbssTbss**dTssdAssdGssdGssdGssdCssdTssdGssdAss**AbssTbssTb** |
| 14 | 30n | **AbssGbssGbss**dTssdTssdAssdGssdGssdGssdCssdTss**GbssAbssAb** |
| 14 | 31n | **GbssGbssTbss**dTssdAssdGssdGssdGssdCssdTssdGss**AbssAbssTb** |
| 14 | 32n | **GbssTbssTbss**dAssdGssdGssdGssdCssdTssdGssdAss**AbssTbssTb** |
| 14 | 30o | **AbssGbss**dGssdTssdTssdAssdGssdGssdGssdCssdTss**GbssAbssAb** |
| 14 | 31o | **GbssGbss**dTssdTssdAssdGssdGssdGssdCssdTssdGss**AbssAbssTb** |
| 14 | 32o | **GbssTbss**dTssdAssdGssdGssdGssdCssdTssdGssdAss**AbssTbssTb** |
| 14 | 30p | **AbssGbss**dGssdTssdTssdAssdGssdGssdGssdCssdTssdGss**AbssAb** |
| 14 | 31p | **GbssGbss**dTssdTssdAssdGssdGssdGssdCssdTssdGssdAss**AbssTb** |
| 14 | 32p | **GbssTbss**dTssdAssdGssdGssdGssdCssdTssdGssdAssdAss**TbssTb** |
| 15 | 19m | AbssGbssdGssdTssdTssdAssdGssdGssdGssdCssdTssGbss AbssAbssTb |
| 15 | 20m | GbssGbssdTssdTssdAssdGssdGssdGssdCssdTssdGssAbss AbssTbssTb |
| 15 | 19n | AbssGbssdGssdTssdTssdAssdGssdGssdGssdCssdTssdGss dAssAbssTb |
| 15 | 20n | GbssGbssdTssdTssdAssdGssdGssdGssdCssdTssdGssdAss dAssTbssTb |
| 15 | 19o | AbssGbssGbssdTssdTssdAssdGssdGssdGssdCssdTssdGss AbssAbssTb |
| 15 | 20o | GbssGbssTbssdTssdAssdGssdGssdGssdCssdTssdGssdAss AbssTbssTb |
| 15 | 19p | AbssGbssGbssTbssdTssdAssdGssdGssdGssdCssdTssdGss AbssAbssTb |
| 15 | 20p | GbssGbssTbssTbssdAssdGssdGssdGssdCssdTssdGssdAss AbssTbssTb |
| 16 | 4ah | AbssGbssdGssdTssdTssdAssdGssdGssdGssdCssdTssdGss dAssAbssTbssTb |
| 16 | 4ai | **AbssGbss**dGssdTssdTssdAssdGssdGssdGssdCssdTssdGss dAssdAss**TbssTb** |
| 16 | 4aj | AbssGbssGbssdTssdTssdAssdGssdGssdGssdCssdTssdGss dAssdAssTbssTb |
| 16 | 4ak | AbssGbssGbssTbssdTssdAssdGssdGssdGssdCssdTssdGss dAssdAssTbssTb |
| 16 | 4al | AbssGbssdGssdTssdTssdAssdGssdGssdGssdCssdTssdGss AbssAbssTbssTb |
| 16 | 4am | AbssGbssGbssdTssdTssdAssdGssdGssdGssdCssdTssdGss dAssAbssTbssTb |
| 16 | 4an | AbssGbssGbssTbssdTssdAssdGssdGssdGssdCssdTssdGss AbssAbssTbssTb |
| 16 | 4ao | AbssGbssGbssTbssTbssdAssdGssdGssdGssdCssdTssdGss |
| | | dAssAbssTbssTb |
| 16 | 4ap | AbssGbssGbssdTssdTssdAssdGssdGssdGssdCssdTssGbss AbssAbssTbssTb |
| 16 | 4aq | AbssGbssGbssdTssdTssdAssdGssdGssdGssdCssdTssdGss AbssAbssTbssTb |
| 16 | 4ar | AbssGbssGbssTbssdTssdAssdGssdGssdGssdCssdTssdGss dAssAbssTbssTb |

**Table 28**

| **L** | **Seq ID No.** | **Sequence, 5'-3'** |
|---|---|---|
| 12 | 46j | **CbssAbss**dAssdGssdCssdAssdAssdGssdGssdCss**AbssTb** |
| 12 | 45j | **AbssCbss**dAssdAssdGssdCssdAssdAssdGssdGss**CbssAb** |
| 12 | 44j | **TbssAbss**dCssdAssdAssdGssdCssdAssdAssdGss**GbssCb** |
| 12 | 47j | **AbssAbss**dGssdCssdAssdAssdGssdGssdCssdAss**TbssTb** |
| 12 | 48j | **AbssGbss**dCssdAssdAssdGssdGssdCssdAssdTss**TbssTb** |
| 12 | 46k | **Cbss**dAssdAssdGssdCssdAssdAssdGssdGssdCss**AbssTb** |
| 12 | 45k | **Abss**dCssdAssdAssdGssdCssdAssdAssdGssdGss**CbssAb** |
| 12 | 44k | **Tbss**dAssdCssdAssdAssdGssdCssdAssdAssdGss**GbssCb** |
| 12 | 47k | **Abss**dAssdGssdCssdAssdAssdGssdGssdCssdAss**TbssTb** |
| 12 | 48k | **Abss**dGssdCssdAssdAssdGssdGssdCssdAssdTss**TbssTb** |
| 12 | 46l | **CbssAbss**dAssdGssdCssdAssdAssdGssdGssdCssdAss**Tb** |
| 12 | 45l | **AbssCbss**dAssdAssdGssdCssdAssdAssdGssdGssdCss**Ab** |
| 12 | 44l | **TbssAbss**dCssdAssdAssdGssdCssdAssdAssdGssdGss**Cb** |
| 12 | 47l | **AbssAbss**dGssdCssdAssdAssdGssdGssdCssdAssdTss**Tb** |
| 12 | 48l | **AbssGbss**dCssdAssdAssdGssdGssdCssdAssdTssdTss**Tb** |
| 11 | 51g | **Tbss**dAssdCssdAssdAssdGssdCssdAssdAss**GbssGb** |
| 11 | 52g | **Abss**dCssdAssdAssdGssdCssdAssdAssdGss**GbssCb** |
| 11 | 53g | **Cbss**dAssdAssdGssdCssdAssdAssdGssdGss**CbssAb** |
| 11 | 54g | **Abss**dAssdGssdCssdAssdAssdGssdGssdCss**AbssTb** |
| 11 | 55g | **Abss**dGssdCssdAssdAssdGssdGssdCssdAssd**TbssTb** |
| 11 | 56g | **Gbss**dCssdAssdAssdGssdGssdCssdAssdTss**TbssTb** |
| 11 | 51h | **TbssAbss**dCssdAssdAssdGssdCssdAssdAssdGss**Gb** |
| 11 | 52h | **AbssCbss**dAssdAssdGssdCssdAssdAssdGssdGss**Cb** |
| 11 | 53h | **CbssAbss**dAssdGssdCssdAssdAssdGssdGssdCss**Ab** |
| 11 | 54h | **AbssAbss**dGssdCssdAssdAssdGssdGssdCssdAss**Tb** |
| 11 | 55h | **AbssGbss**dCssdAssdAssdGssdGssdCssdAssddTss**Tb** |
| 11 | 56h | **GbssCbss**dAssdAssdGssdGssdCssdAssdTssdTss**Tb** |
| 10 | 37d | **Tbss**dAssdCssdAssdAssdGssdCssdAssdAss**Gb** |
| 10 | 38d | **Abss**dCssdAssdAssdGssdCssdAssdAssdGss**Gb** |
| 10 | 39d | **Cbss**dAssdAssdGssdCssdAssdAssdGssdGss**Cb** |
| 10 | 40d | **Abss**dAssdGssdCssdAssdAssdGssdGssdCss**Ab** |
| 10 | 41d | **Abss**dGssdCssdAssdAssdGssdGssdCssdAssd**Tb** |
| 10 | 42d | **Gbss**dCssdAssdAssdGssdGssdCssdAssdTss**Tb** |
| 10 | 43d | **Cbss**dAssdAssdGssdGssdCssdAssdTssdTss**Tb** |
| 13 | 57j | **TbssAbss**dCssdAssdAssdGssdCssdAssdAssdGssdGss**CbssAb** |
| 13 | 58j | **AbssCbss**dAssdAssdGssdCssdAssdAssdGssdGssdCss**AbssTb** |
| 13 | 59j | **CbssAbss**dAssdGssdCssdAssdAssdGssdGssdCssdAss**TbssTb** |
| 13 | 60j | **AbssAbss**dGssdCssdAssdAssdGssdGssdCssdAssdTss**TbssTb** |
| 13 | 57k | **TbssAbssCbss**dAssdAssdGssdCssdAssdAssdGssdGss**CbssAb** |
| 13 | 58k | **AbssCbssAbss**dAssdGssdCssdAssdAssdGssdGssdCss**AbssTb** |
| 13 | 59k | **CbssAbssAbss**dGssdCssdAssdAssdGssdGssdCssdAss**TbssTb** |
| 13 | 60k | **AbssAbssGbss**dCssdAssdAssdGssdGssdCssdAssdTss**TbssTb** |
| 13 | 57l | **TbssAbss**dCssdAssdAssdGssdCssdAssdAssdGss**GbssCbssAb** |
| 13 | 58l | **AbssCbss**dAssdAssdGssdCssdAssdAssdGssdGss**CbssAbssTb** |
| 13 | 59l | **CbssAbss**dAssdGssdCssdAssdAssdGssdGssdCss**AbssTbssTb** |
| 13 | 60l | **AbssAbss**dGssdCssdAssdAssdGssdGssdCssdAss**TbssTbssTb** |
| 14 | 61m | **TbssAbss**dCssdAssdAssdGssdCssdAssdAssdGssdGssdCss**AbssTb** |
| 14 | 62m | **AbssCbss**dAssdAssdGssdCssdAssdAssdGssdGssdCssdAss**TbssTb** |
| 14 | 63m | **CbssAbss**dAssdGssdCssdAssdAssdGssdGssdCssdAssdTss**TbssTb** |
| 14 | 61n | **TbssAbssCbss**dAssdAssdGssdCssdAssdAssdGssdGssdCss**AbssTb** |
| 14 | 62n | **AbssCbssAbss**dAssdGssdCssdAssdAssdGssdGssdCssdAss**TbssTb** |
| 14 | 63n | **CbssAbssAbss**dGssdCssdAssdAssdGssdGssdCssdAssdTss**TbssTb** |
| 14 | 61o | **TbssAbss**dCssdAssdAssdGssdCssdAssdAssdGssdGss**CbssAbssTb** |
| 14 | 62o | **AbssCbss**dAssdAssdGssdCssdAssdAssdGssdGssdCss**AbssTbssTb** |
| 14 | 63o | **CbssAbss**dAssdGssdCssdAssdAssdGssdGssdCssdAss**TbssTbssTb** |
| 14 | 61p | **TbssAbssCbss**dAssdAssdGssdCssdAssdAssdGssdGss**CbssAbssTb** |
| 14 | 62p | **AbssCbssAbss**dAssdGssdCssdAssdAssdGssdGssdCss**AbssTbssTb** |
| 14 | 63p | **CbssAbssAbss**dGssdCssdAssdAssdGssdGssdCssdAss**TbssTbssTb** |
| 15 | 49m | TbssAbssdCssdAssdAssdGssdCssdAssdAssdGssdGssdCss dAssTbssTb |
| 15 | 50m | **TbssAbss**dCssdAssdAssdGssdCssdAssdAssdGssdGssdCss dAss**TbssTb** **AbssCbss**dAssdAssdGssdCssdAssdAssdGssdGssdCssdAss dTss**TbssTb** |
| 15 | 49n | TbssAbssCbssdAssdAssdGssdCssdAssdAssdGssdGssdCss |
| | | dAssTbssTb |
| 15 | 50n | AbssCbssAbssdAssdGssdCssdAssdAssdGssdGssdCssdAss dTssTbssTb |
| 15 | 49o | TbssAbssdCssdAssdAssdGssdCssdAssdAssdGssdGssdCss AbssTbssTb |
| 15 | 50o | AbssCbssdAssdAssdGssdCssdAssdAssdGssdGssdCssdAss TbssTbssTb |
| 15 | 49p | TbssAbssCbssdAssdAssdGssdCssdAssdAssdGssdGssdCss AbssTbssTb |
| 15 | 50p | AbssCbssAbssdAssdGssdCssdAssdAssdGssdGssdCssdAss TbssTbssTb |
| 16 | 5ah | TbssAbssdCssdAssdAssdGssdCssdAssdAssdGssdGssdCss dAssdTssTbssTb |
| 16 | 5ai | TbssAbssCbssdAssdAssdGssdCssdAssdAssdGssdGssdCss dAssdTssTbssTb |
| 16 | 5aj | TbssAbssCbssAbssdAssdGssdCssdAssdAssdGssdGssdCss dAssdTssTbssTb |
| 16 | 5ak | TbssAbssdCssdAssdAssdGssdCssdAssdAssdGssdGssdCss dAssTbssTbssTb |
| 16 | 5al | TbssAbssdCssdAssdAssdGssdCssdAssdAssdGssdGssdCss AbssTbssTbssTb |
| 16 | 5am | TbssAbssCbssdAssdAssdGssdCssdAssdAssdGssdGssdCss dAssTbssTbssTb |
| 16 | 5an | TbssAbssCbssAbssdAssdGssdCssdAssdAssdGssdGssdCss dAssTbssTbssTb |
| 16 | 5ao | TbssAbssCbssdAssdAssdGssdCssdAssdAssdGssdGssdCss AbssTbssTbssTb |
| 16 | 5ap | TbssAbssCbssAbssdAssdGssdCssdAssdAssdGssdGssdCss AbssTbssTbssTb |
| 16 | 5aq | TbssAbssCbssAbssdAssdGssdCssdAssdAssdGssdGssdCss dAssTbssTbssTb |
| 16 | 5ar | TbssAbssCbssdAssdAssdGssdCssdAssdAssdGssdGssdCss AbssTbssTbssTb |

**Table 29**

| **L** | **Seq ID No.** | **Sequence, 5'-3'** |
|---|---|---|
| 12 | 74j | **TbssGbss**dCssdAssdAssdAssdAssdTssdTssdCss**AbssGb** |
| 12 | 75j | **AbssCbss**dAssdTssdTssdGssdCssdAssdAssdAss**AbssTb** |
| 12 | 76j | **CbssAbss**dTssdTssdGssdCssdAssdAssdAssdAss**TbssTb** |
| 12 | 77j | **AbssTbss**dTssdGssdCssdAssdAssdAssdAssdTss**TbssCb** |
| 12 | 78j | **TbssTbss**dGssdCssdAssdAssdAssdAssdTssdTss**CbssAb** |
| 12 | 74k | **TbssGbssCbss**dAssdAssdAssdAssdTssdTssdCss**AbssGb** |
| 12 | 75k | **AbssCbssAbss**dTssdTssdGssdCssdAssdAssdAss**AbssTb** |
| 12 | 76k | **CbssAbssTbss**dTssdGssdCssdAssdAssdAssdAss**TbssTb** |
| 12 | 77k | **AbssTbssTbss**dGssdCssdAssdAssdAssdAssdTss**TbssCb** |
| 12 | 78k | **TbssTbssGbss**dCssdAssdAssdAssdAssdTssdTss**CbssAb** |
| 12 | 74l | **TbssGbss**dCssdAssdAssdAssdAssdTssdTss**CbssAbssGb** |
| 12 | 75l | **AbssCbss**dAssdTssdTssdGssdCssdAssdAss**AbssAbssTb** |
| 12 | 76l | **CbssAbss**dTssdTssdGssdCssdAssdAssdAss**AbssTbssTb** |
| 12 | 77l | **AbssTbss**dTssdGssdCssdAssdAssdAssdAss**TbssTbssCb** |
| 12 | 78l | **TbssTbss**dGssdCssdAssdAssdAssdAssdTss**TbssCbssAb** |
| 11 | 81g | **Abss**dCssdAssdTssdTssdGssdCssdAssdAss**AbssAb** |
| 11 | 82g | **Cbss**dAssdTssdTssdGssdCssdAssdAssdAss**AbssTb** |
| 11 | 83g | **Abss**dTssdTssdGssdCssdAssdAssdAssdAss**TbssTb** |
| 11 | 84g | **Tbss**dTssdGssdCssdAssdAssdAssdAssdTss**TbssCb** |
| 11 | 85g | **Tbss**dGssdCssdAssdAssdAssdAssdTssdTss**CbssAb** |
| 11 | 86g | **Gbss**dCssdAssdAssdAssdAssdTssdTssdCss**AbssGb** |
| 11 | 81h | **AbssCbss**dAssdTssdTssdGssdCssdAssdAssdAss**Ab** |
| 11 | 82h | **CbssAbss**dTssdTssdGssdCssdAssdAssdAssdAss**Tb** |
| 11 | 83h | **AbssTbss**dTssdGssdCssdAssdAssdAssdAssdTss**Tb** |
| 11 | 84h | **TbssTbss**dGssdCssdAssdAssdAssdAssdTssdTss**Cb** |
| 11 | 85h | **TbssGbss**dCssdAssdAssdAssdAssdTssdTssdCss**Ab** |
| 11 | 86h | **GbssCbss**dAssdAssdAssdAssdTssdTssdCssdAss**Gb** |
| 10 | 67d | **Abss**dCssdAssdTssdTssdGssdCssdAssdAss**Ab** |
| 10 | 68d | **Cbss**dAssdTssdTssdGssdCssdAssdAssdAss**Ab** |
| 10 | 69d | **Abss**dTssdTssdGssdCssdAssdAssdAssdAss**Tb** |
| 10 | 70d | **Tbss**dTssdGssdCssdAssdAssdAssdAssdTss**Tb** |
| 10 | 71d | **Tbss**dGssdCssdAssdAssdAssdAssdTssdTss**Cb** |
| 10 | 72d | **Gbss**dCssdAssdAssdAssdAssdTssdTssdCss**Ab** |
| 10 | 73d | **Cbss**dAssdAssdAssdAssdTssdTssdCssdAss**Gb** |
| 13 | 87j | **AbssCbss**dAssdTssdTssdGssdCssdAssdAssdAssdAss**TbssTb** |
| 13 | 88j | **CbssAbss**dTssdTssdGssdCssdAssdAssdAssdAssdTss**TbssCb** |
| 13 | 89j | **AbssTbss**dTssdGssdCssdAssdAssdAssdAssdTssdTss**CbssAb** |
| 13 | 90j | **TbssTbss**dGssdCssdAssdAssdAssdAssdTssdTssdCss**AbssGb** |
| 13 | 87k | **AbssCbssAbss**dTssdTssdGssdCssdAssdAssdAssdAss**TbssTb** |
| 13 | 88k | **CbssAbssTbss**dTssdGssdCssdAssdAssdAssdAssdTss**TbssCb** |
| 13 | 89k | **AbssTbssTbss**dGssdCssdAssdAssdAssdAssdTssdTss**CbssAb** |
| 13 | 90k | **TbssTbssGbss**dCssdAssdAssdAssdAssdTssdTssdCss**AbssGb** |
| 13 | 87l | **AbssCbss**dAssdTssdTssdGssdCssdAssdAssdAss**AbssTbssTb** |
| 13 | 88l | **CbssAbss**dTssdTssdGssdCssdAssdAssdAssdAss**TbssTbssCb** |
| 13 | 89l | **AbssTbss**dTssdGssdCssdAssdAssdAssdAssdTss**TbssCbssAb** |
| 13 | 90l | **TbssTbss**dGssdCssdAssdAssdAssdAssdTssdTss**CbssAbssGb** |
| 14 | 91m | **AbssCbss**dAssdTssdTssdGssdCssdAssdAssdAssdAssdTss**TbssCb** |
| 14 | 92m | **CbssAbss**dTssdTssdGssdCssdAssdAssdAssdAssdTssdTss**CbssAb** |
| 14 | 93m | **AbssTbss**dTssdGssdCssdAssdAssdAssdAssdTssdTssdCss**AbssGb** |
| 14 | 91n | **AbssCbssAbss**dTssdTssdGssdCssdAssdAssdAssdAssdTss**TbssCb** |
| 14 | 92n | **CbssAbssTbss**dTssdGssdCssdAssdAssdAssdAssdTssdTss**CbssAb** |
| 14 | 93n | **AbssTbssTbss**dGssdCssdAssdAssdAssdAssdTssdTssdCss**AbssGb** |
| 14 | 91o | **AbssCbss**dAssdTssdTssdGssdCssdAssdAssdAssdAss**TbssTbssCb** |
| 14 | 92o | **CbssAbss**dTssdTssdGssdCssdAssdAssdAssdAssdTss**TbssCbssAb** |
| 14 | 93o | **AbssTbss**dTssdGssdCssdAssdAssdAssdAssdTssdTss**CbssAbssGb** |
| 14 | 91p | **AbssCbssAbss**dTssdTssdGssdCssdAssdAssdAssdAss**TbssTbssCb** |
| 14 | 92p | **CbssAbssTbss**dTssdGssdCssdAssdAssdAssdAssdTss**TbssCbssAb** |
| 14 | 93p | **AbssTbssTbss**dGssdCssdAssdAssdAssdAssdTssdTss**CbssAbssGb** |
| 15 | 79m | AbssCbssdAssdTssdTssdGssdCssdAssdAssdAssdAssdTssdTss CbssAb |
| 15 | 80m | CbssAbssdTssdTssdGssdCssdAssdAssdAssdAssdTssdTssdCss AbssGb |
| 15 | 79n | AbssCbssAbssdTssdTssdGssdCssdAssdAssdAssdAssdTssdTss CbssAb |
| 15 | 80n | CbssAbssTbssdTssdGssdCssdAssdAssdAssdAssdTssdTssdCss AbssGb |
| 15 | 79o | AbssCbssAbssdTssdTssdGssdCssdAssdAssdAssdAssdTssTbss CbssAb |
| 15 | 80o | CbssAbssTbssdTssdGssdCssdAssdAssdAssdAssdTssdTssCbss AbssGb |
| 15 | 79p | AbssCbssdAssdTssdTssdGssdCssdAssdAssdAssdAssdTssTbss CbssAb |
| 15 | 80p | **CbssAbss**dTssdTssdGssdCssdAssdAssdAssdAssdTssdTss**Cbss** **AbssGb** |
| 16 | 6ah | **AbssCbss**dAssdTssdTssdGssdCssdAssdAssdAssdAssdTss dTssdCss**AbssGb** |
| 16 | 6ai | AbssCbssAbssdTssdTssdGssdCssdAssdAssdAssdAssdTss dTssdCssAbssGb |
| 16 | 6aj | AbssCbssAbssTbssdTssdGssdCssdAssdAssdAssdAssdTss dTssdCssAbssGb |
| 16 | 6ak | AbssCbssAbssdTssdTssdGssdCssdAssdAssdAssdAssdTss dTssCbssAbssGb |
| 16 | 6al | AbssCbssAbssTbssdTssdGssdCssdAssdAssdAssdAssdTss dTssCbssAbssGb |
| 16 | 6am | AbssCbssdAssdTssdTssdGssdCssdAssdAssdAssdAssdTss dTssCbssAbssGb |
| 16 | 6an | AbssCbssdAssdTssdTssdGssdCssdAssdAssdAssdAssdTss TbssCbssAbssGb |
| 16 | 6ao | AbssCbssAbssdTssdTssdGssdCssdAssdAssdAssdAssdTss TbssCbssAbssGb |
| 16 | 6ap | AbssCbssAbssTbssdTssdGssdCssdAssdAssdAssdAssdTss TbssCbssAbssGb |
| 16 | 6aq | AbssCbssAbssdTssdTssdGssdCssdAssdAssdAssdAssTbss TbssCbssAbssGb |
| 16 | 6ar | AbssCbssAbssTbssTbssdGssdCssdAssdAssdAssdAssdTss dTssCbssAbssGb |

The following antisense-oligonucleotides in form of gapmers as listed in **Table 30** to **Table 32** are especially preferred.

**Table 30**

| **L** | **Seq ID No.** | **Sequence, 5'-3'** |
|---|---|---|
| 12 | 16m | **Gb¹sGb¹s**dTsdTsdAsdGsdGsdGsdCsdTs**Gb¹sAb¹** |
| 12 | 15m | **Ab¹sGb¹s**dGsdTsdTsdAsdGsdGsdGsdCsd**Tb¹sGb¹** |
| 12 | 17m | **Gb¹sTb¹s**dTsdAsdGsdGsdGsdCsdTsdGs**Ab¹sAb¹s** |
| 12 | 18m | **Tb¹sTb¹s**dAsdGsdGsdGsdCsdTsdGsdAs**Ab¹sTb¹s** |
| 12 | 16n | **Gb²s**dGsdTsdTsdAsdGsdGsdGsdCsdTs**Gb²sAb²** |
| 12 | 15n | **Ab²s**dGsdGsdTsdTsdAsdGsdGsdGsdCsd**Tb²sGb²** |
| 12 | 17n | **Gb²s**dTsdTsdAsdGsdGsdGsdCsdTsdGs**Ab²sAb²** |
| 12 | 18n | **Tb²s**dTsdAsdGsdGsdGsdCsdTsdGsdAs**Ab²sTb¹** |
| 12 | 16o | **Gb³sGb³s**dTsdTsdAsdGsdGsdGsdCsdTsdGs**Ab³** |
| 12 | 15o | **Ab³sGb³s**dGsdTsdTsdAsdGsdGsdGsdCsddTs**Gb³** |
| 12 | 17o | **Gb³sTb³s**dTsdAsdGsdGsdGsdCsdTsdGsdAs**Ab³** |
| 12 | 18o | **Tb³sTb³s**dAsdGsdGsdGsdCsdTsdGsdAsdAs**Tb³** |
| 12 | 16p | **Gb¹Gb¹**dTsdTsdAsdGsdGsdGsdCsdTs**Gb¹Ab¹** |
| 12 | 15p | **Ab¹Gb¹**dGsdTsdTsdAsdGsdGsdGsdCsd**Tb¹Gb¹** |
| 12 | 17p | **Gb¹Tb¹**dTsdAsdGsdGsdGsdCsdTsdGs**Ab¹Ab¹** |
| 12 | 18p | **Tb¹Tb¹**dAsdGsdGsdGsdCsdTsdGsdAs**Ab¹Tb¹** |
| 12 | 16q | **Gb⁴**dGsdTsdTsdAsdGsdGsdGsdCsdTs**Gb⁴Ab⁴** |
| 12 | 15q | **Ab⁴**dGsdGsdTsdTsdAsdGsdGsdGsdCsd**Tb⁴Gb⁴** |
| 12 | 17q | **Gb⁴**dTsdTsdAsdGsdGsdGsdCsdTsdGs**Ab⁴Ab⁴** |
| 12 | 18q | **Tb⁴**dTsdAsdGsdGsdGsdCsdTsdGsdAs**Ab⁴Tb⁴** |
| 12 | 16r | **Gb⁵Gb⁵**dTsdTsdAsdGsdGsdGsdCsdTsdGs**Ab⁵** |
| 12 | 15r | **Ab⁵Gb⁵**dGsdTsdTsdAsdGsdGsdGsdCsddTs**Gb⁵** |
| 12 | 17r | **Gb⁵Tb⁵**dTsdAsdGsdGsdGsdCsdTsdGsdAs**Ab⁵** |
| 12 | 18r | **Tb⁵Tb⁵**dAsdGsdGsdGsdCsdTsdGsdAsdAs**Tb⁵** |
| 12 | 16s | **Gb¹ssGb¹ss**dTssdTssdAssdGssdGssdGssdCssdTss**Gb¹ssAb¹** |
| 12 | 15s | **Ab¹ssGb¹ss**dGssdTssdTssdAssdGssdGssdGssdCssd**Tb¹ssGb¹** |
| 12 | 17s | **Gb¹ssTb¹ss**dTssdAssdGssdGssdGssdCssdTssdGss**Ab¹ssAb¹** |
| 12 | 18s | **Tb¹ssTb¹ss**dAssdGssdGssdGssdCssdTssdGssdAss**Ab¹ssTb¹** |
| 12 | 16t | **Gb⁶ss**dGssdTssdTssdAssdGssdGssdGssdCssdTss**Gb⁶ssAb⁶** |
| 12 | 15t | **Ab⁶ss**dGssdGssdTssdTssdAssdGssdGssdGssdCssd**Tb⁶ssGb⁶** |
| 12 | 17t | **Gb⁶ss**dTssdTssdAssdGssdGssdGssdCssdTssdGss**Ab⁶ssAb⁶** |
| 12 | 18t | **Tb⁶ss**dTssdAssdGssdGssdGssdCssdTssdGssdAss**Ab⁶ssTb⁶** |
| 12 | 16u | **Gb⁷ssGb⁷ss**dTssdTssdAssdGssdGssdGssdCssdTssdGss**Ab⁷** |
| 12 | 15u | **Ab⁷ssGb⁷ss**dGssdTssdTssdAssdGssdGssdGssdCssddTss**Gb⁷** |
| 12 | 17u | **Gb⁷ssTb⁷ss**dTssdAssdGssdGssdGssdCssdTssdGssdAss**Ab⁷** |
| 12 | 18u | **Tb⁷ssTb⁷ss**dAssdGssdGssdGssdCssdTssdGssdAssdAss**Tb⁷** |
| 12 | 17i | **Gb¹Tb¹**dTsdAsdGsdGsdGsdCsdTsdGsdAs**Ab¹** |
| 12 | 18i | **Tb¹Tb¹**dAsdGsdGsdGsdCsdTsdGsdAsdAs**Tb¹** |
| 11 | 21i | **Gb¹sGb¹s**dTsdTsdAsdGsdGsdGsdCsdTs**Gb¹** |
| 11 | 21j | **Gb¹s**dGsdTsdTsdAsdGsdGsdGsdCs**Tb¹sGb¹** |
| 11 | 22i | **Gb¹s**dTsdTsdAsdGsdGsdGsdCsdTs**Gb¹sAb¹** |
| 11 | 22j | **Gb¹sTb¹s**dTsdAsdGsdGsdGsdCsdTsdGs**Ab¹** |
| 11 | 23i | **Tb¹sTb¹s**dAsdGsdGsdGsdCsdTsdGsdAs**Ab¹** |
| 11 | 23j | **Tb¹s**dTsdAsdGsdGsdGsdCsdTsdGs**Ab¹sAb¹** |
| 11 | 21k | **Gb¹Gb¹**dTsdTsdAsdGsdGsdGsdCsdTs**Gb¹** |
| 11 | 211 | **Gb¹**dGsdTsdTsdAsdGsdGsdGsdCs**Tb¹Gb¹** |
| 11 | 22k | **Gb¹**dTsdTsdAsdGsdGsdGsdCsdTs**Gb¹Ab¹** |
| 11 | 22l | **Gb¹Tb¹**dTsdAsdGsdGsdGsdCsdTsdGs**Ab¹** |
| 11 | 23k | **Tb¹Tb¹**dAsdGsdGsdGsdCsdTsdGsdAs**Ab¹** |
| 11 | 23l | **Tb¹**dTsdAsdGsdGsdGsdCsdTsdGs**Ab¹Ab¹** |
| 11 | 21m | **Gb¹ssGb¹ss**dTssdTssdAssdGssdGssdGssdCssdTss**Gb¹** |
| 11 | 21n | **Gb¹ss**dGssdTssdTssdAssdGssdGssdGssdCss**Tb¹ssGb¹** |
| 11 | 22m | **Gb¹ss**dTssdTssdAssdGssdGssdGssdCssdTss**Gb¹ssAb¹** |
| 11 | 22n | **Gb¹ssTb¹ss**dTssdAssdGssdGssdGssdCssdTssdGss**Ab¹** |
| 11 | 23m | **Tb¹ssTb¹ss**dAssdGssdGssdGssdCssdTssdGssdAss**Ab¹** |
| 11 | 23n | **Tb¹ss**dTssdAssdGssdGssdGssdCssdTssdGss**Ab¹ssAb¹** |
| 10 | 9e | **Gb¹s**dGsdTsdTsdAsdGsdGsdGsdC*s**Tb¹** |
| 10 | 10e | **Gb¹s**dTsdTsdAsdGsdGsdGsdC*sdTs**Gb¹** |
| 10 | 11e | **Tb¹s**dTsdAsdGsdGsdGsdC*sdTsdGs**Ab¹** |
| 10 | 9f | **Gb¹**dGsdTsdTsdAsdGsdGsdGsdCs**Tb¹** |
| 10 | 10f | **Gb¹**dTsdTsdAsdGsdGsdGsdCsdTs**Gb¹** |
| 10 | 11f | **Tb¹**dTsdAsdGsdGsdGsdCsdTsdGs**Ab¹** |
| 10 | 9g | **Gb¹ss**dGssdTssdTssdAssdGssdGssdGssdCss**Tb¹** |
| 10 | 10g | **Gb¹ss**dTssdTssdAssdGssdGssdGssdC*ssdTss**Gb¹** |
| 10 | 11g | **Tb¹ss**dTssdAssdGssdGssdGssdCssdTssdGss**Ab¹** |
| 13 | 26m | **Ab¹sGb¹s**dGsdTsdTsdAsdGsdGsdGsdCsdTs**Gb¹sAb¹** |
| 13 | 27m | **Gb¹sGb¹s**dTsdTsdAsdGsdGsdGsdCsdTsdGs**Ab¹sAb¹** |
| 13 | 28m | **Gb¹sTb¹s**dTsdAsdGsdGsdGsdCsdTsdGsdAs**Ab¹sTb¹** |
| 13 | 29m | **Tb¹sTb¹s**dAsdGsdGsdGsdCsdTsdGsdAsdAs**Tb¹sTb¹** |
| 13 | 26n | **Ab¹sGb¹sGb¹s**dTsdTsdAsdGsdGsdGsdCsdTs**Gb¹sAb¹** |
| 13 | 27n | **Gb¹sGb¹sTb¹s**dTsdAsdGsdGsdGsdCsdTsdGs**Ab¹sAb¹** |
| 13 | 28n | **Gb¹sTb¹sTb¹s**dAsdGsdGsdGsdCsdTsdGsdAs**Ab¹sTb¹** |
| 13 | 29n | **Tb¹sTb¹sAb¹s**dGsdGsdGsdCsdTsdGsdAsdAs**Tb¹sTb¹** |
| 13 | 26o | **Ab¹sGb¹s**dGsdTsdTsdAsdGsdGsdGs**C*b¹sTb¹sGb¹sAb¹** |
| 13 | 27o | **Gb¹sGb¹s**dTsdTsdAsdGsdGsdGsdCsdTs**Gb¹sAb¹sAb¹** |
| 13 | 28o | **Gb¹sTb¹s**dTsdAsdGsdGsdGsdCsdTsdGs**Ab¹sAb¹sTb¹** |
| 13 | 29o | **Tb¹sTb¹s**dAsdGsdGsdGsdCsdTsdGsdAs**Ab¹sTb¹sTb¹** |
| 13 | 26p | **Ab¹Gb¹**dGsdTsdTsdAsdGsdGsdGsdCsdTs**Gb¹Ab¹** |
| 13 | 27p | **Gb¹Gb¹**dTsdTsdAsdGsdGsdGsdCsdTsdGs**Ab¹Ab¹** |
| 13 | 28p | **Gb¹Tb¹**dTsdAsdGsdGsdGsdCsdTsdGsdAs**Ab¹Tb¹** |
| 13 | 29p | **Tb¹Tb¹**dAsdGsdGsdGsdCsdTsdGsdAsdAs**Tb¹Tb¹** |
| 13 | 26q | **Ab¹Gb¹Gb¹**dTsdTsdAsdGsdGsdGsdCsdTs**Gb¹Ab¹** |
| 13 | 27q | **Gb¹Gb¹Tb¹**dTsdAsdGsdGsdGsdCsdTsdGs**Ab¹Ab¹** |
| 13 | 28q | **Gb¹Tb¹Tb¹**dAsdGsdGsdGsdCsdTsdGsdAs**Ab¹Tb¹** |
| 13 | 29q | **Tb¹Tb¹Ab¹**dGsdGsdGsdCsdTsdGsdAsdAs**Tb¹Tb¹** |
| 13 | 26r | **Ab¹Gb¹**dGsdTsdTsdAsdGsdGsdGs**Cb¹Tb¹Gb¹Ab¹** |
| 13 | 27r | **Gb¹Gb¹**dTsdTsdAsdGsdGsdGsdCsdTs**Gb¹Ab¹ Ab¹** |
| 13 | 28r | **Gb¹Tb¹**dTsdAsdGsdGsdGsdCsdTsdGs**Ab¹Ab¹Tb¹** |
| 13 | 29r | **Tb¹Tb¹**dAsdGsdGsdGsdCsdTsdGsdAs**Ab¹Tb¹Tb¹** |
| 13 | 26s | **Ab¹ssGb¹ss**dGssdTssdTssdAssdGssdGssdGssdCssdTss**Gb¹ssAb¹** |
| 13 | 27s | **Gb¹ssGbss**dTssdTssdAssdGssdGssdGssdCssdTssdGss**Ab¹ssAb¹** |
| 13 | 28s | **Gb¹ssTb¹ss**dTssdAssdGssdGssdGssdCssdTssdGssdAss**Ab¹ssTb¹** |
| 13 | 29s | **Tb¹ssTb¹ss**dAssdGssdGssdGssdCssdTssdGssdAssdAss**Tb¹ssTb¹** |
| 13 | 26t | **Ab¹ssGb¹ssGb¹ss**dTssdTssdAssdGssdGssdGssdCssdTss**Gb¹ssAb¹** |
| 13 | 27t | **Gb¹ssGb¹ssTb¹ss**dTssdAssdGssdGssdGssdCssdTssdGss**Ab¹ssAb¹** |
| 13 | 28t | **Gb¹ssTb¹ssTb¹ss**dAssdGssdGssdGssdCssdTssdGssdAss**Ab¹ssTb¹** |
| 13 | 29t | **Tb¹ssTb¹ssAb¹ss**dGssdGssdGssdCssdTssdGssdAssdAss**Tb¹ssTb¹** |
| 13 | 26u | **Ab¹ssGb¹ss**dGssdTssdTssdAssdGssdGssdGss**Cb¹ssTb¹ssGb¹ssAb¹** |
| 13 | 27u | **Gb¹ssGb¹ss**dTssdTssdAssdGssdGssdGssdCssdTss**Gb¹ssAb¹ssAb¹** |
| 13 | 28u | **Gb¹ssTb¹ss**dTssdAssdGssdGssdGssdCssdTssdGss**Ab¹ssAb¹ssTb¹** |
| 13 | 29u | **Tb¹ssTb¹ss**dAssdGssdGssdGssdCssdTssdGssdAss**AbssTbssTb¹** |
| 14 | 30q | **Ab¹sGb¹s**dGsdTsdTsdAsdGsdGsdGsdCsdTs**Gb¹sAb¹sAb¹** |
| 14 | 31q | **Gb¹sGb¹s**dTsdTsdAsdGsdGsdGsdCsdTsdGs**Ab¹sAb¹sTb¹** |
| 14 | 32q | **Gb¹sTb¹s**dTsdAsdGsdGsdGsdCsdTsdGsdAs**Ab¹sTb¹sTb¹** |
| 14 | 30r | **Ab¹sGb¹sGb¹s**dTsdTsdAsdGsdGsdGsdCsdTs**Gb¹sAb¹sAb¹** |
| 14 | 31r | **Gb¹sGb¹sTb¹s**dTsdAsdGsdGsdGsdCsdTsdGs**Ab¹sAb¹sTb¹** |
| 14 | 32r | **Gb¹sTb¹sTb¹s**dAsdGsdGsdGsdCsdTsdGsdAs**Ab¹sTb¹sTb¹** |
| 14 | 30s | **Ab¹sGb¹s**dGsdTsdTsdAsdGsdGsdGsdCsdTs**Gb¹sAb¹sAb¹** |
| 14 | 31s | **Gb¹sGb¹s**dTsdTsdAsdGsdGsdGsdCsdTsdGs**Ab¹sAb¹sTb¹** |
| 14 | 32s | **Gb¹sTb¹s**dTsdAsdGsdGsdGsdCsdTsdGsdAs**Ab¹sTb¹sTb¹** |
| 14 | 30t | **Ab¹sGb¹s**dGsdTsdTsdAsdGsdGsdGsdCsdTsdGs**Ab¹sAb¹** |
| 14 | 31t | **Gb¹sGb¹s**dTsdTsdAsdGsdGsdGsdCsdTsdGsdAs**Ab¹sTb¹** |
| 14 | 32t | **Gb¹sTb¹s**dTsdAsdGsdGsdGsdCsdTsdGsdAsdAs**Tb¹sTb¹** |
| 14 | 30u | **Ab¹Gb¹**dGsdTsdTsdAsdGsdGsdGsdCsdTs**Gb¹Ab¹Ab¹** |
| 14 | 31u | **Gb¹Gb¹**dTsdTsdAsdGsdGsdGsdCsdTsdGs**Ab¹Ab¹Tb¹** |
| 14 | 32u | **Gb¹Tb¹**dTsdAsdGsdGsdGsdCsdTsdGsdAs**Ab¹Tb¹Tb¹** |
| 14 | 30v | **Ab¹Gb¹Gb¹**dTsdTsdAsdGsdGsdGsdCsdTs**Gb¹Ab¹Ab¹** |
| 14 | 31v | **Gb¹Gb¹Tb¹**dTsdAsdGsdGsdGsdCsdTsdGs**Ab¹Ab¹Tb¹** |
| 14 | 32v | **Gb¹Tb¹Tb¹**dAsdGsdGsdGsdCsdTsdGsdAs**Ab¹Tb¹Tb¹** |
| 14 | 30w | **Ab¹Gb¹**dGsdTsdTsdAsdGsdGsdGsdCsdTs**Gb¹Ab¹Ab¹** |
| 14 | 31w | **Gb¹Gb¹**dTsdTsdAsdGsdGsdGsdCsdTsdGs**Ab¹AbTb¹** |
| 14 | 32w | **Gb¹Tb¹**dTsdAsdGsdGsdGsdCsdTsdGsdAs**Ab¹Tb¹Tb¹** |
| 14 | 30x | **Ab¹Gb¹**dGsdTsdTsdAsdGsdGsdGsdCsdTsdGs**Ab¹Ab¹** |
| 14 | 31x | **Gb¹Gb¹**dTsdTsdAsdGsdGsdGsdCsdTsdGsdAs**Ab¹Tb¹** |
| 14 | 32x | **Gb¹Tb¹**dTsdAsdGsdGsdGsdCsdTsdGsdAsdAs**Tb¹Tb¹** |
| 14 | 30y | Ab1ssGb1ssdGssdTssdTssdAssdGssdGssdGssdCssdTssGb1ss Ab1ssAb1 |
| 14 | 31y | Gb^1ssGb^1ssdTssdTssdAssdGssdGssdGssdCssdTssdGssAb^1ss Ab^1ssTb^1 |
| 14 | 32y | Gb1ssTb1ssdTssdAssdGssdGssdGssdCssdTssdGssdAssAb1ss Tb1ssTb1 |
| 14 | 30z | Ab1ssGb1ssGb1ssdTssdTssdAssdGssdGssdGssdCssdTssGb1ss Ab1ssAb1 |
| 14 | 31z | Gb1ssGb1ssTb1ssdTssdAssdGssdGssdGssdCssdTssdGssAb1ss Ab1ssTb1 |
| 14 | 32z | Gb1ssTb1ssTb1ssdAssdGssdGssdGssdCssdTssdGssdAssAb1ss Tb1ssTb1 |
| 14 | 30aa | Ab1ssGb1ssdGssdTssdTssdAssdGssdGssdGssdCssdTssGb1ss Ab1ssAb1 |
| 14 | 31aa | Gb1ssGb1ssdTssdTssdAssdGssdGssdGssdCssdTssdGssAb1ss Ab1ssTb1 |
| 14 | 32aa | Gb1ssTb1ssdTssdAssdGssdGssdGssdCssdTssdGssdAssAb1ss Tb1ssTb1 |
| 14 | 30ab | Ab1ssGb1ssdGssdTssdTssdAssdGssdGssdGssdCssdTssd GssAb1ssAb1 |
| 14 | 31ab | Gb¹ssGb¹ssdTssdTssdAssdGssdGssdGssdCssdTssdGssd AssAb¹ssTb¹ |
| 14 | 32ab | Gb¹ssTb¹ssdTssdAssdGssdGssdGssdCssdTssdGssdAssd AssTb¹ssTb¹ |
| 15 | 19q | **Ab¹sGb¹s**dGsdTsdTsdAsdGsdGsdGsdCsdTs**Gb¹sAb¹sAb¹sTb¹** |
| 15 | 20q | **Gb¹sGb¹s**dTsdTsdAsdGsdGsdGsdCsdTsdGs**Ab¹sAb¹sTb¹sTb¹** |
| 15 | 19r | **Ab¹sGb¹s**dGsdTsdTsdAsdGsdGsdGsdCsdTsdGsdAs**Ab¹sTb¹** |
| 15 | 20r | **Gb¹sGb¹s**dTsdTsdAsdGsdGsdGsdCsdTsdGsdAsdAs**Tb¹sTb¹** |
| 15 | 19s | **Ab¹sGb¹sGb¹s**dTsdTsdAsdGsdGsdGsdCsdTsdGs**Ab¹sAb¹sTb¹** |
| 15 | 20s | **Gb¹sGb¹sTb¹s**dTsdAsdGsdGsdGsdCsdTsdGsdAs**Ab¹sTb¹sTb¹** |
| 15 | 19t | **Ab¹sGb¹sGb¹sTb¹s**dTsdAsdGsdGsdGsdCsdTsdGs**Ab¹sAb¹sTb¹** |
| 15 | 20t | **Gb¹sGb¹sTb¹sTb¹s**dAsdGsdGsdGsdCsdTsdGsdAs**Ab¹sTb¹sTb¹** |
| 15 | 19u | **Ab¹Gb¹**dGsdTsdTsdAsdGsdGsdGsdCsdTs**Gb¹Ab¹Ab¹Tb¹** |
| 15 | 20u | **Gb¹Gb¹**dTsdTsdAsdGsdGsdGsdCsdTsdGs**Ab¹Ab¹Tb¹Tb¹** |
| 15 | 19v | **Ab¹Gb¹**dGsdTsdTsdAsdGsdGsdGsdCsdTsdGsdAs**Ab¹Tb¹** |
| 15 | 20v | **Gb¹Gb¹**dTsdTsdAsdGsdGsdGsdCsdTsdGsdAsdAs**Tb¹Tb¹** |
| 15 | 19w | **Ab¹Gb¹Gb¹**dTsdTsdAsdGsdGsdGsdCsdTsdGs**Ab¹Ab¹Tb¹** |
| 15 | 20w | **Gb¹Gb¹Tb¹**dTsdAsdGsdGsdGsdCsdTsdGsdAs**Ab¹Tb¹Tb¹** |
| 15 | 19x | **Ab¹Gb¹Gb¹Tb¹**dTsdAsdGsdGsdGsdCsdTsdGs**Ab¹Ab¹Tb¹** |
| 15 | 20x | **Gb¹Gb¹Tb¹Tb¹**dAsdGsdGsdGsdCsdTsdGsdAs**Ab¹Tb¹Tb¹** |
| 15 | 19y | Ab¹ssGb¹ssdGssdTssdTssdAssdGssdGssdGssdCssdTssGb¹ssAb¹ss Ab¹ssTb¹ |
| 15 | 20y | Gb1ssGb1ssdTssdTssdAssdGssdGssdGssdCssdTssdGssAb1ssAb1ss Tb1ssTb1 |
| 15 | 19z | **Ab**^1^**ssGb**^1^**ss**dGssdTssdTssdAssdGssdGssdGssdCssdTssdGss dAss**Ab**^1^**ssTb**^1^ |
| 15 | 20z | Gb1ssGb1ssdTssdTssdAssdGssdGssdGssdCssdTssdGssdAss dAssTb1ssTb1 |
| 15 | 19aa | Ab¹ssGb¹ssGb¹ssdTssdTssdAssdGssdGssdGssdCssdTssdGssAb¹ss Ab¹ssTb¹ |
| 15 | 20aa | Gb¹ssGb¹ssTb¹ssdTssdAssdGssdGssdGssdCssdTssdGssdAssAb¹ss Tb¹ssTb¹ |
| 15 | 19ab | Ab1ssGb1ssGbssTb1ssdTssdAssdGssdGssdGssdCssdTssdGssAb1ss Ab1ssTb1 |
| 15 | 20ab | Gb1ssGb1ssTb1ssTb1ssdAssdGssdGssdGssdCssdTssdGssdAssAb1ss Tb1ssTb1 |
| 16 | 4as | **Ab¹sGb¹s**dGsdTsdTsdAsdGsdGsdGsdCsdTsdGsdAs**Ab¹sTb¹sTb¹** |
| 16 | 4at | **Ab¹sGb¹s**dGsdTsdTsdAsdGsdGsdGsdCsdTsdGsdAsdAs**Tb¹sTb¹** |
| 16 | 4au | **Ab¹sGb¹sGb¹s**dTsdTsdAsdGsdGsdGsdCsdTsdGsdAsdAs**Tb¹sTb¹** |
| 16 | 4av | **Ab¹sGb¹sGb¹sTb¹s**dTsdAsdGsdGsdGsdCsdTsdGsdAsdAs**Tb¹sTb¹** |
| 16 | 4aw | **Ab¹sGb¹s**dGsdTsdTsdAsdGsdGsdGsdCsdTsdGs**Ab¹sAb¹sTb¹sTb¹** |
| 16 | 4ax | **Ab¹sGb¹sGb¹s**dTsdTsdAsdGsdGsdGsdCsdTsdGsdAs**Ab¹sTb¹sTb¹** |
| 16 | 4ay | **Ab¹sGb¹sGb¹sTb¹s**dTsdAsdGsdGsdGsdCsdTsdGs**Ab¹sAb¹sTb¹sTb¹** |
| 16 | 4az | **Ab¹sGb¹sGb¹sTb¹sTb¹s**dAsdGsdGsdGsdCsdTsdGsdAs**Ab¹sTb¹sTb¹** |
| 16 | 4ba | **Ab¹sGb¹sGb¹s**dTsdTsdAsdGsdGsdGsdCsdTs**Gb¹sAb¹sAb¹sTb¹sTb¹** |
| 16 | 4bb | **Ab¹sGb¹sGb¹s**dTsdTsdAsdGsdGsdGsdCsdTsdGs**Ab¹sAb¹sTb¹sTb¹** |
| 16 | 4bc | **Ab¹sGb¹sGb¹sTb¹s**dTsdAsdGsdGsdGsdCsdTsdGsdAs**Ab¹sTb¹sTb¹** |
| 16 | 4bd | **Ab¹Gb¹**dGsdTsdTsdAsdGsdGsdGsdCsdTsdGsdAs**Ab¹Tb¹Tb¹** |
| 16 | 4be | **Ab¹Gb¹**dGsdTsdTsdAsdGsdGsdGsdCsdTsdGsdAsdAs**Tb¹Tb¹** |
| 16 | 4bf | **Ab¹Gb¹Gb¹**dTsdTsdAsdGsdGsdGsdCsdTsdGsdAsdAs**Tb¹Tb¹** |
| 16 | 4bg | **Ab¹Gb¹Gb¹Tb¹**dTsdAsdGsdGsdGsdCsdTsdGsdAsdAs**Tb¹Tb¹** |
| 16 | 4bh | **Ab¹Gb¹**dGsdTsdTsdAsdGsdGsdGsdCsdTsdGs**Ab¹Ab¹Tb¹Tb¹** |
| 16 | 4bi | **Ab¹Gb¹Gb¹**dTsdTsdAsdGsdGsdGsdCsdTsdGsdAs**Ab¹Tb¹Tb¹** |
| 16 | 4bj | **Ab¹Gb¹Gb¹Tb¹**dTsdAsdGsdGsdGsdCsdTsdGs**Ab¹Ab¹Tb¹Tb¹** |
| 16 | 4bk | **Ab¹Gb¹Gb¹Tb¹Tb¹**dAsdGsdGsdGsdCsdTsdGsdAs**Ab¹Tb¹Tb¹** |
| 16 | 4bl | **Ab¹Gb¹Gb¹**dTsdTsdAsdGsdGsdGsdCsdTs**Gb¹Ab¹Ab¹Tb¹Tb¹** |
| 16 | 4bm | **Ab¹Gb¹Gb¹**dTsdTsdAsdGsdGsdGsdCsdTsdGs**Ab¹Ab¹Tb¹Tb¹** |
| 16 | 4bn | **Ab¹Gb¹Gb¹Tb¹**dTsdAsdGsdGsdGsdCsdTsdGsdAs**Ab¹Tb¹Tb¹** |
| 16 | 4bo | Ab1ssGb1ssdGssdTssdTssdAssdGssdGssdGssdCssdTssdGssdAss Ab1ssTb1ssTb1 |
| 16 | 4bp | Ab1ssGb1ssdGssdTssdTssdAssdGssdGssdGssdCssdTssdGssdAss dAssTb1ssTb1 |
| 16 | 4bq | Ab1ssGb1ssGb1ssdTssdTssdAssdGssdGssdGssdCssdTssdGssdAss dAssTb1ssTb1 |
| 16 | 4br | Ab¹ssGb¹ssGb¹ssTb¹ssdTssdAssdGssdGssdGssdCssdTssdGssdAss dAssTb¹ssTb¹ |
| 16 | 4bs | Ab1ssGb1ssdGssdTssdTssdAssdGssdGssdGssdCssdTssdGssAb1ss Ab1ssTb1ssTb1 |
| 16 | 4bt | Ab1ssGb1ssGb1ssdTssdTssdAssdGssdGssdGssdCssdTssdGssdAss Ab1ssTb1ssTb1 |
| 16 | 4bu | Ab¹ssGb¹ssGb¹ssTb¹ssdTssdAssdGssdGssdGssdCssdTssdGssAb¹ss Ab¹ssTb¹ssTb¹ |
| 16 | 4bv | Ab¹ssGb¹ssGb¹ssTb¹ssTb¹ssdAssdGssdGssdGssdCssdTssdGssdAss Ab¹ssTb¹ssTb¹ |
| 16 | 4bw | Ab1ssGb1ssGb1ssdTssdTssdAssdGssdGssdGssdCssdTssGb1ssAb1ss Ab1ssTb1ssTb1 |
| 16 | 4bx | Ab1ssGb1ssGb1ssdTssdTssdAssdGssdGssdGssdCssdTssdGssAb1ss Ab1ssTb1ssTb1 |
| 16 | 4by | Ab¹ssGb¹ssGb¹ssTb¹ssdTssdAssdGssdGssdGssdCssdTssdGssdAss Ab¹ssTb¹ssTb¹ |

**Table 31**

| **L** | **Seq ID No.** | **Sequence, 5'-3'** |
|---|---|---|
| 12 | 46m | **C*b¹sAb¹s**dAsdGsdCsdAsdAsdGsdGsdCs**Ab¹sTb¹** |
| 12 | 45m | **Ab¹sC*b¹s**dAsdAsdGsdCsdAsdAsdGsdGs**C*b¹sAb¹** |
| 12 | 44m | **Tb¹sAb¹s**dCsdAsdAsdGsdCsdAsdAsdGs**Gb¹sC*b¹** |
| 12 | 47m | **Ab¹sAb¹s**dGsdCsdAsdAsdGsdGsdCsdAs**Tb¹sTb¹** |
| 12 | 48m | **Ab¹sGb¹s**dCsdAsdAsdGsdGsdCsdAsdTs**Tb¹sTb¹** |
| 12 | 46n | **C*b²s**dAsdAsdGsdCsdAsdAsdGsdGsdCs**Ab²sTb²** |
| 12 | 45n | **Ab²s**dCsdAsdAsdGsdCsdAsdAsdGsdGs**C*b²sAb²** |
| 12 | 44n | **Tb²s**dAsdCsdAsdAsdGsdCsdAsdAsdGs**Gb²sC*b²** |
| 12 | 47n | **Ab²s**dAsdGsdCsdAsdAsdGsdGsdCsdAs**Tb²sTb²** |
| 12 | 48n | **Ab²s**dGsdCsdAsdAsdGsdGsdCsdAsdTs**Tb²sTb²** |
| 12 | 46o | **C*b³sAb³s**dAsdGsdCsdAsdAsdGsdGsdCsdAs**Tb³** |
| 12 | 45o | **Ab³sC*b³s**dAsdAsdGsdCsdAsdAsdGsdGsdCs**Ab³** |
| 12 | 44o | **Tb³sAb³s**dCsdAsdAsdGsdCsdAsdAsdGsdGs**C*b³** |
| 12 | 47o | **Ab³sAb³s**dGsdCsdAsdAsdGsdGsdCsdAsdTs**Tb³** |
| 12 | 48o | **Ab³sGb³s**dCsdAsdAsdGsdGsdCsdAsdTsdTs**Tb³** |
| 12 | 46p | **C*b¹Ab¹**dAsdGsdCsdAsdAsdGsdGsdCs**Ab¹Tb¹** |
| 12 | 45p | **Ab¹Cb¹**dAsdAsdGsdCsdAsdAsdGsdGs**C*b¹Ab¹** |
| 12 | 44p | **Tb¹Ab¹**dCsdAsdAsdGsdCsdAsdAsdGs**Gb¹C*b¹** |
| 12 | 47p | **Ab¹Ab¹**dGsdCsdAsdAsdGsdGsdCsdAs**Tb¹Tb¹** |
| 12 | 48p | **Ab¹Gb¹**dCsdAsdAsdGsdGsdCsdAsdTs**Tb¹Tb¹** |
| 12 | 46q | **C*b⁴**dAsdAsdGsdCsdAsdAsdGsdGsdCs**Ab⁴Tb⁴** |
| 12 | 45q | **Ab⁴**dCsdAsdAsdGsdCsdAsdAsdGsdGs**C*b⁴Ab⁴** |
| 12 | 44q | **Tb⁴**dAsdCsdAsdAsdGsdCsdAsdAsdGs**Gb⁴C*b⁴** |
| 12 | 47q | **Ab⁴**dAsdGsdCsdAsdAsdGsdGsdCsdAs**Tb⁴Tb⁴** |
| 12 | 48q | **Ab⁴**dGsdCsdAsdAsdGsdGsdCsdAsdTs**Tb⁴Tb⁴** |
| 12 | 46r | **C*b⁵Ab⁵**dAsdGsdCsdAsdAsdGsdGsdCsdAs**Tb⁵** |
| 12 | 45r | **Ab⁵C*b⁵**dAsdAsdGsdCsdAsdAsdGsdGsdCs**Ab⁵** |
| 12 | 44r | **Tb⁵Ab⁵**dCsdAsdAsdGsdCsdAsdAsdGsdGs**C*b⁵** |
| 12 | 47r | **Ab⁵Ab⁵**dGsdCsdAsdAsdGsdGsdCsdAsdTs**Tb⁵** |
| 12 | 48r | **Ab⁵Gb⁵**dCsdAsdAsdGsdGsdCsdAsdTsdTs**Tb⁵** |
| 12 | 46s | **C*b¹ssAb¹ss**dAssdGssdCssdAssdAssdGssdGssdCss**Ab¹ssTb¹** |
| 12 | 45s | **Ab¹ssCb¹ss**dAssdAssdGssdCssdAssdAssdGssdGss**C*b¹ssAb¹** |
| 12 | 44s | **Tb¹ssAb¹ss**dCssdAssdAssdGssdCssdAssdAssdGss**Gb¹ssC*b¹** |
| 12 | 47s | **Ab¹ssAb¹ss**dGssdCssdAssdAssdGssdGssdCssdAss**Tb¹ssTb¹** |
| 12 | 48s | **Ab¹ssGb¹ss**dCssdAssdAssdGssdGssdCssdAssdTss**Tb¹ssTb¹** |
| 12 | 46t | **C*b⁶ss**dAssdAssdGssdCssdAssdAssdGssdGssdCss**Ab⁶ssTb⁶** |
| 12 | 45t | **Ab⁶ss**dCssdAssdAssdGssdCssdAssdAssdGssdGss**C*b⁶ssAb⁶** |
| 12 | 44t | **Tb⁶ss**dAssdCssdAssdAssdGssdCssdAssdAssdGss**Gb⁶ssC*b⁶** |
| 12 | 47t | **Ab⁶ss**dAssdGssdCssdAssdAssdGssdGssdCssdAss**Tb⁶ssTb⁶** |
| 12 | 48t | **Ab⁶ss**dGssdCssdAssdAssdGssdGssdCssdAssdTss**Tb⁶ssTb⁶** |
| 12 | 46u | **C*b⁷ssAb⁷ss**dAssdGssdCssdAssdAssdGssdGssdCssdAss**Tb⁷** |
| 12 | 45u | **Ab⁷ssC*b⁷ss**dAssdAssdGssdCssdAssdAssdGssdGssdCss**Ab⁷** |
| 12 | 44u | **Tb⁷ssAb⁷ss**dCssdAssdAssdGssdCssdAssdAssdGssdGss**C*b⁷** |
| 12 | 47u | **Ab⁷ssAb⁷ss**dGssdCssdAssdAssdGssdGssdCssdAssdTss**Tb⁷** |
| 12 | 48u | **Ab⁷ssGb⁷ss**dCssdAssdAssdGssdGssdCssdAssdTssdTss**Tb⁷** |
| 11 | 52i | **Ab¹s**dCsdAsdAsdGsdCsdAsdAsdGs**Gb¹sC*b¹** |
| 11 | 53i | **C*b¹s**dAsdAsdGsdCsdAsdAsdGsdGs**C*b¹sAb¹** |
| 11 | 54i | **Ab¹s**dAsdGsdCsdAsdAsdGsdGsdCs**Ab¹sTb¹** |
| 11 | 55i | **Ab¹s**dGsdCsdAsdAsdGsdGsdCsdAsd**Tb¹sTb¹** |
| 11 | 52j | **Ab¹sC*b¹s**dAsdAsdGsdCsdAsdAsdGsdGs**C*b¹** |
| 11 | 53j | **C*b¹sAb¹s**dAsdGsdCsdAsdAsdGsdGsdCs**Ab¹** |
| 11 | 54j | **Ab¹sAb¹s**dGsdCsdAsdAsdGsdGsdCsdAs**Tb¹** |
| 11 | 55j | **Ab¹sGb¹s**dCsdAsdAsdGsdGsdCsdAsddTs**Tb¹** |
| 11 | 52k | **Ab¹**dCsdAsdAsdGsdCsdAsdAsdGs**Gb¹Cb¹** |
| 11 | 53k | **C*b¹**dAsdAsdGsdCsdAsdAsdGsdGs**Cb¹Ab¹** |
| 11 | 54k | **Ab¹**dAsdGsdCsdAsdAsdGsdGsdCs**Ab¹Tb¹** |
| 11 | 55k | **Ab¹**dGsdCsdAsdAsdGsdGsdCsdAsd**Tb¹Tb¹** |
| 11 | 521 | **Ab¹C*b¹**dAsdAsdGsdCsdAsdAsdGsdGs**C*b¹** |
| 11 | 53l | **C*b¹Ab¹**dAsdGsdCsdAsdAsdGsdGsdCs**Ab¹** |
| 11 | 54l | **A¹bAb¹**dGsdCsdAsdAsdGsdGsdCsdAs**Tb¹** |
| 11 | 55l | **A¹bG¹b**dCsdAsdAsdGsdGsdCsdAsddTs**Tb¹** |
| 11 | 52m | **Ab¹ss**dCssdAssdAssdGssdCssdAssdAssdGss**Gb¹ssC*b¹** |
| 11 | 53m | **C*b¹ss**dAssdAssdGssdCssdAssdAssdGssdGss**Cb¹ssAb¹** |
| 11 | 54m | **Ab¹ss**dAssdGssdCssdAssdAssdGssdGssdCss**Ab¹ssTb¹** |
| 11 | 55m | **Ab¹ss**dGssdCssdAssdAssdGssdGssdCssdAssd**Tb¹ssTb¹** |
| 11 | 52n | **Ab¹ssC*b¹ss**dAssdAssdGssdCssdAssdAssdGssdGss**C*b¹** |
| 11 | 53n | **C*b¹ssAb¹ss**dAssdGssdCssdAssdAssdGssdGssdCss**Ab¹** |
| 11 | 54n | **Ab¹ssAb¹ss**dGssdCssdAssdAssdGssdGssdCssdAss**Tb¹** |
| 11 | 55n | **Ab¹ssGb¹ss**dCssdAssdAssdGssdGssdCssdAssddTss**Tb¹** |
| 10 | 39e | **C*b¹s**dAsdAsdGsdC*sdAsdAsdGsdGs**C*b¹** |
| 10 | 40e | **Ab¹s**dAsdGsdC*sdAsdAsdGsdGsdC*s**Ab¹** |
| 10 | 41e | **Ab¹s**dGsdC*sdAsdAsdGsdGsdC*sdAsd**Tb¹** |
| 10 | 39f | **C*b¹**dAsdAsdGsdCsdAsdAsdGsdGs**C*b¹** |
| 10 | 40f | **Ab¹**dAsdGsdCsdAsdAsdGsdGsdCs**Ab¹** |
| 10 | 41f | **Ab¹**dGsdCsdAsdAsdGsdGsdCsdAsd**Tb¹** |
| 10 | 39g | **C*b¹ss**dAssdAssdGssdCssdAssdAssdGssdGss**C*b¹** |
| 10 | 40g | **Ab¹ss**dAssdGssdCssdAssdAssdGssdGssdCss**Ab¹** |
| 10 | 41g | **Ab¹ss**dGssdCssdAssdAssdGssdGssdCssdAssd**Tb¹** |
| 13 | 57m | **Tb¹sAb¹s**dCsdAsdAsdGsdCsdAsdAsdGsdGs**C*b¹sAb¹** |
| 13 | 58m | **Ab¹sC*b¹s**dAsdAsdGsdCsdAsdAsdGsdGsdCs**Ab¹sTb¹** |
| 13 | 59m | **C*b¹sAb¹s**dAsdGsdCsdAsdAsdGsdGsdC*dAs**Tb¹sTb¹** |
| 13 | 60m | **Ab¹sAb¹s**dGsdCsdAsdAsdGsdGsdC*sdAsdTs**Tb¹sTb¹** |
| 13 | 57n | **Tb¹sAb¹sC*b¹s**dAsdAsdGsdCsdAsdAsdGsdGs**C*b¹sAb¹** |
| 13 | 58n | **Ab¹sC*b¹sAb¹s**dAsdGsdCsdAsdAsdGsdGsdCs**Ab¹sTb¹** |
| 13 | 59n | **C*b¹sAb¹sAb¹s**dGsdCsdAsdAsdGsdGsdCsdAs**Tb¹sTb¹** |
| 13 | 60n | **Ab¹sAb¹sGb¹s**dCsdAsdAsdGsdGsdCsdAsdTs**Tb¹sTb¹** |
| 13 | 57o | **Tb¹sAb¹s**dCsdAsdAsdGsdCsdAsdAsdGs**Gb¹sC*b¹sAb¹** |
| 13 | 58o | **Ab¹sC*b¹s**dAsdAsdGsdCsdAsdAsdGsdGs**C*b¹sAb¹sTb¹** |
| 13 | 59o | **C*b¹sAb¹s**dAsdGsdCsdAsdAsdGsdGsdCs**Ab¹sTb¹sTb¹** |
| 13 | 60o | **Ab¹sAb¹s**dGsdCsdAsdAsdGsdGsdCsdAs**Tb¹sTb¹sTb¹** |
| 13 | 57p | **Tb¹Ab¹**dCsdAsdAsdGsdCsdAsdAsdGsdGs**C*b¹Ab¹** |
| 13 | 58p | **Ab¹C*b¹**dAsdAsdGsdCsdAsdAsdGsdGsdCs**Ab¹Tb¹** |
| 13 | 59p | **C*b¹Ab¹**dAsdGsdCsdAsdAsdGsdGsdCsdAs**Tb¹Tb¹** |
| 13 | 60p | **Ab¹Ab¹**dGsdCsdAsdAsdGsdGsdCsdAsdTs**Tb¹Tb¹** |
| 13 | 57q | **Tb¹Ab¹C*b¹**dAsdAsdGsdCsdAsdAsdGsdGs**C*b¹Ab¹** |
| 13 | 58q | **Ab¹C*b¹Ab¹**dAsdGsdCsdAsdAsdGsdGsdCs**Ab¹Tb¹** |
| 13 | 59q | **C*b¹Ab¹Ab¹**dGsdCsdAsdAsdGsdGsdCsdAs**Tb¹Tb¹** |
| 13 | 60q | **Ab¹Ab¹Gb¹**dCsdAsdAsdGsdGsdCsdAsdTs**Tb¹Tb¹** |
| 13 | 57r | **Tb¹Ab¹**dCsdAsdAsdGsdCsdAsdAsdGs**Gb¹C*b¹Ab¹** |
| 13 | 58r | **Ab¹C*b¹**dAsdAsdGsdCsdAsdAsdGsdGs**C*b¹Ab¹Tb¹** |
| 13 | 59r | **C*b¹Ab¹**dAsdGsdCsdAsdAsdGsdGsdCs**Ab¹Tb¹Tb¹** |
| 13 | 60r | **Ab¹Ab¹**dGsdCsdAsdAsdGsdGsdCsdAs**Tb¹Tb¹Tb¹** |
| 13 | 57s | **Tb¹ssAb¹ss**dCssdAssdAssdGssdCssdAssdAssdGssdGss**C*b¹ssAb¹** |
| 13 | 58s | **Ab¹ssC*b¹ss**dAssdAssdGssdCssdAssdAssdGssdGssdCss**Ab¹ssTb¹** |
| 13 | 59s | **C*b¹ssAb¹ss**dAssdGssdCssdAssdAssdGssdGssdCssdAss**Tb¹ssTb¹** |
| 13 | 60s | **Ab¹ssAb¹ss**dGssdCssdAssdAssdGssdGssdCssdAssdTss**Tb¹ssTb¹** |
| 13 | 57t | **Tb¹ssAb¹ssC*b¹ss**dAssdAssdGssdCssdAssdAssdGssdGss**C*b¹ssAb¹** |
| 13 | 58t | **Ab¹ssC*b¹ssAb¹ss**dAssdGssdCssdAssdAssdGssdGssdCss**Ab¹ssTb¹** |
| 13 | 59t | **C*b¹ssAb¹ssAb¹ss**dGssdCssdAssdAssdGssdGssdCssdAss**Tb¹ssTb¹** |
| 13 | 60t | **Ab¹ssAb¹ssGb¹ss**dCssdAssdAssdGssdGssdCssdAssdTss**Tb¹ssTb¹** |
| 13 | 57u | **Tb¹ssAb¹ss**dCssdAssdAssdGssdCssdAssdAssdGss**Gb¹ssC*b¹ssAb¹** |
| 13 | 58u | **Ab¹ssC*b¹ss**dAssdAssdGssdCssdAssdAssdGssdGss**C*b¹ssAb¹ssTb¹** |
| 13 | 59u | **C*b¹ssAb¹ss**dAssdGssdCssdAssdAssdGssdGssdCss**Ab¹ssTb¹ssTb¹** |
| 13 | 60u | **Ab¹ssAb¹ss**dGssdCssdAssdAssdGssdGssdCssdAss**Tb¹ssTb¹ssTb¹** |
| 14 | 61q | **Tb¹sAb¹s**dCsdAsdAsdGsdCsdAsdAsdGsdGsdCs**Ab¹sTb¹** |
| 14 | 62q | **Ab¹sC*b¹s**dAsdAsdGsdCsdAsdAsdGsdGsdCsdAs**Tb¹sTb¹** |
| 14 | 63q | **C*b¹sAb¹s**dAsdGsdCsdAsdAsdGsdGsdCsdAsdTs**Tb¹sTb¹** |
| 14 | 61r | **Tb¹sAb¹sC*b¹s**dAsdAsdGsdCsdAsdAsdGsdGsdCs**Ab¹sTb¹** |
| 14 | 62r | **Ab¹sC*b¹sAb¹s**dAsdGsdCsdAsdAsdGsdGsdCsdAs**Tb¹sTb¹** |
| 14 | 63r | **C*b¹sAb¹sAb¹s**dGsdCsdAsdAsdGsdGsdCsdAsdTs**Tb¹sTb¹** |
| 14 | 61s | **Tb¹sAb¹s**dCsdAsdAsdGsdCsdAsdAsdGsdGs**C*b¹sAb¹sTb¹** |
| 14 | 62s | **Ab¹sC*b¹s**dAsdAsdGsdCsdAsdAsdGsdGsdCs**Ab¹sTb¹sTb¹** |
| 14 | 63s | **C*b¹sAb¹s**dAsdGsdCsdAsdAsdGsdGsdCsdAs**Tb¹sTb¹sTb¹** |
| 14 | 61t | **Tb¹sAb¹sC*b¹s**dAsdAsdGsdCsdAsdAsdGsdGs**C*b¹sAb¹sTb¹** |
| 14 | 62t | **Ab¹sC*b¹sAb¹s**dAsdGsdCsdAsdAsdGsdGsdCs**Ab¹sTb¹sTb¹** |
| 14 | 63t | **C*b¹sAb¹sAb¹s**dGsdCsdAsdAsdGsdGsdCsdAs**Tb¹sTb¹sTb¹** |
| 14 | 61u | **Tb¹Ab¹**dCsdAsdAsdGsdCsdAsdAsdGsdGsdCs**Ab¹Tb¹** |
| 14 | 62u | **Ab¹C*b¹**dAsdAsdGsdCsdAsdAsdGsdGsdCsdAs**Tb¹Tb¹** |
| 14 | 63u | **C*b¹Ab¹**dAsdGsdCsdAsdAsdGsdGsdCsdAsdTs**Tb¹Tb¹** |
| 14 | 61v | **Tb¹Ab¹C*b¹**dAsdAsdGsdCsdAsdAsdGsdGsdCs**Ab¹Tb¹** |
| 14 | 62v | **Ab¹C*b¹Ab¹**dAsdGsdCsdAsdAsdGsdGsdCsdAs**Tb¹Tb¹** |
| 14 | 63v | **C*b¹Ab¹Ab¹**dGsdCsdAsdAsdGsdGsdCsdAsdTs**Tb¹Tb¹** |
| 14 | 61w | **Tb¹Ab¹**dCsdAsdAsdGsdCsdAsdAsdGsdGs**C*b¹Ab¹Tb¹** |
| 14 | 62w | **Ab¹C*b¹**dAsdAsdGsdCsdAsdAsdGsdGsdCs**Ab¹Tb¹Tb¹** |
| 14 | 63w | **C*b¹Ab¹**dAsdGsdCsdAsdAsdGsdGsdCsdAs**Tb¹Tb¹Tb¹** |
| 14 | 61x | **Tb¹Ab¹C*b¹**dAsdAsdGsdCsdAsdAsdGsdGs**C*b¹Ab¹Tb¹** |
| 14 | 62x | **Ab¹C*b¹Ab¹**dAsdGsdCsdAsdAsdGsdGsdCs**Ab¹Tb¹Tb¹** |
| 14 | 63x | **C*b¹Ab¹Ab¹**dGsdCsdAsdAsdGsdGsdCsdAs**Tb¹Tb¹Tb¹** |
| 14 | 61y | **Tb¹ssAb¹ss**dCssdAssdAssdGssdCssdAssdAssdGssdGssdCss**Ab¹ssTb¹** |
| 14 | 62y | **Ab¹ssC*b¹ss**dAssdAssdGssdCssdAssdAssdGssdGssdCssdAss**Tb¹ssTb¹** |
| 14 | 63y | **C*b¹ssAb¹ss**dAssdGssdCssdAssdAssdGssdGssdCssdAssdTss**Tb¹ssTb¹** |
| 14 | 61z | **Tb¹ssAb¹ssC*b¹ss**dAssdAssdGssdCssdAssdAssdGssdGssdCss**Ab¹ssTb¹** |
| 14 | 62z | **Ab¹ssC*b¹ssAb¹ss**dAssdGssdCssdAssdAssdGssdGssdCssdAss**Tb¹ssTb¹** |
| 14 | 63z | **C*b¹ssAb¹ssAb¹ss**dGssdCssdAssdAssdGssdGssdCssdAssdTss**Tb¹ssTb¹** |
| 14 | 61aa | **Tb¹ssAb¹ss**dCssdAssdAssdGssdCssdAssdAssdGssdGss**C*b¹ssAb¹ssTb¹** |
| 14 | 62aa | **Ab¹ssC*b¹ss**dAssdAssdGssdCssdAssdAssdGssdGssdCss**Ab¹ssTb¹ssTb¹** |
| 14 | 63aa | **C*b¹ssAb¹ss**dAssdGssdCssdAssdAssdGssdGssdCssdAss**Tb¹ssTb¹ssTb¹** |
| 14 | 61ab | **Tb¹ssAb¹ssC*b¹ss**dAssdAssdGssdCssdAssdAssdGssdGss**C*b¹ssAb¹ssTb¹** |
| 14 | 62ab | **Ab¹ssC*b¹ssAb¹ss**dAssdGssdCssdAssdAssdGssdGssdCss**Ab¹ssTb¹ssTb¹** |
| 14 | 63ab | **C*b¹ssAb¹ssAb¹ss**dGssdCssdAssdAssdGssdGssdCssdAss**Tb¹ssTb¹ssTb¹** |
| 15 | 49q | **Tb¹sAb¹s**dCsdAsdAsdGsdCsdAsdAsdGsdGsdCsdAs**Tb¹sTbs¹** |
| 15 | 50q | **Ab¹sC*b¹s**dAsdAsdGsdCsdAsdAsdGsdGsdCsdAsdTs**Tb¹sTb¹** |
| 15 | 49r | **Tb¹sAb¹sC*b¹s**dAsdAsdGsdCsdAsdAsdGsdGsdCsdAs**Tb¹sTb¹s** |
| 15 | 50r | **Ab¹sC*b¹sAb¹s**dAsdGsdCsdAsdAsdGsdGsdCsdAsdTs**Tb¹sTb¹** |
| 15 | 49s | **Tb¹sAb¹s**dCsdAsdAsdGsdCsdAsdAsdGsdGsdCs**Ab¹sTb¹sTbs¹** |
| 15 | 50s | **Ab¹sC*b¹s**dAsdAsdGsdCsdAsdAsdGsdGsdC*sdAs**Tb¹sTb¹sTb¹** |
| 15 | 49t | **Tb¹sAb¹sC*b¹s**dAsdAsdGsdCsdAsdAsdGsdGsdCs**Ab¹sTb¹sTb¹s** |
| 15 | 50t | **Ab¹sC*b¹sAb¹s**dAsdGsdCsdAsdAsdGsdGsdCsdAs**Tb¹sTb¹sTb¹** |
| 15 | 49u | **Tb¹Ab¹**dCsdAsdAsdGsdCsdAsdAsdGsdGsdCsdAs**Tb¹Tb¹** |
| 15 | 50u | **Ab¹C*b¹**dAsdAsdGsdCsdAsdAsdGsdGsdCsdAsdTs**Tb¹Tb¹** |
| 15 | 49v | **Tb¹Ab¹C*b¹**dAsdAsdGsdCsdAsdAsdGsdGsdCsdAs**Tb¹Tb¹** |
| 15 | 50v | **Ab¹C*b¹Ab¹**dAsdGsdCsdAsdAsdGsdGsdCsdAsdTs**Tb¹Tb¹** |
| 15 | 49w | **Tb¹Ab¹**dCsdAsdAsdGsdCsdAsdAsdGsdGsdCs**Ab¹Tb¹Tb¹** |
| 15 | 50w | **Ab¹C*b¹**dAsdAsdGsdCsdAsdAsdGsdGsdCsdAs**Tb¹Tb¹Tb¹** |
| 15 | 49x | **Tb¹Ab¹C*b¹**dAsdAsdGsdCsdAsdAsdGsdGsdCs**Ab¹Tb¹Tb¹** |
| 15 | 50x | **Ab¹C*b¹Ab¹**dAsdGsdCsdAsdAsdGsdGsdCsdAs**Tb¹Tb¹Tb¹** |
| 15 | 49y | Tb1ssAb1ssdCssdAssdAssdGssdCssdAssdAssdGssdGssdCssdAss Tb1ssTb1ss |
| 15 | 50y | AbssC*bssdAssdAssdGssdCssdAssdAssdGssdGssdCssdAssdTssTbss Tb1 |
| 15 | 49z | TbssAbssC*bssdAssdAssdGssdCssdAssdAssdGssdGssdCssdAssTb1ss Tb1 |
| 15 | 50z | Ab¹ssC*b¹ssAb¹ssdAssdGssdCssdAssdAssdGssdGssdCssdAssdTss Tb¹ssTb¹ |
| 15 | 49aa | Tb^1ssAb^1ssdCssdAssdAssdGssdCssdAssdAssdGssdGssdCssAb^1ss Tb^1ssTb^1 |
| 15 | 50aa | Ab1ssC*b1ssdAssdAssdGssdCssdAssdAssdGssdGssdCssdAssTb1ss Tb1ssTb1 |
| 15 | 49ab | Tb^1ssAb^1ssC*b^1ssdAssdAssdGssdCssdAssdAssdGssdGssdCssAb^1ss Tb^1ssTb^1 |
| 15 | 50ab | Ab¹ssC*b¹ssAb¹ssdAssdGssdCssdAssdAssdGssdGssdCssdAssTb¹ss Tb¹ssTb¹ |
| 16 | 5as | **Tb¹sAb¹s**dCsdAsdAsdGsdCsdAsdAsdGsdGsdCsdAsdTs**Tb¹sTb¹** |
| 16 | 5at | **Tb¹sAb¹sC*b¹s**dAsdAsdGsdCsdAsdAsdGsdGsdCsdAsdTs**Tb¹sTb¹** |
| 16 | 5au | **Tb¹sAb¹sC*b¹sAb¹s**dAsdGsdCsdAsdAsdGsdGsdCsdAsdTs**Tb¹sTb¹** |
| 16 | 5av | **Tb¹sAb¹s**dCsdAsdAsdGsdCsdAsdAsdGsdGsdCsdAs**Tb¹sTb¹sTb¹** |
| 16 | 5aw | **Tb¹sAb¹s**dCsdAsdAsdGsdCsdAsdAsdGsdGsdCs**Ab¹sTb¹sTb¹sTb¹** |
| 16 | 5ax | **Tb¹sAb¹sC*b¹s**dAsdAsdGsdCsdAsdAsdGsdGsdCsdAs**Tb¹sTb¹sTb¹** |
| 16 | 5ay | **Tb¹sAb¹sC*b¹sAb¹s**dAsdGsdCsdAsdAsdGsdGsdCsdAs**Tb¹sTb¹sTb¹** |
| 16 | 5az | **Tb¹sAb¹sC*b¹s**dAsdAsdGsdCsdAsdAsdGsdGsdCs**Ab¹sTb¹sTb¹sTb¹** |
| 16 | 5ba | **Tb¹sAb¹sC*b¹sAb¹s**dAsdGsdCsdAsdAsdGsdGsdCs**Ab¹sTb¹sTb¹sTb¹** |
| 16 | 5bb | **Tb¹sAb¹sC*b¹sAb¹s**dAsdGsdCsdAsdAsdGsdGsdCsdAs**Tb¹sTb¹sTb¹** |
| 16 | 5bc | **Tb¹sAb¹sC*b¹s**dAsdAsdGsdCsdAsdAsdGsdGsdCs**Ab¹sTb¹sTb¹sTb¹** |
| 16 | 5bd | **Tb¹Ab¹**dCsdAsdAsdGsdCsdAsdAsdGsdGsdCsdAsdTs**Tb¹Tb¹** |
| 16 | 5be | **Tb¹Ab¹C*b¹**dAsdAsdGsdCsdAsdAsdGsdGsdCsdAsdTs**Tb¹Tb¹** |
| 16 | 5bf | **Tb¹Ab¹C*b¹Ab¹**dAsdGsdCsdAsdAsdGsdGsdCsdAsdTs**Tb¹Tb¹** |
| 16 | 5bg | **Tb¹Ab¹**dCsdAsdAsdGsdCsdAsdAsdGsdGsdCsdAs**Tb¹Tb¹Tb¹** |
| 16 | 5bh | **Tb¹Ab¹**dCsdAsdAsdGsdCsdAsdAsdGsdGsdCs**Ab¹Tb¹Tb¹Tb¹** |
| 16 | 5bi | **Tb¹Ab¹C*b¹**dAsdAsdGsdCsdAsdAsdGsdGsdCsdAs**Tb¹Tb¹Tb¹** |
| 16 | 5b | **Tb¹Ab¹C*b¹Ab¹**dAsdGsdCsdAsdAsdGsdGsdCsdAs**Tb¹Tb¹Tb¹** |
| 16 | 5bk | **Tb¹Ab¹C*b¹**dAsdAsdGsdCsdAsdAsdGsdGsdCs**Ab¹Tb¹Tb¹Tb¹** |
| 16 | 5bl | **Tb¹Ab¹C*b¹Ab¹**dAsdGsdCsdAsdAsdGsdGsdCs**Ab¹Tb¹Tb¹Tb¹** |
| 16 | 5bm | **Tb¹Ab¹C*b¹Ab¹**dAsdGsdCsdAsdAsdGsdGsdCsdAs**Tb¹Tb¹Tb¹** |
| 16 | 5bn | **Tb¹Ab¹C*b¹**dAsdAsdGsdCsdAsdAsdGsdGsdCs**Ab¹Tb¹Tb¹Tb¹** |
| 16 | 5bo | Tb^1ssAb^1ssdCssdAssdAssdGssdCssdAssdAssdGssdGssdCssdAssdTss Tb^1ssTb^1 |
| 16 | 5bp | Tb1ssAb1ssC*b1ssdAssdAssdGssdCssdAssdAssdGssdGssdCssdAssdTss Tb1ssTb1 |
| 16 | 5bq | Tb1ssAb1ssC*b1ssAb1ssdAssdGssdCssdAssdAssdGssdGssdCssdAss dTssTb1ssTb1 |
| 16 | 5br | Tb1ssAb1ssdCssdAssdAssdGssdCssdAssdAssdGssdGssdCssdAssTb1ss Tb1ssTb1 |
| 16 | 5bs | Tb1ssAb1ssdCssdAssdAssdGssdCssdAssdAssdGssdGssdCssAb1ssTb1ss Tb1ssTb1 |
| 16 | 5bt | Tb1ssAb1ssC*b1ssdAssdAssdGssdCssdAssdAssdGssdGssdCssdAssTb1ss Tb1ssTb1 |
| 16 | 5bu | Tb^1ssAb^1ssC*b^1ssAb^1ssdAssdGssdCssdAssdAssdGssdGssdCssdAssTb^1ss Tb^1ssTb^1 |
| 16 | 5bv | Tb1ssAb1ssC*b1ssdAssdAssdGssdCssdAssdAssdGssdGssdCssAb1ss Tb1ssTb1ssTb1 |
| 16 | 5bw | Tb1ssAb1ssC*b1ssAb1ssdAssdGssdCssdAssdAssdGssdGssdCssAb1ss Tb1ssTb1ssTb1 |
| 16 | 5bx | Tb^1ssAb^1ssC*b^1ssAb^1ssdAssdGssdCssdAssdAssdGssdGssdCssdAssTb^1ss Tb^1ssTb^1 |
| 16 | 5by | Tb1ssAb1ssC*b1ssdAssdAssdGssdCssdAssdAssdGssdGssdCssAb1ss Tb1ssTb1ssTb1 |

**Table 32**

| **L** | **Seq ID No.** | **Sequence, 5'-3'** |
|---|---|---|
| 12 | 74m | **Tb¹sGb¹s**dCsdAsdAsdAsdAsdTsdTsdCs**Ab¹sGb¹** |
| 12 | 77m | **Ab¹sTb¹s**dTsdGsdCsdAsdAsdAsdAsdTs**Tb¹sC*b¹** |
| 12 | 78m | **Tb¹sTb¹s**dGsdCsdAsdAsdAsdAsdTsdTs**C*b¹sAb¹** |
| 12 | 74n | **Tb²sGb²sC*b²s**dAsdAsdAsdAsdTsdTsdC*s**Ab²sGb²** |
| 12 | 77n | **Ab²sTb²sTb²s**dGsdCsdAsdAsdAsdAsdTs**Tb²sC*b²** |
| 12 | 78n | **Tb²sTb²sGb²s**dCsdAsdAsdAsdAsdTsdTs**C*b²sAb²** |
| 12 | 74o | **Tb³sGb³s**dCsdAsdAsdAsdAsdTsdTs**C*b³sAb³sGb³** |
| 12 | 77o | **Ab³sTb³s**dTsdGsdCsdAsdAsdAsdAs**Tb³sTb³sC*b³** |
| 12 | 78o | **Tb³sTb³s**dGsdCsdAsdAsdAsdAsdTs**Tb³sC*b³sAb³** |
| 12 | 74p | **Tb¹Gb¹**dCsdAsdAsdAsdAsdTsdTsdCs**Ab¹Gb¹** |
| 12 | 77p | **Ab¹Tb¹**dTsdGsdCsdAsdAsdAsdAsdTs**Tb¹C*b¹** |
| 12 | 78p | **Tb¹Tb¹**dGsdCsdAsdAsdAsdAsdTsdTs**C*b¹Ab¹** |
| 12 | 74q | **Tb⁴Gb⁴C*b⁴**dAsdAsdAsdAsdTsdTsdCs**Ab⁴Gb⁴** |
| 12 | 77q | **Ab⁴Tb⁴Tb⁴**dGsdCsdAsdAsdAsdAsdTs**Tb⁴C*b⁴** |
| 12 | 78q | **Tb⁴Tb⁴Gb⁴**dCsdAsdAsdAsdAsdTsdTs**C*b⁴Ab⁴** |
| 12 | 74r | **Tb⁵Gb⁵**dCsdAsdAsdAsdAsdTsdTs**C*b⁵Ab⁵Gb⁵** |
| 12 | 77r | **Ab⁵Tb⁵**dTsdGsdCsdAsdAsdAsdAs**Tb⁵Tb⁵C*b⁵** |
| 12 | 78r | **Tb⁵Tb⁵**dGsdCsdAsdAsdAsdAsdTs**Tb⁵C*b⁵Ab⁵** |
| 12 | 74s | **Tb¹ssGb¹ss**dCssdAssdAssdAssdAssdTssdTssdCss**Ab¹ssGb¹** |
| 12 | 77s | **Ab¹ssTb¹ss**dTssdGssdCssdAssdAssdAssdAssdTss**Tb¹ssCb¹** |
| 12 | 78s | **Tb¹ssTb¹ss**dGssdCssdAssdAssdAssdAssdTssdTss**C*b¹ssAb¹** |
| 12 | 74t | **Tb⁶ssGb⁶ssC*b⁶ss**dAssdAssdAssdAssdTssdTssdCss**Ab⁶ssGb⁶** |
| 12 | 77t | **Ab⁶ssTb⁶ssTb⁶ss**dGssdCssdAssdAssdAssdAssdTss**Tb⁶ssC*b⁶** |
| 12 | 78t | **Tb⁶ssTb⁶ssGb⁶ss**dCssdAssdAssdAssdAssdTssdTss**C*b⁶ssAb⁶** |
| 12 | 74u | **Tb⁷ssGb⁷ss**dCssdAssdAssdAssdAssdTssdTss**C*b⁷ssAb⁷ssGb⁷** |
| 12 | 77u | **Ab⁷ssTb⁷ss**dTssdGssdCssdAssdAssdAssdAss**Tb⁷ssTb⁷ssC*b⁷** |
| 12 | 78u | **Tb⁷ssTb⁷ss**dGssdCssdAssdAssdAssdAssdTss**Tb⁷ssC*b⁷ssAb⁷** |
| 11 | 84i | **Tb¹s**dTsdGsdCsdAsdAsdAsdAsdTs**Tb¹sC*b¹** |
| 11 | 85i | **Tb¹s**dGsdCsdAsdAsdAsdAsdTsdTs**C*b¹sAb¹** |
| 11 | 86i | **Gb¹s**dCsdAsdAsdAsdAsdTsdTsdCs**Ab¹sGb¹** |
| 11 | 84j | **Tb¹sTb¹s**dGsdCsdAsdAsdAsdAsdTsdTs**C*b¹** |
| 11 | 85j | **Tb¹sGb¹s**dCsdAsdAsdAsdAsdTsdTsdCs**Ab¹** |
| 11 | 86j | **Gb¹sC*b¹s**dAsdAsdAsdAsdTsdTsdCsdAs**Gb¹** |
| 11 | 84k | **Tb¹**dTsdGsdCsdAsdAsdAsdAsdTs**Tb¹C*b¹** |
| 11 | 85k | **Tb¹**dGsdCsdAsdAsdAsdAsdTsdTs**C*b¹Ab¹** |
| 11 | 86k | **Gb¹**dCsdAsdAsdAsdAsdTsdTsdCs**Ab¹Gb¹** |
| 11 | 84l | **Tb¹Tb¹**dGsdCsdAsdAsdAsdAsdTsdTs**C*b¹** |
| 11 | 85l | **Tb¹Gb¹**dCsdAsdAsdAsdAsdTsdTsdCs**Ab¹** |
| 11 | 86l | **Gb¹C*b¹**dAsdAsdAsdAsdTsdTsdCsdAs**Gb¹** |
| 11 | 84m | **Tb¹ss**dTssdGssdCssdAssdAssdAssdAssdTss**Tb¹ssC*b¹** |
| 11 | 85m | **Tb¹ss**dGssdCssdAssdAssdAssdAssdTssdTss**C*b¹ssAb¹** |
| 11 | 86m | **Gb¹ss**dCssdAssdAssdAssdAssdTssdTssdCss**Ab¹ssGb¹** |
| 11 | 84n | **Tb¹ssTb¹ss**dGssdCssdAssdAssdAssdAssdTssdTss**C*b¹** |
| 11 | 85n | **Tb¹ssGb¹ss**dCssdAssdAssdAssdAssdTssdTssdCss**Ab¹** |
| 11 | 86n | **Gb¹ssC*b¹ss**dAssdAssdAssdAssdTssdTssdCssdAss**Gb¹** |
| 10 | 71e | **Tb¹s**dGsdC*sdAsdAsdAsdAsdTsdTs**C*b¹** |
| 10 | 72e | **Gb¹s**dC*sdAsdAsdAsdAsdTsdTsdC*s**Ab¹** |
| 10 | 73e | **C*b¹s**dAsdAsdAsdAsdTsdTsdC*sdAs**Gb¹** |
| 10 | 71f | **Tb¹**dGsdCsdAsdAsdAsdAsdTsdTs**C*b¹** |
| 10 | 72f | **Gb¹**dCsdAsdAsdAsdAsdTsdTsdCs**Ab¹** |
| 10 | 73f | **C*b¹**dAsdAsdAsdAsdTsdTsdCsdAs**Gb¹** |
| 10 | 71g | **Tb¹ss**dGssdCssdAssdAssdAssdAssdTssdTss**C*b¹** |
| 10 | 72g | **Gb¹ss**dCssdAssdAssdAssdAssdTssdTssdCss**Ab¹** |
| 10 | 73g | **C*b¹ss**dAssdAssdAssdAssdTssdTssdCssdAss**Gb¹** |
| 13 | 88m | **C*b¹sAb¹s**dTsdTsdGsdCsdAsdAsdAsdAsdTs**Tb¹sC*b¹** |
| 13 | 89m | **Ab¹sTb¹s**dTsdGsdCsdAsdAsdAsdAsdTsdTs**C*b¹sAb¹** |
| 13 | 90m | **Tb¹sTb¹s**dGsdCsdAsdAsdAsdAsdTsdTsdCs**Ab¹sGb¹** |
| 13 | 88n | **C*b¹sAb¹sTb¹s**dTsdGsdCsdAsdAsdAsdAsdTs**Tb¹sC*b¹** |
| 13 | 89n | **Ab¹sTb¹sTb¹s**dGsdCsdAsdAsdAsdAsdTsdTs**C*b¹sAb¹** |
| 13 | 90n | **Tb¹sTb¹sGb¹s**dCsdAsdAsdAsdAsdTsdTsdCs**Ab¹sGb¹** |
| 13 | 88o | **C*b¹sAb¹s**dTsdTsdGsdCsdAsdAsdAsdAs**Tb¹sTb¹sC*b¹** |
| 13 | 89o | **Ab¹sTb¹s**dTsdGsdCsdAsdAsdAsdAsdTs**Tb¹sC*b¹sAb¹** |
| 13 | 90o | **Tb¹sTb¹s**dGsdCsdAsdAsdAsdAsdTsdTs**C*b¹sAb¹sGb¹** |
| 13 | 88p | **C*b¹Ab¹**dTsdTsdGsdCsdAsdAsdAsdAsdTs**Tb¹C*b¹** |
| 13 | 89p | **Ab¹Tb¹**dTsdGsdCsdAsdAsdAsdAsdTsdTs**C*b¹Ab¹** |
| 13 | 90p | **Tb¹Tb¹**dGsdCsdAsdAsdAsdAsdTsdTsdCs**Ab¹Gb¹** |
| 13 | 88q | **C*b¹Ab¹Tb¹**dTsdGsdCsdAsdAsdAsdAsdTs**Tb¹C*b¹** |
| 13 | 89q | **Ab¹Tb¹Tb¹**dGsdCsdAsdAsdAsdAsdTsdTs**C*b¹Ab¹** |
| 13 | 90q | **Tb¹Tb¹Gb¹**dCsdAsdAsdAsdAsdTsdTsdCs**Ab¹Gb¹** |
| 13 | 88r | **C*b¹Ab¹**dTsdTsdGsdCsdAsdAsdAsdAs**Tb¹Tb¹C*b¹** |
| 13 | 89r | **Ab¹Tb¹**dTsdGsdCsdAsdAsdAsdAsdTs**Tb¹C*b¹Ab¹** |
| 13 | 90r | **Tb¹Tb¹**dGsdCsdAsdAsdAsdAsdTsdTs**C*b¹Ab¹Gb¹** |
| 13 | 88s | **C*b¹ssAb¹ss**dTssdTssdGssdCssdAssdAssdAssdAssdTss**Tb¹ssC*b¹** |
| 13 | 89s | **Ab¹ssTb¹ss**dTssdGssdCssdAssdAssdAssdAssdTssdTss**C*b¹ssAb¹** |
| 13 | 90s | **Tb¹ssTb¹ss**dGssdCssdAssdAssdAssdAssdTssdTssdCss**Ab¹ssGb¹** |
| 13 | 88t | **C*b¹ssAb¹ssTbss**dTssdGssdCssdAssdAssdAssdAssdTss**Tb¹ssC*b¹** |
| 13 | 89t | **Ab¹ssTb¹ssTb¹ss**dGssdCssdAssdAssdAssdAssdTssdTss**C*b¹ssAb¹** |
| 13 | 90t | **Tb¹ssTb¹ssGb¹ss**dCssdAssdAssdAssdAssdTssdTssdCss**Ab¹ssGb¹** |
| 13 | 88u | **C*b¹ssAb¹ss**dTssdTssdGssdCssdAssdAssdAssdAss**Tb¹ssTb¹ssC*b¹** |
| 13 | 89u | **Ab¹ssTb¹ss**dTssdGssdCssdAssdAssdAssdAssdTss**Tb¹ssC*b¹ssAb¹** |
| 13 | 90u | **Tb¹ssTb¹ss**dGssdCssdAssdAssdAssdAssdTssdTss**C*b¹ssAb¹ssGb¹** |
| 14 | 91q | **Ab¹sC*b¹s**dAsdTsdTsdGsdCsdAsdAsdAsdAsdTs**Tb¹sC*b¹** |
| 14 | 92q | **C*b¹sAb¹s**dTsdTsdGsdC*sdAsdAsdAsdAsdTsdTs**C*b¹sAb¹** |
| 14 | 93q | **Ab¹sTb¹s**dTsdGsdCsdAsdAsdAsdAsdTsdTsdCs**Ab¹sGb¹** |
| 14 | 91r | **Ab¹sC*b¹sAb¹s**dTsdTsdGsdCsdAsdAsdAsdAsdTs**Tb¹sC*b¹** |
| 14 | 92r | **C*b¹sAb¹sTb¹s**dTsdGsdCsdAsdAsdAsdAsdTsdTs**C*b¹sAb¹** |
| 14 | 93r | **Ab¹sTb¹sTb¹s**dGsdCsdAsdAsdAsdAsdTsdTsdCs**Ab¹sGb¹** |
| 14 | 91s | **Ab¹sC*b¹s**dAsdTsdTsdGsdCsdAsdAsdAsdAs**Tb¹sTb¹sC*b¹** |
| 14 | 92s | **C*b¹sAb¹s**dTsdTsdGsdCsdAsdAsdAsdAsdTs**Tb¹sC*b¹sAb¹** |
| 14 | 93s | **Ab¹sTb¹s**dTsdGsdCsdAsdAsdAsdAsdTsdTs**C*b¹sAb¹sGb¹** |
| 14 | 91t | **Ab¹sC*b¹sAb¹s**dTsdTsdGsdCsdAsdAsdAsdAs**Tb¹sTb¹sC*b¹** |
| 14 | 92t | **C*b¹sAb¹sTb¹s**dTsdGsdCsdAsdAsdAsdAsdTs**Tb¹sC*b¹sAb¹** |
| 14 | 93t | **Ab¹sTb¹sTb¹s**dGsdCsdAsdAsdAsdAsdTsdTs**C*b¹sAb¹sGb¹** |
| 14 | 91u | **Ab¹C*b¹**dAsdTsdTsdGsdCsdAsdAsdAsdAsdTs**Tb¹C*b¹** |
| 14 | 92u | **C*b¹Ab¹**dTsdTsdGsdCsdAsdAsdAsdAsdTsdTs**C*b¹Ab¹** |
| 14 | 93u | **Ab¹Tb¹**dTsdGsdCsdAsdAsdAsdAsdTsdTsdCs**Ab¹Gb¹** |
| 14 | 91v | **Ab¹C*b¹Ab¹**dTsdTsdGsdCsdAsdAsdAsdAsdTs**Tb¹C*b¹** |
| 14 | 92v | **C*b¹Ab¹Tb¹**dTsdGsdCsdAsdAsdAsdAsdTsdTs**C*b¹Ab¹** |
| 14 | 93v | **Ab¹Tb¹Tb¹**dGsdCsdAsdAsdAsdAsdTsdTsdCs**Ab¹Gb¹** |
| 14 | 91w | **Ab¹C*b¹**dAsdTsdTsdGsdCsdAsdAsdAsdAs**Tb¹Tb¹C*b¹** |
| 14 | 92w | **C*b¹Ab¹**dTsdTsdGsdCsdAsdAsdAsdAsdTs**Tb¹C*b¹Ab¹** |
| 14 | 93w | **Ab¹Tb¹**dTsdGsdCsdAsdAsdAsdAsdTsdTs**C*b¹Ab¹Gb¹** |
| 14 | 91x | **Ab¹C*b¹Ab¹**dTsdTsdGsdCsdAsdAsdAsdAs**Tb¹Tb¹C*b¹** |
| 14 | 92x | **C*b¹Ab¹Tb¹**dTsdGsdCsdAsdAsdAsdAsdTs**Tb¹C*b¹Ab¹** |
| 14 | 93x | **Ab¹Tb¹Tb¹**dGsdCsdAsdAsdAsdAsdTsdTs**C*b¹Ab¹Gb¹** |
| 14 | 91y | **Ab¹ssC*b¹ss**dAssdTssdTssdGssdCssdAssdAssdAssdAssdTss**Tb¹ssC*b¹** |
| 14 | 92y | **C*b¹ssAb¹ss**dTssdTssdGssdCssdAssdAssdAssdAssdTssdTss**C*b¹ssAb¹** |
| 14 | 93y | **Ab¹ssTb¹ss**dTssdGssdCssdAssdAssdAssdAssdTssdTssdCss**Ab¹ssGb¹** |
| 14 | 91z | **Ab¹ssC*b¹ssAb¹ss**dTssdTssdGssdCssdAssdAssdAssdAssdTss**Tb¹ssC*b¹** |
| 14 | 92z | **C*b¹ssAb¹ssTb¹ss**dTssdGssdCssdAssdAssdAssdAssdTssdTss**C*b¹ssAb¹** |
| 14 | 93z | **Ab¹ssTb¹ssTb¹ss**dGssdCssdAssdAssdAssdAssdTssdTssdCss**Ab¹ssGb¹** |
| 14 | 91aa | **Ab¹ssC*b¹ss**dAssdTssdTssdGssdCssdAssdAssdAssdAss**Tb¹ssTb¹ssC*b¹** |
| 14 | 92aa | **C*b¹ssAb¹ss**dTssdTssdGssdCssdAssdAssdAssdAssdTss**Tb¹ssC*b¹ssAb¹** |
| 14 | 93aa | **Ab¹ssTb¹ss**dTssdGssdCssdAssdAssdAssdAssdTssdTss**C*b¹ssAb¹ssGb¹** |
| 14 | 91ab | **Ab¹ssC*b¹ssAb¹ss**dTssdTssdGssdCssdAssdAssdAssdAss**Tb¹ssTb¹ssC*b¹** |
| 14 | 92ab | **C*b¹ssAb¹ssTb¹ss**dTssdGssdCssdAssdAssdAssdAssdTss**TbssCbssAb¹** |
| 14 | 93ab | **Ab¹ssTb¹ssTb¹ss**dGssdCssdAssdAssdAssdAssdTssdTss**C*b¹ssAb¹ssGb¹** |
| 15 | 79q | **Ab¹sC*b¹s**dAsdTsdTsdGsdCsdAsdAsdAsdAsdTsdTs**C*b¹sAb¹** |
| 15 | 80q | **C*b¹sAb¹s**dTsdTsdGsdCsdAsdAsdAsdAsdTsdTsdCs**Ab¹sGb¹** |
| 15 | 79r | **Ab¹sC*b¹sAb¹s**dTsdTsdGsdCsdAsdAsdAsdAsdTsdTs**C*b¹sAb¹** |
| 15 | 80r | **C*b¹sAb¹sTb¹s**dTsdGsdCsdAsdAsdAsdAsdTsdTsdC*s**Ab¹sGb¹** |
| 15 | 79s | **Ab¹sC*b¹sAb¹s**dTsdTsdGsdCsdAsdAsdAsdAsdTs**Tb¹sC*b¹sAb¹** |
| 15 | 80s | **C*b¹sAb¹sTb¹s**dTsdGsdCsdAsdAsdAsdAsdTsdTs**C*b¹sAb¹sGb¹** |
| 15 | 79t | **Ab¹sC*b¹s**dAsdTsdTsdGsdCsdAsdAsdAsdAsdTs**Tb¹sC*b¹sAb¹** |
| 15 | 80t | **C*b¹sAb¹s**dTsdTsdGsdCsdAsdAsdAsdAsdTsdTs**C*b¹sAb¹sGb¹** |
| 15 | 79u | **Ab¹C*b¹**dAsdTsdTsdGsdCsdAsdAsdAsdAsdTsdTs**C*b¹Ab¹** |
| 15 | 80u | **C*b¹Ab¹**dTsdTsdGsdCsdAsdAsdAsdAsdTsdTsdCs**Ab¹Gb¹** |
| 15 | 79v | **Ab¹C*b¹Ab¹**dTsdTsdGsdCsdAsdAsdAsdAsdTsdTs**C*b¹Ab¹** |
| 15 | 80v | **C*b¹Ab¹Tb¹**dTsdGsdCsdAsdAsdAsdAsdTsdTsdCs**Ab¹Gb¹** |
| 15 | 79w | **Ab¹C*b¹Ab¹**dTsdTsdGsdCsdAsdAsdAsdAsdTs**Tb¹C*b¹Ab¹** |
| 15 | 80w | **C*b¹Ab¹Tb¹**dTsdGsdCsdAsdAsdAsdAsdTsdTs**C*b¹Ab¹Gb¹** |
| 15 | 79x | **Ab¹C*b¹**dAsdTsdTsdGsdCsdAsdAsdAsdAsdTs**Tb¹C*b¹Ab¹** |
| 15 | 80x | **C*b¹Ab¹**dTsdTsdGsdCsdAsdAsdAsdAsdTsdTs**C*b¹Ab¹Gb¹** |
| 15 | 79y | Ab1ssC*b1ssdAssdTssdTssdGssdCssdAssdAssdAssdAssdTssdTss C*b1ssAb1 |
| 15 | 80y | C*b¹ssAb¹ssdTssdTssdGssdCssdAssdAssdAssdAssdTssdTssdCss Ab¹ssGb¹ |
| 15 | 79z | Ab¹ssC*b¹ssAb¹ssdTssdTssdGssdCssdAssdAssdAssdAssdTssdTss C*b¹ssAb¹ |
| 15 | 80z | C*b¹ssAb¹ssTb¹ssdTssdGssdCssdAssdAssdAssdAssdTssdTssdCss Ab¹ssGb¹ |
| 15 | 79aa | Ab^1ssC*b^1ssAb^1ssdTssdTssdGssdCssdAssdAssdAssdAssdTssTb^1ss C*b^1ssAb^1 |
| 15 | 80aa | C*b¹ssAb¹ssTb¹ssdTssdGssdCssdAssdAssdAssdAssdTssdTssC*b¹ss Ab¹ssGb¹ |
| 15 | 79ab | Ab^1ssC*b^1ssdAssdTssdTssdGssdCssdAssdAssdAssdAssdTssTb^1ss C*b^1ssAb^1 |
| 15 | 80ab | C*b¹ssAb¹ssdTssdTssdGssdCssdAssdAssdAssdAssdTssdTssC*b¹ss Ab¹ssGb¹ |
| 16 | 6as | **Ab¹sC*b¹s**dAsdTsdTsdGsdCsdAsdAsdAsdAsdTsdTsdCs**Ab¹sGb¹** |
| 16 | 6at | **Ab¹sC*b¹sAb¹s**dTsdTsdGsdCsdAsdAsdAsdAsdTsdTsdCs**Ab¹sGb¹** |
| 16 | 6au | **Ab¹sC*b¹sAb¹sTb¹s**dTsdGsdCsdAsdAsdAsdAsdTsdTsdCs**Ab¹sGb¹** |
| 16 | 6av | **Ab¹sC*b¹sAb¹s**dTsdTsdGsdCsdAsdAsdAsdAsdTsdTs**C*b¹sAb¹sGb¹** |
| 16 | 6aw | **Ab¹sC*b¹sAb¹sTb¹s**dTsdGsdCsdAsdAsdAsdAsdTsdTs**C*b¹sAb¹sGb¹** |
| 16 | 6ax | **Ab¹sC*b¹s**dAsdTsdTsdGsdCsdAsdAsdAsdAsdTsdTs**C*b¹sAb¹sGb¹** |
| 16 | 6ay | **Ab¹sC*b¹s**dAsdTsdTsdGsdCsdAsdAsdAsdAsdTs**Tb¹sC*b¹sAb¹sGb¹** |
| 16 | 6az | **Ab¹sC*b¹sAb¹s**dTsdTsdGsdCsdAsdAsdAsdAsdTs**Tb¹sC*b¹sAb¹sGb¹** |
| 16 | 6ba | **Ab¹sC*b¹sAb¹sTb¹s**dTsdGsdCsdAsdAsdAsdAsdTs**Tb¹sC*b¹sAb¹sGb¹** |
| 16 | 6bb | **Ab¹sC*b¹sAb¹s**dTsdTsdGsdCsdAsdAsdAsdAs**Tb¹sTb¹sC*b¹sAb¹sGb¹** |
| 16 | 6bc | **Ab¹sC*b¹sAb¹sTb¹sTb¹s**dGsdCsdAsdAsdAsdAsdTsdTs**C*b¹sAb¹sGb¹** |
| 16 | 6bd | **Ab¹C*b¹**dAsdTsdTsdGsdCsdAsdAsdAsdAsdTsdTsdCs**Ab¹Gb¹** |
| 16 | 6be | **Ab¹C*b¹Ab¹**dTsdTsdGsdCsdAsdAsdAsdAsdTsdTsdCs**Ab¹Gb¹** |
| 16 | 6bf | **Ab¹C*b¹Ab¹Tb¹**dTsdGsdCsdAsdAsdAsdAsdTsdTsdCs**Ab¹Gb¹** |
| 16 | 6bg | **Ab¹C*b¹Ab¹**dTsdTsdGsdCsdAsdAsdAsdAsdTsdTs**C*b¹Ab¹Gb¹** |
| 16 | 6bh | **Ab¹C*b¹Ab¹Tb¹**dTsdGsdCsdAsdAsdAsdAsdTsdTs**C*b¹Ab¹Gb¹** |
| 16 | 6bi | **Ab¹C*b¹**dAsdTsdTsdGsdCsdAsdAsdAsdAsdTsdTs**C*b¹Ab¹Gb¹** |
| 16 | 6bj | **Ab¹C*b¹**dAsdTsdTsdGsdCsdAsdAsdAsdAsdTs**Tb¹C*b¹Ab¹Gb¹** |
| 16 | 6bk | **Ab¹C*b¹Ab¹**dTsdTsdGsdCsdAsdAsdAsdAsdTs**Tb¹C*b¹Ab¹Gb¹** |
| 16 | 6bl | **Ab¹C*b¹Ab¹Tb¹**dTsdGsdCsdAsdAsdAsdAsdTs**Tb¹C*b¹Ab¹Gb¹** |
| 16 | 6bm | **Ab¹C*b¹Ab¹**dTsdTsdGsdCsdAsdAsdAsdAs**Tb¹Tb¹C*b¹Ab¹Gb¹** |
| 16 | 6bn | **Ab¹C*b¹Ab¹Tb¹Tb¹**dGsdCsdAsdAsdAsdAsdTsdTs**C*b¹Ab¹Gb¹** |
| 16 | 6bo | Ab1ssC*b1ssdAssdTssdTssdGssdCssdAssdAssdAssdAssdTssdTssdCss Ab1ssGb1 |
| 16 | 6bp | Ab1ssC*b1ssAb1ssdTssdTssdGssdCssdAssdAssdAssdAssdTssdTssdCss Ab1ssGb1 |
| 16 | 6bq | Ab^1ssC*b^1ssAb^1ssTb^1ssdTssdGssdCssdAssdAssdAssdAssdTssdTssdCss Ab^1ssGb^1 |
| 16 | 6br | Ab¹ssC*b¹ssAb¹ssdTssdTssdGssdCssdAssdAssdAssdAssdTssdTss C*b¹ssAb¹ssGb¹ |
| 16 | 6bs | Ab¹ssC*b¹ssAb¹ssTb¹ssdTssdGssdCssdAssdAssdAssdAssdTssdTss C*b¹ssAb¹ssGb¹ |
| 16 | 6bt | Ab1ssC*b1ssdAssdTssdTssdGssdCssdAssdAssdAssdAssdTssdTss C*b1ssAb1ssGb1 |
| 16 | 6bu | Ab1ssC*b1ssdAssdTssdTssdGssdCssdAssdAssdAssdAssdTssTb1ss Cb1ssAb1ssGb1 |
| 16 | 6bv | Ab¹ssC*b¹ssAb¹ssdTssdTssdGssdCssdAssdAssdAssdAssdTssTb¹ss C*b¹ssAb¹ssGb¹ |
| 16 | 6bw | Ab1ssC*b1ssAb1ssTb1ssdTssdGssdCssdAssdAssdAssdAssdTssTb1ss C*b1ssAb1ssGb1 |
| 16 | 6bx | Ab^1ssC*b^1ssAb^1ssdTssdTssdGssdCssdAssdAssdAssdAssTb^1ssTb^1ss C*b^1ssAb^1ssGb^1 |
| 16 | 6by | Ab^1ssC*b^1ssAb^1ssTb^1ssTb^1ssdGssdCssdAssdAssdAssdAssdTssdTss C*b^1ssAb^1ssGb^1 |

Also especially preferred are the gapmer antisense-oligonucleotides of **Table 33.**

**Table 33**

| **L** | **Seq ID No.** | **Sequence, 5'-3'** |
|---|---|---|
| 12 | 16m | **Gb¹sGb¹s**dTsdTsdAsdGsdGsdGsdCsdTs**Gb¹sAb¹** |
| 12 | 16o | **Gb³sGb³s**dTsdTsdAsdGsdGsdGsdCsdTsdGs**Ab³** |
| 12 | 16p | **Gb¹Gb¹**dTsdTsdAsdGsdGsdGsdCsdTs**Gb¹Ab¹** |
| 12 | 16q | **Gb⁴**dGsdTsdTsdAsdGsdGsdGsdCsdTs**Gb⁴Ab⁴** |
| 12 | 16r | **Gb⁵Gb⁵**dTsdTsdAsdGsdGsdGsdCsdTsdGs**Ab⁵** |
| 12 | 16s | **Gb¹ssGb¹ss**dTssdTssdAssdGssdGssdGssdCssdTss**Gb¹ssAb¹** |
| 12 | 16t | **Gb⁶ss**dGssdTssdTssdAssdGssdGssdGssdCssdTss**Gb⁶ssAb⁶** |
| 12 | 16u | **Gb⁷ssGb⁷ss**dTssdTssdAssdGssdGssdGssdCssdTssdGss**Ab⁷** |
| 16 | 4as | **Ab¹sGb¹s**dGsdTsdTsdAsdGsdGsdGsdCsdTsdGsdAs**Ab¹sTb¹sTb¹** |
| 16 | 4at | **Ab¹sGb¹s**dGsdTsdTsdAsdGsdGsdGsdCsdTsdGsdAsdAs**Tb¹sTb¹** |
| 16 | 4au | **Ab¹sGb¹sGb¹s**dTsdTsdAsdGsdGsdGsdCsdTsdGsdAsdAs**Tb¹sTb¹** |
| 16 | 4av | **Ab¹sGb¹sGb¹sTb¹s**dTsdAsdGsdGsdGsdCsdTsdGsdAsdAs**Tb¹sTb¹** |
| 16 | 4aw | **Ab¹sGb¹s**dGsdTsdTsdAsdGsdGsdGsdCsdTsdGs**Ab¹sAb¹sTb¹sTb¹** |
| 16 | 4ax | **Ab¹sGb¹sGb¹s**dTsdTsdAsdGsdGsdGsdCsdTsdGsdAs**Ab¹sTb¹sTb¹** |
| 16 | 4ay | **Ab¹sGb¹sGb¹sTb¹s**dTsdAsdGsdGsdGsdCsdTsdGs**Ab¹sAb¹sTb¹sTb¹** |
| 16 | 4az | **Ab¹sGb¹sGb¹sTb¹sTb¹s**dAsdGsdGsdGsdCsdTsdGsdAs**Ab¹sTb¹sTb¹** |
| 16 | 4ba | **Ab¹sGb¹sGb¹s**dTsdTsdAsdGsdGsdGsdCsdTs**Gb¹sAb¹sAb¹sTb¹sTb¹** |
| 16 | 4bb | **Ab¹sGb¹sGb¹s**dTsdTsdAsdGsdGsdGsdCsdTsdGs**Ab¹sAb¹sTb¹sTb¹** |
| 16 | 4bc | **Ab¹sGb¹sGb¹sTb¹s**dTsdAsdGsdGsdGsdCsdTsdGsdAs**Ab¹sTb¹sTb¹** |
| 16 | 4bd | **Ab¹Gb¹**dGsdTsdTsdAsdGsdGsdGsdCsdTsdGsdAs**Ab¹Tb¹Tb¹** |
| 16 | 4be | **Ab¹Gb¹**dGsdTsdTsdAsdGsdGsdGsdCsdTsdGsdAsdAs**Tb¹Tb¹** |
| 16 | 4bf | **Ab¹Gb¹Gb¹**dTsdTsdAsdGsdGsdGsdCsdTsdGsdAsdAs**Tb¹Tb¹** |
| 16 | 4bg | **Ab¹Gb¹Gb¹Tb¹**dTsdAsdGsdGsdGsdCsdTsdGsdAsdAs**Tb¹Tb¹** |
| 16 | 4bh | **Ab¹Gb¹**dGsdTsdTsdAsdGsdGsdGsdCsdTsdGs**Ab¹Ab¹Tb¹Tb¹** |
| 16 | 4bi | **Ab¹Gb¹Gb¹**dTsdTsdAsdGsdGsdGsdCsdTsdGsdAs**Ab¹Tb¹Tb¹** |
| 16 | 4bj | **Ab¹Gb¹Gb¹Tb¹**dTsdAsdGsdGsdGsdCsdTsdGs**Ab¹Ab¹Tb¹Tb¹** |
| 16 | 4bk | **Ab¹Gb¹Gb¹Tb¹Tb¹**dAsdGsdGsdGsdCsdTsdGsdAs**Ab¹Tb¹Tb¹** |
| 16 | 4bl | **Ab¹Gb¹Gb¹**dTsdTsdAsdGsdGsdGsdCsdTs**Gb¹Ab¹Ab¹Tb¹Tb¹** |
| 16 | 4bm | **Ab¹Gb¹Gb¹**dTsdTsdAsdGsdGsdGsdCsdTsdGs**Ab¹Ab¹Tb¹Tb¹** |
| 16 | 4bn | **Ab¹Gb¹Gb¹Tb¹**dTsdAsdGsdGsdGsdCsdTsdGsdAs**Ab¹Tb¹Tb¹** |
| 16 | 4bo | Ab1ssGb1ssdGssdTssdTssdAssdGssdGssdGssdCssdTssdGssdAss Ab1ssTb1ssTb1 |
| 16 | 4bp | Ab1ssGb1ssdGssdTssdTssdAssdGssdGssdGssdCssdTssdGssdAss dAssTb1ssTb1 |
| 16 | 4bq | Ab¹ssGb¹ssGb¹ssdTssdTssdAssdGssdGssdGssdCssdTssdGssdAss dAssTb¹ssTb¹ |
| 16 | 4br | Ab¹ssGb¹ssGb¹ssTb¹ssdTssdAssdGssdGssdGssdCssdTssdGssdAss dAssTb¹ssTb¹ |
| 16 | 4bs | Ab1ssGb1ssdGssdTssdTssdAssdGssdGssdGssdCssdTssdGssAb1ss Ab1ssTb1ssTb1 |
| 16 | 4bt | Ab¹ssGb¹ssGb¹ssdTssdTssdAssdGssdGssdGssdCssdTssdGssdAss Ab¹ssTb¹ssTb¹ |
| 16 | 4bu | Ab¹ssGb¹ssGb¹ssTb¹ssdTssdAssdGssdGssdGssdCssdTssdGssAb¹ss Ab¹ssTb¹ssTb¹ |
| 16 | 4bv | Ab¹ssGb¹ssGb¹ssTb¹ssTb¹ssdAssdGssdGssdGssdCssdTssdGssdAss Ab¹ssTb¹ssTb¹ |
| 16 | 4bw | Ab1ssGb1ssGb1ssdTssdTssdAssdGssdGssdGssdCssdTssGb1ssAb1ss Ab1ssTb1ssTb1 |
| 16 | 4bx | Ab1ssGb1ssGb1ssdTssdTssdAssdGssdGssdGssdCssdTssdGssAb1ss Ab1ssTb1ssTb1 |
| 16 | 4by | Ab¹ssGb¹ssGb¹ssTb¹ssdTssdAssdGssdGssdGssdCssdTssdGssdAss Ab¹ssTb¹ssTb¹ |
| 12 | 46m | **C*b¹sAb¹s**dAsdGsdCsdAsdAsdGsdGsdCs**Ab¹sTb¹** |
| 12 | 46n | **C*b²s**dAsdAsdGsdCsdAsdAsdGsdGsdCs**Ab²sTb²** |
| 12 | 46o | **C*b³sAb³s**dAsdGsdCsdAsdAsdGsdGsdCsdAs**Tb³** |
| 12 | 46p | **C*b¹Ab¹**dAsdGsdCsdAsdAsdGsdGsdCs**Ab¹Tb¹** |
| 12 | 46q | **C*b⁴**dAsdAsdGsdCsdAsdAsdGsdGsdCs**Ab⁴Tb⁴** |
| 12 | 46r | **C*b⁵Ab⁵**dAsdGsdCsdAsdAsdGsdGsdCsdAs**Tb⁵** |
| 12 | 46s | **C*b¹ssAb¹ss**dAssdGssdCssdAssdAssdGssdGssdCss**Ab¹ssTb¹** |
| 12 | 46t | **C*b⁶ss**dAssdAssdGssdCssdAssdAssdGssdGssdCss**Ab⁶ssTb⁶** |
| 12 | 46u | **C*b⁷ssAb⁷ss**dAssdGssdCssdAssdAssdGssdGssdCssdAss**Tb⁷** |
| 16 | 5as | **Tb¹sAb¹s**dCsdAsdAsdGsdCsdAsdAsdGsdGsdCsdAsdTs**Tb¹sTb¹** |
| 16 | 5at | **Tb¹sAb¹sC*b¹s**dAsdAsdGsdCsdAsdAsdGsdGsdCsdAsdTs**Tb¹sTb¹** |
| 16 | 5au | **Tb¹sAb¹sC*b¹sAb¹s**dAsdGsdCsdAsdAsdGsdGsdCsdAsdTs**Tb¹sTb¹** |
| 16 | 5av | **Tb¹sAb¹s**dCsdAsdAsdGsdCsdAsdAsdGsdGsdCsdAs**Tb¹sTb¹sTb¹** |
| 16 | 5aw | **Tb¹sAb¹s**dCsdAsdAsdGsdCsdAsdAsdGsdGsdCs**Ab¹sTb¹sTb¹sTb¹** |
| 16 | 5ax | **Tb¹sAb¹sC*b¹s**dAsdAsdGsdCsdAsdAsdGsdGsdCsdAs**Tb¹sTb¹sTb¹** |
| 16 | 5ay | **Tb¹sAb¹sC*b¹sAb¹s**dAsdGsdCsdAsdAsdGsdGsdCsdAs**Tb¹sTb¹sTb¹** |
| 16 | 5az | **Tb¹sAb¹sC*b¹s**dAsdAsdGsdCsdAsdAsdGsdGsdCs**Ab¹sTb¹sTb¹sTb¹** |
| 16 | 5ba | **Tb¹sAb¹sC*b¹sAb¹s**dAsdGsdCsdAsdAsdGsdGsdCs**Ab¹sTb¹sTb¹sTb¹** |
| 16 | 5bb | **Tb¹sAb¹sC*b¹sAb¹s**dAsdGsdCsdAsdAsdGsdGsdCsdAs**Tb¹sTb¹sTb¹** |
| 16 | 5bc | **Tb¹sAb¹sC*b¹s**dAsdAsdGsdCsdAsdAsdGsdGsdCs**Ab¹sTb¹sTb¹sTb¹** |
| 16 | 5bd | **Tb¹Ab¹**dCsdAsdAsdGsdCsdAsdAsdGsdGsdCsdAsdTs**Tb¹Tb¹** |
| 16 | 5be | **Tb¹Ab¹C*b¹**dAsdAsdGsdCsdAsdAsdGsdGsdCsdAsdTs**Tb¹Tb¹** |
| 16 | 5bf | **Tb¹Ab¹C*b¹Ab¹**dAsdGsdCsdAsdAsdGsdGsdCsdAsdTs**Tb¹Tb¹** |
| 16 | 5bg | **Tb¹Ab¹**dCsdAsdAsdGsdCsdAsdAsdGsdGsdCsdAs**Tb¹Tb¹Tb¹** |
| 16 | 5bh | **Tb¹Ab¹**dCsdAsdAsdGsdCsdAsdAsdGsdGsdCs**Ab¹Tb¹Tb¹Tb¹** |
| 16 | 5bi | **Tb¹Ab¹C*b¹**dAsdAsdGsdCsdAsdAsdGsdGsdCsdAs**Tb¹Tb¹Tb¹** |
| 16 | 5bj | **Tb¹Ab¹C*b¹Ab¹**dAsdGsdCsdAsdAsdGsdGsdCsdAs**Tb¹Tb¹Tb¹** |
| 16 | 5bk | **Tb¹Ab¹C*b¹**dAsdAsdGsdCsdAsdAsdGsdGsdCs**Ab¹Tb¹Tb¹Tb¹** |
| 16 | 5bl | **Tb¹Ab¹C*b¹Ab¹**dAsdGsdCsdAsdAsdGsdGsdCs**Ab¹Tb¹Tb¹Tb¹** |
| 16 | 5bm | **Tb¹Ab¹C*b¹Ab¹**dAsdGsdCsdAsdAsdGsdGsdCsdAs**Tb¹Tb¹Tb¹** |
| 16 | 5bn | **Tb¹Ab¹C*b¹**dAsdAsdGsdCsdAsdAsdGsdGsdCs**Ab¹Tb¹Tb¹Tb¹** |
| 16 | 5bo | **Tb^1ssAb^1ss**dCssdAssdAssdGssdCssdAssdAssdGssdGssdCssdAssdTss **Tb^1ssTb^1** |
| 16 | 5bp | Tb1ssAb1ssC*b1ssdAssdAssdGssdCssdAssdAssdGssdGssdCssdAssdTss Tb1ssTb1 |
| 16 | 5bq | Tb1ssAb1ssC*b1ssAb1ssdAssdGssdCssdAssdAssdGssdGssdCssdAss dTssTb1ssTb1 |
| 16 | 5br | Tb1ssAb1ssdCssdAssdAssdGssdCssdAssdAssdGssdGssdCssdAssTb1ss Tb1ssTb1 |
| 16 | 5bs | Tb1ssAb1ssdCssdAssdAssdGssdCssdAssdAssdGssdGssdCssAb1ssTb1ss Tb1ssTb1 |
| 16 | 5bt | Tb1ssAb1ssC*b1ssdAssdAssdGssdCssdAssdAssdGssdGssdCssdAss Tb1ssTb1ssTb1 |
| 16 | 5bu | Tb1ssAb1ssC*b1ssAb1ssdAssdGssdCssdAssdAssdGssdGssdCssdAss Tb1ssTb1ssTb1 |
| 16 | 5bv | Tb^1ssAb^1ssC*b^1ssdAssdAssdGssdCssdAssdAssdGssdGssdCssAb^1ss Tb^1ssTb^1ssTb^1 |
| 16 | 5bw | Tb1ssAb1ssC*b1ssAb1ssdAssdGssdCssdAssdAssdGssdGssdCssAb1ss Tb1ssTb1ssTb1 |
| 16 | 5bx | Tb1ssAb1ssC*b1ssAb1ssdAssdGssdCssdAssdAssdGssdGssdCssdAss |
| | | Tb¹ssTb¹ssTb¹ |
| 16 | 5by | Tb¹ssAb¹ssC*b¹ssdAssdAssdGssdCssdAssdAssdGssdGssdCssAb¹ss Tb¹ssTb¹ssTb¹ |
| 12 | 74m | **Tb¹sGb¹s**dCsdAsdAsdAsdAsdTsdTsdCs**Ab¹sGb¹** |
| 12 | 74n | **Tb²sGb²sC*b²s**dAsdAsdAsdAsdTsdTsdC*s**Ab²sGb²** |
| 12 | 74o | **Tb³sGb³s**dCsdAsdAsdAsdAsdTsdTs**C*b³sAb³sGb³** |
| 12 | 74p | **Tb¹Gb¹**dCsdAsdAsdAsdAsdTsdTsdCs**Ab¹Gb¹** |
| 12 | 74q | **Tb⁴Gb⁴C*b⁴**dAsdAsdAsdAsdTsdTsdCs**Ab⁴Gb⁴** |
| 12 | 74r | **Tb⁵Gb⁵**dCsdAsdAsdAsdAsdTsdTs**C*b⁵Ab⁵Gb⁵** |
| 12 | 74s | **Tb¹ssGb¹ss**dCssdAssdAssdAssdAssdTssdTssdCss**Ab¹ssGb¹** |
| 12 | 74t | **Tb⁶ssGb⁶ssC*b⁶ss**dAssdAssdAssdAssdTssdTssdCss**Ab⁶ssGb⁶** |
| 12 | 74u | **Tb⁷ssGb⁷ss**dCssdAssdAssdAssdAssdTssdTss**C*b⁷ssAb⁷ssGb⁷** |
| 16 | 6as | **Ab¹sC*b¹s**dAsdTsdTsdGsdCsdAsdAsdAsdAsdTsdTsdCs**Ab¹sGb¹** |
| 16 | 6at | **Ab¹sC*b¹sAb¹s**dTsdTsdGsdCsdAsdAsdAsdAsdTsdTsdCs**Ab¹sGb¹** |
| 16 | 6au | **Ab¹sC*b¹sAb¹sTb¹s**dTsdGsdCsdAsdAsdAsdAsdTsdTsdCs**Ab¹sGb¹** |
| 16 | 6av | **Ab¹sC*b¹sAb¹s**dTsdTsdGsdCsdAsdAsdAsdAsdTsdTs**C*b¹sAb¹sGb¹** |
| 16 | 6aw | **Ab¹sC*b¹sAb¹sTb¹s**dTsdGsdCsdAsdAsdAsdAsdTsdTs**C*b¹sAb¹sGb¹** |
| 16 | 6ax | **Ab¹sC*b¹s**dAsdTsdTsdGsdCsdAsdAsdAsdAsdTsdTs**C*b¹sAb¹sGb¹** |
| 16 | 6ay | **Ab¹sC*b¹s**dAsdTsdTsdGsdCsdAsdAsdAsdAsdTs**Tb¹sC*b¹sAb¹sGb¹** |
| 16 | 6az | **Ab¹sC*b¹sAb¹s**dTsdTsdGsdCsdAsdAsdAsdAsdTs**Tb¹sC*b¹sAb¹sGb¹** |
| 16 | 6ba | **Ab¹sC*b¹sAb¹sTb¹s**dTsdGsdCsdAsdAsdAsdAsdTs**Tb¹sC*b¹sAb¹sGb¹** |
| 16 | 6bb | **Ab¹sC*b¹sAb¹s**dTsdTsdGsdCsdAsdAsdAsdAs**Tb¹sTb¹sC*b¹sAb¹sGb¹** |
| 16 | 6bc | **Ab¹sC*b¹sAb¹sTb¹sTb¹s**dGsdCsdAsdAsdAsdAsdTsdTs**C*b¹sAb¹sGb¹** |
| 16 | 6bd | **Ab¹C*b¹**dAsdTsdTsdGsdCsdAsdAsdAsdAsdTsdTsdCs**Ab¹Gb¹** |
| 16 | 6be | **Ab¹C*b¹Ab¹**dTsdTsdGsdCsdAsdAsdAsdAsdTsdTsdCs**Ab¹Gb¹** |
| 16 | 6bf | **Ab¹C*b¹Ab¹Tb¹**dTsdGsdCsdAsdAsdAsdAsdTsdTsdCs**Ab¹Gb¹** |
| 16 | 6bg | **Ab¹C*b¹Ab¹**dTsdTsdGsdCsdAsdAsdAsdAsdTsdTs**C*b¹Ab¹Gb¹** |
| 16 | 6bh | **Ab¹C*b¹Ab¹Tb¹**dTsdGsdCsdAsdAsdAsdAsdTsdTs**C*b¹Ab¹Gb¹** |
| 16 | 6bi | **Ab¹C*b¹**dAsdTsdTsdGsdCsdAsdAsdAsdAsdTsdTs**C*b¹Ab¹Gb¹** |
| 16 | 6bj | **Ab¹C*b¹**dAsdTsdTsdGsdCsdAsdAsdAsdAsdTs**Tb¹C*b¹Ab¹Gb¹** |
| 16 | 6bk | **Ab¹C*b¹Ab¹**dTsdTsdGsdCsdAsdAsdAsdAsdTs**Tb¹C*b¹Ab¹Gb¹** |
| 16 | 6bl | **Ab¹C*b¹Ab¹Tb¹**dTsdGsdCsdAsdAsdAsdAsdTs**Tb¹C*b¹Ab¹Gb¹** |
| 16 | 6bm | **Ab¹C*b¹Ab¹**dTsdTsdGsdCsdAsdAsdAsdAs**Tb¹Tb¹C*b¹Ab¹Gb¹** |
| 16 | 6bn | **Ab¹C*b¹Ab¹Tb¹Tb¹**dGsdCsdAsdAsdAsdAsdTsdTs**C*b¹Ab¹Gb¹** |
| 16 | 6bo | Ab¹ssC*b¹ssdAssdTssdTssdGssdCssdAssdAssdAssdAssdTssdTssdCss Ab¹ssGb¹ |
| 16 | 6bp | Ab¹ssC*b¹ssAb¹ssdTssdTssdGssdCssdAssdAssdAssdAssdTssdTssdCss |
| | | Ab¹ssGb¹ |
| 16 | 6bq | Ab1ssC*b1ssAb1ssTb1ssdTssdGssdCssdAssdAssdAssdAssdTssdTssdCss Ab1ssGb1 |
| 16 | 6br | Ab1ssC*b1ssAb1ssdTssdTssdGssdCssdAssdAssdAssdAssdTssdTss C*b1ssAb1ssGb1 |
| 16 | 6bs | Ab¹ssC*b¹ssAb¹ssTb¹ssdTssdGssdCssdAssdAssdAssdAssdTssdTss C*b¹ssAb¹ssGb¹ |
| 16 | 6bt | Ab1ssC*b1ssdAssdTssdTssdGssdCssdAssdAssdAssdAssdTssdTss C*b1ssAb1ssGb1 |
| 16 | 6bu | Ab1ssC*b1ssdAssdTssdTssdGssdCssdAssdAssdAssdAssdTssTb1ss Cb1ssAb1ssGb1 |
| 16 | 6bv | Ab^1ssC*b^1ssAb^1ssdTssdTssdGssdCssdAssdAssdAssdAssdTssTb^1ss C*b^1ssAb^1ssGb^1 |
| 16 | 6bw | Ab¹ssC*b¹ssAb¹ssTb¹ssdTssdGssdCssdAssdAssdAssdAssdTssTb¹ss C*b¹ssAb¹ssGb¹ |
| 16 | 6bx | Ab¹ssC*b¹ssAb¹ssdTssdTssdGssdCssdAssdAssdAssdAssTb¹ssTb¹ss C*b¹ssAb¹ssGb¹ |
| 16 | 6by | Ab¹ssC*b¹ssAb¹ssTb¹ssTb¹ssdGssdCssdAssdAssdAssdAssdTssdTss C*b¹ssAb¹ssGb¹ |

The **Seq. ID No. 98** represents Homo sapiens ephrin B2 (EFNB2), transcript variant 1, mRNA (NCBI Reference Sequence: NM_004093.4) written in the DNA code, i.e. represented in G, C, A, T code, and not in the RNA code.

The **Seq. ID No. 99** represents Homo sapiens ephrin B2 (EFNB2), transcript variant 2, mRNA (NCBI Reference Sequence: NM_001372056.1) written in the DNA code, i.e. represented in G, C, A, T code, and not in the RNA code.

The **Seq. ID No. 100** represents Homo sapiens ephrin B2 (EFNB2), transcript variant 1, mRNA (NCBI Reference Sequence: NM_001372057.1) written in the DNA code, i.e. represented in G, C, A, T code, and not in the RNA code.

The **Seq. ID No. 101** represents the Homo sapiens chromosome 13, GRCh38.p13 Primary Assembly (NCBI Reference Sequence: NC_000013.11) (NC_000013.11:c106535662-106489745 Homo sapiens chromosome 13, GRCh38.p13 Primary Assembly).

### Pharmaceutical Compositions

The antisense-oligonucleotides of the present invention are preferably administered in form of their pharmaceutically active salts optionally using substantially nontoxic pharmaceutically acceptable carriers, excipients, adjuvants, solvents or diluents. The medications of the present invention are prepared in a conventional solid or liquid carrier or diluents and a conventional pharmaceutically-made adjuvant at suitable dosage level in a known way. The preferred preparations and formulations are in administrable form which is suitable for infusion or injection (intrathecal, intracerebroventricular, intracranial, intravenous, intraparenchymal, intratumoral, intra- or extraocular, intraperitoneal, intramuscular, subcutaneous), local administration into the brain, inhalation, local administration into a solid tumor or oral application. However also other application forms are possible such as absorption through epithelial or mucocutaneous linings (oral mucosa, rectal and vaginal epithelial linings, nasopharyngial mucosa, intestinal mucosa), rectally, transdermally, topically, intradermally, intragastrically, intracutaneously, intravaginally, intravasally, intranasally, intrabuccally, percutaneously, sublingually application, or any other means available within the pharmaceutical arts.

The administrable formulations, for example, include injectable liquid formulations, retard formulations, powders especially for inhalation, pills, tablets, film tablets, coated tablets, dispersible granules, dragees, gels, syrups, slurries, suspensions, emulsions, capsules and deposits. Other administratable galenical formulations are also possible like a continuous injection through an implantable pump or a catheter into the brain.

As used herein the term "pharmaceutically acceptable" refers to any carrier which does not interfere with the effectiveness of the biological activity of the antisense-oligonucleotides as active ingredient in the formulation and that is not toxic to the host to which it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and the active compound can be administered to the subject at an effective dose. An "effective dose" refers to an amount of the antisense-oligonucleotide as active ingredient that is sufficient to affect the course and the severity of the disease, leading to the reduction or remission of such pathology. An "effective dose" useful for treating and/or preventing these diseases or disorders may be determined using methods known to one skilled in the art. Furthermore, the antisense-oligonucleotides of the present invention may be mixed and administered together with liposomes, complex forming agents, receptor targeted molecules, solvents, preservatives and/or diluents.

Preferred are pharmaceutical preparations in form of **infusion** solutions or solid matrices for continuous release of the active ingredient, especially for continuous infusion for **intrathecal** administration, intracerebroventricular administration or intracranial administration of at least one antisense-oligonucleotide of the present invention. Also preferred are pharmaceutical preparations in form of solutions or solid matrices suitable for local administration into the brain. For fibrotic diseases of the lung, inhalation formulations are especially preferred.

A ready-to-use sterile solution comprises for example at least one antisense-oligonucleotide at a concentration ranging from 1 to 100 mg/ml, preferably from 5 to 25 mg/ml and an isotonic agent selected, for example, amongst sugars such as sucrose, lactose, mannitol or sorbitol. A suitable buffering agent, to control the solution pH to 6 to 8 (preferably 7 - 8), may be also included. Another optional ingredient of the formulation can be a non-ionic surfactant, such as Tween 20 or Tween 80.

A sterile lyophilized **powder** to be reconstituted for use comprises at least one antisense-oligonucleotide, and optionally a bulking agent (e.g. mannitol, trehalose, sorbitol, glycine) and/or a cryoprotectent (e.g. trehalose, mannitol). The solvent for reconstitution can be water for injectable compounds, with or without a buffering salt to control the pH to 6 to 8.

Aerosol preparations suitable for **inhalation** may include solutions and solids in powder form, which may be in combination with a pharmaceutically acceptable carrier such as inert compressed gas, e.g. nitrogen.

A particularly preferred pharmaceutical composition is a lyophilized (freeze-dried) preparation (lyophilisate) suitable for administration by inhalation or for intravenous administration. To prepare the preferred lyophilized preparation at least one antisense-oligonucleotide of the invention is solubilized in a 4 to 5% (w/v) mannitol solution and the solution is then lyophilized. The mannitol solution can also be prepared in a suitable buffer solution as described above.

Further examples of suitable cryo- / lyoprotectants (otherwise referred to as bulking agents or stabilizers) include thiol-free albumin, immunoglobulins, polyalkyleneoxides (e.g. PEG, polypropylene glycols), trehalose, glucose, sucrose, sorbitol, dextran, maltose, raffinose, stachyose and other saccharides (cf. for instance WO 97/29782), while mannitol is used preferably. These can be used in conventional amounts in conventional lyophilization techniques. Methods of lyophilization are well known in the art of preparing pharmaceutical formulations.

For administration by inhalation the particle diameter of the lyophilized preparation is preferably between 2 to 5 µm, more preferably between 3 to 4 µm. The lyophilized preparation is particularly suitable for administration using an inhalator, for example the OPTINEB^{®} or VENTA-NEB^{®} inhalator (NEBU-TEC, Elsenfeld, Germany). The lyophilized product can be rehydrated in sterile distilled water or any other suitable liquid for inhalation administration. Alternatively, for intravenous administration the lyophilized product can be rehydrated in sterile distilled water or any other suitable liquid for intravenous administration.

After rehydration for administration in sterile distilled water or another suitable liquid the lyophilized preparation should have the approximate physiological osmolality of the target tissue for the rehydrated peptide preparation i.e. blood for intravenous administration or lung tissue for inhalation administration. Thus it is preferred that the rehydrated formulation is substantially isotonic.

The preferred dosage concentration for either intravenous, oral, or inhalation administration is between 10 to 2000 µmol/ml, and more preferably is between 200 to 800 µmol / ml.

For **oral** administration in the form of tablets or capsules, the at least one antisense-oligonucleotide may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like.

Additionally, the compositions of the present invention may be formulated in sustained release form to provide the rate controlled release of the at least one antisense-oligonucleotide to optimize the therapeutic effects. Suitable dosage forms for sustained release include implantable biodegradable matrices for sustained release containing the at least one antisense-oligonucleotide, layered tablets containing layers of varying disintegration rates or controlled release polymeric matrices impregnated with the at least one antisense-oligonucleotide.

Liquid form preparations include solutions, suspensions and emulsions. As an example may be mentioned water or water-propylene glycol solutions for parenteral injections or addition of sweeteners and opacifiers for oral solutions, suspensions and emulsions.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5% to about 95% by weight of the total composition, preferably from about 25% to about 75% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates, such as magnesium stearate, calcium stearate or potassium stearate, stearic acid; high melting point waxes; and water soluble lubricants, such as sodium chloride, sodium benzoate, sodium acetate, sodium oleate, polyethylene glycols and D,L-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1% to about 7% by weight of the total composition, more preferably from about 0.2 to 5% by weight, and most preferably from about 0.5 to about 2% by weight.

In the pharmaceutical compositions disclosed herein the antisense-oligonucleotides are incorporated preferably in the form of their salts and optionally together with other components which increase stability of the antisense-oligonucleotides, increase recruitment of RNase H, increase target finding properties, enhance cellular uptake and the like. In order to achieve these goals, the antisense-oligonucleotides may be chemically modified instead of or in addition to the use of the further components useful for achieving these purposes. Thus the antisense-oligonucleotides of the invention may be chemically linked to moieties or components which enhance the activity, cellular distribution or cellular uptake etc. of the antisense-oligonucleotides. Such moieties include lipid moieties such as a cholesterol moiety, cholic acid, a thioether, hexyl-S-tritylthiol, a thiocholesterol, an aliphatic chain, e.g., dodecandiol or undecyl residues, a phospholipid such as dihexadecyl-rac-glycerol or triethylammonium-1,2-di-O-hexadecyl-rac-glycero-3H-phosphonate, a polyamine or a polyethylene glycol chain, or adamantine acetic acid, a palmityl moiety, or an octadecylamine or hexylamino-carbonyl-oxycholesterol moiety. The present invention also includes antisense-oligonucleotides which are chimeric compounds. "Chimeric" antisense-oligonucleotides in the context of this invention, are antisense-oligonucleotides, which contain two or more chemically distinct regions, one is the oligonucleotide sequence as disclosed herein which is connected to a moiety or component for increasing cellular uptake, increasing resistance to nuclease degradation, increasing binding affinity for the target nucleic acid, increasing recruitment of RNase H and so on. For instance, the additional region or moiety or component of the antisense-oligonucleotide may serve as a substrate for enzymes capable of cleaving RNA:DNA hybrids or RNA:RNA molecules. By way of example, RNase H is a cellular endoribonuclease which cleaves the RNA strand of an RNA:DNA duplex. Activation of RNase H, therefore, results in cleavage of the RNA target which is the mRNA coding for the TGF-R_{II}, thereby greatly enhancing the efficiency of antisense-oligonucleotide inhibition of gene expression. Consequently, comparable results can often be obtained with shorter oligonucleotides when chimeric oligonucleotides are used.

### Indications

The present invention relates to the use of the antisense-oligonucleotides disclosed herein for suppression of ephrin-B2 function or for renal protective effects or for controlling nephrin function.

The present invention relates to the use of the antisense-oligonucleotides disclosed herein for use in the prophylaxis and treatment of nephropathy. The present invention relates to the use of the antisense-oligonucleotides disclosed herein for use in the prophylaxis and treatment of diabetic nephropathy. The present invention relates to the use of the antisense-oligonucleotides disclosed herein for use in the prophylaxis and treatment of proteinuria in diabetes. and/or nephropathy. For the treatment of proteinuria in diabetes and/or diabetic nephropathy db/db mice with uninephrectomy (UNx) surgery will be used as a model animal of type 2 diabetes with kidney complications. The mice will receive 20 mg/kg KGW ASO in a volume of 1 ml/kg KGW i.p. from the age of 14 weeks, twice a week for maximum 4 weeks. Albumin uria and structure of the kidneys will be analyzed.

### Description of the Figures:

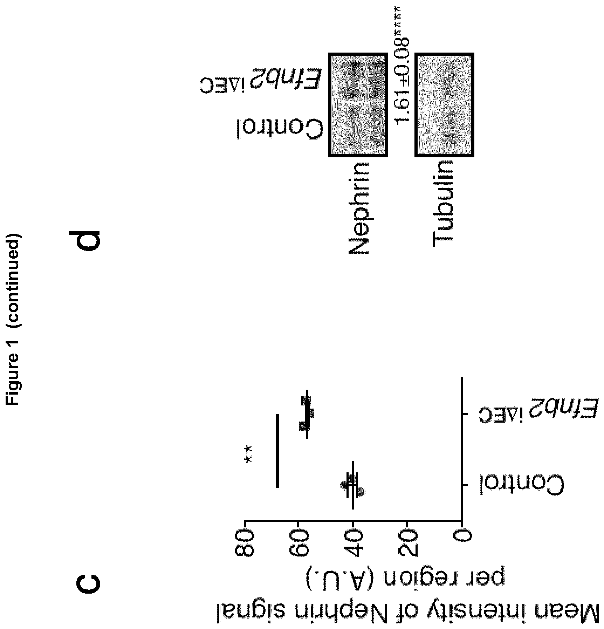

- Figure 1: **Ephrin-B2/EphB4 forward signaling controls Nephrin expression in the glomerulus. a** Staining of ephrin-B2, CD31, Nephrin and nuclei (DAPI) in the glomerulus of control and *Efnb2 ^{iΔEC}* mice. Arrowheads indicated Nephrin positive podocytes, while arrows indicated CD31 positive ECs. Enlarged images of dash boxes in the middle panels are shown in right panels. **b, c** Staining of Nephrin, CD31, and DAPI in the glomerulus of control and *Efnb2 ^{iΔEC}* mice. Quantified mean intensity of Nephrin in b) was shown in c). n=3, Line represents mean +/- SEM. **p<0.01 by Student's t-test. **d** Western blotting analysis of control and *Efnb2 ^{iΔEC}* mice kidney lysate. Intensity of Nephrin quantified was shown. The value represents mean +/- SEM n=5, ****p<0.0001 by Student's t-test.
- Figure 2: **Ephrin-B2 is secreted from ECs to podocytes. a** Diagram of tagged version of ephrin-B2 protein and epitope of antibodies. **b** Immunoprecipitation of N-terminal tagged GFP-flag-ephrin-B2 or GFP from HEK293 cell lysate and its culture conditioned medium with flag antibody. Upper panels showed bands detected with an anti-flag antibody. Lower panels showed bands detected with an ephrin-B2 antibody raised against its cytoplasmic tail. **c,** CFP-ephrin-B2 visualized with a GFP- antibody was confirmed within podocytes (arrowheads) as well as ECs. tTA negative animals were used as a negative control. **d** Eprhin-B2 was detected in the blood plasma of control mice, which was signigicantly decreased in that of *Efnb2*^{iΔEC} mice. **e, f** Staining of pEphB4, Nephrin, and DAPI in the glomerulus of control and *Efnb2 ^{iΔEC}* mice. Quantified mean intensity of pEphB4 in d) was shown in e). n=3, Line represent mean +/- SEM. **p<0.01 by Student's t-test.
- Figure 3: **Ephrin-B2/EphB4 forward signaling regulates Nephrin phosphorylation in the glomerulus. a** Immunoprecipitation of endogenous EphB4 with Nephrin from mouse kidney lysate. **b, c** Staining of pNephrin, CD31, and DAPI in the glomerulus of control and *Efnb2 ^{iΔEC}* mice. Quantified mean intensity of pNephrin in b) was shown in c). n=4, Line represents mean +/- SEM. ***p<0.001 by Student's t-test.
- Figure 4: **Ephrin-B2 expression is increased in the diabetic nephropathy patients. a, b,** Staining of ephrin-B2, Nephrin and DAPI in the glomerulus of hyperoxaluria and diabetic nephrophathy patients. Relative ratio of ephrin-B2 expression in podocyte normalized with DAPI in a) was shown in b). n=3, Line represents mean +/- SEM. *p<0.05 by Student's t-test.
- Figure 5: **Diabetic kidney dysfunction is ameliorated in *Efnb2*^{iΔEC} mutants. a** Light microscopy analysis of hematoxylin and eosin (HE)- stained sections of control and *Efnb2 ^{iΔEC}* mice kidneys. **b** Quantification of blood glucose levels in diabetic control and *Efnb2 ^{iΔEC}* mice. Line represents mean +/- SEM. n=8 for control, n=6 for *Efnb2 ^{iΔEC}* mice at the end point of experiments. n.s, by Student's t-test. **c** Quantification of body weight of diabetic control and *Efnb2 ^{iΔEC}* mice at the end point of experiments. Line represents mean +/- SEM. n=6. n.s. by Student's t-test. **d,** Quantification of serum creatinine levels in diabetic control (n=3) and *Efnb2 ^{iΔEC}* mice (n=4) at the end point of experiments. Line represents mean +/- SEM. n.s by Student's t-test. **e** Quantification of consecutive urine samples from control and *Efnb2 ^{iΔEC}* mice using UACR. n=5, non-diabetic control, n=5 diabetic control, n=4 diabetic *Efnb2 ^{iΔEC}* mice, **p<0.01, *p<0.05, by two way ANOVA. Line represents mean +/- SEM. **f, g** Staining of Nephrin, CD31, and DAPI in the glomerulus of non-diabetic control, diabetic control and *Efnb2 ^{iΔEC}* mice. Quantified mean intensity of Nephrin in f) was shown in g). n=6 for non-diabetic control, n=6 for diabetic control, n=8 for diabetic *Efnb2 ^{iΔEC}* mice. Line represents mean +/- SEM. ***p<0.001 by two way ANOVA. **h** Immunogold EM staining shows Nephrin (arrowheads) localizes to the base of the foot processes and beneath the slit diaphragm of podocytes from diabetic control and *Efnb2 ^{iΔEC}* mice.
- Figure 6: **Ephrin-B2 knockdown by ASO** a) The relative expression of Efnb2 mRNA, gene encoded ephrin-B2, was reduced ASO transfection into cultured ECs isolated from Efnb2 loxed mice, named LOX. The mRNA levels were analyzed by real-time RT-PCR (n=3). Data represents mean 15 ± S.E.M. one-way ANOVA. *p< 0.05, **p< 0.01, ***p< 0.001. b) Sequences of ASO against Efnb2 are shown. For negative control, ephrin-B2 KO cells, named KO were used.

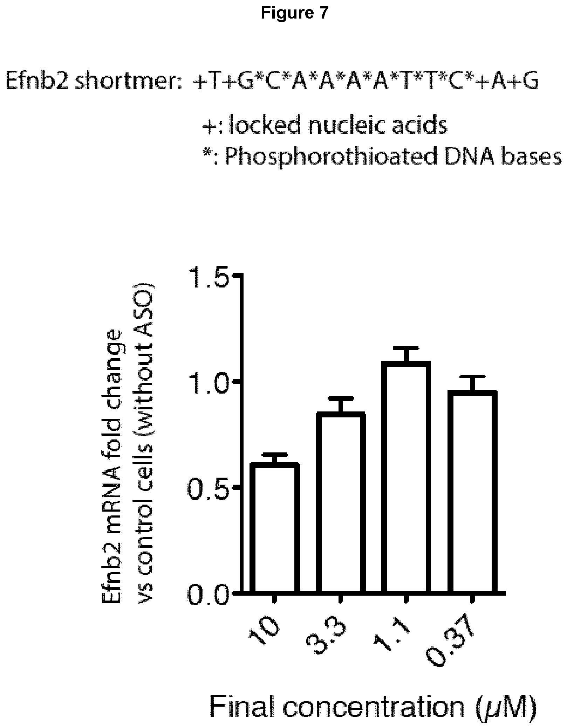

- Figure 7: Diagram showing the results for efnb2 shortmer Seq. ID 74p.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Material and Methods

Oligonucleotides having the following sequences were used as references: Ref.1=Gb¹sC*b¹sdGsdGsdAsdCsdAsdCsdGsdCsC*b¹sGb¹(Seq. ID No. 97)

### Example 1

### Mice

Male mice were used for all experiments. *Cdh5-Cre ERT2* mice were bred with *Efnb2 IoxP*-flanked homozygous mice (*Efnb2*^{Iox/Iox})*.* The *Efnb2*^{Iox/Iox}*-Cdh5-Cre ERT2* negative mice were used as control littermates. To induce the genetic silencing of ephrin-B2, Tamoxifen (Sigma, T5648) was injected intraperitoneally at a dose of 50µl of 3mg/ml in *Efnb2*^{iΔEC} and control littermates for three consecutive days from P21 to P23. To examine the specific expression of Cre in the glomerulus, *Cdh5-Cre ERT2* mice were bred with R26-stop-EYFP mutant mice.

Experiments involving animals were conducted in accordance with institutional guidelines and laws, and following the protocols approved by the local animal ethics committees and authorities (Regierungspraesidium Darmstadt).

LOX is the name of cell line, isolated endothelial cells from ephrin-B2 floxed/floxed mice carrying T antigen. Then the cells were infected the plasmid DNA encoding Cre to KO Efnb2, the gene encoding ephrin-B2.

### Diabetic model mouse

To induce diabetes, Streptozotocin (Sigma, S0130) dissolved in Sodium-Citrate buffer, pH 4.5 (Sigma, C8532) and at 40mg/Kg body weight was intraperitoneally injected from P35 to P39. The mice were provided with 10% sucrose water (Sigma, S0389) to prevent and treat hypoglycemia when required. Subsequent to Streptozotocin injections, the mice were fed with western-type high fat diet until the end point of the experiments (ssniff Spezialdiäten GmbH, E15126-34) to aid in the development of diabetes. Mice were checked for blood glucose values prior to and at the end of the protocol by drawing blood via the tail-vein. The control non-diabetic mice were only fed the western-type high fat diet from 5 weeks of age until 18 weeks of age.

### Immunostaining

Mouse kidney sections were prepared by cryosectioning (10µm thickness) and fixed for 10 min using 4% paraformaldehyde solution in PBS (pH 7.4). Fixed sections were washed 3x with PBS and incubated overnight at 4°C with the appropriate primary antibodies against ephrin-B2 (Sigma, HPA0008999, dilution 1:100), CD31 (BD Pharmingen, 557355, dilution 1:100), Nephrin (R&D Systems, AF3159, dilution 1:100), phospho-EphB4 (Signalway Antibody, 12720, dilution 1:100), phospho-Nephrin (Abcam, ab80299, dilution 1:100), or GFP (Invitrogen, A21311, dilution 1:200). After incubation with the primary antibodies, the sections were washed 3x with PBS and incubated for 2 hours at room temperature with Biotin-SP- conjugated AffiniPure Goat Anti-Rabbit IgG (H+L) (Jackson ImmunoResearch Laboratories Inc., 705-065-147, dilution 1:200) and additional relevant Alexa Fluor secondary antibodies. The sections were washed 3x with PBS and incubated for an additional two hours with streptavidin Alexa Fluor conjugated secondary antibodies (Thermo Fisher Scientific, Invitrogen, Life Technologies) for biotin. Following washing 3x with PBS, sections were counterstained with DAPI (Thermo Fisher Scientific, Invitrogen, Life Technologies; D3571) solution in PBS for 15min. Sections were washed and mounted using Fluoromount-G^{®} mounting medium (SouthernBiotech, 0100-01).

Specimens for human study analysis were taken from patients after informed consent. The use of these specimens was approved by the IRB of the Washington University School of Medicine, in adherence to the Declaration of Helsinki.

Formaldehyde-fixed paraffin-embedded kidney tissue samples were deparafinized, and antigen retrieval was performed by boiling the slides in EDTA buffer (pH 8) for 15 min, blocked with 5% BSA / 5% FBS / 0.1% Tween-20 for 30 min, and treated with rabbit anti-ephrin-B2 antibody (Sigma, HPA0008999, dilution 1:100) and goat anti-Nephrin antibody (R&D Systems, AF3159, dilution 1:100) at 4°C overnight. Then slides were incubated with AlexaFluor 546 donkey anti-goat IgG (1:500 dilution; Invitrogen), AlexaFluor 488 donkey anti-rabbit IgG, and Hoechst 33342 (1:500 dilution; Thermo Fisher Scientific) at room temperature for 2 hours. Images were taken using a Leica SP8 microscope. To measure mean signal intensity of the pixels in the glomerulus, Volocity (Improvision) was used for quantitative image analysis. Photoshop CS, and Illustrator CS (Adobe) software were used for image processing and in compliance with general guidelines for image processing.

### Hemotoxylin and Eosin (HE), and PAM staining

Kidneys collected at experimental end-point were dehydrated and paraffin mounted. Following sectioning using a microtome (3-4µm thick), slides were placed at 60°C to enable the paraffin to melt and fix the tissue onto the slide. The kidney sections were then re-hydrated by immersion in xylene (10 min, 3x), 100% ethanol (5 min, 3x), 95% ethanol (5 min, 1x), 80% ethanol (5 min, 1x) and deionized water (5minutes, 1x) sequentially. The sections were immersed in hemotoxylin (Waldeck; 2E-038) solution for 5 min, after which they were washed with running tap water till the appropriate levels of blue color corresponding to hemotoxylin had developed. Following this, sections were placed in eosin (Waldeck; 2C-140) solution for 1 min and washed 3x in 95% ethanol for 3 min. Stained kidney sections were again dehydrated in 100% ethanol (3 min, 3x) and xylene (5 min, 3x) followed by mounting using the Entellan^{®} New (Merck Millipore; 107961) mounting medium.

### Nephrin staining for immunoelectron microcopy

For EM, small pieces (transmission EM) of kidney tissue were fixed in 4% paraformaldehyde and 0.2% gluteraldehyde overnight. Samples were incubated with 20% donkey serum and 0.1% photo-flo (Electron Microscopy Sciences) in PBS for 30 min and then were incubated with an anti-Nephrin antibody (R&D systems, AF3159 dilution 1:100) at 4°C overnight followed by rabbit anti-goat Fab'(Nanoprobes, 2006, dilution 1:100) at 4°C overnight. After washing in 0.1M phosphate buffer and fixing with 1% glutaraldehyde, signal was enhanced with HQ Silver enhancement kit (Nanoprobes 2012). After refixing with OsO₄, the samples were dehydrated in a graded ethanol series and embedded in epoxy resin. Ultrathin sections were examined by EM (Hitachi, H-7650)

### UACR measurement and serum creatinine assay

Urine albumin values and urine creatinine values were determined using the Mouse Albumin ELISA Kit (Shibayagi Co.Ltd, AKRAL-121) and Creatinine Assay Kit (AbCam, ab65340) according to the manufacturers instruction. The optical density (O.D.) read-out of the ELISA plates was measured at 450 nm for albumin values and at 570 nm for creatinine values using a 96-well plate reader. The UACR values were obtained by dividing the final values of albumin in milligrams/liter (mg/l) by the creatinine values obtained in grams/liter (g/l) for each time point per mouse. The serum creatinine values were determined using a Creatinine Assay Kit (AbCam, ab65340) as described above. Serum stored at - 80°C was thawed on ice and used for the assay without any dilution.

### Western blotting analysis with kidney lysate

Collected whole kidney tissue was weighed, cut into small pieces and suspended in 3mL/gram tissue of RIPA buffer (50 mM Tris-HCl, pH 7.4; 1% NP-40; 0.5% Nadeoxycholate; 0.1% SDS; 150 mM NaCl; 2 mM EDTA; Protease inhibitor (Sigma, P2714, 1:100)) and homogenized with a hand-held sonicator. The samples were then centrifuged at 10000xg for 10 min. The supernatant was transferred into a new tube and spun again at 10000xg for 30 min at 4°C. The supernatant was then used for further analysis. Protein concentration was determined using a BCA protein assay (Pierce) and 2 mg of protein was suspended in 3x SDS loading buffer, boiled and used for western blot analysis using anti-Nephrin (R&D systems, AF3159, dilution 1:1000) and anti-a-tubulin (Sigma, T5168, 1:20000) antibodies. Signal intensity was measured with ImageJ.

### Immunoprecipitation of EphB4 from kidney lysates

Kidney lysates were prepared as for western blotting, then incubated with 2 µg of an anti-EphB4 antibody (R&D systems, MAB446) for 1hr at 4°C with rotation. Then 20µl of Dynabeads G (Thermo Fisher Scientific, Invitrogen, Life Technologies; 10004D), pre-washed with wash buffer (50mM Tris-HCI pH 8.0, EDTA 1mM, NaCl 150mM, NP-40 1%, Protease inhibitor (Sigma, P2714, 1:100) and Phosphatase inhibitor (Merck Millipore, 524625)), were added. After 1hr incubation at 4°C, the Dynabeads G were separated using a magnetic separator and washed 5x with wash buffer. The beads were re-suspended in 30µl of 1X SDS sample buffer for western blotting analysis using anti-Nephrin (R&D Systems, AF3159, dilution 1:1000) and anti-EphB4 (R&D systems, AF446, 1:1000) antibodies.

### Immunoprecipitation of ephrin-B2 from cultured ECs

*Efnb2* lox/lox cells and KO cells (1 × 10⁶ cells/10-cm dish)¹⁵ were seeded and incubated for 24 hours. Cells were washed with ice-cold PBS and lysed with 800 µl of lysis buffer (50 mM Tris-HCl [pH 7.4], 1% NP-40, 150 mM NaCl, protease inhibitor cocktail (Sigma, P2714, 1:100), phosphatase inhibitor cocktail (Calbiochem, 524629, 1:50). The lysates were centrifuged at 20000× g for 10 min at 4°C, and the supernatant was incubated with protein G sepharose beads crosslinked with 2 µg of ephrin-B2 antibody (R&D systems AF 496) at 4°C for 1 hr. The beads were washed three times with an excess of lysis buffer and eluted with 60 µl of 1× SDS sample buffer. Then, 20 µl of each eluate was subjected to SDS-PAGE, followed by immunoblotting with anti-ephrin-B2 antibody (Sigma, HPA008999).

### Transfection of cDNA

pEGFP (clontech) pBabe-puro-GFP-flag-ephrin-B2, pcDNA3-HA-Capns1 (kindly gifted by Dr. Claudio Schneider), or pCMV-Rbpj-myc-Flag (kindly provided by the Lead Discovery Center GmbH, Dortmund) were transfected into HEK293 cells with Polyethylenimine (PEI; Polysciences Inc., 24765). 200µl of transfection mix was prepared by mixing 100µl of 150mM NaCl solution in distilled water with 2µg plasmid DNA, followed by vortexing the mix and subsequently addition of 100µl of PEI-NaCl solution. The PEI-NaCl-plasmid transfection mix was incubated for 10 min and was added to cells. The transfection mix was incubated with cells for 6-8hours. The cells were washed and fresh cell culture medium was added. For HUVECs, jet-PEI^{®} DNA Transfection reagent (PolyPlus, 101-10) was used according to the manufacturers instructions.

### Immunoprecipitation of tagged proteins

48 hours after transfection, cells were lysed with lysis buffer (20 mM Tris-HCl [pH 7.4], 1 mM EDTA, 1mM DTT, 1% CHAPS, 150mM NaCland 1× Protease inhibitor (Sigma, P2714)). Cell lysate or the medium was clarified by centrifugation at 10000 × g for 10 min at 4°C. The supernatants were incubated with Flag-M2 magnetic beads (Sigma, M8823) for 2 hours at 4°C. The Flag-M2 magnetic beads were separated using a magnetic separator and washed 3 times with lysis buffer. The beads were suspended in SDS sample buffer for western blotting analysis using anti-Flag-M2 HRP conjugated (Sigma, A8592, dilution 1:10,000), anti-ephrin-B2 (Sigma, HPA008999, dilution 1:1000) or anti-HA-HRP conjugated (Cell Signaling Technology, 2999 dilution 1:10000) antibodies.

### Example 2

### Transfection of ASOs

ASOs were transfected into Lox cells with TransIT-X2^{®} Dynamic Delivery System (Mirus Bio, Madison, WI). 50 µL of ASO mix was prepared by mixing 6 µl of ASO in Opti-MEM, followed by vortexing the mix and subsequently addition of 3 µl of TransIT-X2. The ASO transfection mix was incubated for 30 min and was added to cells (final concentration 100 nM). The transfection mix was incubated with cells for 48hours.

### RT-qPCR

mRNA was extracted with Quick-RNA Miniprep Kit (R1054, Zymo Research, Freiburg Germany). Reverse transcription was performed with SuperScript IV VILO (11756050, ThermoFisherScientific, MA). quantitative PCR was performed with Power SYBR Green Master Mix (4367659, Thermo Fisher Scientific) with following primers: mouse beta-actin_Fwd 5'-GAAATCGTGCGTGACATCAAAG-3', mouse beta-actin_Rvr 5'-TGTAGTTTCATGGATGCCACAG-3', mouse Efnb2-1_Fwd 5'-ATTATTTGCCCCAAAGTGGACTC-3', mouse Efnb2-1_Rvs 5'-GCAGCGGGGTATTCTCCTTC-3'

### Example 2.1

Result for antisense oligonucteotide Seq. ID 74p are shown in Fig. 7.

**Table 34**

| Format | 96 well | |
|---|---|---|
| cells | LOX | 2×10^{∧}4/well |
| Medium | LOX cell medium | |

Free uptake 10, 3.3, 1.1, 0.37, 0.12 µM

**Table 35**

| Shortmer concentrations | | | |
|---|---|---|---|
| | | Conc (µM) | Final (µM) |
| A | 60 µL of stock | 30 | 10 |
| B | 30 µL of stock + 60 µLof opti-MEM | 10 | 3,33333333 |
| C | 30 µL of B + 60 µLof opti-MEM | 3,33333333 | 1,11111111 |
| D | 30 µL of C + 60 µLof opti-MEM | 1,11111111 | 0,37037037 |
| E | 30 µL of D + 60 µLof opti-MEM | 0,37037037 | 0,12345679 |

| | | | |
|---|---|---|---|
| Mix 60 µL of shortmer antisense oligonucteotide Seq. ID 74p + 300 µL cell Divide 120 µL × 3 Incubate for 48 hr at 33°C | | | |

Analyse with Cells-to-CT kit
- qPCR: StepOnePlus^{™} Real-Time PCR System Power up SYBR Green Internal control: B2M (beta-2-Microglobulin)

### Example 3: Synthesis of gapmer antisense-oligonucleotides

### Abbreviations

- Pybop:: (Benzotriazol-1-yl-oxy)tripyrrolidinophosphonium-hexafluorophosphate
- DCM:: Dichloromethane
- DMF:: Dimethylformamide
- DIEA:: Diisopropylethylamine
- DMAP:: 4-Dimethylaminopyridine
- DMT:: 4,4'-dimethoxytrityl
- LCAA:: Long Chain Alkyl Amino
- TRIS:: Tris(hydroxymethyl)-aminomethan
- TRIS-HCl:: Tris(hydroxymethyl)-aminomethan hydrochloride
- DEPC:: Diethylpyrocarbonate

### Gapmer antisense-oligonucleotide synthesis and purification

The antisense-oligonucleotides in form of gapmers were prepared on an ABI 394 synthesizer (Applied Biosystems) according to the phosphoramidite oligomerization chemistry using 500 Ǻ controlled pore glass (CPG) loaded with Unylinker^{™} Chemgenes (Wilmington, MA, USA) as solid support to give a 3 µmοl synthesis scale.

Alternatively, the antisense-oligonucleotides can be prepared on an ABI 3900 or an Expedite^{™} (Applied Biosystems) according to the phosphoramidite oligomerization chemistry. On the ABI3900, the solid support can be polystyrene loaded with UnySupport (Glen Research, Sterling, Virginia, USA) to give a synthesis scale of 0.2 µmol.

As DNA building-units 5'-O-DMT-2'-deoxythymidine-3'-[(2-cyanoethyl)-(N,N-diisopropyl)]-phosphoramidite, 5'-O-DMT-N⁴-benzoyl-2'-deoxycytidine-3'-(2-cyanoethyl-N,N-diisopropyl)phosphoramidite, 5'-O-DMT-N⁶-benzoyl-2'-deoxyadenosine-3'-(2-cyanoethyl-N,N-diisopropyl)phosphoramidite and 5'-O-DMT-N²-isobutyryl-2'-deoxyguanosine-3'-(2-cyanoethyl-N,N-diisopropyl)phosphoramidite were used, which correspond to the 5'-O-(4,4'-dimethoxytrityl)-2'-O,3'-O-(2-cyanoethyl-N,N-diisopropyl) phosphoramidite monomers of deoxy thymidine (dT), 4-N-benzoyl-2'-deoxy-cytidine (dC^{Bz}), 6-N-benzoyl-2'-deoxy-adenosine (dA^{Bz}) and 2-N-isobutyryl-2'-deoxy-guanosine (dG^{iBU}).

As LNA-building-units (β-D-oxy-LNA) 5'-O-DMT-2'-O,4'-C-methylene-N²-dimethylformamidine-guanosine-3'-(2-cyanoethyl-N,N-diisopropyl)phosphoramidite (LNA-G^{DMF}), 5'-O-DMT-2'-O,4'-C-methylene-thymidine 3'-[(2-cyanoethyl)-(N,N-diisopropyl)]phosphoramidite (LNA-T),5'-O-DMT-2'-O,4'-C-methylene-N⁶-benzoyla denosine-3'-(2-cyanoethyl-N,N-diisopropyl)phosphoramidite (LNA-A^{Bz}), 5'-O-DMT-2'-O-,4'-C-methylene-5-methyl-N⁴-benzoylcytidine-3'-(2-cyanoethyl-N,N-diisopropyl)phosphoramidite (LNA-C*^{Bz}) were used.

The phosphoramidites of the LNAs were dissolved in dry acetonitrile to give 0.07 M-oligonucleotide except LNA-C*^{Bz} which was dissolved in a mixture of THF/acetonitrile (25/75 (v/v)). In case of LNA-A^{Bz} (MW = 885.9 g/mole, CAS No. [206055-79-0]) 100 mg were dissolved in 1.6 ml anhydrous acetonitrile. In case of LNA-C*^{Bz} (MW = 875.9 g/mol, CAS No. [206055-82-5]) 100 mg were dissolved in 1.6 ml THF/acetonitrile 25/75 (v/v). In case of LNA-G^{DMF} (MW = 852.9 g/mol, CAS No. [709641-79-2]) 100 mg were dissolved in 1.7 ml anhydrous acetonitrile. In case of LNA-T (MW = 772.8 g/mol, CAS No. [206055-75-6]) 100 mg was dissolved in 1.8 ml anhydrous acetonitrile.

The β-D-thio-LNAs 5'-O-DMT-2'-deoxy-2'-mercapto-2'-S,4'-C-methylene-N⁶-benzoyladenosine-3'-[(2-cyanoethyl-N,N-diisopropyl)]-phosphoramidite, 5'-O-DMT-2'-deoxy-2'-mercapto-2'-S,4'-C-methylene-5-methyl-N⁴-benzoylcytidine-3'-(2-cyanoethyl-N,N-diisopropyl)phosphoramidite, 5'-O-DMT-2'-deoxy-2'-mercapto-2'-S,4'-C-methylene-N²-dimethyformamidineguanosine-3'-[(2-cyanoethyl)-(N,N-diisopropyl)]-phosphoramidite, and 5'-O-DMT-2'-deoxy-2'-mercapto-2'-S,4'-C-methylene-thymidine-3'-[(2-cyanoethyl)-(N,N-diisopropyl)]-phosphoramidite were synthesized as described in J. Org. Chem. 1998, 63, 6078 ― 6079.

The synthesis of the β-D-amino-LNA 5'-O-DMT-2'-deoxy-2'-amino-2'-N,4'-C-methylene-5-methyl-N⁴-benzoylcytidine-3'-[(2-cyanoethyl-N,N-diisopropyl)]-phosphoramidite, 5'-O-DMT-2'-deoxy-2'-amino-2'-N,4'-C-methylene-N²-dimethyformamidineguanosine-3'-[(2-cyanoethyl)-(N,N-diisopropyl)]-phosphoramidite, 5'-O-DMT-2'-deoxy-2'-amino-2'-N,4'-C-methylene-thymidine-3'-[(2-cyanoethyl)-(N,N-diisopropyl)]-phosphoramidite, 5'-O-DMT-2'-deoxy-2'-amino-2'-N,4'-C-methylene-N⁶-benzoyladenosine-3'-[(2-cyanoethyl-N,N-diisopropyl)]-phosphoramidite, 5'-O-DMT-2'-deoxy-2'-methylamino-2'-N,4'-C-methylene-5-methyl-N⁴-benzoylcytidine-3'-(2-cyanoethyl-N,N-diisopropyl)phosphoramidite, 5'-O-DMT-2'-deoxy-2'-methylamino-2'-N,4'-C-methylene-N²-dimethyformamidineguanosine-3'-[(2-cyanoethyl)-(N,N-diisopropyl)]-phosphoramidite, 5'-O-DMT-2'-deoxy-2'-methylamino-2'-N,4'-C-methylene-thymidine-3'-[(2-cyanoethyl)-(N,N-diisopropyl)]-phosphoramidite, and 5'-O-DMT-2'-deoxy-2'-amino-2'-N,4'-C-methylene-N⁶-benzoyladenosine-3'-(2-cyanoethyl-N,N-diisopropyl)phosphoramidites was carried out according to the literature procedure (J. Org Chem. 1998, 63, 6078 - 6079).

The α-L-oxy-LNAs α-L-5'-O-DMT-2'-O,4'-C-methylene-N⁶-benzoyladenosine-3'-(2-cyanoethyl-N,N-diisopropyl)phosphoramidite, α-L-5'-O-DMT-2'-O-,4'-C-methylene-5-methyl-N⁴-benzoylcytidine-3'-(2-cyanoethyl-N,N-diisopropyl)phosphoramidite, α-L-5'-O-DMT-2'-O,4'-C-methylene-N²-dimethyformamidineguanosine-3'-[(2-cyanoethyl)-(N,N-diisopropyl)]-phosphoramidite, and α-L-5'-O-DMT-2'-0,4'-C-methylene-thymidine-3'-[(2-cyanoethyl)-(N,N-diisopropyl)]-phosphoramidite were performed similarly to the procedures described in the literature (J. Am. Chem. Soc. 2002, 124, 2164-2176; Angew. Chem. Int. Ed. 2000, 39, 1656 - 1659).

The synthesis of β-D-ENA LNAs 5'-O-DMT-2'-O,4'-C-ethylene-N²-dimethylformamidine-guanosine-3'-(2-cyanoethyl-N,N-diisopropyl)phosphoramidite, 5'-O-DMT-2'-O,4'-C-ethylene-thymidine 3'-[(2-cyanoethyl)-(N,N-diisopropyl)]phosphoramidite, 5'-O-DMT-2'-O,4'-C-ethylene-N⁶-benzoyladenosine-3'-(2-cyanoethyl-N,N-diisopropyl)phosphoramidite, and 5'-O-DMT-2'-O-,4'-C-ethylene-5-methyl-N⁴-benzoylcytidine-3'-(2-cyanoethyl-N,N-diisopropyl)-phosphoramidite was carried out according to the literature procedure (Nucleic Acids Research 2001, Supplement No. 1, 241-242).

The synthesis of β-D-ENA LNAs 5'-O-DMT-2'-O,4'-C-ethylene-N²-dimethylformamidine-guanosine-3'-(2-cyanoethyl-N,N-diisopropyl)phosphoramidite, 5'-O-DMT-2'-O,4'-C-ethylene-thymidine 3'-[(2-cyanoethyl)-(N,N-diisopropyl)]phosphoramidite, 5'-O-DMT-2'-O,4'-C-ethylene-N⁶-benzoyladenosine-3'-(2-cyanoethyl-N,N-diisopropyl)phosphoramidite, and 5'-O-DMT-2'-O-,4'-C-ethylene-5-methyl-N⁴-benzoylcytidine-3'-(2-cyanoethyl-N,N-diisopropyl)-phosphoramidite was carried out according to the literature procedure (Nucleic Acids Research 2001, Supplement No. 1, 241-242).

The (*β*-(benzoylmercapto)ethyl)pyrrolidinolthiophosphoramidites for the synthesis of the oligonucleotide with phosphorodithioate backbone were prepared in analogy to the protocol reported by Caruthers (J. Org. Chem. 1996, 61, 4272-4281).

The "phosphoramidites-C3" (3-(4,4'-dimethoxytrityloxy)propyl-1-[(2-cyanoethyl)-(N,N-diisopropyl)]-phosphoramidite and the "3'-Spacer C3 CPG" (1-dimethoxytrityloxy-propanediol-3-succinoyl)-long chain alkylamino-CPG were purchased.

### General procedure

### Preparation of the LNA-solid support:

1) Preparation of the LNA succinyl hemiester (WO2007/112754)
   5'-O-DMT-3'-hydroxy-nucleoside monomer, succinic anhydride (1.2 eq.) and DMAP (1.2 eq.) were dissolved in 35 ml dichloromethane (DCM). The reaction was stirred at room temperature overnight. After extractions with NaH₂PO₄ 0.1 M pH 5.5 (2×) and brine (1×), the organic layer was further dried with anhydrous NaSO₄ filtered and evaporated. The hemiester derivative was obtained in 95% yield and was used without any further purification.
2) Preparation of the LNA-support (WO2007/112754)
   The above prepared hemiester derivative (90 µmol) was dissolved in a minimum amount of DMF, DIEA and pyBOP (90 µmol) were added and mixed together for 1 min. This pre-activated mixture was combined with LCAA-CPG (500 Å, 80-120 mesh size, 300 mg) in a manual synthesizer and stirred. After 1.5 hours at room temperature, the support was filtered off and washed with DMF, DCM and MeOH. After drying, the loading was determined to be 57 µmol/g.

### Elongation of the oligonucleotide (Coupling)

4,5-Dicyanoimidazole (DCl) as described in WO2007/112754 was employed for the coupling of the phosphoramidites. Instead of DCI other reagents, such as 5-ethylthio-1H-tetrazole (ETT) (0.5 M in acetonitrile), 5-benzylthio-1H-tetrazole or saccharin-1-methylimidazol can be used as activator. 0.25 M DCI in acetonitrile was used for the coupling with LNA.

### Capping

10% acetic anhydride (Ac₂O) in THF (HPLC grade) and 10% N-methylimidazol (NMI) in THF/pyridine (8:1) (HPLC grade) were added and allowed to react.

### Oxidation

Phosphorous(III) to Phosphorous(V) is normally done with e.g. iodine/THF/pyridine/H₂O using 0.02 M iodine in THF/Pyridine/H₂O or 0.5 M 1S)-(+)-(10-camphorsulfonyl)-oxaziridine (CSO) in anhydrous acetonitrile.

In the case that a phosphorthioate internucleoside linkage is prepared, a thiolation step is performed using 0.2 M 3H-1,2-benzothiole-3-one 1,1-dioxide (Beaucage reagent) in anhydrous acetonitrile. The thiolation can also be carried out by using a 0.05 M solution of 3-((dimethylamino-methylidene)-amino)-3H-1,2,4-dithiazole-3-thione (DDTT) in anhydrous acetonitrile/pyridine (1:1 v/v) or by using xanthane chloride (0.01 M in acetonitrile/pyridine 10%) as described in WO2007/112754. Alternatively, other reagents for the thiolation step such as xanthane hydride (5-imino-(1,2,4)dithiazolidine-3-thione), phenylacetyl disulfide (PADS) can be applied.

In the case that a phosphordithioate internucleoside linkage is prepared, the resulting thiophosphite triester was oxidized to the phosphorothiotriester by addition of 0.05 M DDTT (3-((Dimethylamino-methylidene)amino)-3H-1,2,4-dithiazole-3-thione) in pyridine/acetonitrile (4:1 v/v).

### Cleavage from the solid support and deprotection

At the end of the solid phase synthesis, the final 5'-O-(4,4'-dimethoxytrityl) group can be removed on the synthesizer using the "Deblock" reagent or the group may be still present while the oligonucleotide is cleaved from the solid support. The DMT groups were removed with trichloroacetic acid.

### Removal of 5'-O-(4,4'-dimethoxytrityl) group on solid support:

Upon completion of the solid phase synthesis antisense-oligonucleotides were treated with a 20% diethylamine solution in acetonitrile for 20 min. to remove the cyanoethyl protecting groups on the phosphate backbone. Subsequently, the antisense-oligonucleotides were cleaved from the solid support and deprotected using 1 to 5 mL concentrated aqueous ammonia for 16 hours at 55 °C. The solid support was separated from the antisense-oligonucleotides by filtration or centrifugation.

If the oligonucleotides contain phosphorodithioate triester, the thiol-groups were deprotected with thiophenol:triethylamine:dioxane, 1:1:2, v/v/v for 24 h, then the oligonucleotides were cleaved from the solid support using aqueous ammonia for 1-2 hours at room temperature, and further deprotected for 4 hours at 65 °C.

### Removal of 5'-O-(4,4'-dimethoxytrityl) group after cleavage from solid support:

The oligonucleotides were cleaved from the solid support using aqueous ammonia for 1-2 hours at room temperature, and further deprotected for 4 hours at 65 °C. The oligonucleotides were purified by reverse phase HPLC (RP-HPLC), and then the DMT-group is removed with trichloroacetic acid.

If the oligonucleotides contain phosphorodithioate triester, the cleavage from the solid-support and the deprotection of the thiol-group were performed by the addition of 850 µl ammonia in concentrated ethanol (ammonia/ethanol 3:1 v/v) at 55 °C for 15 - 16h.

### Terminal groups

### Terminal groups at the 5'-end of the antisense oligonucleotide:

The solid supported oligonucleotide was treated with 3% trichloroacetic acid in dichloromethane (w/v) to completely remove the 5'-DMT protection group. Further, the compound was converted with an appropriate terminal group with cyanoethyl-N,N-diisopropyl)phosphoramidite-moiety. After the oxidation of the phosphorus(III) to phosphorus(V), the deprotection, detachment from the solid support and deprotection sequence were performed as described above.

### Purification

The crude antisense-oligonucleotides were purified by anion-exchange high-performance liquid chromatography (HPLC) on an AKTA Explorer chromatography system (GE Healthcare, Freiburg, Germany) and a column packed with Source Q15 (GE Healthcare). Buffer A was 10 mM sodium perchlorate, 20 mM Tris, 1 mM EDTA, pH 7.4 and contained 20% acetonitrile and buffer B was the same as buffer A with the exception of 500 mM sodium perchlorate. A gradient of 15%B to 55%B within 32 column volumes (CV) was employed. UV traces at 280 nm were recorded. Appropriate fractions were pooled and precipitated with 3 M NaOAc, pH= 5.2 and 70% ethanol. Finally, the pellet was washed with 70% ethanol.

### Analytics

Identity of the antisense-oligonucleotides was confirmed by electrospray ionization mass spectrometry (ESI-MS) and purity was determined by analytical OligoPro Capillary Electrophoresis (CE).

The purification of the dithioate was performed on an Amersham Biosciences P920 FPLC instrument fitted with a Mono Q 10/100 GL column. The buffers were prepared with DEPC-treated water, and their compositions were as follows: Buffer A: 25 mM Tris-HCl, 1 mM EDTA, pH 8.0; Buffer B: 25 mM Tris-HCl, 1 mM EDTA, 1 M NaCl, pH 8.0.

### Example 3.1: C*b¹sAb¹sdAsdGsdCsdAsdAsdGsdGsdCsAb¹sTb¹ (Seq. ID No. 46m)

### Step 1 (Coupling).

5'-O-DMT-2'-O,4'-C-methylene-thymidine-3'-O-succinoyl- linked LCAA CPG (0.2 µmol) was treated with 1400 µl 3% trichloroacetic acid in dichloromethane for 60 s to completely remove the 5'-DMT protection group. After several washes with a total amount of 800 µl acetonitrile, the coupling reaction was carried out with 80 µl 5'-O-DMT-2'-O,4'-C-methylene-N⁶-benzoyladenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N-methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 2 (Coupling):

The coupling was carried out with 80 µl 5'-O-DMT-N4-benzoyl-2'-deoxycytidine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 3 (Coupling)

The coupling was carried out with 80 µl 5'-O-DMT-N2-isobutyryl-2'-deoxyguanosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 4 (Coupling)

The coupling was carried out with 80 µl 5'-O-DMT-N2-isobutyryl-2'-deoxyguanosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 5 (Coupling)

The coupling was carried out with 80 µl 5-O-DMT-N6-benzoyl-2-deoxyadenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 6 (Coupling)

The coupling was carried out with 80 µl 5'-O-DMT-N6-benzoyl-2'-deoxyadenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 7 (Coupling)

The coupling was carried out with 80 µl 5'-O-DMT-N4-benzoyl-2'-deoxycytidine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 8 (Coupling)

The coupling was carried out with 80 µl 5'-O-DMT-N2-isobutyryl-2'-deoxyguanosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 9 (Coupling)

The coupling was carried out with 80 µl 5'-O-DMT-N6-benzoyl-2'-deoxyadenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 10 (Coupling):

The coupling was carried out with 80 µl 5'-O-DMT-2'-O,4'-C-methylene-N6-benzoyladenosine-3'-(2-cyanoethyl-N,N-diisopropyl)phosphoramidites (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 11 (Coupling)

The coupling was carried out with 80 µl 5'-O-DMT-2'-O-,4'-C-methylene-5-methyl-N4-benzoylcytidine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 12 (Deprotection and Cleavage):

Upon completion of the solid phase synthesis, the antisense-oligonucleotides were treated with a 20% diethylamine solution in acetonitrile for 20 min. to remove the cyanoethyl protecting groups on the phosphate backbone.

Subsequently, the antisense-oligonucleotides were cleaved from the solid support and further deprotected using 5 mL concentrated aqueous ammonia for 16 hours at 55°C. The solid support was separated from the antisense-oligonucleotides by filtration or centrifugation.

### Step 13 (Purification):

The crude antisense-oligonucleotide was purified by anion-exchange high-performance liquid chromatography (HPLC) according to the general procedure as described above.

### Example 3.2: Tb¹sGb¹sdCsdAsdAsdAsdAsdTsdTsdCsAb¹sGb¹s (Seq. ID No. 74m)

### Step 1 (Coupling)

5'―O―DMT-2'-O,4'-C-methylene-N2-dimethyformamidineguanosine 3'-O succinoyl-linked LCAA CPG (0.2 µmol) was treated with 1400 µl 3% trichloroacetic acid in dichloromethane for 60 s to completely remove the 5'-DMT protection group. After several washes with a total amount of 800 µl acetonitrile, the coupling reaction was carried out with 80 µl 5'―O―DMT―2'―O,4'―C―methylene―N6― benzoyladenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 2 (Coupling):

The coupling was carried out with 80 µl 5'-O-DMT-N4-benzoyl-2'-deoxycytidine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 3 (Coupling)

The coupling was carried out with 80 µl 5'―O―DMT-2'-deoxythymidine-3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 4 (Coupling)

The coupling was carried out with 80 µl 5'―O―DMT-2'-deoxythymidine-3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 5 (Coupling)

The coupling was carried out with 80 µl 5'―O―DMT―N6―benzoyl-2'-deoxyadenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 6 (Coupling)

The coupling was carried out with 80 µl 5'―O―DMT―N6―benzoyl-2'-deoxyadenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 7 (Coupling)

The coupling was carried out with 80 µl 5'―O―DMT―N6―benzoyl-2'-deoxyadenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 8 (Coupling)

The coupling was carried out with 80 µl 5'―O―DMT―N6―benzoyl-2'-deoxyadenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 9 (Coupling)

The coupling was carried out with 80 µl 5'-O-DMT-N4-benzoyl-2'-deoxycytidine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 10 (Coupling):

The coupling was carried out with 80 µl 5'―O―DMT-2'-O,4'-C-methylene-N2-dimethyformamidineguanosine-3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 11 (Coupling)

The coupling was carried out with 80 µl 5'―O―DMT-2'-O,4'-C-methylene―thymidine 3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazole (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 12 (Deprotection and Cleavage):

Upon completion of the solid phase synthesis, the antisense-oligonucleotides were treated with a 20% diethylamine solution in acetonitrile (Biosolve BV, Valkenswaard, The Netherlands) for 20 min. to remove the cyanoethyl protecting groups on the phosphate backbone.

Subsequently, the antisense-oligonucleotides were cleaved from the solid support and further deprotected using 5 mL concentrated aqueous ammonia for 16 hours at 55°C. The solid support was separated from the antisense-oligonucleotides by filtration or centrifugation.

### Step 13 (Purification):

The crude antisense-oligonucleotides were purified by anion-exchange high-performance liquid chromatography (HPLC) on an AKTA Explorer System (GE Healthcare, Freiburg, Germany) and a column packed with Source Q15 (GE Helthcare). Buffer A was 10 mM sodium perchlorate, 20 mM Tris, 1 mM EDTA, pH 7.4 and contained 20% acetonitrile and buffer B was the same as buffer A with the exception of 500 mM sodium perchlorate. A gradient of 15%B to 55%B within 32 column volumes (CV) was employed. UV traces at 280 nm were recorded. Appropriate fractions were pooled and precipitated with 3 M NaOAc, pH= 5.2 and 70% ethanol. Finally, the pellet was washed with 70% ethanol.

### Example 3.3: Gb¹sGb¹sdTsdTsdAsdGsdGsdGsdCsdTsGb¹sAb¹s (Seq. ID No. 16m)

### Step 1 (Coupling).

5'―O―DMT―2'―O,4'―C―methylene―N6―benzoxyladenosine-3'-O―succinoyl-linked LCAA CPG (0.2 µmol) was treated with 1400 µl 3% trichloroacetic acid in dichloromethane for 60 s to completely remove the 5'-DMT protection group. After several washes with a total amount of 800 µl acetonitrile, the coupling reaction was carried out with 80 µl 5' O DMT-2'-O,4'-C-methylene-N2-dimethyformamidineguanosine-3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 2 (Coupling):

The coupling was carried out with 80 µl 5'―O―DMT-2'-deoxythymidine-3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 3 (Coupling)

The coupling was carried out with 80 µl 5'-O-DMT-N4-benzoyl-2'-deoxycytidine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 4 (Coupling)

The coupling was carried out with 80 µl 5'―O―DMT-N2-isobutyryl-2'-deoxyguanosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 5 (Coupling)

The coupling was carried out with 80 µl 5'―O―DMT-N2-isobutyryl-2'-deoxyguanosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 6 (Coupling)

The coupling was carried out with 80 µl 5'―O―DMT-N2-isobutyryl-2'-deoxyguanosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 7 (Coupling)

The coupling was carried out with 80 µl 5'―O―DMT―N6―benzoyl-2'-deoxyadenosine-3'-(2-cyanoethyl-N,N-diisopropyl)phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 8 (Coupling)

The coupling was carried out with 80 µl 5'―O―DMT-2'-deoxythymidine-3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 9 (Coupling)

The coupling was carried out with 80 µl 5'―O―DMT-2'-deoxythymidine-3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 10 (Coupling):

The coupling was carried out with 80 µl 5'―O―DMT-2'-O,4'-C-methylene-N2-dimethyformamidineguanosine-3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile.

The compound was treated with 1400 µl 3% trichloroacetic acid in dichloromethane for 60 s to completely remove the 5'-DMT protection group.

### Step 11 (Coupling)

The coupling was carried out with 80 µl 5'―O―DMT-2'-O,4'-C-methylene-N2-dimethyformamidineguanosine-3'-[(2-cyanoethyl)-(N,N-diisopropyl)]-phosphoramidite (0.07 M) and 236 µl DCI in acetonitrile (0.25 M). The coupling reaction was allowed to take place for 250 sec., and excess reagents were flashed out with 800 µl acetonitrile, and 640 µl of Beaucage (0.2 M) were inserted to the column for 180 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

### Step 12 (Deprotection and Cleavage):

Upon completion of the solid phase synthesis, the antisense-oligonucleotides were treated with a 20% diethylamine solution in acetonitrile (Biosolve BV, Valkenswaard, The Netherlands) for 20 min. to remove the cyanoethyl protecting groups on the phosphate backbone.

Subsequently, the antisense-oligonucleotides were cleaved from the solid support and further deprotected using 5 mL concentrated aqueous ammonia for 16 hours at 55°C. The solid support was separated from the antisense-oligonucleotides by filtration or centrifugation.

### Step 13 (Purification):

The crude antisense-oligonucleotides were purified by anion-exchange high-performance liquid chromatography (HPLC) on an AKTA Explorer System (GE Healthcare, Freiburg, Germany) and a column packed with Source Q15 (GE Helthcare). Buffer A was 10 mM sodium perchlorate, 20 mM Tris, 1 mM EDTA, pH 7.4 and contained 20% acetonitrile and buffer B was the same as buffer A with the exception of 500 mM sodium perchlorate. A gradient of 15%B to 55%B within 32 column volumes (CV) was employed. UV traces at 280 nm were recorded. Appropriate fractions were pooled and precipitated with 3 M NaOAc, pH= 5.2 and 70% ethanol. Finally, the pellet was washed with 70% ethanol.

### Example 3.4

The antisense oligonucleotides of Seq. ID No. 15m, 17m, 18m, 21i, 22i, 23i, 21j, 22j, 23j, 26m, 27m, 28m, 29m, 26n, 27n, 28n, 29n, 26o, 27o, 28o, 29o, 30q, 31q, 32q, 30r, 31r, 32r, 30s, 31s, 32s, 30t, 31t, 32t, 19q, 20q, 19r, 20r, 19s, 20s, 19t, 20t, 4as, 4at, 4au, 4av, 4aw, 4ax, 4ay, 4az, 4ba, 4bb and 4bc were synthesized according to the general procedure with the appropriate DNA and LNA building units as exemplified in example 3.3.

### Example 3.5

The antisense oligonucleotides of Seq. ID No. 45m, 47m, 48m, 44m, 52i, 53i, 54i, 55i, 52j, 53j, 54j, 55j, 57m, 58m, 60m, 59m, 57n, 58n, 60n, 59n, 57o, 58o, 60o, 59o, 61q, 62q, 63q, 61r, 62r, 63r, 61s, 62s, 63s, 61t, 62t, 63t, 49q, 50q, 49r, 50r, 49s, 50s, 49t, 50t, 5as, 5at, 5au, 5av, 5aw, 5ax, 5ay, 5az, 5ba, 5bb and 5bc were synthesized according to the general procedure with the appropriate DNA and LNA building units as exemplified in example 3.1.

### Example 3.6

The antisense oligonucleotides of Seq. ID No. 74m, 77m, 78m, 84i, 85i, 86i, 84j, 85j, 86j, 88m, 89m, 90m, 88n, 89n, 90n, 88o, 89o, 90o,91q, 92q, 93q, 91r, 92r, 93r, 91s, 92s, 93s, 91t, 92t, 93t, 79q, 80q, 79r, 80r, 79s, 80s, 79t, 80t, 6as, 6at, 6au, 6av, 6aw, 6ax, 6ay, 6az, 6ba, 6bb, 6bc were synthesized according to the general procedure with the appropriate DNA and LNA building units as exemplified in example 3.2.

**Example 3.7: Gb¹Gb¹**dTsdTsdAsdGsdGsdGsdCsdTs**Gb¹Ab¹** (Seq. ID. 16p) The LNA was bound to CPG according to the general procedure. The coupling reaction and capping step were also carried out as described in example 3.3.

### Example 3.8

The antisense oligonucleotides of Seq. ID No. 15p, 17p, 18p, 21k, 22k, 23k, 21l, 221, 231, 9f, 10f, 11f, 26p, 27p, 28p, 29p, 26q, 27q, 28q, 29q, 26r, 27r, 28r, 29r, 30u, 31u, 32u, 30v, 31v, 32v, 30w, 31w, 32w, 30x, 31x, 32x, 19u, 20u, 19v, 20v, 19w, 20w, 19x, 20x, 4bd, 4be, 4bf, 4bg, 4bh, 4bi, 4bj, 4bk, 4bl, 4bm and 4bn were synthesized according to the general procedure with the appropriate DNA and LNA building units as exemplified in example 3.3.

### Example 3.9

The antisense oligonucleotides of Seq. ID No. 46p, 45p, 47p, 48p, 44p, 52k, 53k, 54k, 55k, 521, 531, 541, 551, 39f, 40f, 41f, 57p, 58p, 60p, 59p, 57q, 58q, 60q, 59q, 57r, 58r, 60r, 59r, 61u, 62u, 63u, 61v, 62v, 63v, 61w, 62w, 63w, 61x, 62x, 63x, 49u, 50u, 49v, 50v, 49w, 50w, 49x, 50x, 5bd, 5be, 5bf, 5bg, 5bh, 5bi, 5bj, 5bk, 5bl, 5bm and 5bn were synthesized according to the general procedure with the appropriate DNA and LNA building units as exemplified in example 3.1.

### Example 3.10

The antisense oligonucleotides of Seq. ID No. 74p, 77p, 78p, 84k, 85k, 86k, 84l, 85l, 86l, 71f, 72f, 73f, 88p, 89p, 90p, 88q, 89q, 90q, 88r, 89r, 90r, 91u, 92u, 93u, 91v, 92v, 93v, 91w, 92w, 93w, 91x, 92x, 93x, 79u, 80u, 79v, 80v, 79w, 80w, 79x, 80x, 6bd, 6be, 6bf, 6bg, 6bh, 6bi, 6bj, 6bk, 6bl, 6bm and 6bn were synthesized according to the general procedure with the appropriate DNA and LNA building units as exemplified in example 3.2.

### Example 3.11:

**Gb¹ssGb¹ss**dTssdTssdAssdGssdGssdGssdCssdTss**Gb¹ssAb¹** (Seq. ID. 16s) 5'―O―DMT―2'―O,4'―C―methylene―N6―benzoxyladenosine-3'-O―succinoyl-linked LCAA CPG (0.2 µmol) was treated with 1400 µl 3% trichloroacetic acid in dichloromethane for 60 s to completely remove the 5'-DMT protection group. After several washes with a total amount of 800 µl acetonitrile, the coupling reaction was carried out with 38 µl 5'―O―DMT-2'-O,4'-C-methylene-N2-dimethyformamidineguanosine-3'-[((β-benzoylmercapto)ethyl]pyrrolidinolthiophos-phoramidite (0.15 M) in 10% dichloromethane (v/v) in acetonitrile and 236 µl DCI in acetonitrile (0.25 M). The coupling reaction was allowed to take place for 250 sec., and excess reagents were flashed out with 800 µl acetonitrile, and 900 µl of DDTT (0.05 M in pyridine/acetonitrile 4:1 v/v) were inserted to the column for 240 s The system was flushed with 320 µl acetonitrile. For the capping step, 448 µl of acetic anhydride in THF (1:9 v/v) and 448 µl N methylimidazol (NMI)/THF/pyridine (1:8:1) were added and allowed to react for 45 sec. At the end of this cycle, the system was washed with 480 µl acetonitrile. The compound was treated with 1400 µl 3% trichloroacetic acid in dichloromethane for 60 s to completely remove the 5'-DMT protection group.

The further elongation of the oligonucleotide was performed in the same way as described in the previous examples.

Upon completion of the solid phase synthesis, the antisense-oligonucleotides were treated 850 µl ammonia in concentrated ethanol (ammonia/ethanol 3:1 v/v) at 55 °C for 15 - 16h in order to cleave antisense-oligonucleotide from the solid-support and to deprotect the thiol-group.

Next, the crude antisense-oligonucleotide was purified by anion-exchange chromatogtraophy using a Mono Q 10/100 GL column. The buffers were prepared with DEPC-treated water, and their compositions were as follows: Buffer A: 25 mM Tris-HCl, 1 mM EDTA, pH 8.0; Buffer B: 25 mM Tris-HCl, 1 mM EDTA, 1 M NaCl, pH 8.0.

### Example 3.12

The antisense oligonucleotides of Seq. ID No. 15s, 17s, 18s, 21m, 22m, 23m, 21n, 22n, 23n, 9g, 10g, 11g, 26s, 27s, 28s, 29s, 26t, 27t, 28t, 29t, 26u, 27u, 28u, 29u, 30y, 31y, 32y, 30z, 31z, 32z, 30aa, 31aa, 32aa, 30ab, 31ab, 32ab, 19y, 20y, 19z, 20z, 19aa, 20aa, 19ab, 20ab, 4bo, 4bp, 4bq, 4br, 4bs, 4bt, 4bu, 4bv, 4bw, 4bx, 4by, 46s, 45s, 47s, 48s, 44s, 52m, 53m, 54m, 55m, 52n, 53n, 54n, 55n, 39g, 40g, 41g, 57s, 58s, 60s, 59s, 57t, 58t, 60t, 59t, 57u, 58u, 60u, 59u, 61y, 62y, 63y, 61z, 62z, 63z, 61aa, 62aa, 63aa, 61ab, 62ab, 63ab, 49y, 50y, 49z, 50z, 49aa, 50aa, 49ab, 50ab, 5bo, 5bp, 5bq, 5br, 5bs, 5bt, 5bu, 5bv, 5bw, 5bx, 5by,74s, 77s, 78s, 84m, 85m, 86m, 84n, 85n, 86n, 71g, 72g, 73g, 88s, 89s, 90s, 88t, 89t, 90t, 88u, 89u, 90u, 91y, 92y, 93y, 91z, 92z, 93z, 91aa, 92aa, 93aa, 91ab, 92ab, 93ab, 79y, 80y, 79z, 80z, 79aa, 80aa, 79ab, 80ab, 6bo, 6bp, 6bq, 6br, 6bs, 6bt, 6bu, 6bv, 6bw, 6bx and 6by were synthesized according to the general procedure with the appropriate DNA and LNA building units as exemplified in example 3.11.

### Example 3.13

The antisense oligonucleotides of Seq. ID No. 9e, 10e, 11e, 39e, 40e, 41e, 71e, 72e and 73e were synthesized according to the general procedure with the appropriate DNA and LNA building units as exemplified in example 3.11.

### Example 3.14

The other oligonucleotides of Seq. ID No. 16n, 15n, 17n, 18n, 46n, 45n, 47n, 48n, 44n, 74n, 77n, 78n, 16o, 15o, 17o, 18o, 46o, 45o, 47o, 48o, 44o, 74o, 77o, 78o, 16t, 15t, 17t, 18t, 46t, 45t, 47t, 48t, 44t, 74t, 77t, 78t, 16u, 15u, 17u, 18u, 46u, 45u, 47u, 48u, 44u, 74u, 77u, 78u, 16q, 15q, 17q, 18q, 46q, 45q, 47q, 48q, 44q, 74q, 77q, 78q, 16r, 15r, 17r, 18r, 46r, 45r, 47r, 48r, 44r, 74r, 77r and 78r were synthesized according to the general procedure and as shown in the previous examples. The preparation of the antisense-oligonucleotide including β-D-thio-LNA, β-D-amino-LNA, α-L-oxy-LNA, β-D-ENA, β-D-(NH)-LNA, or (β-D-(NCH₃

## Claims

1. Antisense-oligonucleotide consisting of 10 to 28 nucleotides and at least two of the 10 to 28 nucleotides are LNAs, and the antisense-oligonucleotide is capable of hybridizing with a region of the gene encoding *Efnb2,* or with a region of the mRNA encoding *Efnb2,* wherein the region of the gene encoding *Efnb2,* or the region of the mRNA encoding *Efnb2,* comprises the sequence AATTCAGCCCTAACCT (Seq. ID No. 1), AAATGCCTTGCTTGTA (Seq. ID No. 2), or CTGAATTTTGCAATGT (Seq. ID No. 3), and the antisense-oligonucleotide comprises a sequence capable of hybridizing with said sequence AATTCAGCCCTAACCT (Seq. ID No. 1), AAATGCCTTGCTTGTA (Seq. ID No. 2), or CTGAATTTTGCAATGT (Seq. ID No. 3), and salts and optical isomers of said antisense-oligonucleotide.

2. Antisense-oligonucleotide according to claim 1, wherein the antisense-oligonucleotide hybridizes selectively only with the sequence AATTCAGCCCTAACCT (Seq. ID No. 1) of the region of the gene encoding *Efnb2,* or of the region of the mRNA encoding the *Efnb2*; or wherein the antisense-oligonucleotide hybridizes selectively only with the sequence AAATGCCTTGCTTGTA (Seq. ID No. 2) of the region of the gene encoding *Efnb2,* or of the region of the mRNA encoding the *Efnb2*; or wherein the antisense-oligonucleotide hybridizes selectively only with the sequence CTGAATTTTGCAATGT (Seq. ID No. 3) of the region of the gene encoding *Efnb2* or of the region of the mRNA encoding the *Efnb2.*

3. Antisense-oligonucleotide according to claim 1 or 2, wherein the antisense-oligonucleotide has a length of 12 to 16 nucleotides and/or wherein the antisense-oligonucleotide has a gapmer structure with 1 to 5 LNA units at the 3' terminal end and 1 to 5 LNA units at the 5' terminal end and/or wherein the antisense-oligonucleotide has phosphate, phosphorothioate and/or phosphorodithioate as internucleotide linkages.

4. Antisense-oligonucleotide according to any one of the claims 1 - 3, wherein the antisense-oligonucleotide is represented by the following general formula (S1) 5'-N¹-**TAGGGCTG**-N²-3' (Seq. ID No. 7) or general formula (S1A) 5'-N^{1A}-**TTAGGGCT**-N^{2A}-3' (Seq. ID No. 34) or general formula (S1B) 5'-N^{1B}-**GTTAGGGC**-N^{2B}-3' (Seq. ID No. 35), wherein
N¹ represents: AATTCTAGACCCCAGAGGT-, ATTCTAGACCCCAGAGGT-, TTCTAGACCCCAGAGGT-, TCTAGACCCCAGAGGT-, CTAGACCCCAGAGGT-, TAGACCCCAGAGGT-, AGACCCCAGAGGT-, GACCCCAGAGGT-, ACCCCAGAGGT-, CCCCAGAGGT-, CCCAGAGGT-, CCAGAGGT-, CAGAGGT-, AGAGGT-, GAGGT-, AGGT-, GGT-, GT-, or T-;
N² represents: -AATTCTTGAAACTTGATGG, ―AATTCTTGAAACTTGATG, -AATTCTTGAAACTTGAT, ―AATTCTTGAAACTTGA, ―AATTCTTGAAACTTG, -AATTCTTGAAACTT, -AATTCTTGAAACT, -AATTCTTGAAAC, -AATTCTTGAAA, -AATTCTTGAA, ―AATTCTTGA, -AATTCTTG, ―AATTCTT, -AATTCT, ―AATTC, -AATT, ―AAT, ―AA, or ―A;
N^{1A} represents: AAATTCTAGACCCCAGAGG-, AATTCTAGACCCCAGAGG-, ATTCTAGACCCCAGAGG-, TTCTAGACCCCAGAGG-, TCTAGACCCCAGAGG-, CTAGACCCCAGAGG-, TAGACCCCAGAGG-, AGACCCCAGAGG-, GACCCCAGAGG-, ACCCCAGAGG-, CCCCAGAGG-, CCCAGAGG-, CCAGAGG-, CAGAGG-, AGAGGT-, GAGG-, AGG-, GG-, or G-;
N^{2A} represents: ―GAATTCTTGAAACTTGATG, -GAATTCTTGAAACTTGAT, ―GAATTCTTGAAACTTGA, ―GAATTCTTGAAACTTG, -GAATTCTTGAAACTT, -GAATTCTTGAAACT, -GAATTCTTGAAAC, -GAATTCTTGAAA, -GAATTCTTGAA, ―GAATTCTTGA, -GAATTCTTG, ―GAATTCTT, -GAATTCT, ―GAATTC, -GAATT, -GAAT, ―GAA, ―GA, or ―G;
N^{1B} represents: GAAATTCTAGACCCCAGAG-, AAATTCTAGACCCCAGAG-, AATTCTAGACCCCAGAG-, ATTCTAGACCCCAGAG-, TTCTAGACCCCAGAG-, TCTAGACCCCAGAG-, CTAGACCCCAGAG-, TAGACCCCAGAG-, AGACCCCAGAG-, GACCCCAGAG-, ACCCCAGAG-, CCCCAGAG-, CCCAGAG-, CCAGAG-, CAGAG-, AGAG-, GAG-, AG-, or G-;
N^{2B} represents: -TGAATTCTTGAAACTTGAT, ―TGAATTCTTGAAACTTGA, -TGAATTCTTGAAACTTG, -TGAATTCTTGAAACTT, -TGAATTCTTGAAACT, -TGAATTCTTGAAAC, -TGAATTCTTGAAA, -TGAATTCTTGAA, ―TGAATTCTTGA, -TGAATTCTTG, ―TGAATTCTT, -TGAATTCT, ―TGAATTC, -TGAATT, ―TGAAT, ―TGAA, ―TGA, ―TG or ―T;
and salts and optical isomers of the antisense-oligonucleotide.

5. Antisense-oligonucleotide according to any one of the claims 1 ― 3, wherein the antisense-oligonucleotide is represented by the following general formula (S2) 5'-N³- **AGCAAGGC** ―N⁴-3' (Seq. ID No. 64) or general formula (S2A) 5'-N^{3A}-**AAGCAAGG** ―N^{4A}-3' (Seq. ID No. 65) or general formula (S2B) 5'-N^{3B}-**GCAAGGCA**-N^{4B}-3' (Seq. ID No. 66), wherein
N³ represents: GACCAGGGACGATCATACA-, ACCAGGGACGATCATACA-, CCAGGGACGATCATACA-, CAGGGACGATCATACA-, AGGGACGATCATACA-, GGGACGATCATACA-, GGACGATCATACA-, GACGATCATACA-, ACGATCATACA-, CGATCATACA-, GATCATACA-, ATCATACA-, TCATACA-, CATACA-, ATACA-, TACA-, ACA-, CA-, or A-;
N⁴ represents: -ATTTACAGTAACTTTACAA, -ATTTACAGTAACTTTACA, -ATTTACAGTAACTTTAC, -ATTTACAGTAACTTTA, -ATTTACAGTAACTTT, -ATTTACAGTAACTT, -ATTTACAGTAACT, -ATTTACAGTAAC, -ATTTACAGTAA, -ATTTACAGTA, -ATTTACAGT, -ATTTACAGT, -ATTTACAG, -ATTTACA, -ATTTAC, -ATTTA, -ATTT, -ATT, -AT, or -A;
N^{3A} represents: TGACCAGGGACGATCATAC-, GACCAGGGACGATCATAC-, ACCAGGGACGATCATAC-, CCAGGGACGATCATAC-, CAGGGACGATCATAC-, AGGGACGATCATAC-, GGGACGATCATAC-, GGACGATCATAC-, GACGATCATAC-, ACGATCATAC-, CGATCATAC-, GATCATAC-, ATCATAC-, TCATAC-, CATAC-, ATAC-, TAC-, AC-, or C-;
N^{4A} represents: -CATTTACAGTAACTTTACA, -CATTTACAGTAACTTTAC, -CATTTACAGTAACTTTA, -CATTTACAGTAACTTT, -CATTTACAGTAACTT, -CATTTACAGTAACT, -CATTTACAGTAAC, -CATTTACAGTAA, -CATTTACAGTA, -CATTTACAGT, -CATTTACAGT, -CATTTACAG, -CATTTACA, -CATTTAC, -CATTTA, -CATTT, -CATT, -CAT, -CA or -C;
N^{3B} represents: ACCAGGGACGATCATACAA-, CCAGGGACGATCATACAA-, CAGGGACGATCATACAA-, AGGGACGATCATACAA-, GGGACGATCATACAA-, GGACGATCATACAA-, GACGATCATACAA-, ACGATCATACAA-, CGATCATACAA-, GATCATACAA-, ATCATACAA-, TCATACAA-, CATACAA-, ATACAA-, TACAA-, ACAA-, CAA-, AA-, or A-;
N^{4B} represents: ―TTTACAGTAACTTTACAAA, -TTTACAGTAACTTTACAA, -TTTACAGTAACTTTACA, -TTTACAGTAACTTTAC, -TTTACAGTAACTTTA, -TTTACAGTAACTTT, -TTTACAGTAACTT, -TTTACAGTAACT, -TTTACAGTAAC, -TTTACAGTAA, -TTTACAGTA, -TTTACAGT, -TTTACAGT, -TTTACAG, -TTTACA, -TTTAC, -TTTA, -TTT, -TT, or ―T;
and salts and optical isomers of the antisense-oligonucleotide.

6. Antisense-oligonucleotide according to any one of the claims 1 - 3, wherein the antisense-oligonucleotide is represented by the following general formula (S3) 5'-N⁵-**GCAAAATT**-N⁶-3' (Seq. ID No. 94) or general formula (S3A) 5'-N^{5A}-**CAAAATTC** -N^{6A}-3' (Seq. ID No. 95) or general formula (S3B) 5'-N^{5B}-**AAAATTCA-**N^{6B}-3' (Seq. ID No. 96), wherein
N⁵ represents: AGCTGTAGCTAAATACATT-, GCTGTAGCTAAATACATT-, CTGTAGCTAAATACATT-, TGTAGCTAAATACATT-, GTAGCTAAATACATT-, TAGCTAAATACATT-, AGCTAAATACAT-, GCTAAATACATT-, GCTAAATACATT-, CTAAATACATT-, TAAATACATT-, AAATACAT-, AATACAT-, ATACATT-, TACATT-, ACATT-, CATT-, ATT-, TT- or T-;
N⁶ represents: -CAGATTTTATACAAAACAT, -CAGATTTTATACAAAACA, -CAGATTTTATACAAAAC, -CAGATTTTATACAAAA, -CAGATTTTATACAAA, -CAGATTTTATACAA, -CAGATTTTATACA, -CAGATTTTATAC, -CAGATTTTATA, -CAGATTTTAT, -CAGATTTTA, -CAGATTTT, -CAGATTT, -CAGATT, -CAGAT, -CAGA, -CAG, -CA, or -C;
N^{5A} represents: GCTGTAGCTAAATACATTG-, CTGTAGCTAAATACATTG-, TGTAGCTAAATACATTG-, GTAGCTAAATACATTG-, TAGCTAAATACATTG-, AGCTAAATACATG-, GCTAAATACATTG-, GCTAAATACATTG-, CTAAATACATTG-, TAAATACATTG-, AAATACATG-, AATACATG-, ATACATTG-, TACATTG-, ACATTG-, CATTG-, ATTG-, TTG-, TG-, or G-;
N^{6A} represents: -AGATTTTATACAAAACATC, -AGATTTTATACAAAACAT, ―AGATTTTATACAAAACA, -AGATTTTATACAAAAC, ―AGATTTTATACAAAA, -AGATTTTATACAAA, ―AGATTTTATACAA, -AGATTTTATACA, -AGATTTTATAC, -AGATTTTATA, -AGATTTTAT, -AGATTTTA, -AGATTTT, -AGATTT, -AGATT, -AGAT, -AGA, -AG, or -A;
N^{5B} represents: CTGTAGCTAAATACATTGC-, TGTAGCTAAATACATTGC-, GTAGCTAAATACATTGC-, TAGCTAAATACATTGC-, AGCTAAATACATGC-, GCTAAATACATTGC-, GCTAAATACATTGC-, CTAAATACATTGC-, TAAATACATTGC-, AAATACATGC-, AATACATGC-, ATACATTGC-, TACATTGC-, ACATTGC-, CATTGC-, ATTGC-, TTGC-, TGC-, GC- or C-;
N^{6B} represents: -GATTTTATACAAAACATCT, -GATTTTATACAAAACATC -GATTTTATACAAAACAT, -GATTTTATACAAAACA,-GATTTTATACAAAAC, -GATTTTATACAAAA, -GATTTTATACAAA, -GATTTTATACAA, -GATTTTATACA, -GATTTTATAC, -GATTTTATA, -GATTTTAT, -GATTTTA, -GATTTT, -GATTT, -GATT, -GAT, -GA, or -G;
and salts and optical isomers of the antisense-oligonucleotide.

7. Antisense-oligonucleotide according to any one of the claims 1 - 6, wherein the last 2 to 4 nucleotides at the 5' terminal end are LNA nucleotides and the last 2 to 4 nucleotides at the 3' terminal end are LNA nucleotides and between the LNA nucleotides at the 5' terminal end and the LNA nucleotides at the 3' terminal end at least 6 consecutive nucleotides are present which are non-LNA nucleotides or which are DNA nucleotides.

8. Antisense-oligonucleotide according to any one of the claims 1 - 7, wherein the LNA nucleotides are linked to each other through a phosphorothioate group or a phosphorodithioate group or wherein all nucleotides are linked to each other through a phosphate group or a phosphorothioate group or a phosphorodithioate group.

9. Antisense-oligonucleotide according to any one of the claims 1 - 8, wherein the LNA nucleotides are selected from the following group: 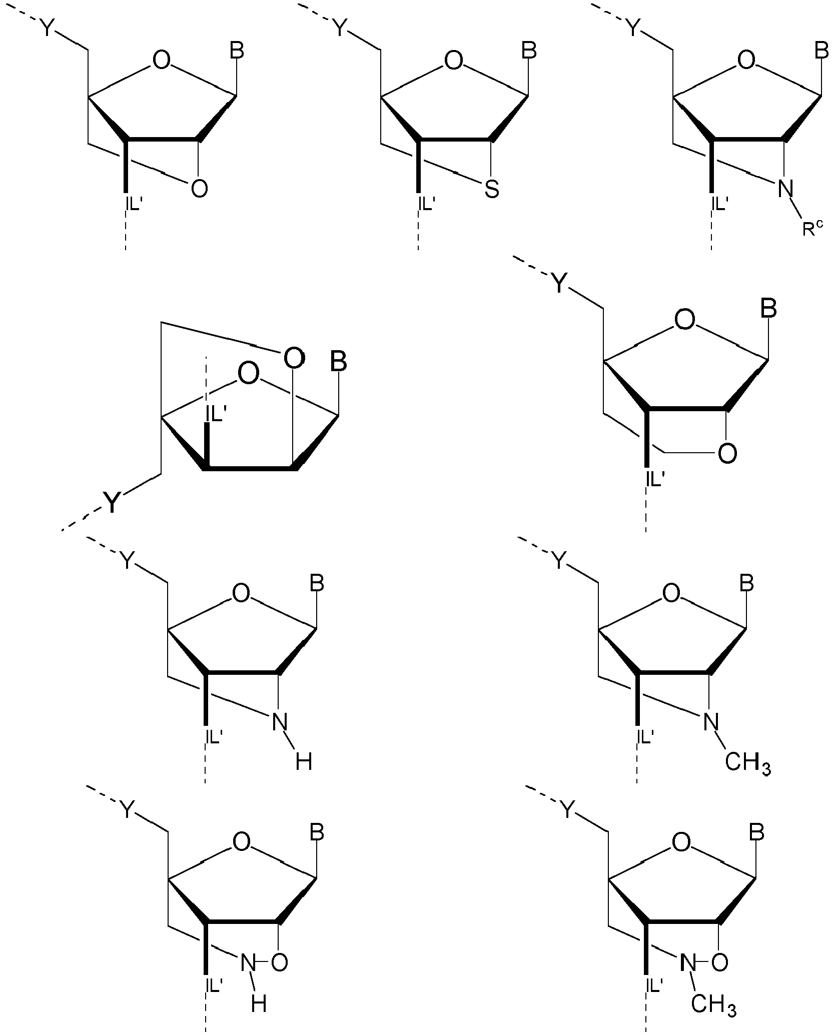 wherein
IL' represents ―X"―P(=X')(X")―;
X' represents =O or =S;
X⁻ represents ―O⁻, -OH, ―OR^{H}, ―NHR^{H}, ―N(R^{H})₂, ―OCH₂CH₂OR^{H}, ―OCH₂CH₂SR^{H}, ―BH₃⁻, ―R^{H}, -SH, ―SR^{H}, or ―S⁻;
X" represents -O-, -NH-, ―NR^{H}―, ―CH₂―, or ―S―;
Y is ―O―, ―NH―, ―NR^{H}―, ―CH₂― or ―S―;
R^{C} and R^{H} are independently of each other selected from hydrogen and C₁₋₄-alkyl;
B represents a nucleobase selected from the following group:
adenine, thymine, guanine, cytosine, uracil, 5-methylcytosine, 5-hydroxymethyl cytosine, N⁴-methylcytosine, xanthine, hypoxanthine, 7-deazaxanthine, 2-aminoadenine, 6-methyladenine, 6-methylguanine, 6-ethyladenine, 6-ethylguanine, 2-propyladenine, 2-propylguanine, 6-carboxyuracil, 5,6-dihydrouracil, 5-propynyl uracil, 5-propynyl cytosine, 6-aza uracil, 6-aza cytosine, 6-aza thymine, 5-uracil, 4-thiouracil, 8-fluoroadenine, 8-chloroadenine, 8-bromoadenine, 8-iodoadenine, 8-aminoadenine, 8-thioladenine, 8-thioalkyladenine, 8-hydroxyladenine, 8-fluoroguanine, 8-chloroguanine, 8-bromoguanine, 8-iodoguanine, 8-aminoguanine, 8-thiolguanine, 8-thioalkylguanine, 8-hydroxylguanine, 5-fluorouracil, 5-bromouracil, 5-chlorouracil, 5-iodouracil, 5-trifluoromethyluracil, 5-fluorocytosine, 5-bromocytosine, 5-chlorocytosine, 5-iodocytosine, 5-trifluoromethylcytosine, 7-methylguanine, 7-methyladenine, 8-azaguanine, 8-azaadenine, 7-deazaguanine, 7-deazaadenine, 7-deaza-8-azaadenine, 3-deazaguanine, 3-deazaadenine, 2-thiouracil, 2-thiothymine and 2-thiocytosine.

10. Antisense-oligonucleotide according to any one of the claims 1 - 9 having one of the following gapmer structures: 2-8-2, 2-8-3, 3-8-2, 3-8-3, 4-8-2, 2-8-4, 3-8-4, 4-8-3, 4-8-4, 2-9-2, 2-9-3, 3-9-2, 3-9-3, 4-9-2, 2-9-4, 4-9-3, 3-9-4, 3-10-3, 2-10-4, 4-10-2, 2-11-3, 3-11-2, 2-11-2.

11. Antisense oligonucleotide according to any one of the claims 1 - 10, wherein the antisense oligonucleotides bind with 100% complementarity to the region of the gene encoding *Efnb2* or to the mRNA encoding *Efnb2* and do not bind to any other region in the human transcriptome.

12. Antisense oligonucleotide according to any one of the claims 1 - 11, wherein the antisense-oligonucleotide is represented by the following sequence GGTTAGGGCT(Seq. ID No. 9), GTTAGGGCTG (Seq. ID No. 10), TTAGGGCTGA (Seq. ID No. 11), AGGTTAGGGCTG (Seq. ID No. 15), GGTTAGGGCTGA (Seq. ID No. 16), GTTAGGGCTGAA (Seq. ID No. 17), TTAGGGCTGAAT (Seq. ID No. 18), AGGTTAGGGCTGAAT (Seq. ID No. 19), GGTTAGGGCTGAATT (Seq. ID No. 20), GGTTAGGGCTG (Seq. ID No. 21), GTTAGGGCTGA (Seq. ID No. 22), TTAGGGCTGAA (Seq. ID No. 23), AGGTTAGGGCTGA (Seq. ID No. 26), GGTTAGGGCTGAA (Seq. ID No. 27), GTTAGGGCTGAAT (Seq. ID No. 28), TTAGGGCTGAATT (Seq. ID No. 29), AGGTTAGGGCTGAA (Seq. ID No. 30), GGTTAGGGCTGAAT (Seq. ID No. 31), GTTAGGGCTGAATT (Seq. ID No. 32), AGGTTAGGGCTGAATT (Seq. ID No. 4), CAAGCAAGGC (Seq. ID No. 39), AAGCAAGGCA (Seq. ID No. 40), AGCAAGGCAT (Seq. ID No. 41), TACAAGCAAGGC (Seq. ID No. 44), ACAAGCAAGGCA (Seq. ID No. 45), CAAGCAAGGCAT (Seq. ID No. 46), AAGCAAGGCATT (Seq. ID No. 47), AGCAAGGCATTT (Seq. ID No. 48), TACAAGCAAGGCATT (Seq. ID No. 49), ACAAGCAAGGCATTT (Seq. ID No. 50), ACAAGCAAGGC (Seq. ID No. 52), CAAGCAAGGCA (Seq. ID No. 53), AAGCAAGGCAT (Seq. ID No. 54), AGCAAGGCATT (Seq. ID No. 55), TACAAGCAAGGCA (Seq. ID No. 57), ACAAGCAAGGCAT (Seq. ID No. 58), CAAGCAAGGCATT (Seq. ID No. 59), AAGCAAGGCATTT (Seq. ID No. 60), TACAAGCAAGGCAT (Seq. ID No. 61), ACAAGCAAGGCATT (Seq. ID No. 62) CAAGCAAGGCATTT (Seq. ID No. 63) TACAAGCAAGGCATTT (Seq. ID No. 5), TGCAAAATTC (Seq. ID No. 71), GCAAAATTCA (Seq. ID No. 72), CAAAATTCAG (Seq. ID No. 73), TGCAAAATTCAG (Seq. ID No. 74), ATTGCAAAATTC (Seq. ID No. 77), TTGCAAAATTCA (Seq. ID No. 78), ACATTGCAAAATTCA (Seq. ID No. 79), CATTGCAAAATTCAG (Seq. ID No. 80), ACATTGCAAAATTCAG (Seq. ID No. 6), TTGCAAAATTC (Seq. ID No. 84), TGCAAAATTCA (Seq. ID No. 85), GCAAAATTCAG (Seq. ID No. 86), CATTGCAAAATTC (Seq. ID No. 88), ATTGCAAAATTCA (Seq. ID No. 89), TTGCAAAATTCAG (Seq. ID No. 90), ACATTGCAAAATTC (Seq. ID No. 91), CATTGCAAAATTCA (Seq. ID No. 92), or ATTGCAAAATTCAG (Seq. ID No. 93), and salts and optical isomers of said antisense-oligonucleotide.

13. Antisense-oligonucleotide selected from the following group:
| **L** | **Seq ID No.** | **Sequence, 5'-3'** |
|---|---|---|
| 12 | 16m | **Gb¹sGb¹s**dTsdTsdAsdGsdGsdGsdCsdTs**Gb¹sAb¹** |
| 12 | 16o | **Gb³sGb³s**dTsdTsdAsdGsdGsdGsdCsdTsdGs**Ab³** |
| 12 | 16p | **Gb¹Gb¹**dTsdTsdAsdGsdGsdGsdCsdTs**Gb¹Ab¹** |
| 12 | 16q | **Gb⁴**dGsdTsdTsdAsdGsdGsdGsdCsdTs**Gb⁴Ab⁴** |
| 12 | 16r | **Gb⁵Gb⁵**dTsdTsdAsdGsdGsdGsdCsdTsdGs**Ab⁵** |
| 12 | 16s | **Gb¹ssGb¹ss**dTssdTssdAssdGssdGssdGssdCssdTss**Gb¹ssAb¹** |
| 12 | 16t | **Gb⁶ss**dGssdTssdTssdAssdGssdGssdGssdCssdTss**Gb⁶ssAb⁶** |
| 12 | 16u | **Gb7ssGb⁷ss**dTssdTssdAssdGssdGssdGssdCssdTssdGss**Ab⁷** |
| 16 | 4as | **Ab¹sGb¹s**dGsdTsdTsdAsdGsdGsdGsdCsdTsdGsdAs**Ab¹sTb¹sTb¹** |
| 16 | 4at | **Ab¹sGb¹s**dGsdTsdTsdAsdGsdGsdGsdCsdTsdGsdAsdAs**Tb¹sTb¹** |
| 16 | 4au | **Ab¹sGb¹sGb¹s**dTsdTsdAsdGsdGsdGsdCsdTsdGsdAsdAs**Tb¹sTb¹** |
| 16 | 4av | **Ab¹sGb¹sGb¹sTb¹s**dTsdAsdGsdGsdGsdCsdTsdGsdAsdAs**Tb¹sTb¹** |
| 16 | 4aw | **Ab¹sGb¹s**dGsdTsdTsdAsdGsdGsdGsdCsdTsdGsA**b¹sAb¹sTb¹sTb¹** |
| 16 | 4ax | **Ab¹sGb¹sGb¹s**dTsdTsdAsdGsdGsdGsdCsdTsdGsdAs**Ab¹sTb¹sTb¹** |
| 16 | 4ay | **Ab¹sGb¹sGb¹sTb¹s**dTsdAsdGsdGsdGsdCsdTsdGs**Ab¹sAb¹sTb¹sTb¹** |
| 16 | 4az | **Ab¹sGb¹sGb¹sTb¹sTb¹s**dAsdGsdGsdGsdCsdTsdGsdAs**Ab¹sTb¹sTb¹** |
| 16 | 4ba | **Ab¹sGb¹sGb¹s**dTsdTsdAsdGsdGsdGsdCsdTs**Gb¹sAb¹sAb¹sTb¹sTb¹** |
| 16 | 4bb | **Ab¹sGb¹sGb¹s**dTsdTsdAsdGsdGsdGsdCsdTsdGs**Ab¹sAb¹sTb¹sTb¹** |
| 16 | 4bc | **Ab¹sGb¹sGb¹sTb¹s**dTsdAsdGsdGsdGsdCsdTsdGsdAs**Ab¹sTb¹sTb¹** |
| 16 | 4bd | **Ab¹Gb¹**dGsdTsdTsdAsdGsdGsdGsdCsdTsdGsdAs**Ab¹Tb¹Tb¹** |
| 16 | 4be | **Ab¹Gb¹**dGsdTsdTsdAsdGsdGsdGsdCsdTsdGsdAsdAs**Tb¹Tb¹** |
| 16 | 4bf | **Ab¹Gb¹Gb¹**dTsdTsdAsdGsdGsdGsdCsdTsdGsdAsdAs**Tb¹Tb¹** |
| 16 | 4bg | **Ab¹Gb¹Gb¹Tb¹**dTsdAsdGsdGsdGsdCsdTsdGsdAsdAsT**b¹Tb¹** |
| 16 | 4bh | **Ab¹Gb¹**dGsdTsdTsdAsdGsdGsdGsdCsdTsdGs**Ab¹Ab¹Tb¹Tb¹** |
| 16 | 4bi | **Ab¹Gb¹Gb¹**dTsdTsdAsdGsdGsdGsdCsdTsdGsdAs**Ab¹Tb¹Tb¹** |
| 16 | 4bj | **Ab¹Gb¹Gb¹Tb¹**dTsdAsdGsdGsdGsdCsdTsdGsAb**¹Ab¹Tb¹Tb¹** |
| 16 | 4bk | **Ab¹Gb¹Gb¹Tb¹Tb¹**dAsdGsdGsdGsdCsdTsdGsdAs**Ab¹Tb¹Tb¹** |
| 16 | 4bl | **Ab¹Gb¹Gb¹**dTsdTsdAsdGsdGsdGsdCsdTs**Gb¹Ab¹Ab¹Tb¹Tb¹** |
| 16 | 4bm | **Ab¹Gb¹Gb¹**dTsdTsdAsdGsdGsdGsdCsdTsdGs**Ab¹Ab¹Tb¹Tb¹** |
| 16 | 4bn | **Ab¹Gb¹Gb¹Tb¹**dTsdAsdGsdGsdGsdCsdTsdGsdAs**Ab¹Tb¹Tb¹** |
| 16 | 4bo | Ab1ssGb1ssdGssdTssdTssdAssdGssdGssdGssdCssdTssdGssdAss Ab1ssTb1ssTb1 |
| 16 | 4bp | Ab¹ssGb¹ssdGssdTssdTssdAssdGssdGssdGssdCssdTssdGssdAss dAssTb¹ssTb¹ |
| 16 | 4bq | Ab¹ssGb¹ssGb¹ssdTssdTssdAssdGssdGssdGssdCssdTssdGssdAss dAssTb¹ssTb¹ |
| 16 | 4br | Ab¹ssGb¹ssGb¹ssTb¹ssdTssdAssdGssdGssdGssdCssdTssdGssdAss dAssTb¹ssTb¹ |
| 16 | 4bs | Ab1ssGb1ssdGssdTssdTssdAssdGssdGssdGssdCssdTssdGssAb1ss Ab1ssTb1ssTb1 |
| 16 | 4bt | Ab¹ssGb¹ssGb¹ssdTssdTssdAssdGssdGssdGssdCssdTssdGssdAss Ab¹ssTb¹ssTb¹ |
| 16 | 4bu | Ab¹ssGb¹ssGb¹ssTb¹ssdTssdAssdGssdGssdGssdCssdTssdGssAb¹ss Ab¹ssTb¹ssTb¹ |
| 16 | 4bv | Ab¹ssGb¹ssGb¹ssTb¹ssTb¹ssdAssdGssdGssdGssdCssdTssdGssdAss Ab¹ssTb¹ssTb¹ |
| 16 | 4bw | Ab¹ssGb¹ssGb¹ssdTssdTssdAssdGssdGssdGssdCssdTssGb¹ssAb¹ss Ab¹ssTb¹ssTb¹ |
| 16 | 4bx | Ab1ssGb1ssGb1ssdTssdTssdAssdGssdGssdGssdCssdTssdGssAb1ss Ab1ssTb1ssTb1 |
| 16 | 4by | Ab¹ssGb¹ssGb¹ssTb¹ssdTssdAssdGssdGssdGssdCssdTssdGssdAss Ab¹ssTb¹ssTb¹ |
| 12 | 46m | **C*b¹sAb¹s**dAsdGsdCsdAsdAsdGsdGsdCs**Ab¹sTb¹** |
| 12 | 46n | **C*b²s**dAsdAsdGsdCsdAsdAsdGsdGsdCs**Ab²sTb²** |
| 12 | 46o | **C*b³sAb³s**dAsdGsdCsdAsdAsdGsdGsdCsdAs**Tb³** |
| 12 | 46p | **C*b¹Ab¹**dAsdGsdCsdAsdAsdGsdGsdCs**Ab¹Tb¹** |
| 12 | 46q | **C*b⁴**dAsdAsdGsdCsdAsdAsdGsdGsdCs**Ab⁴Tb⁴** |
| 12 | 46r | **C*b⁵Ab⁵**dAsdGsdCsdAsdAsdGsdGsdCsdAsTb⁵ |
| 12 | 46s | **C*b¹ssAb¹ss**dAssdGssdCssdAssdAssdGssdGssdCss**Ab¹ssTb¹** |
| 12 | 46t | **C*b⁶ss**dAssdAssdGssdCssdAssdAssdGssdGssdCss**Ab⁶ssTb⁶** |
| 12 | 46u | **C*b⁷ssAb⁷ss**dAssdGssdCssdAssdAssdGssdGssdCssdAss**Tb⁷** |
| 16 | 5as | **Tb¹sAb¹s**dCsdAsdAsdGsdCsdAsdAsdGsdGsdCsdAsdTs**Tb¹sTb¹** |
| 16 | 5at | **Tb¹sAb¹sC*b¹s**dAsdAsdGsdCsdAsdAsdGsdGsdCsdAsdTs**Tb¹sTb¹** |
| 16 | 5au | **Tb¹sAb¹sC*b¹sAb¹s**dAsdGsdCsdAsdAsdGsdGsdCsdAsdTs**Tb¹sTb¹** |
| 16 | 5av | **Tb¹sAb¹s**dCsdAsdAsdGsdCsdAsdAsdGsdGsdCsdAs**Tb¹sTb¹sTb¹** |
| 16 | 5aw | **Tb¹sAb¹s**dCsdAsdAsdGsdCsdAsdAsdGsdGsdCs**Ab¹sTb¹sTb¹sTb¹** |
| 16 | 5ax | **Tb¹sAb¹sC*b¹s**dAsdAsdGsdCsdAsdAsdGsdGsdCsdAs**Tb¹sTb¹sTb¹** |
| 16 | 5ay | **Tb¹sAb¹sC*b¹sAb¹s**dAsdGsdCsdAsdAsdGsdGsdCsdAs**Tb¹sTb¹sTb¹** |
| 16 | 5az | **Tb¹sAb¹sC*b¹s**dAsdAsdGsdCsdAsdAsdGsdGsdCs**Ab¹sTb¹sTb¹sTb¹** |
| 16 | 5ba | **Tb¹sAb¹sC*b¹sAb¹s**dAsdGsdCsdAsdAsdGsdGsdCs**Ab¹sTb¹sTb¹sTb¹** |
| 16 | 5bb | **Tb¹sAb¹sC*b¹sAb¹s**dAsdGsdCsdAsdAsdGsdGsdCsdAs**Tb¹sTb¹sTb¹** |
| 16 | 5bc | **Tb¹sAb¹sC*b¹s**dAsdAsdGsdCsdAsdAsdGsdGsdCsA**b¹sTb¹sTb¹sTb¹** |
| 16 | 5bd | **Tb¹Ab¹**dCsdAsdAsdGsdCsdAsdAsdGsdGsdCsdAsdTs**Tb¹Tb¹** |
| 16 | 5be | **Tb¹Ab¹C*b¹**dAsdAsdGsdCsdAsdAsdGsdGsdCsdAsdTs**Tb¹Tb¹** |
| 16 | 5bf | **Tb¹Ab¹C*b¹Ab¹**dAsdGsdCsdAsdAsdGsdGsdCsdAsdTs**Tb¹Tb¹** |
| 16 | 5bg | **Tb¹Ab¹**dCsdAsdAsdGsdCsdAsdAsdGsdGsdCsdAs**Tb¹Tb¹Tb¹** |
| 16 | 5bh | **Tb¹Ab¹**dCsdAsdAsdGsdCsdAsdAsdGsdGsdCs**Ab¹Tb¹Tb¹Tb¹** |
| 16 | 5bi | **Tb¹Ab¹C*b¹**dAsdAsdGsdCsdAsdAsdGsdGsdCsdAs**Tb¹Tb¹Tb¹** |
| 16 | 5bj | **Tb¹Ab¹C*b¹Ab¹**dAsdGsdCsdAsdAsdGsdGsdCsdAs**Tb¹Tb¹Tb¹** |
| 16 | 5bk | **Tb¹Ab¹C*b¹**dAsdAsdGsdCsdAsdAsdGsdGsdCs**Ab¹Tb¹Tb¹Tb¹** |
| 16 | 5bl | **Tb¹Ab¹C*b¹Ab¹**dAsdGsdCsdAsdAsdGsdGsdCs**Ab¹Tb¹Tb¹Tb¹** |
| 16 | 5bm | **Tb¹Ab¹C*b¹Ab¹**dAsdGsdCsdAsdAsdGsdGsdCsdAs**Tb¹Tb¹Tb¹** |
| 16 | 5bn | **Tb¹Ab¹C*b¹**dAsdAsdGsdCsdAsdAsdGsdGsdCs**Ab¹Tb¹Tb¹Tb¹** |
| 16 | 5bo | **Tb^1ssAb^1ss**dCssdAssdAssdGssdCssdAssdAssdGssdGssdCssdAssdTss **Tb^1ssTb^1** |
| 16 | 5bp | Tb1ssAb1ssC*b1ssdAssdAssdGssdCssdAssdAssdGssdGssdCssdAssdTss Tb1ssTb1 |
| 16 | 5bq | Tb1ssAb1ssC*b1ssAb1ssdAssdGssdCssdAssdAssdGssdGssdCssdAss dTssTb1ssTb1 |
| 16 | 5br | Tb1ssAb1ssdCssdAssdAssdGssdCssdAssdAssdGssdGssdCssdAssTb1ss Tb1ssTb1 |
| 16 | 5bs | Tb1ssAb1ssdCssdAssdAssdGssdCssdAssdAssdGssdGssdCssAb1ssTb1ss Tb1ssTb1 |
| 16 | 5bt | Tb1ssAb1ssC*b1ssdAssdAssdGssdCssdAssdAssdGssdGssdCssdAssTb1ss Tb1ssTb1 |
| 16 | 5bu | Tb^1ssAb^1ssC*b^1ssAb^1ssdAssdGssdCssdAssdAssdGssdGssdCssdAssTb^1ss Tb^1ssTb^1 |
| 16 | 5bv | Tb^1ssAb^1ssC*b^1ssdAssdAssdGssdCssdAssdAssdGssdGssdCssAb^1ss Tb^1ssTb^1ssTb^1 |
| 16 | 5bw | Tb1ssAb1ssC*b1ssAb1ssdAssdGssdCssdAssdAssdGssdGssdCssAb1ss Tb1ssTb1ssTb1 |
| 16 | 5bx | Tb^1ssAb^1ssC*b^1ssAb^1ssdAssdGssdCssdAssdAssdGssdGssdCssdAssTb^1ss Tb^1ssTb^1 |
| 16 | 5by | Tb^1ssAb^1ssC*b^1ssdAssdAssdGssdCssdAssdAssdGssdGssdCssAb^1ss Tb^1ssTb^1ssTb^1 |
| 12 | 74m | **Tb¹sGb¹s**dCsdAsdAsdAsdAsdTsdTsdCs**Ab¹sGb¹** |
| 12 | 74n | **Tb²sGb²sC*b²s**dAsdAsdAsdAsdTsdTsdC*s**Ab²sGb²** |
| 12 | 74o | **Tb³sGb³s**dCsdAsdAsdAsdAsdTsdTs**C*b³sAb³sGb³** |
| 12 | 74p | **Tb¹Gb¹**dCsdAsdAsdAsdAsdTsdTsdCs**Ab¹Gb¹** |
| 12 | 74q | **Tb⁴Gb⁴C*b⁴**dAsdAsdAsdAsdTsdTsdCs**Ab⁴Gb⁴** |
| 12 | 74r | **Tb⁵Gb⁵**dCsdAsdAsdAsdAsdTsdTsC***b⁵Ab⁵Gb⁵** |
| 12 | 74s | **Tb¹ssGb¹ss**dCssdAssdAssdAssdAssdTssdTssdCss**Ab¹ssGb¹** |
| 12 | 74t | **Tb⁶ssGb⁶ssC*b⁶ss**dAssdAssdAssdAssdTssdTssdCss**Ab⁶ssGb⁶** |
| 12 | 74u | **Tb⁷ssGb⁷ss**dCssdAssdAssdAssdAssdTssdTss**C*b⁷ssAb⁷ssGb⁷** |
| 16 | 6as | **Ab¹sC*b¹s**dAsdTsdTsdGsdCsdAsdAsdAsdAsdTsdTsdCs**Ab¹sGb¹** |
| 16 | 6at | **Ab¹sC*b¹sAb¹s**dTsdTsdGsdCsdAsdAsdAsdAsdTsdTsdCs**Ab¹sGb¹** |
| 16 | 6au | **Ab¹sC*b¹sAb¹sTb¹s**dTsdGsdCsdAsdAsdAsdAsdTsdTsdCs**Ab¹sGb¹** |
| 16 | 6av | **Ab¹sC*b¹sAb¹s**dTsdTsdGsdCsdAsdAsdAsdAsdTsdTs**C*b¹sAb¹sGb¹** |
| 16 | 6aw | **Ab¹sC***b**¹sAb¹sTb¹s**dTsdGsdCsdAsdAsdAsdAsdTsdTs**C*b¹sAb¹sGb¹** |
| 16 | 6ax | **Ab¹sC*b¹s**dAsdTsdTsdGsdCsdAsdAsdAsdAsdTsdTs**C*b¹sAb¹sGb¹** |
| 16 | 6ay | **Ab¹sC*b¹s**dAsdTsdTsdGsdCsdAsdAsdAsdAsdTs**Tb¹sC*b¹sAb¹sGb¹** |
| 16 | 6az | **Ab¹sC*b¹sAb¹s**dTsdTsdGsdCsdAsdAsdAsdAsdTs**Tb¹sC*b¹sAb¹sGb¹** |
| 16 | 6ba | **Ab¹sC*b¹sAb¹sTb¹s**dTsdGsdCsdAsdAsdAsdAsdTs**Tb¹sC*b¹sAb¹sGb¹** |
| 16 | 6bb | **Ab¹sC*b¹sAb¹s**dTsdTsdGsdCsdAsdAsdAsdAs**Tb¹sTb¹sC*b¹sAb¹sGb¹** |
| 16 | 6bc | **Ab¹sC*b¹sAb¹sTb¹sTb¹s**dGsdCsdAsdAsdAsdAsdTsdTs**C*b¹sAb¹sGb¹** |
| 16 | 6bd | **Ab¹C*b¹**dAsdTsdTsdGsdCsdAsdAsdAsdAsdTsdTsdCs**Ab¹Gb¹** |
| 16 | 6be | **Ab¹C*b¹Ab¹**dTsdTsdGsdCsdAsdAsdAsdAsdTsdTsdCs**Ab¹Gb¹** |
| 16 | 6bf | **Ab¹C*b¹Ab¹Tb¹**dTsdGsdCsdAsdAsdAsdAsdTsdTsdCs**Ab¹Gb¹** |
| 16 | 6bg | **Ab¹C*b¹Ab¹**dTsdTsdGsdCsdAsdAsdAsdAsdTsdTs**C*b¹Ab¹Gb¹** |
| 16 | 6bh | **Ab¹C*b¹Ab¹Tb¹**dTsdGsdCsdAsdAsdAsdAsdTsdTs**C*b¹Ab¹Gb¹** |
| 16 | 6bi | **Ab¹C*b¹**dAsdTsdTsdGsdCsdAsdAsdAsdAsdTsdTs**C*b¹Ab¹Gb¹** |
| 16 | 6bj | **Ab¹C^{*}b¹**dAsdTsdTsdGsdCsdAsdAsdAsdAsdTs**Tb¹C*b¹Ab¹Gb¹** |
| 16 | 6bk | **Ab¹C*b¹Ab¹**dTsdTsdGsdCsdAsdAsdAsdAsdTs**Tb¹C*b¹Ab¹Gb¹** |
| 16 | 6bl | **Ab¹C*b¹Ab¹Tb¹**dTsdGsdCsdAsdAsdAsdAsdTs**Tb¹C*b¹Ab¹Gb¹** |
| 16 | 6bm | **Ab¹C*b¹Ab¹**dTsdTsdGsdCsdAsdAsdAsdAs**Tb¹Tb¹C*b¹Ab¹Gb¹** |
| 16 | 6bn | **Ab¹C*b¹Ab¹Tb¹Tb¹**dGsdCsdAsdAsdAsdAsdTsdTs**C*b¹Ab¹Gb¹** |
| 16 | 6bo | Ab^1ssC*b^1ssdAssdTssdTssdGssdCssdAssdAssdAssdAssdTssdTssdCss Ab^1ssGb^1 |
| 16 | 6bp | Ab1ssC*b1ssAb1ssdTssdTssdGssdCssdAssdAssdAssdAssdTssdTssdCss Ab1ssGb1 |
| 16 | 6bq | Ab1ssC*b1ssAb1ssTb1ssdTssdGssdCssdAssdAssdAssdAssdTssdTssdCss Ab1ssGb1 |
| 16 | 6br | Ab1ssC*b1ssAb1ssdTssdTssdGssdCssdAssdAssdAssdAssdTssdTss C*b1ssAb1ssGb1 |
| 16 | 6bs | Ab¹ssC*b¹ssAb¹ssTb¹ssdTssdGssdCssdAssdAssdAssdAssdTssdTss C*b¹ssAb¹ssGb¹ |
| 16 | 6bt | Ab1ssC*b1ssdAssdTssdTssdGssdCssdAssdAssdAssdAssdTssdTss C*b1ssAb1ssGb1 |
| 16 | 6bu | Ab1ssC*b1ssdAssdTssdTssdGssdCssdAssdAssdAssdAssdTssTb1ss Cb1ssAb1ssGb1 |
| 16 | 6bv | Ab¹ssC*b¹ssAb¹ssdTssdTssdGssdCssdAssdAssdAssdAssdTssTb¹ss C*b¹ssAb¹ssGb¹ |
| 16 | 6bw | Ab¹ssC*b¹ssAb¹ssTb¹ssdTssdGssdCssdAssdAssdAssdAssdTssTb¹ss C*b¹ssAb¹ssGb¹ |
| 16 | 6bx | Ab¹ssC*b¹ssAb¹ssdTssdTssdGssdCssdAssdAssdAssdAssTb¹ssTb¹ss C*b¹ssAb¹ssGb¹ |
| 16 | 6by | Ab¹ssC*b¹ssAb¹ssTb¹ssTb¹ssdGssdCssdAssdAssdAssdAssdTssdTss C*b¹ssAb¹ssGb¹ |
wherein
b¹ is β-D-oxy-LNA, b² is β-D-thio-LNA, b³ is β-D-amino-LNA,
b⁴ is α-L-oxy-LNA, b⁵ is β-D-ENA, b⁶ is β-D-(NH)-LNA, b⁷ is β-D-(NCH₃)-LNA, d is 2-deoxy;
C* is methyl-C (5-methylcytosine);
dC* is 5-methyl-2'-deoxycytidine;
s represents the internucleotide linkage phosphorothioate group (-O-P(O)(S⁻)-O-);
ss represents the internucleotide linkage phosphorodithioate group (-O-P(S)(S⁻)-O-), wherein
nucleotides in **bold** are LNA nucleotides, and
nucleotides not in bold are non-LNA nucleotides.

14. Antisense-oligonucleotide according to any one of the claims 1 - 13 for use in the prophylaxis and treatment of nephropathy and/or proteinuria in diabetes and/or diabetic nephropathy.

15. Pharmaceutical composition containing at least one antisense-oligonucleotide according to any one of claims 1 - 13 together with at least one pharmaceutically acceptable carrier, excipient, adjuvant, solvent or diluent.
